# EUROPEAN PATENT SPECIFICATION

(11) **EP 2 509 590 B1**
(45) Date of publication and mention of the grant of the patent: **30.10.2019**
(21) Application number: 10836767.3
(22) Date of filing: 10.12.2010
(51) Int. Cl.: A61K 31/27, C07D 213/75, C07D 213/82, C07D 333/38, C07D 233/90, C07C 311/29, C07C 323/31, C07C 323/62, C07C 235/64

(54) **HISTONE ACETYLTRANSFERASE ACTIVATORS AND USES THEREOF**
HISTON-ACETYLTRANSFERASE-AKTIVATOREN UND VERWENDUNGEN DAVON
ACTIVATEURS D'HISTONE ACÉTYLTRANSFÉRASE ET UTILISATION DE CEUX-CI

(30) Priority: 10.12.2009 US 285287 P; 26.03.2010 US 317765 P; 15.06.2010 US 355110 P; 15.06.2010 US 354964 P; 09.07.2010 US 363009 P
(43) Date of publication of application: 17.10.2012
(73) Proprietor: The Trustees of Columbia University in the City of New York, New York, NY 10027 (US)
(72) Inventor: FENG, Yan, Shanghai 200032 (CN); FA, Mauro, New York NY 10032 (US); ARANCIO, Ottavio, New York NY 10025 (US); DENG, Shixian, White Plains NY 10605 (US); LANDRY, Donald, W., New York NY 10027 (US); FRANCIS, Yitshak, New York NY 10025 (US)
(74) Representative: Barker Brettell LLP
(86) International application number: PCT/US2010/059925
(87) International publication number: WO 2011/072243

(56) References cited:
- US-A- 3 254 120
- US-A- 5 696 138
- US-A1- 2005 227 915
- US-A1- 2006 167 107
- US-A1- 2007 265 296
- US-A1- 2009 076 155
- US-A1- 2009 111 863
- US-A1- 2009 264 414
- MOHAMMAD MOVASSAGHI ET AL: "Single-Step Synthesis of Pyrimidine Derivatives", JOURNAL OF THE AMERICAN CHEMICAL SOCIETY, vol. 128, no. 44, 1 November 2006 (2006-11-01), pages 14254-14255, XP055060594, ISSN: 0002-7863, DOI: 10.1021/ja066405m
- HE H ET AL: "Copper-catalyzed N-arylation of sulfonamides with aryl bromides and iodides using microwave heating", TETRAHEDRON LETTERS, ELSEVIER, AMSTERDAM, NL, vol. 44, no. 16, 14 April 2003 (2003-04-14), pages 3385-3386, XP004417082, ISSN: 0040-4039, DOI: 10.1016/S0040-4039(03)00569-0
- HOSSEINZADEH ET AL: "Copper-catalyzed arylation of phenylurea using KF/Al2O3", TETRAHEDRON LETTERS, ELSEVIER, AMSTERDAM, NL, vol. 49, no. 5, 4 December 2007 (2007-12-04), pages 840-843, XP022413155, ISSN: 0040-4039, DOI: 10.1016/J.TETLET.2007.11.180
- MANTELINGU ET AL.: 'Activation of p300 Histone Acetyltransferase by Small Molecules Altering Enzyme Structure: Probed by Surface-Enhanced Raman Spectroscopy.' J. PHYS. CHEM. B vol. 111, 2007, pages 4527 - 4534

## Description

### GOVERNMENT SUPPORT

This invention was made with government support under R01-NS049442 awarded by the National Institute of Neurological Disorders and Stroke (NINDS), under POlAG17490 awarded by the National Institute of Health (NIH), under U01AG031294 awarded by the NIH, and under U01AG14351 awarded by the NIH. The government has certain rights in the invention.

### BACKGROUND OF THE INVENTION

Cognitive neurodegenerative disorders are characterized by synaptic dysfunction, cognitive abnormalities, and/or the presence of inclusion bodies throughout the CNS containing, for example, but not limited to native beta-amyloid fragments, native and phosphorylated Tau, native and phosphorylated alpha-synuclein, lipofuscin, cleaved TARDBP (TDB-43), in various percentages and in relation to the specific disease.

Alzheimer's disease (AD) is a neurodegenerative disorder characterized by memory loss, synaptic dysfunction and accumulation of amyloid β-peptides (Aβ). It is caused in part by increased levels of amyloid-β-peptide 1-42 (Aβ42). Although Alzheimer's Disease (AD) was described almost a century ago, the molecular mechanisms that lead to its development are still unknown. From a neuropathological point of view it is characterized by the presence of amyloid plaques and neurofibrillary tangles associated with neuronal degeneration, whereas the clinical hallmark is a progressive memory loss associated with a number of neuropsychiatric symptoms

Histone Acetyltransferases (HATs) are involved in histone acetylation (leading to gene activation), chromosome decondensation, DNA repair and non-histone substrate modification.
US 2006/167107 A1 discloses modulators (inhibitors/activators) of histone acetyltransferases. US 3,254,120 A discloses compositions of matter which are pharmacologically effective in relaxing spasm.
US 2009/0111863 A1 discloses bishydroxyaryl compounds and pharmaceutically acceptable salts, their synthesis and their use in the treatment of Aβ disease.

### SUMMARY OF THE INVENTION

The invention is directed to compounds with histone acetyltransferase (HAT) activity, high selectivity, and blood-brain-barrier (BBB) permeability. In one aspect, the compound is a compound of Formula (I), 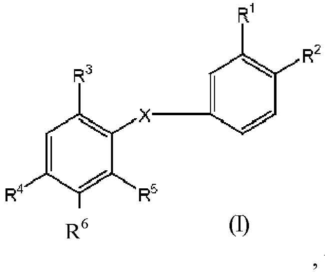 wherein,
R¹ is CF₃;
R² is Cl, or CN;
R³ is H, O-methyl, O-ethyl, S-ethyl, O-cyclopentyl, OCH₂CH₂N(CH₃)₂, or CH₂CH₂CH₂N(CH₃)₂;
R⁴ is H; C(=O)NH-phenyl, wherein phenyl is substituted with one or more halo or CF₃;
R⁵ is H, C₁-C₅-alkyl, OH, OCH₃, O-ethyl, OCH₂CH₂N(CH₃)₂, CH₂CH₂CH₂N(CH₃)₂, SCH₂CH₂N(CH₃)₂, or OCH₂C(=O)O-alkyl;
R⁶ is H, O-methyl, O-ethyl, OCH₂CH₂N(CH₃)₂; and
X is CONH, SONH, SO₂NH, NHC(=O)NH, or NHCO, or a pharmaceutically acceptable salt or hydrate thereof wherein the compound is: 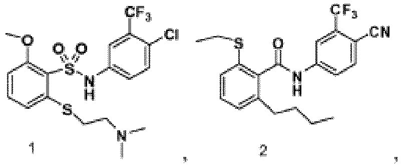 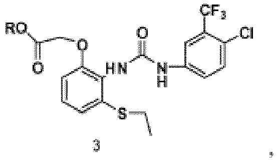 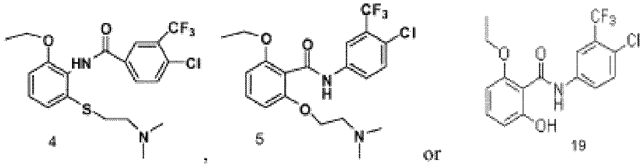 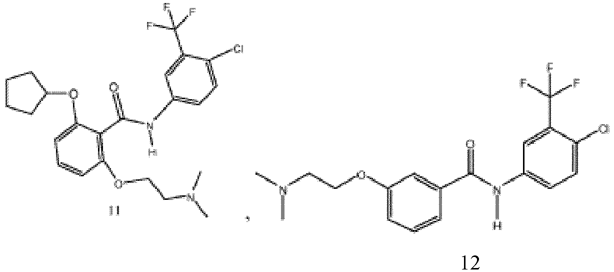 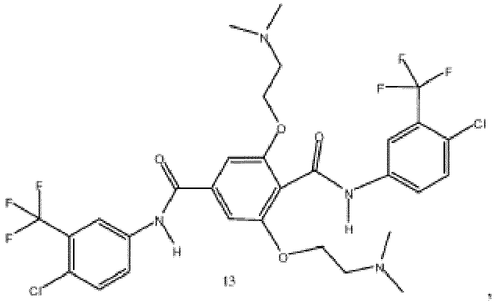 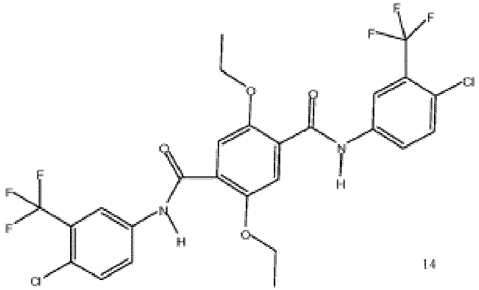 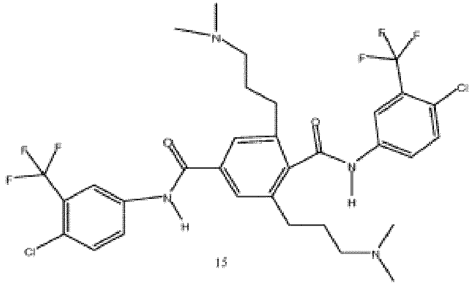 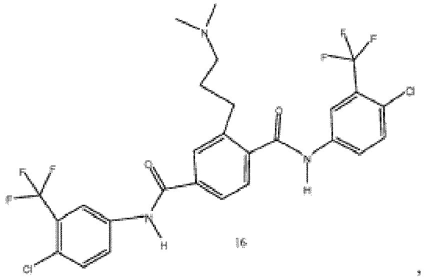 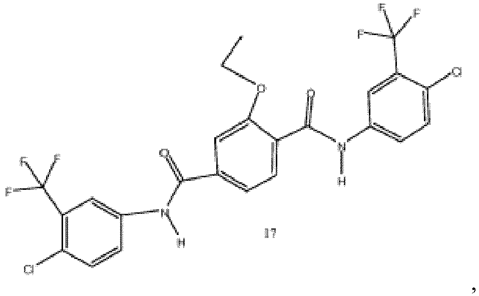 or 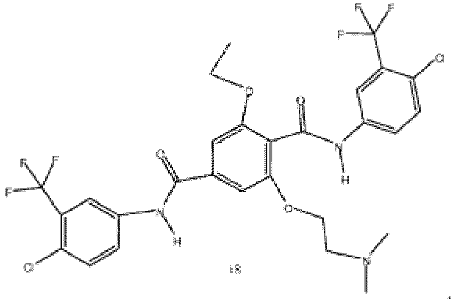 wherein R of compound 3 is H, Methyl, Ethyl, n-Propyl, Isopropyl, n-butyl, t- butyl, C₈H₁₈,C₁₅H₂₆, C₁₅H₂₈, C₁₅H₃₀, or C₁₅H₃₂.

The description also discloses a reference compound of Formula (II), 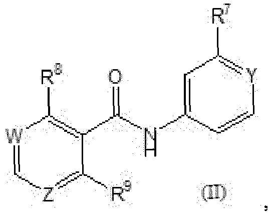 wherein,
R⁷ is H or CF₃;
R⁸ is O-ethyl or S-methyl;
R⁹ is butyl or OCH₂CH₂N(CH₃)₂;
Y is C-Cl, C-CN, C-NO₂, or N;
W is CH or N; and
Z is CH or N, or a pharmaceutically acceptable salt or hydrate thereof.

The description also discloses a reference compound of Formula (III), 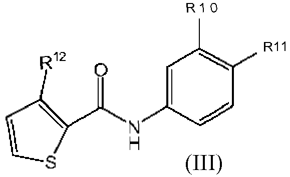 , wherein
R¹⁰ is CF₃;
R¹¹ is CN; and
R¹² is O-ethyl, or a pharmaceutically acceptable salt or hydrate thereof

The description also discloses a reference compound of Formula (IV), 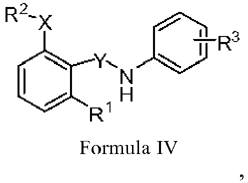 wherein
X is S, S(O)₂, NH, O, or C;
Y is -C(O), S(O)₂, or NH-C(O);
R^{I} is H, Methyl, Ethyl, n-Propyl, Isopropyl, n-butyl, t-butyl, C₈H₁₈,C₁₅H₂₆, C₁₅H₂₈, C₁₅H₃₀, C₁₅H₃₂, SR⁴, or OR⁴;
R² is H, methyl, ethyl, n-propyl, isopropyl, n-butyl, t-butyl, or (C₁-C₆ alkyl)-CO₂R⁶;
R³ is H, methyl, ethyl, n-propyl, isopropyl, n-butyl, t-butyl, CF₃, CCl₃, Cl₃, F, Cl, I, NO₂, or CN;
R⁴ is (C₁-C₆ alkyl)-N(R⁵)₂- or C₁-C₆ alkyl;
R⁵ is independently hydrogen, C₁-C₆ alkyl, or C₃-C₈ cycloalkyl; and
R⁶ is hydrogen, C₁-C₆ alkyl, or C₃-C₈ cycloalkyl, or a pharmaceutically acceptable salt or hydrate thereof.

The description also discloses a reference compound of Formula (V), 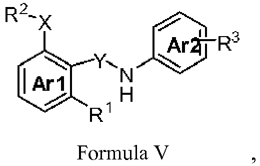 wherein
X is S, S(O)₂, NH, O, or C;
Y is -C(O), S(O)₂, or NH-C(O);
AR1 is a 5-membered aromatic ring or a 6-membered aromatic ring containing 1-2 Nitrogens;
AR2 is a 5-membered aromatic ring, a 6-membered aromatic ring or a 6-membered aromatic ring containing 1-2 Nitrogens;
R¹ is H, Methyl, Ethyl, n-Propyl, Isopropyl, n-butyl, t-butyl, C₈H₁₈,C₁₅H₂₆, C₁₅H₂₈, C₁₅H₃₀, C₁₅H₃₂, SR⁴, or OR⁴;
R² is H, methyl, ethyl, n-propyl, isopropyl, n-butyl, t-butyl, or (C₁-C₆ alkyl)-CO₂R⁶;
R³ is H, methyl, ethyl, n-propyl, isopropyl, n-butyl, t-butyl, CF₃, CCl₃, Cl₃, F, Cl, I, NO₂, or CN;
R⁴ is (C₁-C₆ alkyl)-N(R⁵)₂- or C₁-C₆ alkyl;
R⁵ is independently hydrogen, C₁-C₆ alkyl, or C₃-C₈ cycloalkyl; and
R⁶ is hydrogen, C₁-C₆ alkyl, or C₃-C₈ cycloalkyl, or a pharmaceutically acceptable salt or hydrate thereof.

In one embodiment of the invention, the compound of Formula (I) is YF2 compound:

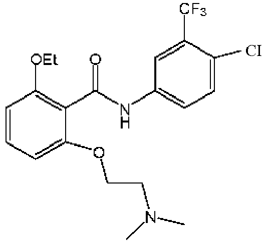

In other embodiments, the compound of Formula (I) is 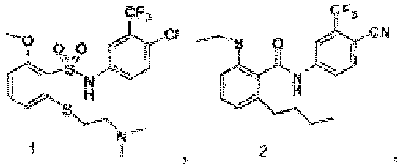 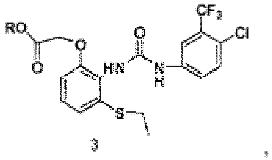 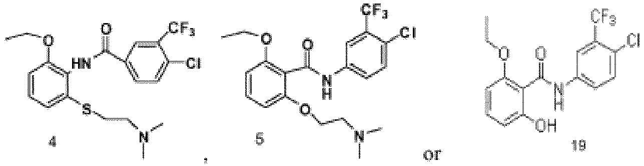 wherein R of compound 3 is H, Methyl, Ethyl, n-Propyl, Isopropyl, n-butyl, t- butyl, C₈H₁₈,C₁₅H₂₆, C₁₅H₂₈, C₁₅H₃₀, or C₁₅H₃₂.

In some embodiments, the compound of Formula (I) is: 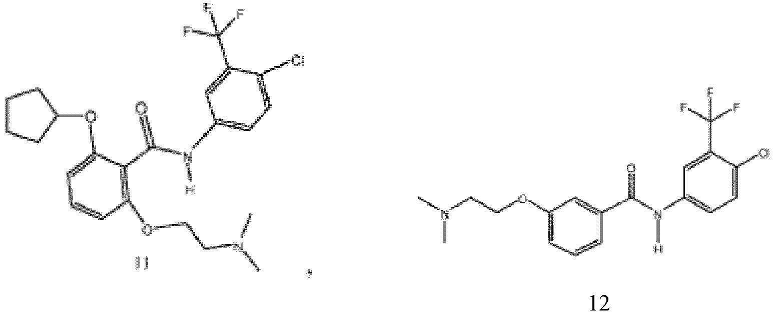 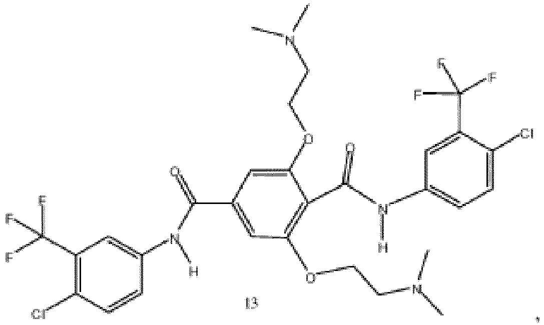 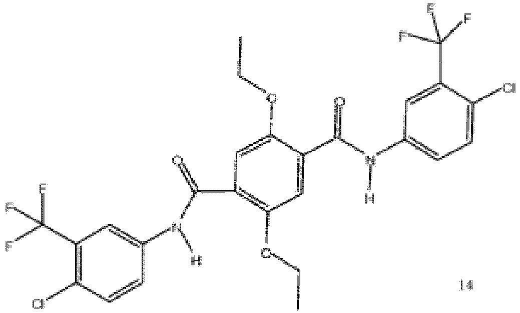 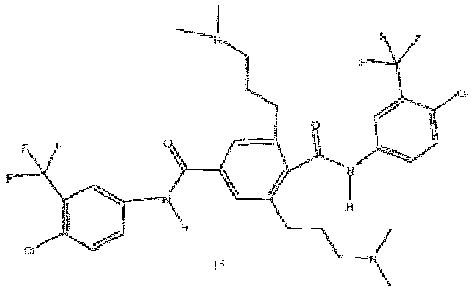 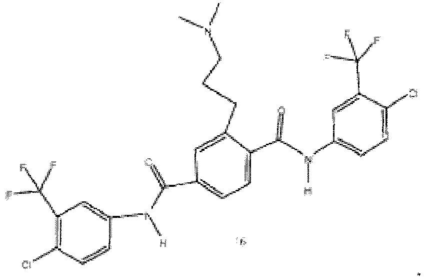 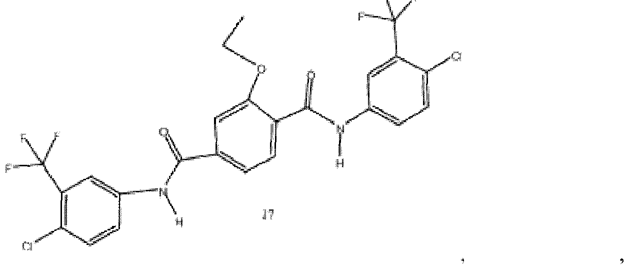 or

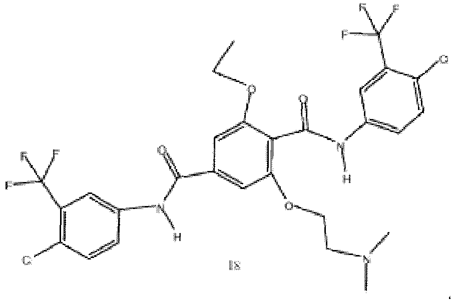

In some embodiments, the refefrence compound of Formula (II) is:

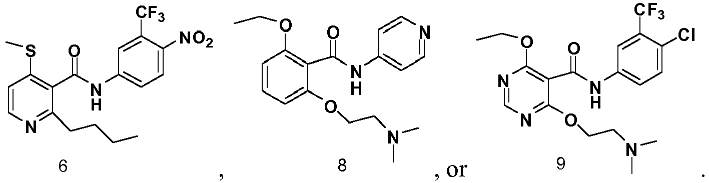

In some embodiments, the reference compound of Formula (V) is:

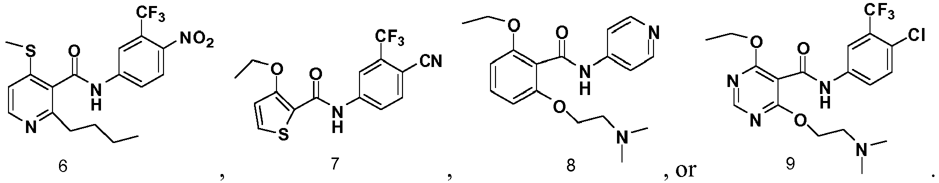

An aspect of the invention provides a method for screening compounds of Formula (I) in a non-human animal to treat conditions associated with accumulated amyloid-beta peptide deposits comprising (a) administering a HAT Activator compound of Formula (I), to a non-human animal model of amyloid-beta peptide deposit accumulation; and (b) selecting a HAT Activator compound of Formula (I) that can modulate histone acetylation after administration of the HAT Activator compound in a non-human animal model of amyloid-beta peptide deposit accumulation.

An aspect of the invention further provides a method for identifying a histone acetyltransferase (HAT) activator compound of Formula (I) in a non-human animal to treat conditions associated with accumulated amyloid-beta peptide deposits, wherein the method comprises selecting a HAT Activator compound of Formula (I) having one or more of the following features: (a) the EC₅₀ of the compound is no more than about 1000 nM; (b) the histone acetylation activity *in vitro* targets histone protein H2, H3, and/or H4; (c) the compound penetrates the blood brain barrier; (d) or a combination thereof. In one embodiment, the compound has a molecular mass less than about 500 Da, has a polar surface area less than about 90 Å², has less than 8 hydrogen bonds, or a combination thereof, in order to penetrate the blood brain barrier.

An aspect of the invention provides a compound of Formula (I) for use in reducing amyloid beta (Aβ) protein deposits in a subject by administering to the subject an effective amount of a composition comprising a HAT Activator compound of Formula (I)thereby decreasing Aβ protein deposits in the subject. In one embodiment, the subject exhibits abnormally elevated levels of amyloid beta plaques. In another embodiment, the subject is afflicted with Alzheimer's disease, Lewy body dementia, inclusion body myositis, or cerebral amyloid angiopathy. In a further embodiment, the Aβ protein deposit comprises an Aβ₄₀ isomer, an Aβ42 isomer, or a combination of isomers. In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

An aspect of the invention further provides a compound of Formula (I) for use in treating Alzheimer's Disease in a subject by administering to a subject a therapeutic amount of a pharmaceutical composition comprising a compound of Formula (I), preferably a HAT activator compound of Formula (I). In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

The description also discloses a compound of Formula (I) for use in treating Alzheimer's Disease in a subject by administering to a subject a therapeutic amount of a pharmaceutical composition comprising a compound of Formula (I). In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof In further embodiments, histone acetylation comprises acetylation of histone lysine residues H3K4, H3K9, H3K14, H4K5, H4K8, H4K12, H4K16, or a combination thereof.

Another aspect of the invention provides a compound of Formula (I) for use in increasing memory retention in a subject afflicted with a neurodegenerative disease by administering to a subject a therapeutic amount of a pharmaceutical composition comprising a compound of Formula (I),. In one embodiment, the neurodegenerative disease comprises Adrenoleukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, Familial fatal insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseasesm Progressive Supranuclear Palsy, Rett's syndrome, Tau-positive FrontoTemporal dementia, Tau-negative FrontoTemporal dementia, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia (multiple types with varying characteristics), Spinal muscular atrophy, Steele-Richardson-Olszewski disease, Tabes dorsalis, or Toxic encephalopathy. In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

An aspect of the invention further provides a compound of Formula (I) for use in increasing synaptic plasticity in a subject afflicted with a neurodegenerative disease, by administering to a subject a therapeutic amount of a composition that increases histone acetylation in the subject, wherein the composition comprises a compound of Formula (I). In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In one embodiment, the neurodegenerative disease comprises Adrenoleukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, Familial fatal insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseasesm Progressive Supranuclear Palsy, Rett's syndrome, Tau-positive FrontoTemporal dementia, Tau-negative FrontoTemporal dementia, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia (multiple types with varying characteristics), Spinal muscular atrophy, Steele-Richardson-Olszewski disease, Tabes dorsalis, or Toxic encephalopathy. In another embodiment, synaptic plasticity comprises learning, memory, or a combination thereof. In some embodiments, synaptic plasticity comprises long term potentiation (LTP). In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

The description further discloses a compound of Formula (I) for use in treating Alzheimer's Disease in a subject by administering to a subject a therapeutic amount of a pharmaceutical composition comprising a compound of Formula (I). In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

An aspect of the invention provides a compound of Formula (I) for use in ameliorating symptoms of Parkinson's Disease in a subject by administering to a subject a therapeutic amount of a pharmaceutical composition comprising a compound of Formula (I) or a HAT activator compound of Formula (I). In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof. In one embodiment, the symptoms of Parkinson's Disease comprise tremor, bradykinesia, dyskinesia, rigidity, postural instability, dystonia, akathisia, dementia, impaired gross motor coordination, or a combination of the listed symptoms. In another embodiment, the postural instability comprises impaired imbalance, impaired coordination, or a combination thereof.

An aspect of the invention provides a compound of Formula (I) for use in treating Huntington's Disease in a subject by administering to a subject a therapeutic amount of a pharmaceutical composition comprising a compound of Formula (I) or a HAT activator compound of Formula (I). In one embodiment, the HAT activator compound can be a compound of Formula (I), Formula (II), Formula (III), Formula (IV), Formula (V), or Formula (VI). In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

An aspect of the invention provides for a compound of Formula (I) for use in treating a neurodegenerative disease in a subject by administering to a subject a therapeutic amount of a pharmaceutical composition comprising a compound of Formula (I), thereby treating the neurodegenerative disease in the subject. In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In one embodiment, the neurodegenerative disease comprises Adrenoleukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, Familial fatal insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseasesm Progressive Supranuclear Palsy, Rett's syndrome, Tau-positive FrontoTemporal dementia, Tau-negative FrontoTemporal dementia, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia (multiple types with varying characteristics), Spinal muscular atrophy, Steele-Richardson-Olszewski disease, Tabes dorsalis, or Toxic encephalopathy. In another embodiment, synaptic plasticity comprises learning, memory, or a combination thereof. In some embodiments, synaptic plasticity comprises long term potentiation (LTP). In some embodiments, the compound is compound 1, compound 2, compound 3, compound 4, compound 5compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

An aspect of the invention provides for a compound of Formula (I) for use in decreasing inclusion bodies in a subject afflicted with a neurodegenerative disorder, by administering to the subject an effective amount of a composition comprising a HAT Activator compound of Formula (I), thereby decreasing inclusion bodies in the subject. In one embodiment, the inclusion bodies comprise beta-amyloid peptides, native and phosphorylated Tau proteins, native and phosphorylated alpha-synuclein, lipofuscin, cleaved TARDBP (TDB-43), or a combination thereof In another embodiment, the subject exhibits abnormally elevated levels of amyloid beta plaques. In a further embodiment, the beta-amyloid peptides comprises an Aβ₄₀ isomer, an Aβ₄₂ isomer, or a combination of isomers. In one embodiment, the compound is YF2. In other embodiments, the effective amount is at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, or at least about 100 mg/kg body weight. In some embodiments, the composition crosses the blood brain barrier. In one embodiment, the neurodegenerative disease comprises Adrenoleukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, Familial fatal insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseasesm Progressive Supranuclear Palsy, Rett's syndrome, Tau-positive FrontoTemporal dementia, Tau-negative FrontoTemporal dementia, Refsum's disease, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia (multiple types with varying characteristics), Spinal muscular atrophy, Steele-Richardson-Olszewski disease, Tabes dorsalis, or Toxic encephalopathy. In another embodiment, synaptic plasticity comprises learning, memory, or a combination thereof In some embodiments, synaptic plasticity comprises long term potentiation (LTP). In some embodiments, the compound is compound 1, compound 2, compound 3, compound 4, compound 5, compound 11, compound 12, compound 13, compound 14, compound 15, compound 16, compound 17, compound 18, or compound 19. In other embodiments, the compound increases histone acetylation. In further embodiments, histone acetylation comprises acetylation of histones H2B, H3, H4, or a combination thereof. In further embodiments, histone acetylation comprises acetylation of histone lysine residues H3K4, H3K9, H3K14, H4K5, H4K8, H4K12, H4K16, or a combination thereof.

### BRIEF DESCRIPTION OF THE FIGURES

**FIG. 1** is photograph of a western blot that shows histone 3 acetylation levels of mice hippocampus.
**FIG. 2** is a bar graph showing that contextual fear conditioning of Aβ42 infused memory deficit mice.
**FIG. 3** is the chemical structure of lead compound YF2, a HAT agonist/activator.
**FIG. 4** is the chemical structure of compound 6J (CTB), N-(4-chloro-3-trifluoromethyl-phenyl)-2-ethoxy-benzamide. 6J had no solubility and precipitated as soon as it was put in H₂O.
**FIG. 5** is the chemical structure of compound MOM (YF1). YF1 was administered 25mg/kg to WT mice (i.p.). The mice liver and hippocampus were extracted lhr after treatment. Hippocampus and liver had no increase in AcH3 levels.
**FIG. 6** is a photograph of a western blot showing acetylation levels of H3 in the liver and hippocampus.
**FIG. 7** is a photograph of a western blot showing acetylation levels of H3 in the liver, cortex, and hippocampus of mice that were administered by gavage and i.p. 25mg/kg of the HAT agonist, MOM. Mice were subsequently subjected to fear conditioning treatment to see if the drug is active after induction of learning.
**FIG. 8** is a photograph of a western blot showing acetylation levels of H3 in the cortex and hippocampus. Mice were administered with MOM via cannula (100 µg/µl per side) or mice were administered YF2 (50 mg/kg, i.p.).

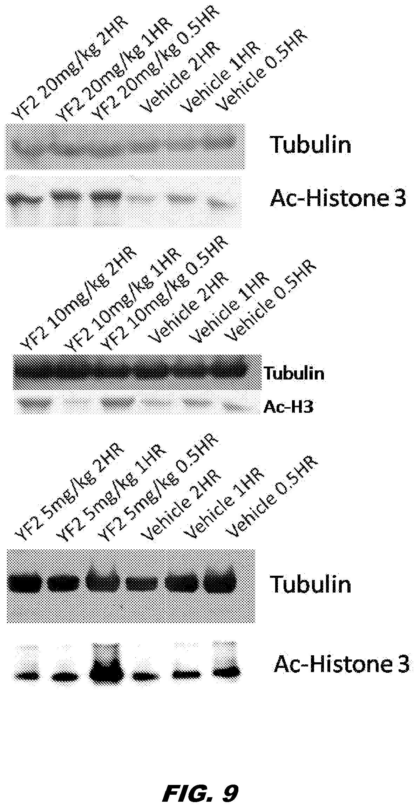

**FIG. 9** is a photograph of a western blot showing acetylation levels of H3 in the hippocampus. Mice were administered with YF2 (i.p. dissolved in saline) at 5 mg/kg, 10 mg/kg, or 20 mg/kg.
**FIG. 10** is a bar graph demonstrating cued fear conditioning responses after administration of the HAT activator, YF2, to mice treated with amyloid-beta (Aβ or A-beta).
**FIG. 11** is a bar graph demonstrating contextual fear conditioning responses after administration of the HAT activator, YF2, to mice treated with amyloid-beta (Aβ or A-beta).
**FIG. 12** is a bar graph demonstrating the assessment of sensory threshold in mice treated with vehicle, YF2 alone, Aβ alone, or YF2 plus Aβ.
**FIG. 13** is a graph showing the kinetics of the HAT agonist, YF2, in the blood. YF2 was administered (20 mg/kg. i.p.) to mice and then blood was sampled from tails at different time points.
**FIG. 14** is a graph showing the results from the radial arm water maze for mice that were administered the HAT agonist, YF2.
**FIG. 15** is a graph showing the results from platform trials for mice that were administered the HAT activator, YF2.
**FIG. 16** is a graph showing the speed of mice during the radial arm water maze that were administered the HAT activator, YF2.
**FIG. 17** is a graph that shows that TSA reverses CA1-LTP impairment in slices from 3-4 month old APP/PS1 mice. Summary graph showing that 30 min HDAC inhibition ameliorates LTP in APP/PS1 mice with no effect on WT littermates. *p<0.05 compared to vehicle-treated APP/PS1 slices (two-way ANOVA). The horizontal bar represents TSA application. The 3 arrows correspond to the θ-burst. TSA-treated APP/PS1 and WT mice (n=7 and n=8, respectively), vehicle-treated APP/PS1 and WT mice (n=6).
**FIG. 18** is a graph that shows that HDAC inhibition improves contextual FC in 3-4 month-old APP/PS1 mice. **(Baseline)** APP/PS1 and WT littermates treated with TSA or vehicle show no difference in immediate freezing in the training chamber. **(24 hrs)** Contextual FC performed 24 hrs after training shows a reduction of freezing in APP/PS1 mice treated with vehicle compared to vehicle-treated WT littermates. Treatment with TSA ameliorates deficit in freezing responses in APP/PS1 mice, and has no effect in WT mice. * p<0.05 vs. APP/PS1-vehicle. (n=13 for all groups).
**FIG. 19** is a schematic of a CBP/Gal4 hybrid and a reporter plasmid in which luciferase expression is driven by a Gal4 yeast promoter. CBP is CREB binding protein.

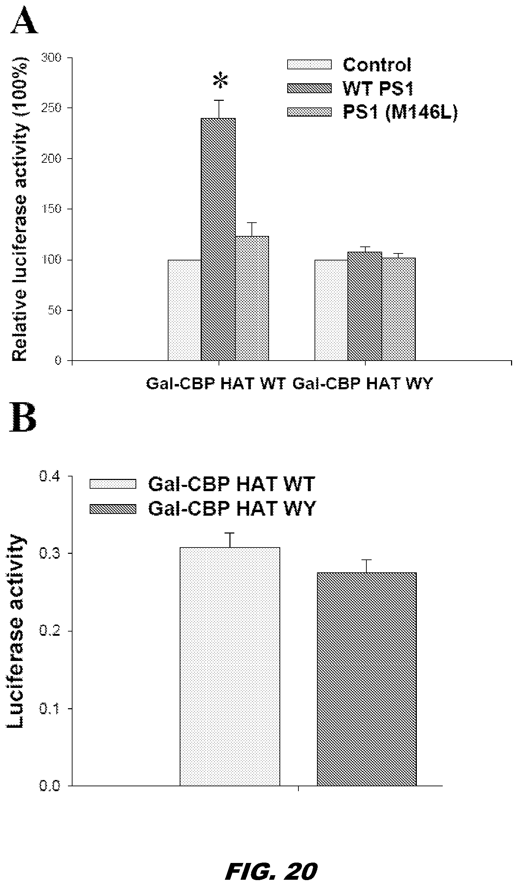

**FIG. 20** are graphs that show the Role of CBP and its HAT region on transcription following PS1 stimulation. **FIG. 20A** depicts the relative luciferase activity in F11 cells cotransfected with 2 µg of a promoter-reporter construct containing Gal4 DNA binding sites upstream of the luciferase gene and constructs containing either functional CBP (WT-CBP) or a mutant lacking HAT activity (WY-CBP) linked to the DNA binding domain of Gal4. Cells were transfected with WT or mutated PS1. Values are expressed relative to the level of luciferase in control, neural cells transfected with Gal-luciferase and Gal-CBP. n=3 per each group. t=7.788, p= 0.0015 vs. control; * p<0.05 vs. control. **FIG. 20B** shows basal luciferase activity in F11 cells co-transfected with 2µg of Gal4 DNA binding sites upstream of the luciferase gene and constructs containing either WT-CBP or WY-CBP linked to the DNA binding domain of Gal4. No significant difference was found.
**FIG. 21** shows photographs of western blots showing that endogenous expression of CBP and PCAF is reduced in APP/PS1 mice. A decrease in PCAF and CBP levels, but not p300 or GCN5, was observed in Aβ-infused mice. 4 month old APP/PS1 mice displayed reduced CBP levels compared to WT controls.
**FIG. 22** is a photograph of a western blot showing acetylated H4 expression is reduced in APP/PS1 mice 1 hour after contextual FC. Treatment with TSA, 2 hours before training, rescued the histone 4 acetylation level defect of Tg mice. n=3 per each group.
**FIG. 23** is a schematic showing that CBP is recruited to act as a bridge between DNA-bound phosphorylated CREB and the basal transcription machinery located at the start site of transcription. In addition, CBP acts as a HAT, making the chromatin more accessible and increasing the transcription of memory associated genes. Unlike CBP, HDACs remove an acetyl group from histones, thus restricting access of the transcriptional machinery to the DNA (figure was modified from [B1]).

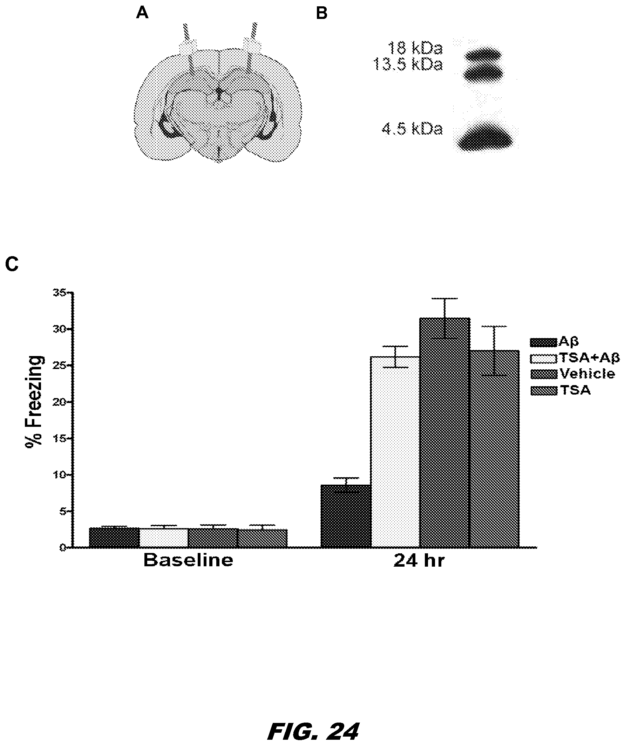

**FIG. 24** depicts HDAC inhibition improves contextual fear conditioning in mice in which dorsal hippocampi were infused with a preparation containing oligomeric Aβ42. **FIG. 24A** is a schematic representation of cannulas implanted bilaterally into the dorsal hippocampi. **FIG. 24B** is a photograph of a Tris-Tricine PAGE Western blot depicting the analysis of Aβ preparation in non-denaturing/non-reducing conditions, showing different bands corresponding to monomers (4.5 kDa), trimers (13.5 kDa) and tetramers (18 kDa). **FIG. 24C** is a graph that represents: **(Baseline)** Bilateral infusions of human Aβ42 (200 nM in a final volume of 1 µl slowly over 1 min) into dorsal hippocampi, 15 min prior to training, do not affect immediate freezing in the training chamber either in conjunction with TSA or vehicle injected (i.p) 2 hrs before training. **(24 hrs)** Contextual fear conditioning performed 24 hrs after training shows a reduction of freezing in Aβ-infused mice injected with vehicle compared to vehicle-infused mice injected with vehicle. Treatment with TSA ameliorates deficit in freezing responses in Aβ-infused mice, and has no effect in vehicle-infused mice. (Aβ-infused mice with TSA injection: n=14, Aβ-infused mice with vehicle injection: n=12, vehicle-infused mice with TSA injection: n=8 and vehicle-infused mice with vehicle injection: n=11).

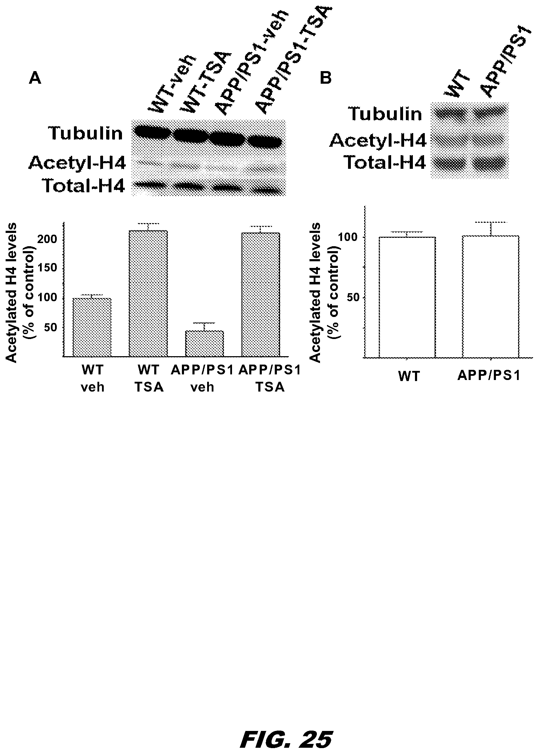

**FIG. 25** depicts histone acetylation reduction in APP/PS1 mice. **FIG. 25A** is photograph of a western blot (top) and the normalized results in graphic form (bottom). Western blotting of protein extracts from APP/PS1 and WT mice, which were injected with vehicle and TSA 2 hours before training, and euthanized 1 hour after contextual fear conditioning. Vehicle-treated APP/PS1 animals showed a decrease in acetylated H4 levels. However, TSA-treated APP/PS1 mice showed an enhancement of H4 acetylation, reaching the same levels of acetylation as TSA-treated WT mice. Results were normalized against vehicle-treated WT mice. n=4 per each group except for TSA-treated WT mice with n=5. **FIG. 25B** is photograph of a western blot (top) and the normalized results in graphic form (bottom). Basal acetylation levels of H4 in APP/PS1 mice and WT littermates, which were exposed to the context without receiving an electrical shock, were similar n=3 per each group. The data shown in **FIG. 25A** and **FIG. 25B** are presented as a ratio of acetylated-H4 to total H4.

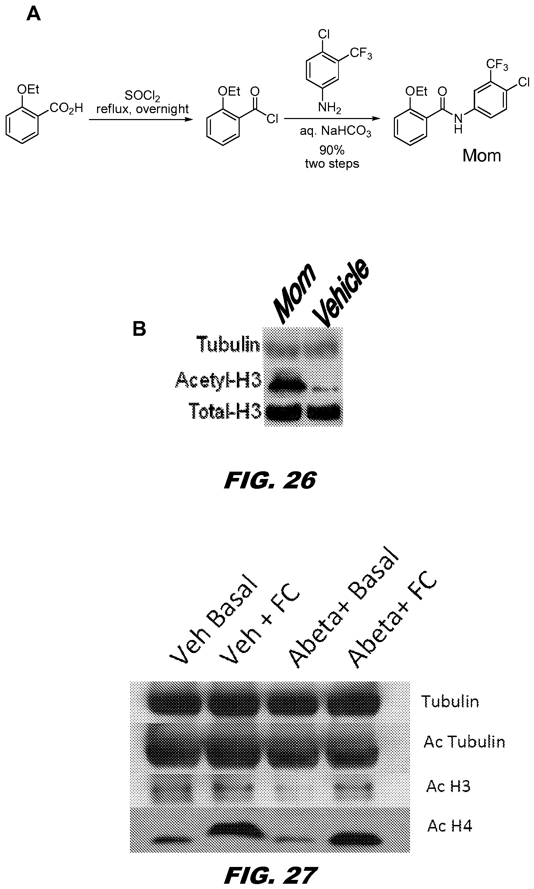

**FIG. 26** is a synthetic scheme showing the synthesis and HAT activity assessment of the agonist MOM. **FIG. 26A** shows that the o-ethoxybenzolic acid was refluxed in SOCl₂ (1.66 g, 10 mmol) overnight. Removal of the organic solvent *in vacuum* gave the corresponding benzoyl chloride, which was dissolved in 20 mL of CH₂Cl₂, followed by addition of 4-chloro-3-trifluoromethylaniline (1.96 g, 10 mmol). The resulting solution was put in an ice-H₂O bath and added 20 mL of saturated NaHCO₃ aqueous solution dropwise. The mixture was then allowed to react at room temperature for 4 hours. The organic phase was separated and washed with 2N HCl, H₂O and brine. After dried over Na₂SO₄, the organic solvent was removed *in vacuum* to yield an off-white crystalline as the desired product (3.1 g, yield 90%). **FIG. 26B** is a photograph of a western blot showing that endogenous levels of acetylated H3 are increased by MOM (100 µg in 1 µl infused into dorsal hippocampi through cannulas) in mouse hippocampi which were harvested 2 hrs after the agonist administration.
**FIG. 27** is a photograph of a western blot showing that histone acetylation is reduced in Aβ (A-beta) infused mice. Vehicle-treated A-beta infused animals showed a decrease in acetylated H3/4 levels both before and after following associative memory.
**FIG. 28** is a schematic of histone acetylation and the role of HATs in such. YF2 is an example of a compound that activates HAT.
**FIG. 29** is a synthetic scheme for the HAT Activator compound, YF2.

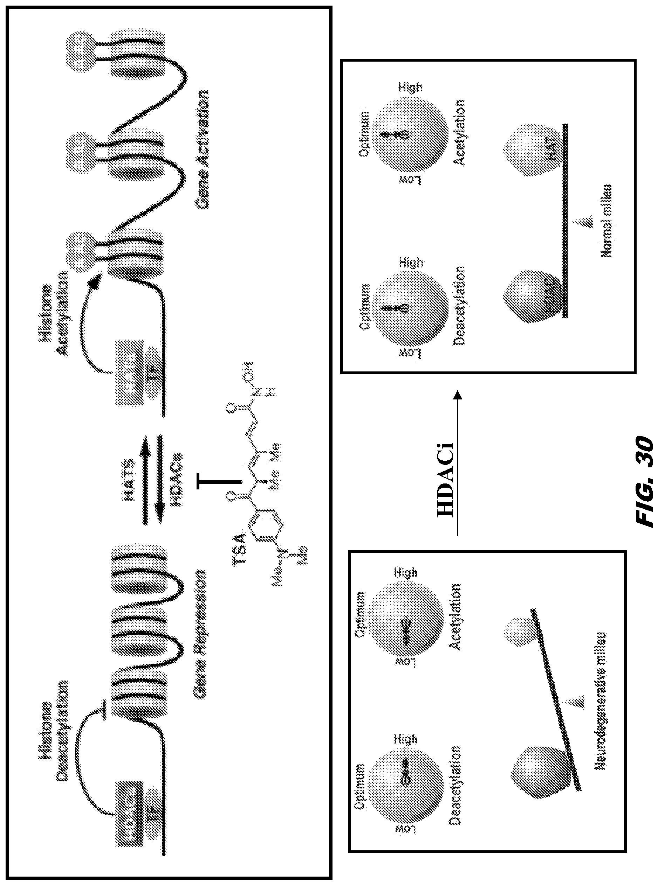

**FIG. 30** is a schematic showing the role of a HDAC inhibitor (HDACi; e.g., TSA) in histone acetylation and deacetylation.

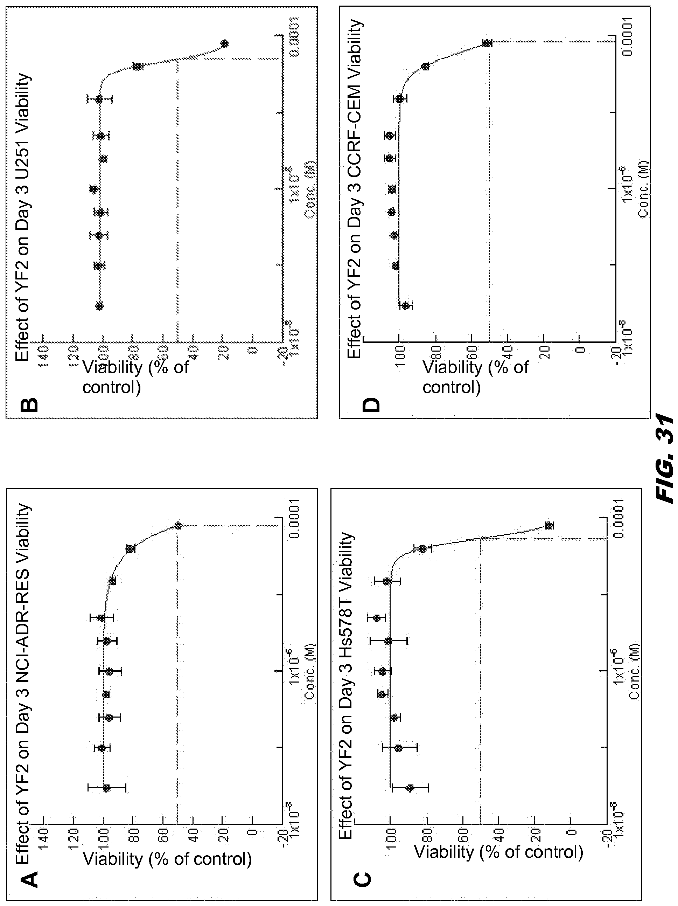

**FIG. 31** are graphs showing the effect of YF2 on cancer cell viability in NCI-ADR-RES cells (**FIG. 31A**; ovarian cancer), U251 cells (**FIG. 31B**; glioblastoma cells), Hs578T cells (**FIG. 31C**; breast cancer cells), and CCRF-CEM cell (**FIG. 31D**; leukemia cells). Values represent an average of 3 wells. Error bars represent standard deviations.

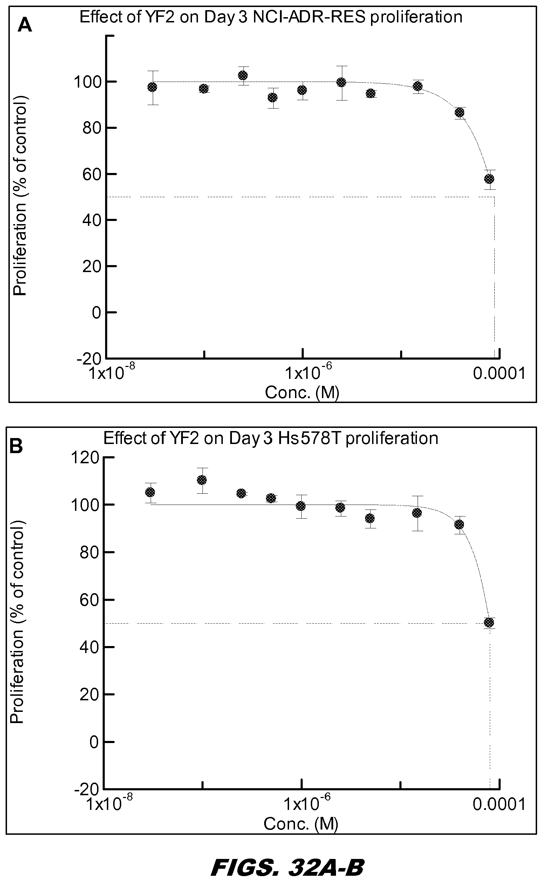

**FIGS. 32A-B** are graphs showing the effect of YF2 on cancer cell proliferation in NCI-ADR-RES cells (**FIG. 32A**; ovarian cancer) and U251 cells (**FIG. 32B**; glioblastoma cells).

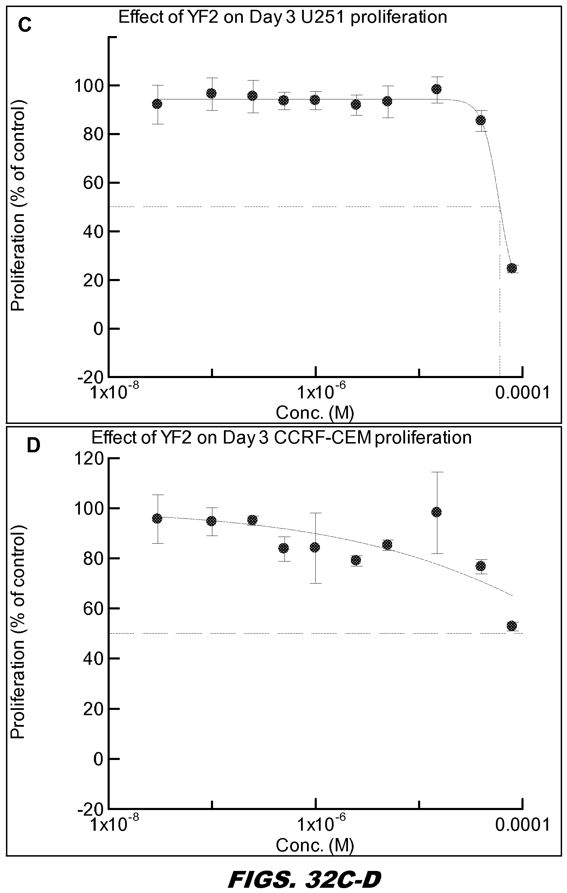

**FIGS. 32C-D** are graphs showing the effect of YF2 on cancer cell proliferation in Hs578T cells (**FIG. 32C**; breast cancer cells) and CCRF-CEM cell (**FIG. 32D**; leukemia cells).

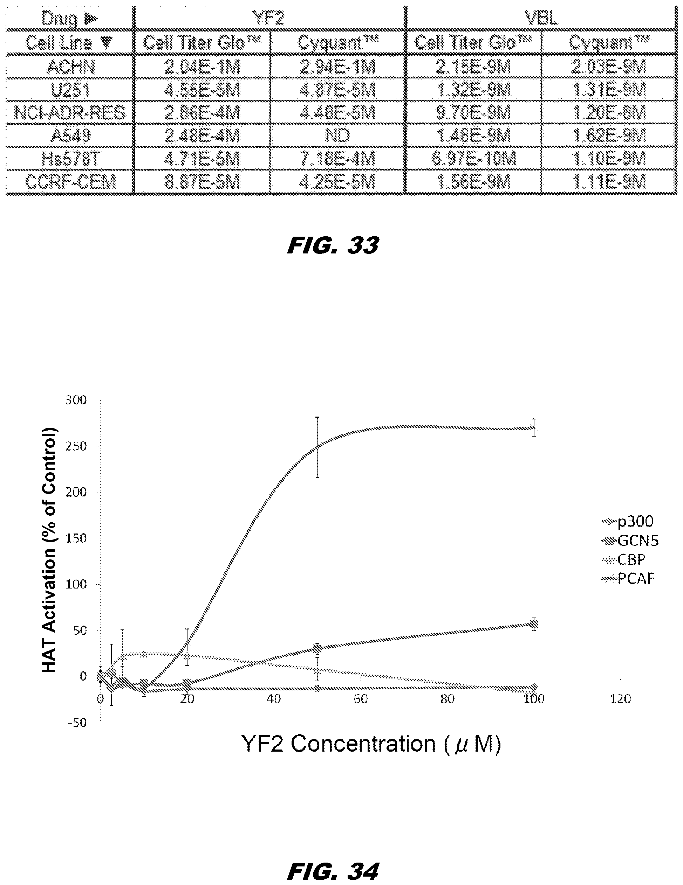

**FIG. 33** is a table showing the IC₅₀ measurements for YF2 and Vinblastine (VBL) on cell viability (Cell Titer Glo column) and cell proliferation (Cyquant column) in NCI-ADR-RES cells (ovarian cancer), U251 cells (glioblastoma cells), Hs578T cells (breast cancer cells), CCRF-CEM cells (leukemia cells), ACHN cells (human renal carcinoma cells), and A549 cells (human lung carcinoma cells).
**FIG. 34** is a graph showing YF2 specificity. YF2 activates CREB Binding Protein (CBP) and the histone acetyltransferase, GCN5.

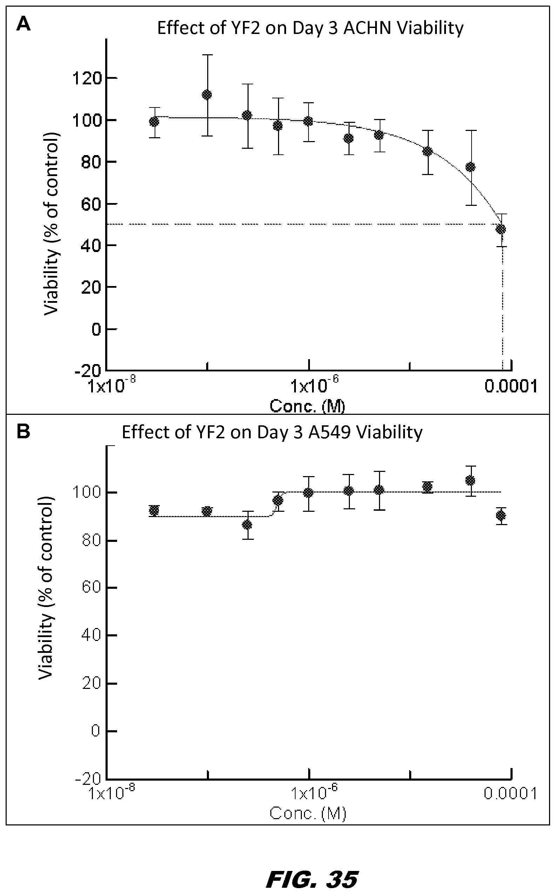

**FIG. 35** are graphs showing the effect of YF2 on cancer cell viability in ACHN cells **(****FIG. 35A**; human renal carcinoma cells) and A549 cells **(****FIG. 35B****;** human lung carcinoma cells). Values represent an average of 3 wells. Error bars represent standard deviations.

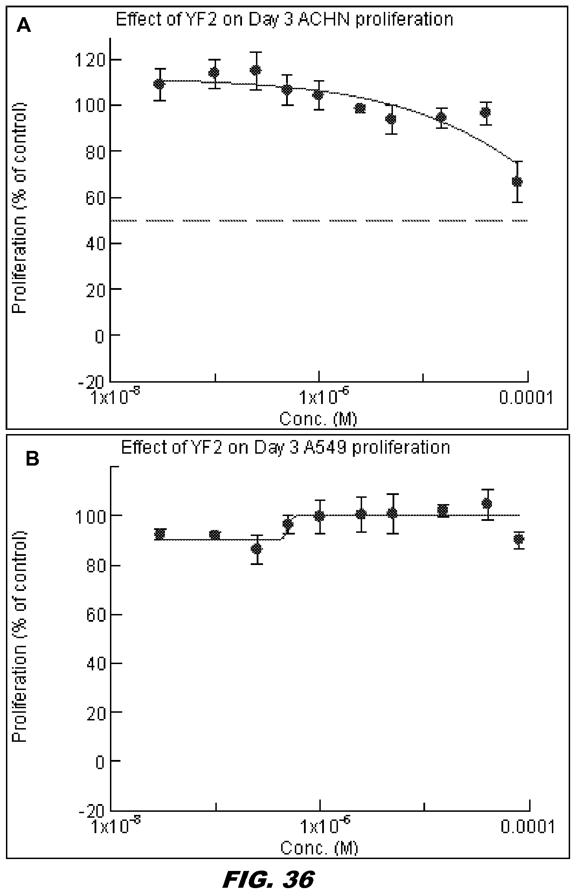

**FIG. 36** are graphs showing the effect of YF2 on cancer cell proliferation in ACHN cells **(****FIG. 36A**; human renal carcinoma cells) and A549 cells **(****FIG. 36B****;** human lung carcinoma cells).
   The use of certain compounds for cancer treatment has been described in the description and the drawings.
**FIG. 37** is a synthetic scheme for the HAT Activator compound, 10.
**FIG. 38** is a synthetic scheme for the HAT Activator compound, 20.
**FIG. 39** is a synthetic scheme for the HAT Activator compound, 11.
**FIG. 40** is a synthetic scheme for the HAT Activator compound, 12.
**FIG. 41** is a synthetic scheme for the HAT Activator compound, 14.
**FIG. 42** is a synthetic scheme for the HAT Activator compound, 13.
**FIG. 43** is a synthetic scheme for the HAT Activator compound, 15. The synthetic scheme is continued to a second page at the hatched line.
**FIG. 44** is a synthetic scheme for the HAT Activator compound, 16.
**FIG. 45** is a synthetic scheme for the HAT Activator compound, 17.
**FIG. 46** is a synthetic scheme for the HAT Activator compound, 18.

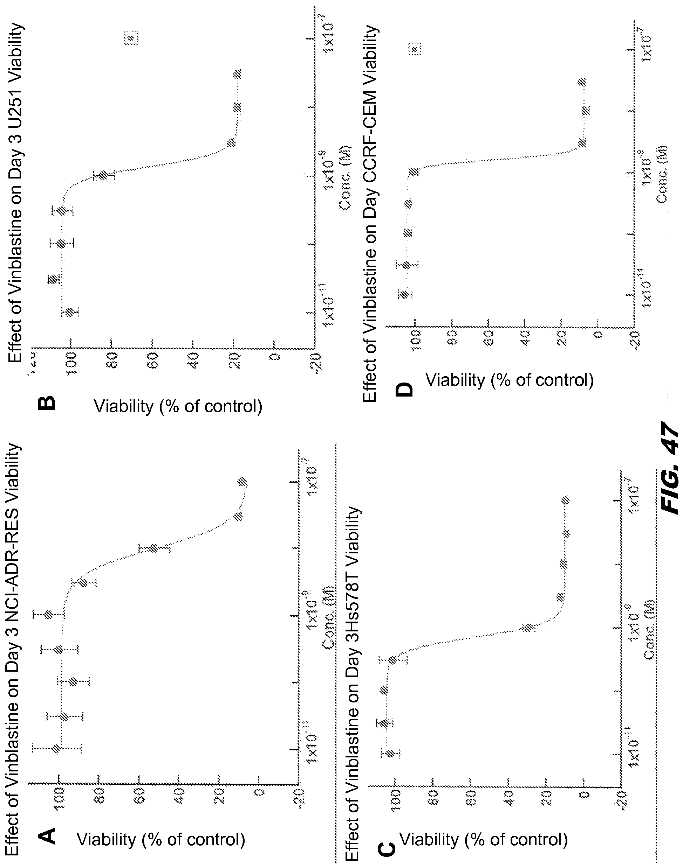

**FIG. 47** are graphs showing the effect of Vinblastine on cancer cell viability in NCI-ADR-RES cells **(****FIG. 47A**; ovarian cancer), U251 cells **(****FIG. 47B****;** glioblastoma cells), Hs578T cells **(****FIG. 47C****;** breast cancer cells), and CCRF-CEM cell **(****FIG. 47D****;** leukemia cells). Values represent an average of 3 wells. Error bars represent standard deviations.

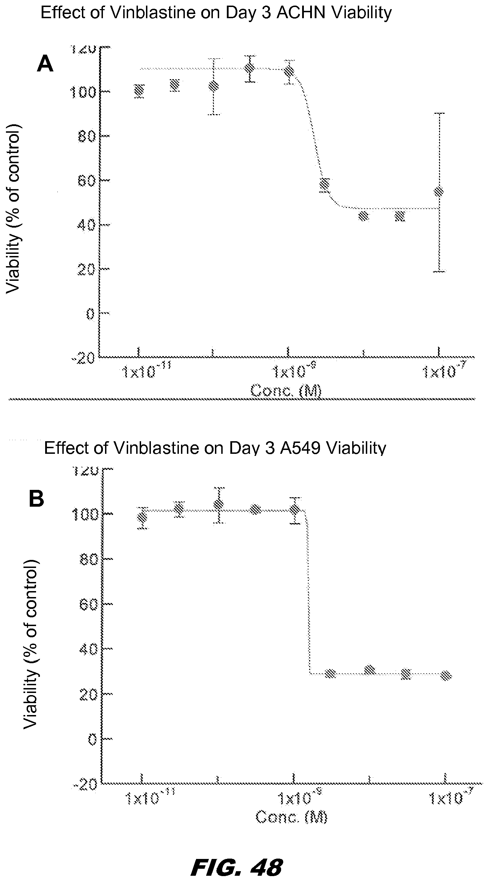

**FIG. 48** are graphs showing the effect of Vinblastine on cancer cell viability in ACHN cells **(****FIG. 48A****;** human renal carcinoma cells) and A549 cells **(****FIG. 48B****;** human lung carcinoma cells). Values represent an average of 3 wells. Error bars represent standard deviations.

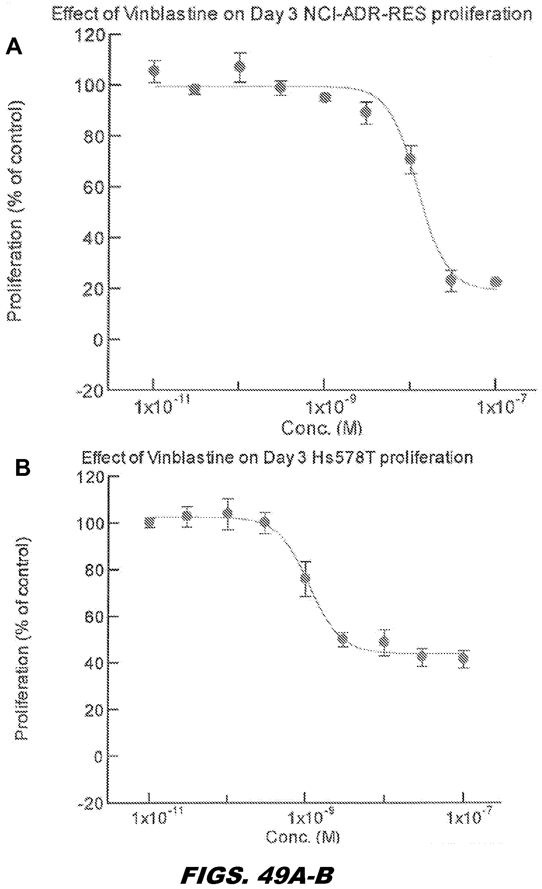

**FIGS. 49A-B** are graphs showing the effect of Vinblastine on cancer cell proliferation in NCI-ADR-RES cells **(****FIG. 49A****;** ovarian cancer) and U251 cells **(****FIG. 49B****;** glioblastoma cells). Values represent an average of 3 wells. Error bars represent standard deviations.

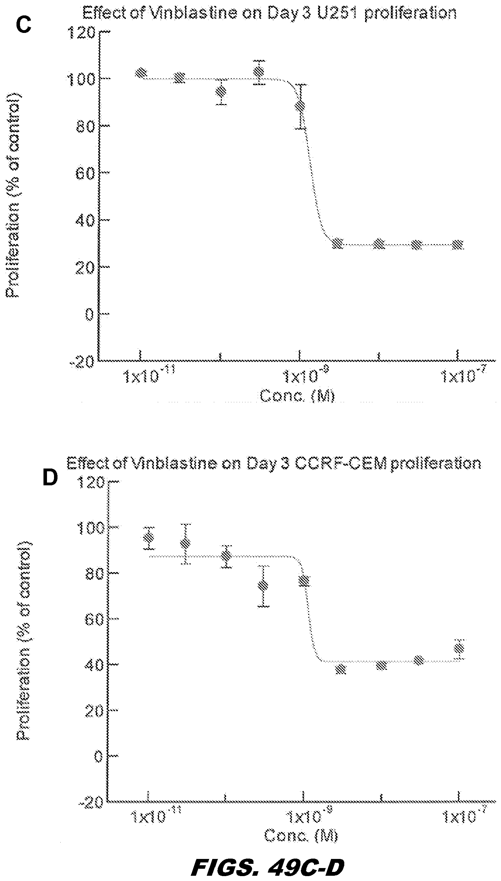

**FIGS. 49C-D** are graphs showing the effect of Vinblastine on cancer cell proliferation in Hs578T cells **(****FIG. 49C****;** breast cancer cells) and CCRF-CEM cell (**FIG. 49D**; leukemia cells). Values represent an average of 3 wells. Error bars represent standard deviations.

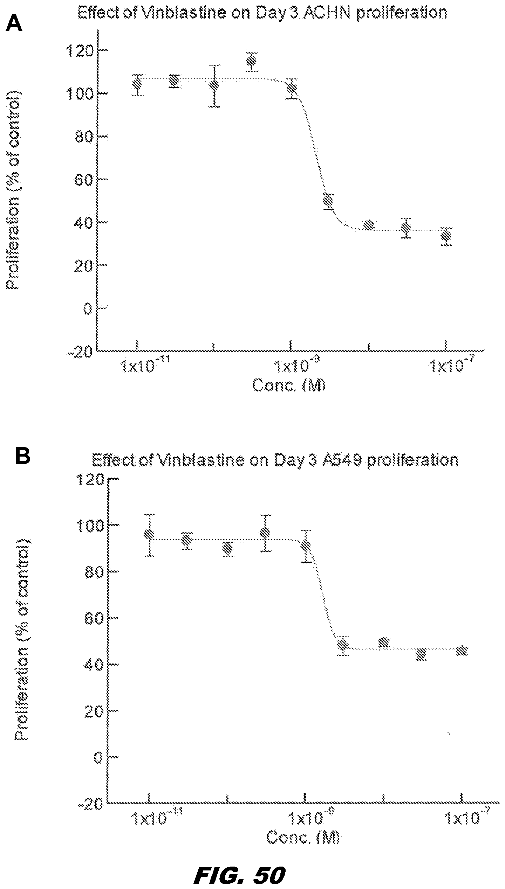

**FIG. 50** are graphs showing the effect of Vinblastine on cancer cell proliferation in ACHN cells **(****FIG. 50A****;** human renal carcinoma cells) and A549 cells **(****FIG. 50B****;** human lung carcinoma cells). Values represent an average of 3 wells. Error bars represent standard deviations.
**FIG. 51** is a synthetic scheme for the HAT Activator compound, 19.
**FIG. 52** is a graph showing the effect of OA2 (YF2 compound) on HDAC 1 activity.
**FIG. 53** is a graph showing the effect of OA2 (YF2 compound) on HDAC3/NCOR2 activity.
**FIG. 54** is a graph showing the effect of OA2 (YF2 compound) on HDAC5FL activity.
**FIG. 55** is a graph showing the effect of OA2 (YF2 compound) on HDAC7 activity.
**FIG. 56** is a graph showing the effect of OA2 (YF2 compound) on HDAC8 activity.
**FIG. 57** is a graph showing the effect of OA2 (YF2 compound) on HDAC10 activity.
**FIG. 58** is a graph showing the effect of OA2 (YF2 compound) on HDAC11 activity.
**FIG. 59** is a graph showing the effect of OA2 (YF2 compound) on Sirtuin1 activity.
**FIG. 60** is a graph showing the effect of OA2 (YF2 compound) on Sirtuin2 activity.
**FIG. 61** is a graph showing the effect of the HDAC inhibitor, SAHA, on HDAC1 activity.
**FIG. 62** is a graph showing the effect of the HDAC inhibitor, SAHA, on HDAC3/NCOR2 activity.
**FIG. 63** is a graph showing the effect of the HDAC inhibitor, SAHA, on HDAC6 activity.
**FIG. 64** shows that an acute administration of YF2 increased specific acetylation of H3, H4, and H2B in hippocampal lysates. n=3 and p<0.05 per group.
**FIG. 65** is a photograph of a western blot showing that YF2 (i.p., 5mg/kg) was able to rescue the Aβ-induced decrease in H3 acetylation levels (n=3 per each group, p<0.05). Aβ (200nM in a final volume of 1µl over 1min, 75 min before mice were sacrificed) was infused through cannulas onto dorsal hippocampi.
**FIGS. 66A-B** are graphs that show the beneficial effect of YF2 on Aβ₄₂-induced synaptic and cognitive dysfunction. YF2 ameliorates the LTP deficit in Aβ₄₂-treated slices **(****FIG. 66A**; the graph represents the average of the last 5min of recording at 60min after the tetanus) P<0.05. YF2 ameliorates the contextual fear memory deficit in Aβ₄₂-infused mice, P<0.01 **(****FIG. 66B**).
**FIG. 66C** is a graph that shows the beneficial effect of YF2 on Aβ₄₂-induced cognitive dysfunction using a water maze/reference memory test. YF2 ameliorates the reference memory deficit in Aβ₄₂-infused mice, P<0.01.
**FIG. 67** shows graphs depicting the beneficial effect of YF2 against memory defect in APP/PS1 mice using fear conditioning or reference memory tests. YF2 ameliorates the reference **(****FIG. 67A****)** and contextual fear **(****FIG. 67B****)** memory deficit in APP/PS1 mice compared to WT littermates (P<0.02 for both tests).

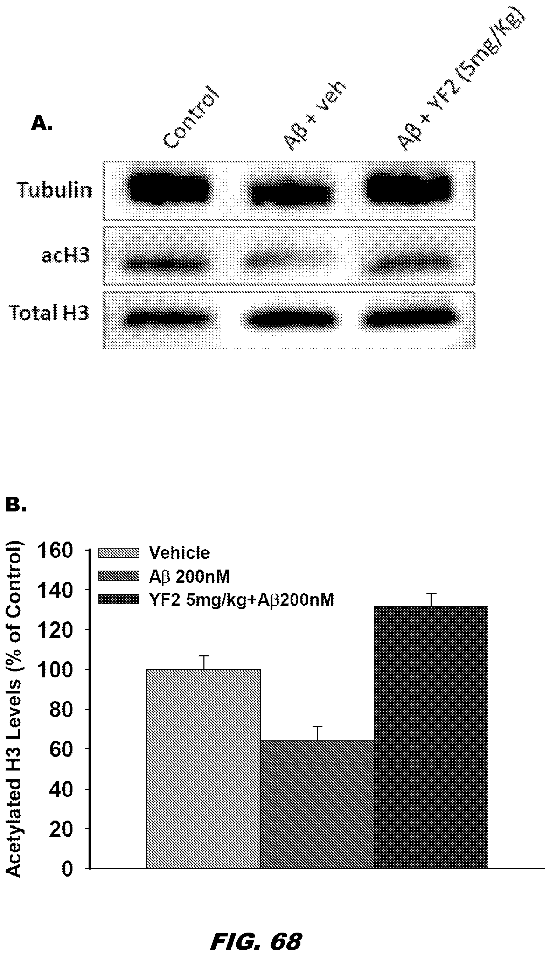

**FIGS. 68A-B** show that YF2 rescues the reduction in acetylated H3 levels after infusion of Aβ42.

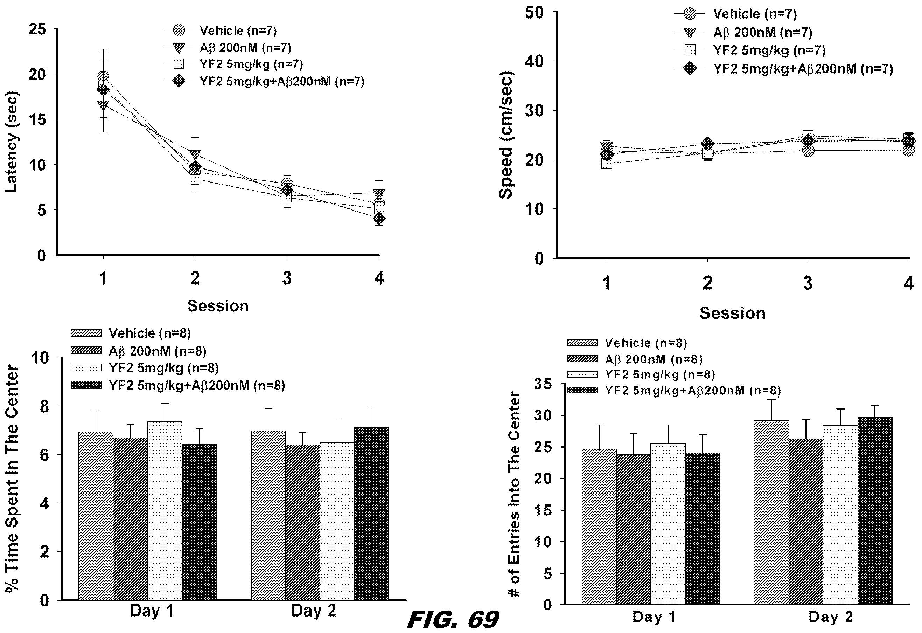

**FIG. 69** are graphs that show the Latency and speed to reach a visible platform and open field assessment of YF2 in mice infused with Aβ42.

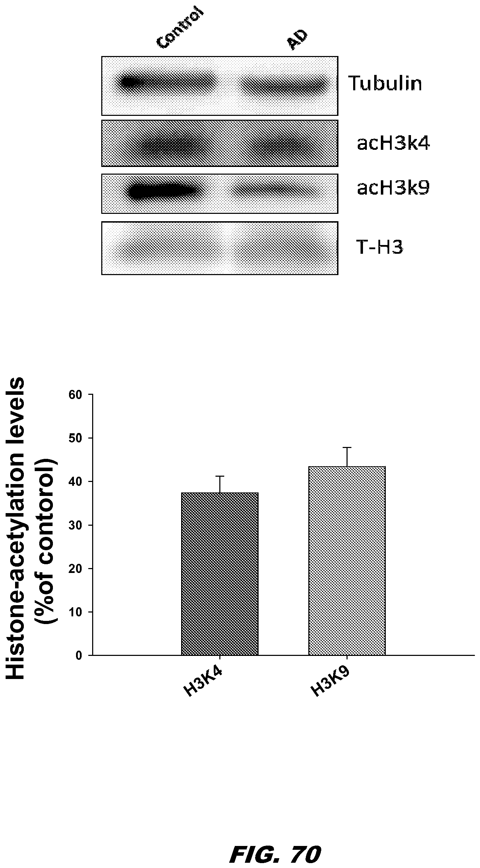

**FIG. 70** shows that histone acetylation levels are reduced in AD patients. Compared to control, Alzheimer's disease patients showed a decrease in acetylation of histone residues important for memory.
**FIG. 71** is a graph showing pharmacokinetic properties of YF2. The amount of YF2 in the brain is higher than that in the plasma. YF2 is rapidly absorbed in the brain (see Table 14). The elimination half-lives of YF2 in the brain and plasma are ∼40 min. The distribution of YF2 to the brain is fast. YF2 does not induce any adverse effects up to 300mg/kg (i.p.) in acute toxicity experiments.
**FIG. 72A** are dose-response curves for human CBP, p300, PCAF and GCN5 activation by different YF2 concentrations.
**FIG. 72B** is a table that represents the calculated EC₅₀ values of CBP, p300, PCAF, and GCN5.

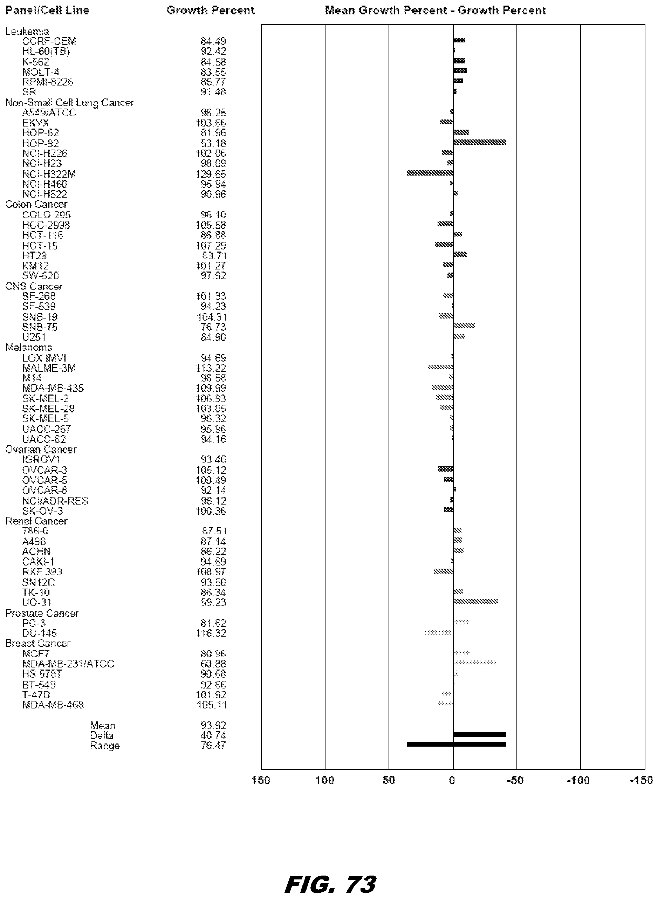

**FIG. 73** is a graph showing NIH cell lines growth inhibition values for 10µM YF2 treatment.
**FIG. 74** is a graph showing the effect of OA2 (YF2 compound) on HDAC6 activity.

### DETAILED DESCRIPTION OF THE INVENTION

Early amnesic changes in Alzheimer's disease (AD) are thought to be linked to synaptic dysfunction. β-amyloid (Aβ), a peptide that is present in high amounts in the disease, has been found to inhibit memory^{[P1,P2]} and its electrophysiological model, long-term potentiation (LTP)^{[P3-P8]}. Memory is known to be modulated by epigenetics through regulation of gene expression. Epigenetics is defined as the mechanism that changes gene expression by 'marking' DNA or its associated proteins, through processes such as DNA methylation and histone (H) modification, without changing the DNA sequence itself^{[P9]}. Modification of histones by, for example, the addition or removal of acetyl or methyl functional groups causes the chromatin structure to open or close, so that the information contained within the DNA is made more or less accessible to transcription factors. Hence, it is not surprising that deregulation of one of the epigenetic mechanisms might lead to memory disruption. For instance, reduction of histone acetylation causes the chromatin structure to close, so that the information contained within the DNA might be less accessible to transcription factors and memory formation^{[P9]}.

The main strategy that is currently used to up-regulate histone acetylation involves inhibition of histone deacetylases (HDACs), enzymes that remove an acetyl group from histones. However, the pleiotropic effect of nonspecific HDAC inhibition may hamper the therapeutic potential of HDAC inhibitors^{[P10-P13]}.

HATs share a highly conserved motif containing an acetyl-CoA binding site. HATs can be involved in the pathology of cancer, asthma, neurodegenerative diseases and viral infection. This indicates that specific HAT activators are potential tools for pharmacological research and might find therapeutic applications. So far only one group has reported HAT activators. However, their compounds are neither soluble, nor membrane permeant, which makes them not good drug candidates for the treatment for the diseases described above.

This invention is about the synthesis of a new class of HAT activators, which have good potency, excellent solubility, reasonable pharmacokinetic profiles and good membrane and Blood-brain-Barrier (BBB) permeability, hence can be used as a new medicine with minimum amount of side effect for patients with neurodegenerative diseases and cancers.

The invention provides for compounds with histone acetyltransferase activity, HAT activation potency, high selectivity, reasonable pharmacokinetics and good permeability across the blood-brain-barrier (BBB). These compounds may be used to minimize the side effects for AD patients, the third most costly disease in the U.S., such as improving cognition or memory in AD and Alzheimer's-like pathologies, as well as minimize the side effects for subjects afflicted with other neurodegenerative diseases. The compounds of the invention may also be developed as anti-cancer drugs. The use of certain compounds for cancer treatment has been described in the description and the drawings.

In some embodiments, the invention provides methods for identifying HAT activators that can acetylate histone proteins thus increasing gene expression in a subject resulting in enhanced memory and cognition.

In further embodiments, the invention provides for the utilization of HAT agonists as memory enhancers in normal subjects (for example, a subject not afflicted with a neurodegenerative disease). In further embodiments, the invention provides for the utilization of HAT agonists as memory enhancers in aging subjects (for example, a subject who is >55 years old). In further embodiments, the invention provides for the utilization of HAT agonists as memory enhancers for other conditions associated with cognitive decrease/impairment. Non-limiting examples of conditions associated with cognitive decrease/impairment include a variety of syndromes associated with mental retardation and syndromes associated with learning disabilities, Parkinson's disease, Pick's disease, a Lewy body disease, amyotrophic lateral sclerosis, Huntington's disease, Creutzfeld-Jakob disease, Down syndrome, multiple system atrophy, neuronal degeneration with brain iron accumulation type I (Hallervorden-Spatz disease), pure autonomic failure, REM sleep behavior disorder, mild cognitive impairment (MCI), cerebral amyloid angiopathy (CAA), mild cognitive deficits, aging, vascular dementias mixed with Alzheimer's disease, a neurodegenerative disease characterized by abnormal amyloid deposition, and any combination thereof.

To shrink the candidate pool of HAT activator compounds to be tested in animal models of neurodegenerative diseases, such as animals that exhibit elevated levels of inclusion bodies, for example Aβ accumulation animal models (e.g., animal models of AD), or, for example, a mouse model for Huntington's disease, HAT activators can first be screened or selected based on their possession of certain characteristics, such as having one or more of: an EC₅₀ no greater than about 100 nM; a histone acetylation activity *in vitro*; and the ability to penetrate the BBB.

In some embodiments, the candidate pool of HAT activators to be tested in animal models of neurodegenerative diseases, such as, but not limited to, animals that exhibit elevated levels of inclusion bodies, for example Aβ accumulation animal models (e.g., animal models of AD), or, for example, a mouse model for Huntington's disease, can first be screened or selected based on "medicinal chemistry" strategies. For example, based on the structure analysis of reported HAT activators, a class of structurally related, but nevertheless formally independent scaffolds, can be generated to be deemed as HAT activator candidates. Compounds derived from these scaffolds can first be screened and optimized on computational models. Compounds with highest score will be synthesized and tested for potency. At this stage, the synthetic effort will be guided by the testing results of potency/selectivity. Compounds with satisfactory potency and selectivity (lead compounds) will be further studied for PK, bioavailability/brain penetration and off-target activities (safety). Selected compounds can be tested in the animal models of neurodegenerative diseases, such as, but not limited to, animals that exhibit elevated levels of inclusion bodies, for example Aβ accumulation animal models (e.g., animal models of AD), or a mouse model for Huntington's disease to determine whether they increase gene expression in a subject resulting in enhanced memory and cognition. As used herein, a HAT activator compound does not necessarily preclude the possibility that the compound may also be able to inhibit other HATs.

### Acetylation and Methylation of DNA and Histones

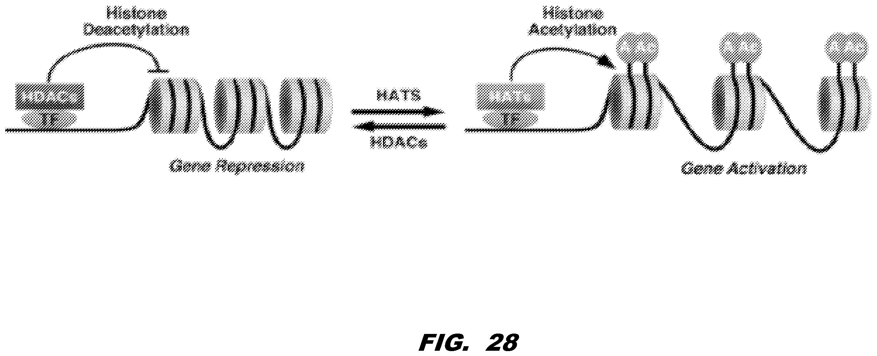

Eukaryotic DNA is highly organized and packaged into the nucleus. The organization and packaging are achieved through the addition of proteins, including core histones H2A, H2B, H3 and H4, which form a complex structure, the chromatin, together with DNA (see **FIG. 28**). The modification of core histones is of fundamental importance to conformational changes of the chromatin. The level of acetylation is related to transcription activity, and then the acetylation induces an open chromatin confirmation that allows the transcription machinery access to promoters. Histone deacetylase (HDAC) and histone acetyltransferase (HAT) are enzymes that influence transcription by selectively deacetylating or acetylating the ε-amino groups of lysine located near the amino termini of core histone proteins. Chromatin acetylation correlates with transcriptional activity (euchromatin), whereas deacetylation correlates with gene silencing. Interestingly, it was shown that increased acetylation of H3 in area CA1 of the hippocampus (an area in the brain that plays an important role in long-tem memory) occurs following associative memory. Additionally, by inhibiting HDAC, they were able to manipulate changes in the chromatin and enhance the formation of long-tem memory.

Histone acetylation and deacetylation are increasingly recognized for their contribution to the tight regulation of gene activation and silencing, respectively. Hence, it is not surprising that deregulation of these mechanisms might lead to the disruption of memory-associated gene expression, resulting in a number of syndromes associated with mental retardation.

**Histones.** The DNA is firstly wrapped around an octamer complex of histones (H) to form nucleosomal units, giving the appearance of beads on a string [B31]. In turn, these nucleosomal units, fold into a higher-order chromatin fiber [B32]. Each histone-octamer complex contains two copies of histones H3 and H4 bordered by two copies of histones 2A and 2B [B32]. H1 and its avian variant H5 are linker histones that bind the nucleosome and both the entry and exit sites of the DNA, thus locking the DNA into place and allowing the formation of higher order structure. Every histone has a globular domain, which mediates histone-histone interactions, and an N-terminal 'tail' extension. The histone cores and in particular their tails, are targets for a considerable number of covalent modifications, such as acetylation, ubiquitination, sumoylation, phosphorylation, citrullination, ADP-ribosylation, and methylation [B33]. Histone modifications associated with active gene transcription, such as H3 Lys4 methylation and H3 Lys56 acetylation, were found to lead to gene expression. On the other hand, histone modifications associated with the inactivation of gene transcription, such as H3 Lys27 methylation and H2A Lys119 ubiquitination were found to cause gene silencing. Of particular interest for this application are histone 2B, 3 and 4 because they have been shown to be involved in memory processes [B19, B25].

**HATs and HDACs.** Histone modifications and their combinations have been proposed to be involved in gene regulation by modifying the chromatin accessibility and by acting as docking sites for transcription factors and modifying enzymes [B34, B35]. One of the most studied histone modifications is the acetylation of the evolutionary-conserved lysine residues on the histone N-termini by histone acetyltransferase (HAT). In contrast, histone deacetylation, catalyzed by histone deacetylase (HDAC), was found to package the DNA into a more condensed form, limiting the access of transcription factors and thus acting as a gene silencer [B36]. The HATs involved in the regulation of gene expression include at least three groups of enzymes [B37] . The general control non-derepressible 5 (Gcn5) is the founding member of the Gcn5 N-acetyltransferases (GNATs). The GNAT family members include Gcn5, PCAF, Elp3, HAT1m Hpa2 and Nut1. The MYST family is named after the founding members of the family: Morf, Ybf2, Sas2 and Tip60 [B37]. In addition, other proteins including CBP/p300, Taf1 and a number of nuclear receptor co-activators have been shown to possess intrinsic HAT activity. However, these proteins do not contain a consensus domain and therefore represent an 'orphan class' of HAT enzymes [B37].

HDACs form repressor complexes with transcription activators and with other HDACs [B38]. Mammalian HDACs can be divided into the classical and the silent information regulator 2 (Sir2)-related protein (sirtruin) families [B39]. In humans, members of the classical family have another subdivision, which include class I, II and IV, that share sequence similarity and require Zn+ for deacetylase activity. Class I HDACs (HDAC 1-3, HDAC8) are related to the yeast gene repressor Rpd3p, and are subunits of at least two distinct co-repressor complexes, the Sin3 complex and the NuRD complex. Class II HDACs (HDAC4-7, 9 and 10) are similar to the yeast Hdalp HDAC, they act as gene repressors and have been implicated in various roles in cell differentiation and development. Class IV comprises HDAC11, which has some features of both class I and II HDACs. The sirtruin family includes class III HDACs (SIRT1-7), which are similar to yeast Sir2. Class III HDACs are biochemically and structurally distinct from the classical family and require NAD⁺ as a cofactor. HDACs appear to be involved in gene silencing and heterochromatin formation at centromeres and telomeres (for a review see [B40]).

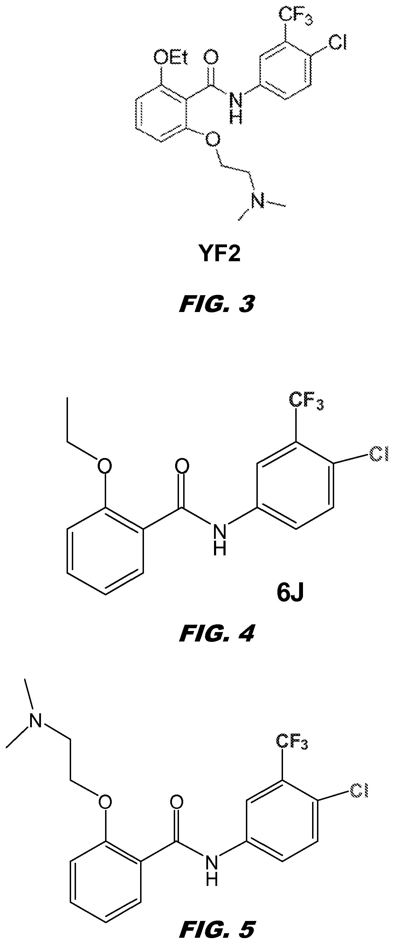

Alterations in epigenetic modifications including acetylation and methylation of DNA and histones may contribute to gene expression changes in cancer and neurological diseases. Addition of acetyl group on histones by Histone Acetyltransferases (HATs) enhances gene expression, while its removal by Histone Deacytylases (HDAC) reduces gene expression. Reduction in histone acetylation has been found in a variety of ailments such as tumors, mood disorders, and neurodegenerative diseases. Examples of HATs include, but are not limited to GCN5, GCN5L, PCAF, HAT1, ELP3, HPA2, ESA1, SAS2, SAS3, TIP60, HBO1, MOZ, MORF, MOF, SRC1, SRC3, TIF2, GRIP1, ATF-2 [see Lee and Workman (2007) Nat Rev Mol Cell Biol., 8(4):284-95, Marmorstein (2001) J Molec Biol. 311: 433-444; and Kimura et al., (2005) J Biochem. 138(6): 647-662]. In one embodiment, the HAT activator compound of the invention is directed to GCN5, GCN5L, HAT1, PCAF, or a combination thereof. In some embodiments, the HAT activator compound of the invention is directed to proteins that possess intrinsic HAT activity, such as nuclear receptor co-activators (for example, CBP/p300 and Tafl). In another embodiment, the acetylation of H2, H3, and/or H4 histones is increased. In some embodiments, the HAT Activator compound is YF2, depicted in **FIG. 3****.**

Increasing histone acetylation has been shown to improve outcome in a wide variety of diseases as diverse as asthma, infectious disease and psychiatric diseases. Although clinical trials of several HDAC inhibitors are currently underway, the alternative strategy where by histone acetylation is increased by HAT activation has not been extensively explored. For example, compounds in U.S. Patent Publication No. US2009076155 and PCT Publication No. WO2004053140 have poor solubility and membrane permeability. Furthermore, the compounds disclosed in the patent applications do not disclose any data for the treatment of any diseases.

No HAT activator is currently in drug trials, however several HDAC inhibitors are currently in clinical trials. Some of these HDAC inhibitors (HDACi) have shown therapeutic efficacy in preclinical trials. Without being bound by theory, HAT activators can be a useful drug candidate with a role similar to HDACi. However, previously available HAT activators had little solubility and membrane permeability, making them unsuitable as drugs.

Several HDACi are in trials for cancer some of which are, for example, 4SC-202 (Nycomed, Germany), which is in a Preclinical stage; AR-42 (Arno therapeutics, Parsippany, NJ) which is in a Preclinical stage; Belinostat (TopoTarget, Rockaway, NJ) which is in Phase II clinical trials; and Entinostat (Bayer Schering) which is in Phase II clinical trials. For example, in Table 3 of Lane and Chabner (2009, J Clin Oncol., 27(32):5459-68), selected clinical trials of HDAC inhibitors are discussed, which include Vorinostat, Depsipeptide, and MGCD0103. In Table 2 of Lane and Chabner (2009, J Clin Oncol., 27(32):5459-68), selected HDAC inhibitors in clinical use or development are discussed, which include hydroxamic acid compounds (e.g., Vorinostat, Trichostatin A, LAQ824, Panobinostat, Belinostat, and ITF2357), cyclic tetrapeptide compounds (e.g., Depsipeptide), benzamide compounds (e.g., Entinostat and MGCD0103), and short-chain aliphatic acid compounds (e.g., valproic acid, phenyl butyrate, and pivanex).

Some HDACi are or were being developed for neurological diseases, such as an HDACi from Merck (Whitehouse Station, NJ) that is being used for the treatment of neurodegenerative diseases; and HDACi from TopoTarget (Rockaway, NJ) that was being used for the treatment of Huntington's disease, now discontinued; isovaleramide NPS-1776 (NPS Pharmaceutical, Bedminster, New Jersey) that was being used for bipolar disorder, epilepsy, and migraines, now discontinued; and a histone acetyltransferase inhibitor for cancer from TopoTarget A/S (København, Denmark), which was discontinued in the preclinical stage.

Here, the synthesis of a new class of HAT activators with improved solubility and membrane permeability are described and its potency in-vitro as well as in an animal model are shown (see EXAMPLES). *In vitro* and behavioral data show that a HAT activator compound of the invention, YF2, can acetylate histone H3 in brain and ameliorate memory deficits in a mouse model of Alzheimer's disease. For example, the HAT activator shown in **FIG. 3** can be used as adjuvant therapy in several cancers, psychiatric and neurodegenerative diseases and may improve efficacy and safety of treatment for these disorders. Furthermore, The HAT activator compound, YF2, has a moiety which was not mentioned in the above-referenced patent applications that significantly improves the solubility and membrane and Blood-brain-Barrier (BBB) permeability. *See* Abel and Zukin (2008) Current Opinion in Pharmacology 8:57-64; and Lee and Workman (2007) Nat Rev Mol Cell Biol 8:284-295.

In one embodiment described in the description and the drawings, a HAT activator compound can be used to treat a cancer in a subject in need thereof. Non-limiting examples of cancers include B cell lymphoma, colon cancer, lung cancer, renal cancer, bladder cancer, T cell lymphoma, myeloma, leukemia, chronic myeloid leukemia, acute myeloid leukemia, chronic lymphocytic leukemia, acute lymphocytic leukemia, hematopoietic neoplasias, thymoma, lymphoma, sarcoma, lung cancer, liver cancer, non-Hodgkins lymphoma, Hodgkins lymphoma, uterine cancer, renal cell carcinoma, hepatoma, adenocarcinoma, breast cancer, pancreatic cancer, liver cancer, prostate cancer, head and neck carcinoma, thyroid carcinoma, soft tissue sarcoma, ovarian cancer, primary or metastatic melanoma, squamous cell carcinoma, basal cell carcinoma, brain cancer, angiosarcoma, hemangiosarcoma, bone sarcoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, testicular cancer, uterine cancer, cervical cancer, gastrointestinal cancer, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, Waldenstroom's macroglobulinemia, papillary adenocarcinomas, cystadenocarcinoma, bronchogenic carcinoma, bile duct carcinoma, choriocarcinoma, seminoma, embryonal carcinoma, Wilms' tumor, lung carcinoma, epithelial carcinoma, cervical cancer, testicular tumor, glioma, astrocytoma, medulloblastoma, craniopharyngioma, ependymoma, pinealoma, hemangioblastoma, acoustic neuroma, oligodendroglioma, meningioma, retinoblastoma, leukemia, melanoma, neuroblastoma, small cell lung carcinoma, bladder carcinoma, lymphoma, multiple myeloma, and medullary carcinoma.

In one embodiment of the invention, a HAT activator compound can be used to treat a neurodegenerative disease in a subject in need thereof. Non-limiting examples of neurodegenerative diseases include Adrenoleukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, Familial fatal insomnia, Frontotemporal lobar degeneration, Huntington's disease, HIV-associated dementia, Kennedy's disease, Krabbe's disease, Lewy body dementia, Neuroborreliosis, Machado-Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseasesm Progressive Supranuclear Palsy, Refsum's disease, Rett's syndrome, Tau-positive FrontoTemporal dementia, Tau-negative FrontoTemporal dementia, Sandhoff disease, Schilder's disease, Subacute combined degeneration of spinal cord secondary to Pernicious Anaemia, Spielmeyer-Vogt-Sjogren-Batten disease (also known as Batten disease), Spinocerebellar ataxia (multiple types with varying characteristics), Spinal muscular atrophy, Steele-Richardson-Olszewski disease, Tabes dorsalis, and Toxic encephalopathy.

### Gene Transcription and Memory

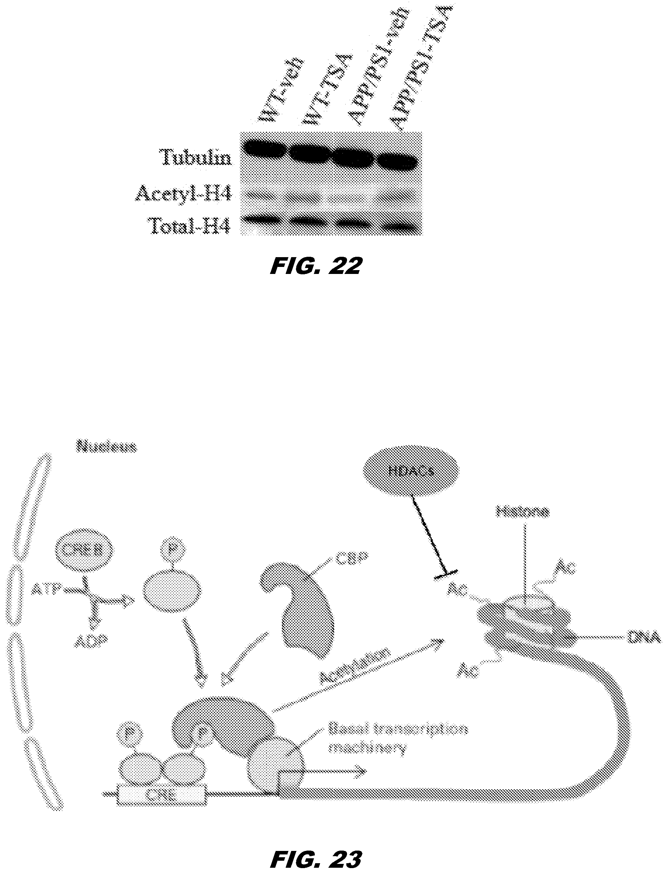

A schematic representation of the processes involved in gene transcription and memory is shown in **FIG. 23**. In the brain, CREB phosphorylation is required for CREB ability to bind to CBP and to stimulate memory associated gene expression. CBP functions as a co-activator that facilitates interactions with the basal transcription machinery and through its HAT activity catalyzes acetylation of the histones, causing a loss in chromosomal repression and increase in the transcription of memory associated genes. In agreement with this scheme, mutations in HAT domain CBP were found to cause LTM impairment. For instance, Korzus *et a*l. demonstrated that inducible dominant-negative CBP mice, with no CBP HAT activity, exhibited normal short-tem memory while LTM was impaired [B8]. Moreover, the impaired LTM was rescued by suppression of the transgene expression and by HDAC inhibitor administration [B8].

The relevance of histone deacetylation in memory processes is underscored by a study from Levenson *et al.* [B26]. In this work acetylation of H3 in area CA1 of the hippocampus (an area in the brain that plays important roles in LTM) was found to be increased following training for contextual fear conditioning, a form of associative memory in which mice associate a novel context with an aversive stimulus. However, HDAC inhibition enhanced LTM [B26]. In agreement with this study, HDAC inhibition may be beneficial in certain neurodegenerative disorders, such as Huntington's disease, spinal muscular atrophy, amyotrophic lateral sclerosis, ischemia and Rubinstein-Taybi syndrome [B19, B25, B41, B42]. Interestingly, the effect of HDAC inhibitors are likely to depend upon specific HDACs, as neuron-specific overexpression of HDAC2, but not that of HDAC1, reduced dendritic spine density, synapse number, synaptic plasticity and memory formation [B43]. Conversely, HDAC2 deficiency increased synapse number and facilitated memory, similar to chronic treatment with HDAC inhibitors in mice [B43]. Taken together, these findings indicate that HDAC inhibition may provide a therapeutic avenue for memory impairment in neurodegenerative diseases characterized by cognitive disorders such as AD.

### Transcriptional machinery and AD

Recent studies have linked CREB/CBP dysfunction to AD. APP(K670N:M671L) transgenic animals showed a decrease in CREB phosphorylation at the downstream level of the α-7-nicotinic-receptor (α7-nAChR)/ERK/MAPK cascade [B44, B45]. Perfusion of hippocampal slices with a preparation containing oligomeric Aβ42 revealed a reduction of the tetanus-induced increase in CREB phosphorylation, providing a clue to the mechanisms underlying the Aβ-mediated changes in LTP [B12]. Consistent with these results Aβ blocked the glutamate-induced increase in CREB phosphorylation in rat hippocampal cultures [B11]. Gong et al. also found that rolipram, a selective PDE IV inhibitor which increases cAMP levels and therefore CREB phosphorylation, ameliorates deficits in both LTP and contextual learning in the APP/PS1 double-transgenic mouse [B46]. This protective effect is possibly due to CREB activation that might lead to CBP recruitment and thus histone acetylation. Another investigation demonstrated that in PS1 and 2 conditional double knockout (PS cDKO) mice showed impaired memory and LTP that were amplified with age. In addition PS cDKO mice showed a reduction in CBP levels and in CRE-dependent gene expression in the cerebral cortex, which is likely to contribute to subsequent neuronal degeneration [B15]. In addition, a recent study has demonstrated that wild-type (WT) PS1 stimulates the transcriptional ability of CBP and p300, whereas the AD associated mutant of PS1 (M146L) does not have this effect [B18, B47]. In these experiments a response to WT PS1 was observed with a construct containing the 721-1679 region of CBP, which contains the CBP acetyltransferase domain [B18]. Moreover, it was shown that CBP loss and histone deacetylation takes place during neuronal death, which was induced by an APP-directed antibody in primary cortical neurons [B48].

### Alzheimer's Disease - An example of a Neurodegenerative Disease

An emerging view of the processes involved in memory impairment indicates that the subtlety and variability of the earliest amnesic symptoms, occurring in the absence of any other clinical signs of brain injury, could be due to discrete changes in the function of a single synapse, produced at least in part, by Aβ [B11, B27-B29].

Several evidences have shown that LTM and synaptic plasticity rely on gene expression after an early induction phase characterized by the activation of a number of pathways (for a review, see [B30]). More recently, a fine regulation of memory-related genes and long-term synaptic plasticity has been discovered to involve epigenetic factors [B6]. Indeed, epigenetic modifications, such as DNA methylation and histone post-translational modifications, profoundly affect the ability of polymerases to interact with the open reading frame of DNA without changing the DNA sequence itself. Hence, it would not be surprising that deregulation of epigenetic mechanisms might lead to the disruption of memory-associated gene expression and synaptic plasticity [B6], contributing to the pathogenesis of diseases characterized by cognitive disorders, such as AD.

The process of memory storage has been described as a dialogue between genes and synapses [B2]. The formation of long-term memory (LTM) is dependent upon gene transcription [B3], synthesis of new proteins [B4] and structural changes of the synapse [B5]. In addition, the proper regulation of gene expression in LTM is modulated by epigenetics [B6]. Epigenetics is defined as the mechanism that changes gene expression by 'marking' DNA or its associated proteins, through processes such as histone modification and DNA methylation, without changing the DNA sequence itself [B7]. The N-terminal tails of histone proteins are known to undergo posttranslational modifications, such as histone acetylation, ubiquitination, sumoylation, phosphorylation, citrullination, ADP-ribosylation, and methylation that can dictate the transitions between transcriptionally active or transcriptionally silent chromatin states [B7]. Hence, it is not surprising that deregulation of one of these mechanisms might lead to disruption of memory associated gene expression and cognitive disorders.

Alzheimer's disease (AD) is characterized by neuronal loss, extracellular senile plaques and intracellular neurofibrillary tangles, leading to memory loss. AD purportedly begins as a synaptic disorder produced at least in part, by Aβ (Selkoe, D.J. Alzheimer's disease is a synaptic failure. Science (New York, N.Y 298, 789-791 (2002)). Aβ-induced reduction in long-term-potentiation (LTP), a physiological correlate of synaptic plasticity that is thought to underlie learning and memory, and phosphorylation of the memory transcription factor CREB, are ameliorated by nitric oxide (NO) donors and cGMP-analogs (Puzzo, D., et al. Amyloid-beta peptide inhibits activation of the nitric oxide/cGMP/cAMP-responsive element-binding protein pathway during hippocampal synaptic plasticity. J Neurosci 25, 6887-6897 (2005)). Vice-versa, genetic ablation of NO-synthase 2 (NOS2) results in worsening of the AD phenotype in mice expressing mutated amyloid precursor protein (APP) (Colton, C.A., et al. NO synthase 2 (NOS2) deletion promotes multiple pathologies in a mouse model of Alzheimer's disease. Proceedings of the National Academy of Sciences of the United States of America 103, 12867-12872 (2006)). These findings show that up-regulation of the NO pathway can be protective in AD.

Alzheimer's disease (AD) is a chronic progressive neurodegenerative disorder, in which the earliest stages are thought to be linked to synaptic dysfunction leading to memory disorders. In this regard, β-amyloid (Aβ) has been found to inhibit memory^{[A1,A2]} and its cellular model, long-term potentiation (LTP^{)[A3-A8]}. Aβ is the proteolytic product of a larger precursor protein, the amyloid precursor protein (APP), which in its mutant form has been found to be implicated in familial AD (FAD)^{[A9]}. Subsequently, two AD associated genes, presenilin 1 (PS1) and presenilin 2 (PS2)^{[A10,A11]}, were found to be involved in FAD as well^{[A12]}. Presenilins are part of the γ- secretase complex responsible for cleaving APP and producing the Aβ42 peptide^{[A13]}.

AD is characterized neuropathologically by neuronal loss, extracellular senile plaques (SPs) and intracellular neurofibrillary tangles (NFTs). SPs are chiefly comprised of Aβ aggregates. The major component of NFTs is the microtubule binding protein *tau.* Clinically, AD is characterized by cognitive dysfunction and begins as a synaptic disorder that involves progressively larger areas of the brain over time [S1]. An emerging view of the processes involved in synaptic impairment shows that the subtlety and variability of the earliest amnesic symptoms, occurring in the absence of any other clinical signs of brain injury, can be due to discrete changes in the function of a single synapse, produced at least in part, by Aβ [S5, S7, S10, S11].

An important target for developing a causal therapy for Alzheimer's disease is represented by synapses. Synaptic alterations are highly correlated with the severity of clinical dementia [S1, S2], whereas other important variables such as senile plaques and neurofibrillary tangles are involved to a lesser extent [S1]. The importance of synaptic alterations in AD has been confirmed by studies of transgenic (Tg) mouse models of AD [S3] as well as of long-term potentiation (LTP), a widely studied cellular model of learning and memory (L&M) [S4], which is impaired following application of amyloid-β (Aβ) both in slices and *in vivo* [S3, S5-S12]. Aβ has been found to markedly inhibit LTP. Electrophysiological studies using Tg, human Aβ producing mice have often revealed significant deficits in basal synaptic transmission and/or LTP in the hippocampus [S23-S30].

Regulation of gene expression in long-term memory is known to be modulated by epigenetics. Epigenetics is defined as the mechanism that changes gene expression by 'marking' DNA or its associated proteins, through processes such as DNA methylation and histone modification, without changing the DNA sequence itself^{[A14]}. Modification of histones by, for example, the addition or removal of acetyl or methyl functional groups causes the chromatin structure to open or close, so that the information contained within the DNA is made more or less accessible to transcription factors. Hence, it is not surprising that deregulation of one of the epigenetic mechanisms might lead to disruption of memory associated gene expression. Studies of the mechanisms underlying synaptic and memory dysfunction in AD have indicated central roles for the transcription factor CREB (CRE binding protein) and the coactivator CREB binding protein (CBP).

Recent studies have linked the transcriptional machinery to Alzheimer's disease (AD), a pathology characterized by profound memory disorders. Both the transcription factor CREB (CRE binding protein) and the coactivator CREB binding protein (CBP), two molecules that are known to be associated with chromatin and memory processes [B8-B10], are likely to play central roles in the mechanisms underlying synaptic and memory dysfunction in AD. Drugs enhancing CREB phosphorylation were shown to ameliorate memory and long-term potentiation (LTP), a type of synaptic plasticity that is thought to underlie learning and memory, both in mouse models of AD and following exposure to sublethal doses of Aβ42, a proteolytic product of the amyloid precursor protein (APP) [B11-B14]. In addition, cerebral CBP levels are reduced in mice lacking functional presenilin 1 (PS1) and presenilin 2 (PS2) [B15], two genes that have been implicated in AD [B16, B17]. Moreover, wild-type (WT) PS1 stimulates CBP transcriptional ability, whereas PS1(M146L) mutation does not have this effect [B18].

Following exposure to sublethal doses of Aβ₄₂ and in mouse models of AD, drugs enhancing CREB phosphorylation were shown to ameliorate LTP and memory^{[A5,A15-A17]}. Dysregulation of CBP histone acetyltransferase (HAT) activity disrupts memory associated gene expression^{[A18]}. In addition, cerebral CBP levels are reduced in mice lacking functional PS1 and PS2^{[A19]}. Moreover, wild-type (WT) PS1 stimulates CBP transcriptional ability, whereas PS1(M146L) mutation does not have this effect^{[A20]}.

NO is a central molecule in cellular biochemical processes. The gas has been established as an important messenger molecule in various steps of brain physiology, from development to synaptic plasticity and learning and memory. In AD research, NO has been found to have a protective effect on Aβ-induced damage of the nervous system [S38-S40]. Studies performed on PC12 cells, sympathetic neurons and hippocampal neurons, have shown that treatment with the NO generator S-nitroso penicillamine exerts a neuroprotective effect due to the inhibition of the pro-apoptotic factor caspase-2 by nitrosylation [S39], whereas inhibition of NO synthesis by N-nitro-L-arginine methyl ester does not protect against Aβ-induced neurotoxicity. Aβ has been found to impair NO generation by decreasing NMDA receptor signal transduction [S38], by subtracting NADPH availability to NO-synthase (NOS) [S41], or by inhibiting the phosphorylation of the serine-threonine kinase Akt [S42]. Moreover, i-NOS deletion enhances AD pathology in the APP mice [S43]. Thus, drugs enhancing the NO-cascade have a beneficial effect against AD [S44].

Despite the neuroprotective function of NO is clear and indisputable, the gas has also been viewed as a major agent of neuropathology and cell death when it is produced in high quantity. High amounts of NO lead to generation of significant quantity of peroxinitrites that are responsible for oxidative and nitrosative stress in Aβ-induced cell death [S45-S51]. In fact, release of low amounts of NO by the constitutive forms of NOS that include both the neuronal and the endothelial isoforms, n-NOS and e-NOS, promotes synaptic plasticity and learning, whereas uncontrolled production of high amounts of the gas by the inducible form of NOS (i-NOS) can promote oxidative and nitrosative stress via production of peroxinitrite [S45-S51]. Thus, both Aβ-induced downregulation of the NO cascade which blocks plasticity and memory and generation of peroxinitrites leading to cell death, can play roles in AD. The current status of drug research exploiting these discoveries is focused both on finding ways to upregulate the NO cascade and therefore elicit neuroprotection, as well as on finding ways to block peroxinitrite toxic effects in order to limit neuropathology [S52].

### HAT Activators optimized for CNS diseases and Cancer

None of the commercially available HAT activators were developed to have the characteristics required for administration in a chronic disease of the CNS, for example neurodegenerative diseases (such as AD, Parkinson's Disease, Huntington's Disease), or for cancer. Thus, in some embodiments, the invention provides methods for identifying an agent or compound for the treatment of neurodegenerative diseases (such as AD, Huntington's Disease, Parkinson's Disease, other Aβ-accumulation related neurodegenerative disorders or diseases characterized by elevated levels of inclusion bodies) that comprise selecting the agent or compound on the basis of having one or more characteristics that make the compound optimized for treating CNS diseases. For example, the characteristics can comprise: an EC₅₀ no greater than about 100 nM; histone acetylation activity *in vitro*; the ability to penetrate the BBB; or a combination thereof. In one embodiment, the HAT Activator compound is YF2, depicted in FIG. 3.

In some embodiments, the invention provides methods for identifying or designing agents or compounds for the treatment of neurodegenerative diseases, treatment of conditions associated with, but not limited to, elevated inclusion bodies, (e.g., conditions associated with Aβ, alpha-synuclein, lipofuscin, cleaved TARDBP-TDP-43, and/or Tau protein accumulation), where computer aided-medicinal chemistry methods are used to identify and/or design agents or compounds tailored to satisfy one or more of the characteristics mentioned above and/or to suit the strengths of various bioassays described herein.

The invention generally provides methods for identifying compounds which can be used for treating a neurodegenerative disease in a subject. The invention provides methods for identifying compounds which can be used for treating subjects that exhibit abnormally elevated amyloid beta plaques, or elevated Tau protein levels, or elevated alpha-synuclein levels, or inclusions, or lipofuscin level or inclusions, or cleaved TARDBP-TDP-43 level or inclusion, or accumulation of cleaved TARDBP / TDP-43 inclusions. In addition, the invention provides methods for identifying compounds which can be used for the treatment of Alzheimer's disease, Lewy body dementia, inclusion body myositis, cerebral amyloid angiopathy, Huntington's Disease, Parkinson's Disease, and cancer. The methods can comprise the identification of test compounds or agents (e.g., peptides (such as antibodies or fragments thereof), small molecules, nucleic acids (such as siRNA or antisense RNA), or other agents) that can bind to a HAT polypeptide molecule and/or activate or enhance the biological activity of a HAT polypeptide or its expression. In one embodiment, the compound is a HAT activator (for example a HAT activator compound having Formula (I). In another embodiment, the HAT Activator compound is YF2, depicted in FIG. 3.

The term "modulate", as it appears herein, refers to a change in the activity or expression of a protein molecule. For example, modulation can cause an increase or a decrease in protein activity, binding characteristics, or any other biological, functional, or immunological properties of a secretase protein molecule.

In one embodiment, a HAT activator compound can be a peptide fragment of a HAT protein that binds to a histone acetyltransferase protein. For example, the HAT activator molecule can encompass any portion of at least about 8 consecutive amino acids of SEQ ID NO: 1, 3, or 5. The fragment can comprise at least about 10 amino acids, at least about 20 amino acids, at least about 30 amino acids, at least about 40 amino acids, a least about 50 amino acids, at least about 60 amino acids, or at least about 75 amino acids of SEQ ID NO: 1, 3, or 5. In one embodiment, the peptide fragment is directed to a HAT protein, such as GCN5, GCN5L, HAT1, or PCAF.

The polypeptide sequence of a HAT protein, human HAT1, is depicted in SEQ ID NO: 1. The nucleotide sequence of human HAT1 is shown in SEQ ID NO: 2. Sequence information related to HAT1 is accessible in public databases by GenBank Accession numbers NM_003642 (for mRNA) and NP_003633 (for protein). HAT1 is also known as KAT1 (K(lysine) acetyltransferase 1). The protein encoded by this gene is a type B histone acetyltransferase (HAT) that is involved in the rapid acetylation of newly synthesized cytoplasmic histones, which are in turn imported into the nucleus for de novo deposition onto nascent DNA chains. Histone acetylation, particularly of histone H4, plays an important role in replication-dependent chromatin assembly.

SEQ ID NO: 1 is the human wild type amino acid sequence corresponding to the HAT protein, the HAT1 enzyme (residues 1-419):

SEQ ID NO: 2 is the human wild type nucleotide sequence corresponding to HAT protein, the HAT1 enzyme (residues 1-1682), wherein the underscored ATG denotes the beginning of the open reading frame:

The polypeptide sequence of a HAT protein, human PCAF, is depicted in SEQ ID NO: 3. The nucleotide sequence of human PCAF is shown in SEQ ID NO: 4. Sequence information related to PCAF is accessible in public databases by GenBank Accession numbers NM_003884 (for mRNA) and NP_003875 (for protein). PCAF is also known as KAT2B (K(lysine) acetyltransferase 2B). CBP and p300 are large nuclear proteins that bind to many sequence-specific factors involved in cell growth and/or differentiation, including c-jun and the adenoviral oncoprotein E1A. The protein encoded by this gene associates with p300/CBP. It has in vitro and in vivo binding activity with CBP and p300, and competes with E1A for binding sites in p300/CBP. It has histone acetyl transferase activity with core histones and nucleosome core particles, indicating that this protein plays a direct role in transcriptional regulation

SEQ ID NO: 3 is the human wild type amino acid sequence corresponding to the HAT protein, the PCAF enzyme (residues 1-832):

SEQ ID NO: 4 is the human wild type nucleotide sequence corresponding to HAT protein, the PCAF enzyme (residues 1-4824), wherein the underscored ATG denotes the beginning of the open reading frame:

The polypeptide sequence of a HAT protein, human GCN5L, is depicted in SEQ ID NO: 5. The nucleotide sequence of human GCN5L is shown in SEQ ID NO: 6. Sequence information related to GCN5L is accessible in public databases by GenBank Accession numbers NM_021078 (for mRNA) and NP_066564.2 (for protein). GCN5L is also known as KAT2A (K(lysine) acetyltransferase 2A). KAT2A, or GCN5, is a histone acetyltransferase (HAT) that functions primarily as a transcriptional activator. It also functions as a repressor of NF-kappa-B by promoting ubiquitination of the NF-kappa-B subunit RELA in a HAT-independent manner (Mao et al., Genes Dev. 2009 Apr 1;23(7):849-61).

SEQ ID NO: 5 is the human wild type amino acid sequence corresponding to the HAT protein, the GCN5L enzyme (residues 1-837):

SEQ ID NO: 6 is the human wild type nucleotide sequence corresponding to HAT protein, the GCN5L enzyme (residues 1-3127), wherein the underscored ATG denotes the beginning of the open reading frame:

Fragments include all possible amino acid lengths between and including about 8 and 100 about amino acids, for example, lengths between about 10 and 100 amino acids, between about 15 and 100 amino acids, between about 20 and 100 amino acids, between about 35 and 100 amino acids, between about 40 and 100 amino acids, between about 50 and 100 amino acids, between about 70 and 100 amino acids, between about 75 and 100 amino acids, or between about 80 and 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England). The HAT peptide fragments can be isolated from a natural source, genetically engineered, or chemically prepared. These methods are well known in the art.

A HAT Activator compound can also be a protein, such as an antibody (monoclonal, polyclonal, humanized, and the like), or a binding fragment thereof, directed against a histone acetyltransferase enzyme, such as GCN5, GCN5L, PCAF, or HAT1. An antibody fragment can be a form of an antibody other than the full-length form and includes portions or components that exist within full-length antibodies, in addition to antibody fragments that have been engineered. Antibody fragments can include, but are not limited to, single chain Fv (scFv), diabodies, Fv, and (Fab')₂, triabodies, Fc, Fab, CDR1, CDR2, CDR3, combinations of CDR's, variable regions, tetrabodies, bifunctional hybrid antibodies, framework regions, constant regions, and the like (*see*, Maynard et al., (2000) Ann. Rev. Biomed. Eng. 2:339-76; Hudson (1998) Curr. Opin. Biotechnol. 9:395-402). Antibodies can be obtained commercially, custom generated, or synthesized against an antigen of interest according to methods established in the art (Janeway et al., (2001) Immunobiology, 5th ed., Garland Publishing).

Inhibition of RNA encoding a HAT protein can effectively modulate the expression of a HAT gene (e.g., GCN5, GCN5L, PCAF, or HAT1) from which the RNA is transcribed. Inhibitors are selected from the group comprising: siRNA, interfering RNA or RNAi; dsRNA; RNA Polymerase III transcribed DNAs; ribozymes; and antisense nucleic acid, which can be RNA, DNA, or artificial nucleic acid.

Antisense oligonucleotides, including antisense DNA, RNA, and DNA/RNA molecules, act to directly block the translation of mRNA by binding to targeted mRNA and preventing protein translation. For example, antisense oligonucleotides of at least about 15 bases and complementary to unique regions of the DNA sequence encoding a HAT polypeptide can be synthesized, e.g., by conventional phosphodiester techniques (Dallas et al., (2006) Med. Sci. Monit.12(4):RA67-74; Kalota et al., (2006) Handb. Exp. Pharmacol. 173:173-96; Lutzelburger et al., (2006) Handb. Exp. Pharmacol. 173:243-59).

siRNA comprises a double stranded structure containing from about 15 to about 50 base pairs, for example from about 21 to about 25 base pairs, and having a nucleotide sequence identical or nearly identical to an expressed target gene or RNA within the cell. Antisense nucleotide sequences include, but are not limited to: morpholinos, 2'-O-methyl polynucleotides, DNA, RNA and the like. RNA polymerase III transcribed DNAs contain promoters, such as the U6 promoter. These DNAs can be transcribed to produce small hairpin RNAs in the cell that can function as siRNA or linear RNAs that can function as antisense RNA. The HAT activator compound can contain ribonucleotides, deoxyribonucleotides, synthetic nucleotides, or any suitable combination such that the target RNA and/or gene is inhibited. In addition, these forms of nucleic acid can be single, double, triple, or quadruple stranded. (see for example Bass (2001) Nature, 411, 428 429; Elbashir et al., (2001) Nature, 411, 494 498; and PCT Publication Nos. WO 00/44895, WO 01/36646, WO 99/32619, WO 00/01846, WO 01/29058, WO 99/07409, WO 00/44914).

In some embodiments, a HAT Activator compound can be a small molecule that binds to a histone acetyltransferase enzyme, such as GCN5, GCN5L, PCAF, or HAT1, and disrupts its function. Small molecules are a diverse group of synthetic and natural substances generally having low molecular weights. They can be isolated from natural sources (for example, plants, fungi, microbes and the like), are obtained commercially and/or available as libraries or collections, or synthesized. Candidate small molecules that interact with a HAT protein can be identified via *in silico* screening or high-through-put (HTP) screening of combinatorial libraries. Most conventional pharmaceuticals, such as aspirin, penicillin, and many chemotherapeutics, are small molecules, can be obtained commercially, can be chemically synthesized, or can be obtained from random or combinatorial libraries as described below (Werner et al., (2006) Brief Funct. Genomic Proteomic 5(1):32-6).

Knowledge of the primary sequence of a molecule of interest, such as a HAT polypeptide, and the similarity of that sequence with other proteins of the same histone acetyltransferase family (such as the GNAT family, the MYST family or the GCN5 family [see Lee and Owrkman (2007) Nat Rev Mol Cell Biol., 8(4):284-95, Marmorstein (2001) J Molec Biol. 311: 433-444; and Kimura et al., (2005) J Biocehm. 138(6): 647-662]), can provide information as to the inhibitors or antagonists of the protein of interest. Identification and screening antagonists can be further facilitated by determining structural features of the protein, e.g., using X-ray crystallography, neutron diffraction, nuclear magnetic resonance spectrometry, and other techniques for structure determination. These techniques provide for the rational design or identification of antagonists, in addition to protein agonists.

The invention provides methods for screening and identifying compounds useful for treating a neurodegenerative disease in a subject. The invention provides methods for identifying compounds which can be used for treating subjects that exhibit, for example, abnormally elevated amyloid beta plaques, or elevated Tau protein levels, or elevated alpha-synuclein levels, or inclusions, or lipofuscin level or inclusions, or cleaved TARDBP-TDP-43 level or inclusion, or accumulation of cleaved TARDBP / TDP-43 inclusions, or a combination thereof. In one embodiment, the method comprises selecting a HAT Activator compound that comprises one or both of the following features: (a) the EC₅₀ of the compound is no more than about 1000 nM; (b) the compound penetrates the blood brain barrier; (c) the compound enhances histone acetylation (for example acetylates histone protein H3 or H4), or a combination thereof. In a further embodiment, the compound, for example the HAT Activator, has an EC₅₀ of at least about 0.1 nM, at least about 1 nM, at least about 5 nM, at least about 10 nM, at least about 25 nM, at least about 50 nM, at least about 100 nM, at least about 200 nM, at least about 300 nM, at least about 400 nM, at least about 500 nM, at least about 600 nM, at least about 700 nM, at least about 800 nM, or at least about 900 nM. In some embodiments, the HAT Activator compound can have a molecular mass less than about 500 Da in order to penetrate the blood brain barrier. In other embodiments, the HAT Activator compound can have a polar surface area less than about 90 Å² and should have 8 or fewer hydrogen bonds in order to penetrate the blood brain barrier. The screening and identifying of the compound can comprise in silico screening, molecular docking, in vivo screening, in vitro screening, or a combination thereof.

Test compounds, such as HAT Activator compounds, can be screened from large libraries of synthetic or natural compounds (see Wang et al., (2007) Curr Med Chem, 14(2):133-55; Mannhold (2006) Curr Top Med Chem, 6 (10):1031-47; and Hensen (2006) Curr Med Chem 13(4):361-76). Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means (Blondelle et al., (1996) Tib Tech 14:60).

Methods for preparing libraries of molecules are well known in the art and many libraries are commercially available. Libraries of interest in the invention include peptide libraries, randomized oligonucleotide libraries, synthetic organic combinatorial libraries, and the like. Degenerate peptide libraries can be readily prepared in solution, in immobilized form as bacterial flagella peptide display libraries or as phage display libraries. Peptide ligands can be selected from combinatorial libraries of peptides containing at least one amino acid. Libraries can be synthesized of peptoids and non-peptide synthetic moieties. Such libraries can further be synthesized which contain non-peptide synthetic moieties, which are less subject to enzymatic degradation compared to their naturally-occurring counterparts. Libraries are also meant to include for example but are not limited to peptide-on-plasmid libraries, polysome libraries, aptamer libraries, synthetic peptide libraries, synthetic small molecule libraries, neurotransmitter libraries, and chemical libraries. The libraries can also comprise cyclic carbon or heterocyclic structure and/or aromatic or polyaromatic structures substituted with one or more of the functional groups described herein.

Small molecule combinatorial libraries can also be generated and screened. A combinatorial library of small organic compounds is a collection of closely related analogs that differ from each other in one or more points of diversity and are synthesized by organic techniques using multi-step processes. Combinatorial libraries include a vast number of small organic compounds. One type of combinatorial library is prepared by means of parallel synthesis methods to produce a compound array. A compound array can be a collection of compounds identifiable by their spatial addresses in Cartesian coordinates and arranged such that each compound has a common molecular core and one or more variable structural diversity elements. The compounds in such a compound array are produced in parallel in separate reaction vessels, with each compound identified and tracked by its spatial address. Examples of parallel synthesis mixtures and parallel synthesis methods are provided in U.S. Ser. No. 08/177,497, filed Jan. 5, 1994 and its corresponding PCT published patent application WO95/18972, published Jul. 13, 1995 and U.S. Pat. No. 5,712,171 granted Jan. 27, 1998 and its corresponding PCT published patent application WO96/22529.

Examples of chemically synthesized libraries are described in Fodor et al., (1991) Science 251:767-773; Houghten et al., (1991) Nature 354:84-86; Lam et al., (1991) Nature 354:82-84; Medynski, (1994) BioTechnology 12:709-710; Gallop et al., (1994) J. Medicinal Chemistry 37(9):1233-1251; Ohlmeyer et al., (1993) Proc. Natl. Acad. Sci. USA 90:10922-10926; Erb et al., (1994) Proc. Natl. Acad. Sci. USA 91:11422-11426; Houghten et al., (1992) Biotechniques 13:412; Jayawickreme et al., (1994) Proc. Natl. Acad. Sci. USA 91:1614-1618; Salmon et al., (1993) Proc. Natl. Acad. Sci. USA 90:11708-11712; PCT Publication No. WO 93/20242, dated Oct. 14, 1993; and Brenner et al., (1992) Proc. Natl. Acad. Sci. USA 89:5381-5383.

Examples of phage display libraries are described in Scott et al., (1990) Science 249:386-390; Devlin et al., (1990) Science, 249:404-406; Christian, et al., (1992) J. Mol. Biol. 227:711-718; Lenstra, (1992) J. Immunol. Meth. 152:149-157; Kay et al., (1993) Gene 128:59-65; and PCT Publication No. WO 94/18318.

*In vitro* translation-based libraries include but are not limited to those described in PCT Publication No. WO 91/05058; and Mattheakis et al., (1994) Proc. Natl. Acad. Sci. USA 91:9022-9026.

In one non-limiting example, non-peptide libraries, such as a benzodiazepine library (see e.g., Bunin et al., (1994) Proc. Natl. Acad. Sci. USA 91:4708-4712), can be screened. Peptoid libraries, such as that described by Simon et al., (1992) Proc. Natl. Acad. Sci. USA 89:9367-9371, can also be used. Another example of a library that can be used, in which the amide functionalities in peptides have been permethylated to generate a chemically transformed combinatorial library, is described by Ostresh et al. (1994), Proc. Natl. Acad. Sci. USA 91:11138-11142.

The three dimensional geometric structure of an active site, for example that of a HAT polypeptide can be determined by known methods in the art, such as X-ray crystallography, which can determine a complete molecular structure. Solid or liquid phase NMR can be used to determine certain intramolecular distances. Any other experimental 52 method of structure determination can be used to obtain partial or complete geometric structures. The geometric structures can be measured with a complexed ligand, natural or artificial, which can increase the accuracy of the active site structure determined. In one embodiment, a compound that binds to a HAT protein, such as GCN5, GCN5L, PCAF, or HAT1, can be identified via: (1) providing an electronic library of test compounds; (2) providing atomic coordinates listed in PDB Entry No. 1YGH or 2RC4, for at least 20 amino acid residues for the acetyltransferase active site of the HAT protein (the HAT domain), wherein the coordinates have a root mean square deviation therefrom, with respect to at least 50% of Cα atoms, of not greater than about 2 Å, in a computer readable format; (3) converting the atomic coordinates into electrical signals readable by a computer processor to generate a three dimensional model of the HAT protein; (4) performing a data processing method, wherein electronic test compounds from the library are docked onto the three dimensional model of the HAT protein; and determining which test compound fits into the active site of the three dimensional model of the HAT protein, thereby identifying which compound would bind to a HAT protein. In another embodiment, the method can further comprise: synthesizing or obtaining the compound determined to dock to the active site of the HAT protein; contacting the HAT protein with the compound under a condition suitable for binding; and determining whether the compound modulates HAT protein expression or mRNA expression, or HAT protein activity using a diagnostic assay.

Methods for predicting the effect on protein conformation of a change in protein sequence, are known in the art, and the skilled artisan can thus design a variant which functions as an antagonist according to known methods. One example of such a method is described by Dahiyat and Mayo in Science (1997) 278:82 87, which describes the design of proteins *de novo.* The method can be applied to a known protein to vary only a portion of the polypeptide sequence. Similarly, Blake (U.S. Pat. No. 5,565,325) teaches the use of known ligand structures to predict and synthesize variants with similar or modified function.

Other methods for preparing or identifying peptides that bind to a target are known in the art. Molecular imprinting, for instance, can be used for the de novo construction of macromolecular structures such as peptides that bind to a molecule. See, for example, Kenneth J. Shea, Molecular Imprinting of Synthetic Network Polymers: The De Novo synthesis of Macromolecular Binding and Catalytic Sites, TRIP Vol. 2, No. 5, May 1994; Mosbach, (1994) Trends in Biochem. Sci., 19(9); and Wulff, G., in Polymeric Reagents and Catalysts (Ford, W. T., Ed.) ACS Symposium Series No. 308, pp 186-230, American Chemical Society (1986). One method for preparing mimics of a HAT protein involves the steps of: (i) polymerization of functional monomers around a known substrate (the template) that exhibits a desired activity; (ii) removal of the template molecule; and then (iii) polymerization of a second class of monomers in, the void left by the template, to provide a new molecule which exhibits one or more desired properties which are similar to that of the template. Other binding molecules such as polysaccharides, nucleosides, drugs, nucleoproteins, lipoproteins, carbohydrates, glycoproteins, steroids, lipids, and other biologically active materials can also be prepared. This method is useful for designing various biological mimics that are more stable than their natural counterparts, because they are prepared by the free radical polymerization of functional monomers, resulting in a compound with a nonbiodegradable backbone. Other methods for designing such molecules include , e.g., drug design based on structure activity relationships, which require the synthesis and evaluation of a number of compounds and molecular modeling.

The description also provides *in vivo* and *in vitro* methods for identifying a compound that binds to a HAT protein. In one embodiment, the method comprises: (a) obtaining a tissue and/or cells that express a HAT protein (such as GCN5, GCN5L, PCAF, or HAT1); (b) contacting the tissue and/or cell with a ligand source for an effective period of time; (c) measuring a secondary messenger response, wherein the response is indicative of a ligand binding to a HAT protein; (d) isolating the ligand from the ligand source; and (e) identifying the structure of the ligand that binds a HAT protein, thereby identifying which compound would bind to a HAT protein. As used herein, the term "ligand source" can be any compound library described herein, or a library of neurotransmitters that can be used to screen for compounds that would act as an agonist of a HAT protein (such as GCN5, GCN5L, PCAF, or HAT1). Screening compound libraries listed herein [also see U.S. Patent Application Publication No. 2005/0009163], in combination with *in vivo* animal studies and functional assays can be used to identify HAT Activator compounds that can be used to treat subjects afflicted with abnormal Aβ deposits, such as AD or to treat cancer.

A HAT Activator compound can be a compound that increases the activity and/or expression of a HAT molecule (e.g., GCN5, GCN5L, PCAF, or HAT1) *in vivo* and/or *in vitro.* HAT Activator compounds can be compounds that exert their effect on the activity of a HAT protein via the expression, via post-translational modifications, or by other means. In one embodiment, a HAT Activator compound can increase HAT protein or mRNA expression, or acetyltransferase activity by at least about 10%, at least about 20%, at least about 30%, at least about 40%, at least about 50%, at least about 60%, at least about 70%, at least about 75%, at least about 80%, at least about 85%, at least about 90%, at least about 95%, at least about 97%, at least about 99%, or 100%.

Test compounds or agents which bind to a HAT molecule (such as GCN5, GCN5L, PCAF, or HAT1), and/or have a stimulatory effect on the activity or the expression of a HAT molecule, can be identified by various assays. The assay can be a binding assay comprising direct or indirect measurement of the binding of a test compound or a known HAT ligand to the active site of a HAT protein. The assay can also be an activity assay comprising direct or indirect measurement of the activity of a HAT molecule. The assay can also be an expression assay comprising direct or indirect measurement of the expression of a HAT mRNA or protein. The various screening assays can be combined with an *in vivo* assay comprising measuring the effect of the test compound on cognitive and synaptic function in an animal model for neurodegenerative disorders, such as, but not imited to, AD or Huntington's Disease.

The diagnostic assay of the screening methods of the invention can also involve monitoring the expression of a HAT molecule. For example, inhibitors of the expression of a HAT molecule can be identified via contacting a HAT-positive cell or tissue with a test compound and determining the expression of a HAT protein or HAT mRNA in the cell. The protein or mRNA expression level of a HAT molecule in the presence of the test compound is compared to the protein or mRNA expression level of a HAT protein in the absence of the test compound. The test compound can then be identified as an inhibitor of expression of a HAT protein (such as GCN5, GCN5L, PCAF, or HAT1) based on this comparison. Acivators of the expression of a HAT molecule can also be identified via contacting a HAT-positive cell or tissue with a test compound and determining the expression of a HAT protein or HAT mRNA in the cell. The protein or mRNA expression level of a HAT molecule in the presence of the test compound is compared to the protein or mRNA expression level of a HAT protein in the absence of the test compound. The test compound can then be identified as an activator of expression of a HAT protein (such as GCN5, GCN5L, PCAF, or HAT1) based on this comparison. For example, when expression of HAT protein or mRNA is statistically or significantly more in the presence of the test compound than in its absence, the compound is identified as an activator of the expression of a HAT protein or mRNA. In other words, the test compound can also be said to be a HAT Activator compound (such as an agonist). The expression level of a HAT protein or mRNA in cells can be determined by methods described herein.

Determining the ability of a test compound to bind to a HAT molecule or a variant thereof can be accomplished using real-time Bimolecular Interaction Analysis (BIA) [McConnell, (1992); Sjolander, (1991)]. BIA is a technology for studying biospecific interactions in real time, without labeling any of the interactants (e.g., BIA-core™). Changes in optical phenomenon surface plasmon resonance (SPR) can be used as an indication of real-time reactions between biological molecules.

Exemplary HAT Activator compounds optimized for CNS disorders and Cancer

The invention provides for compounds that bind to a HAT activator protein, such as GCN5, GCN5L, PCAF, or HAT1. These compounds can be identified by the screening methods and assays described herein, and enhance the activity or expression of HAT activator proteins. In one embodiment, the invention encompasses a compound of Formula (I): 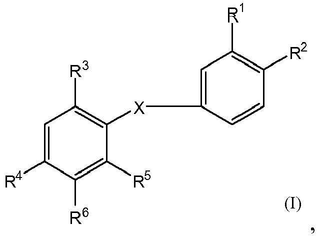 wherein,
R¹ is CF₃;
R² is Cl, or CN;
R³ is H, O-methyl, O-ethyl, S-ethyl, O-cyclopentyl, OCH₂CH₂N(CH₃)₂, or CH₂CH₂CH₂N(CH₃)₂;
R⁴ is H; C(=O)NH-phenyl, wherein phenyl is substituted with one or more halo or CF₃;
R⁵ is H, C₁-C₅-alkyl, OH, OCH₃, O-ethyl, OCH₂CH₂N(CH₃)₂, CH₂CH₂CH₂N(CH₃)₂, SCH₂CH₂N(CH₃)₂, or OCH₂C(=O)O-alkyl;
R⁶ is H, O-methyl, O-ethyl, OCH₂CH₂N(CH₃)₂; and
X is CONH, SONH, SO₂NH, NHC(=O)NH, or NHCO, or a pharmaceutically acceptable salt or hydrate thereof.

In one embodiment of the compound of Formula (I),
R¹ is CF3;
R² is Cl, or CN;
R³ is H, O-(C₁-C₂)-alkyl, O-(C₃-C₆)-cycloalkyl, O-(C₁-C₂)-alkyl-CO₂-(C₁-C₂)-alkyl, or S-(C₁-C₂)-alkyl;
R⁴ is H;
R⁵ is H, (C₁-C₆)-alkyl, OH, OCH₂CH₂N(CH₃)₂, S-(C₁-C₂)-alkyl, or SCH₂CH₂N(CH₃)₂;
R⁶ is H, or OCH₂CH₂N(CH₃)₂; and
X = SO₂NH, CONH, NHCO, or NHCONH, or a pharmaceutically acceptable salt or hydrate thereof.

In particular embodiments, the compound of Formula (I) is:

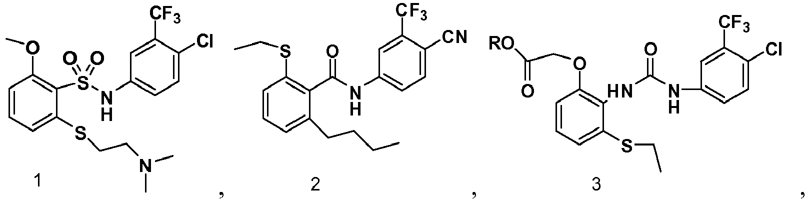

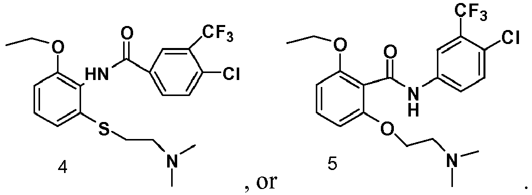

In some embodiments, the compound of Formula (I) is

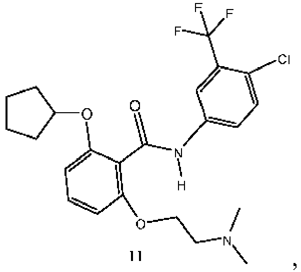

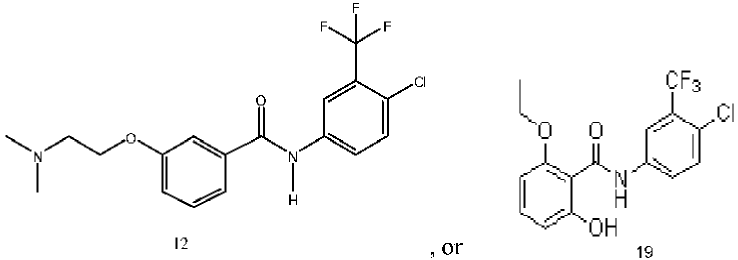

In another embodiment of the compound of Formula (I),
R¹ is CF₃,
R² is Cl;
R³ is H, O-(C₁-C₂)-alkyl, O-(C₃-C₆)-cycloalkyl, or O-(C₁-C₂)-alkyl-CO₂-(C₁-C₂)-alkyl;
R⁴ is H;
R⁵ is H, (C₁-C₆)-alkyl, OH, OCH₂CH₂N(CH₃)₂, S-(C₁-C₂)-alkyl, or SCH₂CH₂N(CH₃)₂;
R⁶ is H, or OCH₂CH₂N(CH₃)₂; and
X is SO₂NH, CONH, NHCO, or NHCONH, or a pharmaceutically acceptable salt or hydrate thereof.

In particular embodiments, the compound of Formula (I) is: 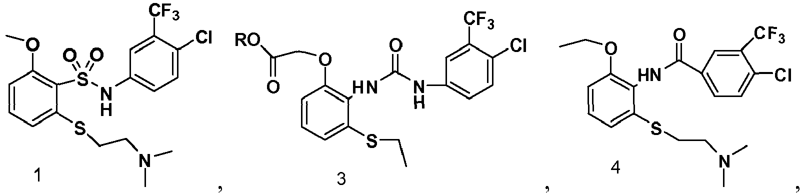 or

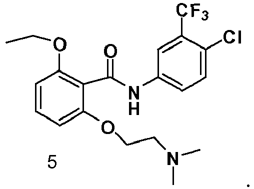

In some embodiments, the compound is,

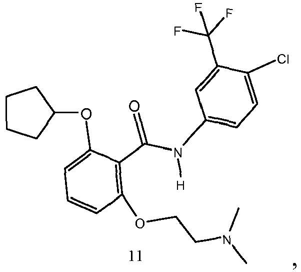

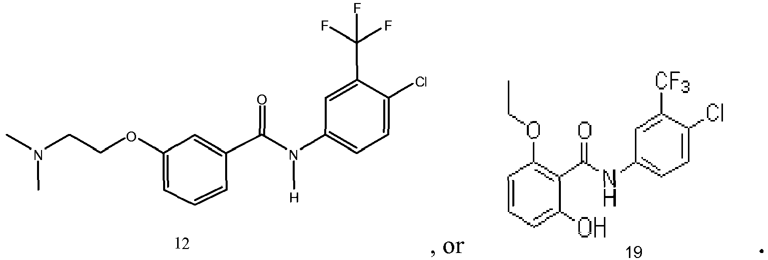

In a further embodiment of the compound of Formula (I),
R¹ is CF3;
R² is CN;
R³ is S-(C₁-C₂)-alkyl, or OCH₂CH₂N(CH₃)₂;
R⁴ is H;
R⁵ is H, or (C₁-C₄)-alkyl;
R⁶ is H; and
X is CONH, or a pharmaceutically acceptable salt or hydrate thereof.

In particular embodiments, the compound of Formula (I) is:

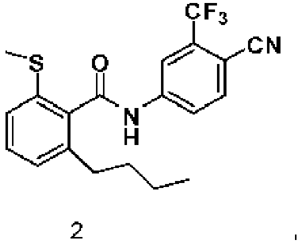

In some embodiments of the compound of Formula (I),
R¹ is CF₃;
R² is Cl;
R³ is H, O-(C₁-C₂)-alkyl, OCH₂CH₂N(CH₃)₂, or CH₂CH₂CH₂N(CH₃)₂;
R⁴ is C(O)NH-(3-CF₃, 4-Cl-phenyl);
R⁵ is H, O-(C₁-C₂)-alkyl, OCH₂CH₂N(CH₃)₂, or CH₂CH₂CH₂N(CH₃)₂;
R⁶ is H, or O-(C₁-C₂)-alkyl; and
X = CONH, or a pharmaceutically acceptable salt or hydrate thereof.

In particular embodiments, the compound of Formula (I) is: 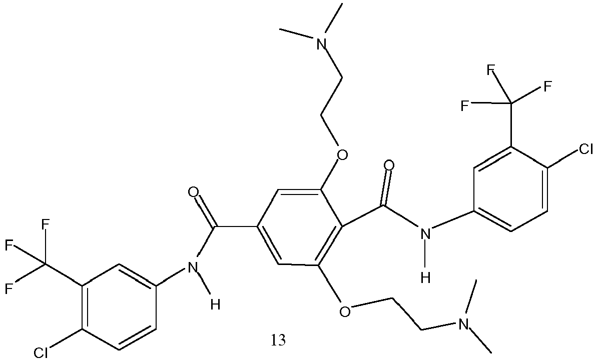 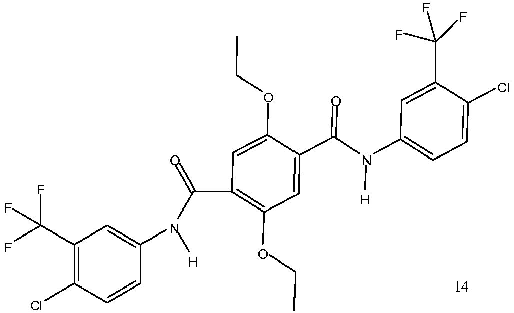 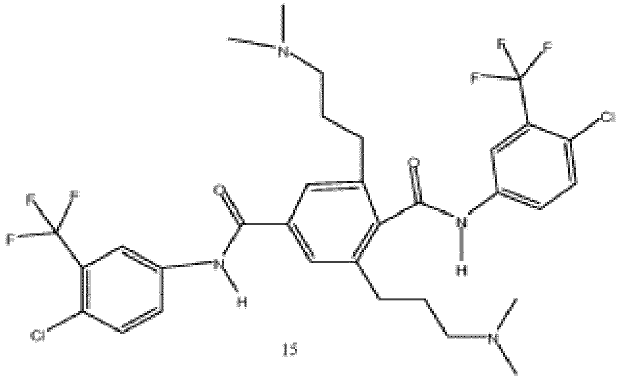 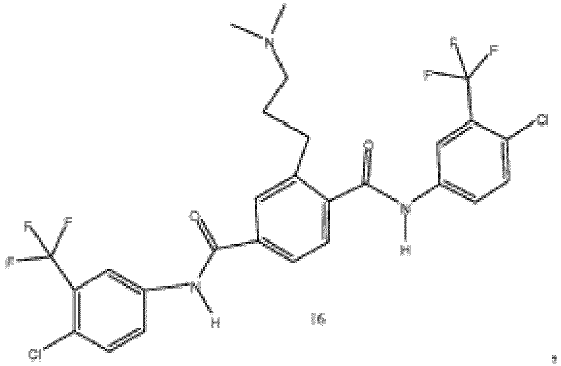 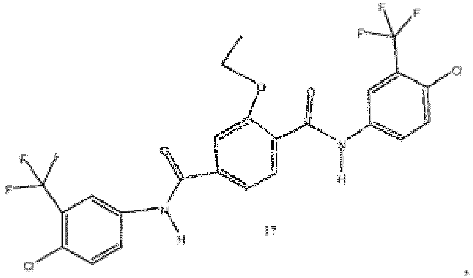 or

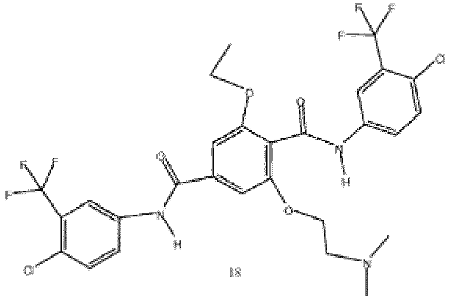

In one embodiment of the compound of Formula (I),
R¹ is CF₃;
R² is Cl;
R³ is O-(C₁-C₂)-alkyl;
R⁴ is H;
R⁵ is OCH₂CH₂N(CH₃)₂; and
X is CONH, or a pharmaceutically acceptable salt or hydrate thereof.

In another embodiment of the compound of Formula (I),
R¹ is CF₃;
R² is Cl;
R³ is H, 0-(C₁-C₂)-alkyl;
R⁴ is H;
R⁵ is SCH₂CH₂N(CH₃)₂;
R⁶ is H; and
X = SO₂NH, or a pharmaceutically acceptable salt or hydrate thereof.

The description discloses reference HAT Activator compounds of Formula (II), wherein: 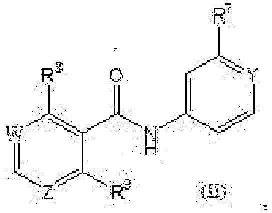 wherein,
R⁷ is H or CF₃;
R⁸ is O-ethyl or S-methyl;
R⁹ is butyl or OCH₂CH₂N(CH₃)₂;
Y is C-Cl, C-CN, C-NO₂, or N;
W is CH or N; and
Z is CH or N, or a pharmaceutically acceptable salt or hydrate thereof.

The reference compound of Formula (II) is:

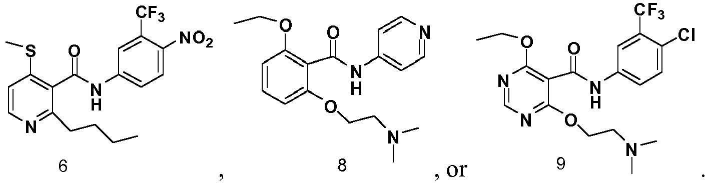

The description also discloses reference HAT Activator compounds of Formula (III), wherein: 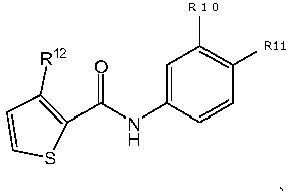 wherein
R¹⁰ is CF₃;
R¹¹ is CN; and
R¹² is O-ethyl, or a pharmaceutically acceptable salt or hydrate thereof

The description also discloses HAT Activator reference compounds of Formula (IV), wherein: 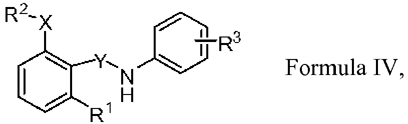 wherein,
X is S, S(O)₂, NH, O, or C;
Y is -C(O), S(O)₂, or NH-C(O);
R^{I} is H, Methyl, Ethyl, n-Propyl, Isopropyl, n-butyl, t-butyl, C₈H₁₈, C₁₅H₂₆, C₁₅H₂₈, C₁₅H₃₀, C₁₅H₃₂, SR⁴, or OR⁴;
R² is H, methyl, ethyl, n-propyl, isopropyl, n-butyl, t-butyl, or (C₁-C₆ alkyl)-CO₂R⁶;
R³ is H, methyl, ethyl, n-propyl, isopropyl, n-butyl, t-butyl, CF₃, CCl₃, Cl₃₅ F, Cl, I, NO₂, or CN;
R⁴ is (C₁-C₆ alkyl)-N(R⁵)₂- or C₁-C₆ alkyl;
R⁵ is independently hydrogen, C₁-C₆ alkyl, or C₃-Cs cycloalkyl; and
R⁶ is hydrogen, C₁-C₆ alkyl, or C₃-Cs cycloalkyl, or a pharmaceutically acceptable salt or hydrate thereof

In one embodiment, X is O, while Y is N-C=O. In another embodiment, X is S, while Y is N-C=O.

In specific embodiments, the compound is YF2:

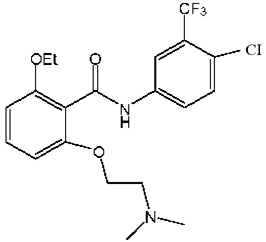

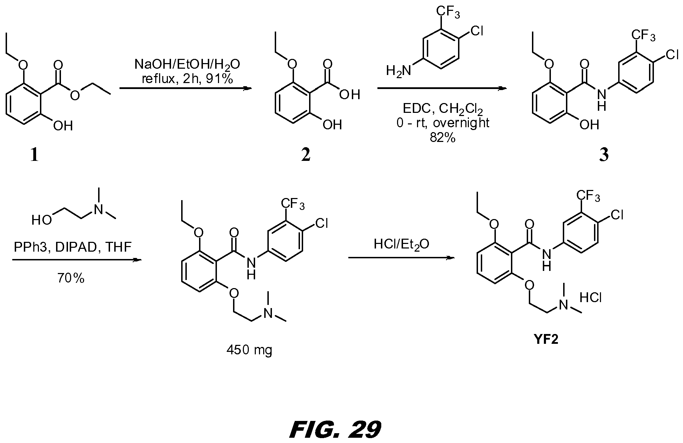

In one embodiment, the HAT Activator compound, YF2, can be synthesized according to the scheme depicted in **FIG. 29****.** Other analogs of HAT Activator compounds having Formula I can be similarly synthesized.

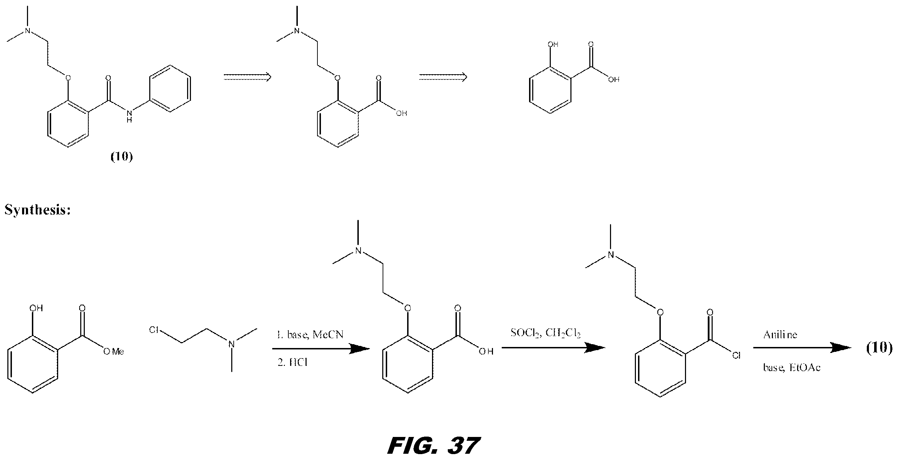

In one embodiment, the HAT Activator compound, 10, can be synthesized according to the scheme depicted in **FIG. 37****.**

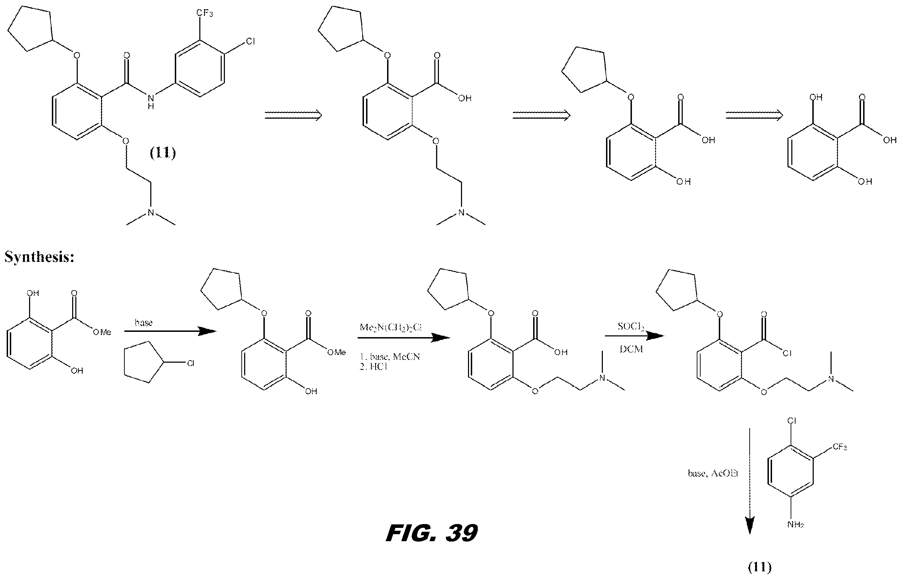

In one embodiment, the HAT Activator compound, 11, can be synthesized according to the scheme depicted in **FIG. 39****.**

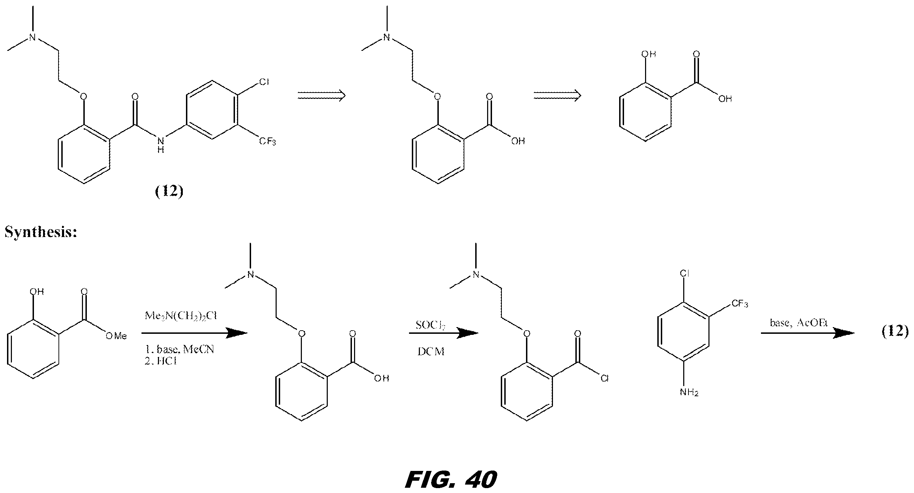

In one embodiment, the HAT Activator compound, 12, can be synthesized according to the scheme depicted in **FIG. 40****.**

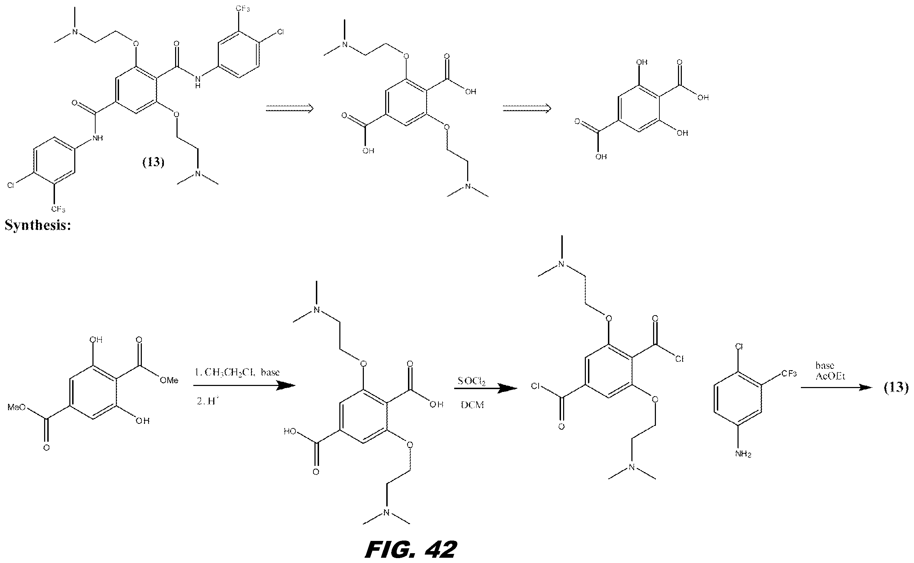

In one embodiment, the HAT Activator compound, 13, can be synthesized according to the scheme depicted in **FIG. 42****.**

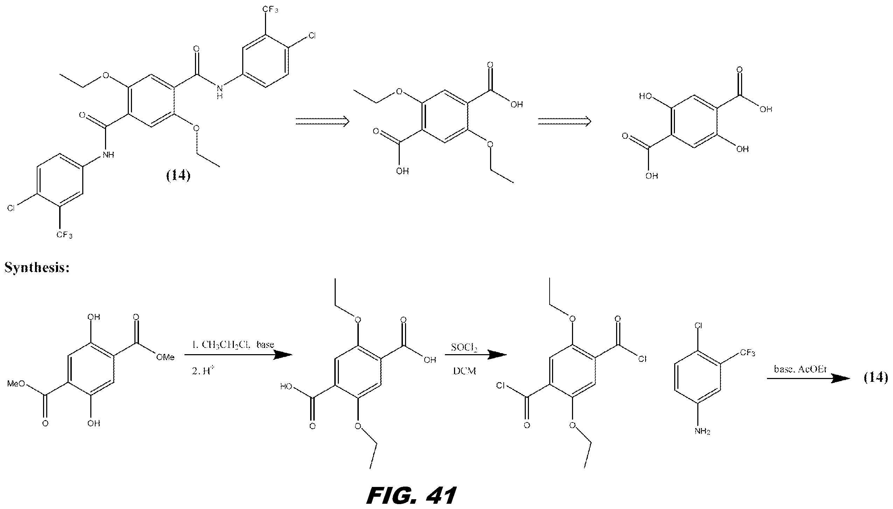

In one embodiment, the HAT Activator compound, 14, can be synthesized according to the scheme depicted in **FIG. 41****.**

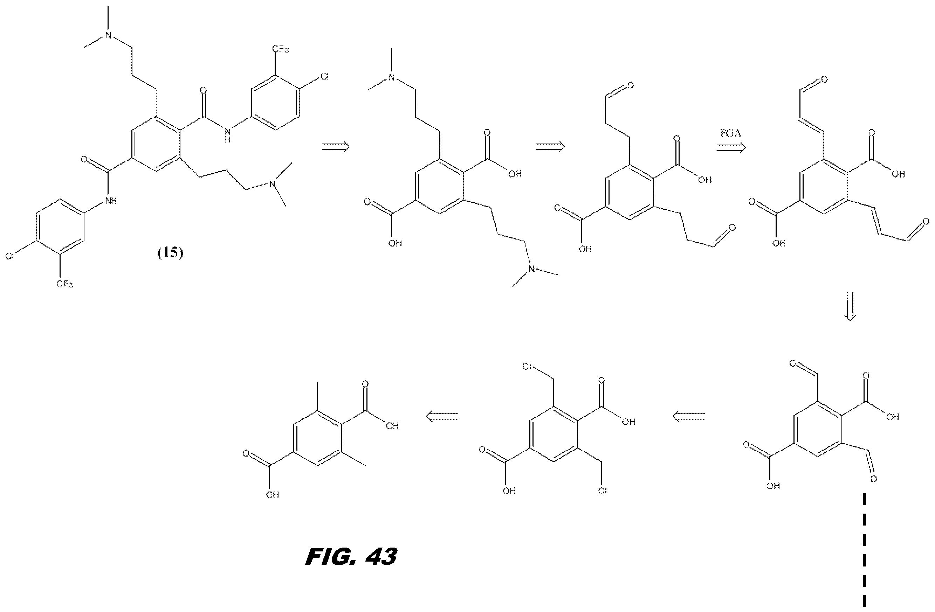

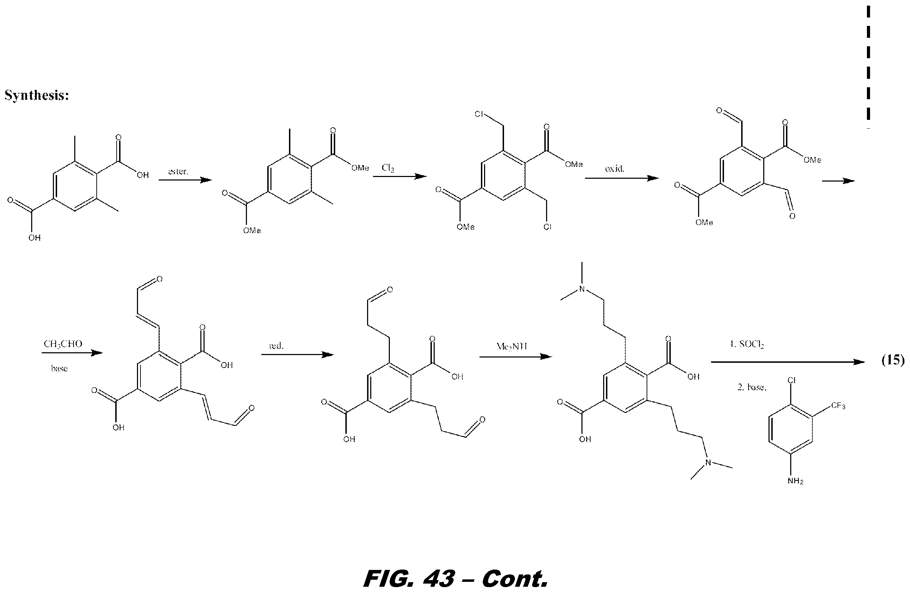

In one embodiment, the HAT Activator compound, 15, can be synthesized according to the scheme depicted in **FIG. 43****.**

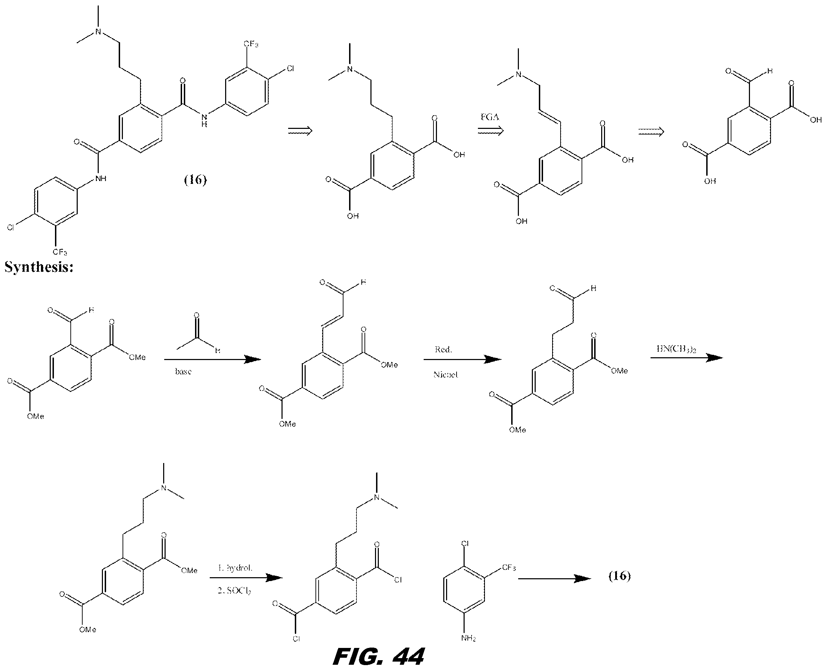

In one embodiment, the HAT Activator compound, 16, can be synthesized according to the scheme depicted in **FIG. 44****.**

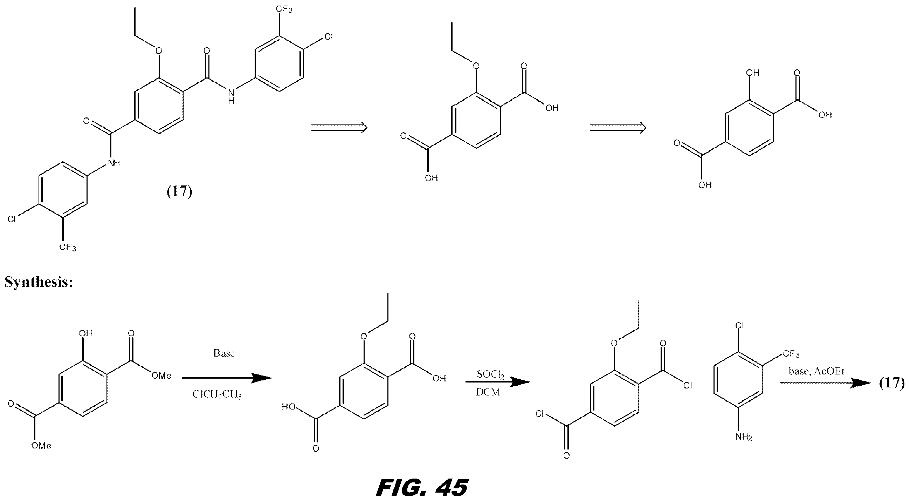

In one embodiment, the HAT Activator compound, 17, can be synthesized according to the scheme depicted in **FIG. 45****.**

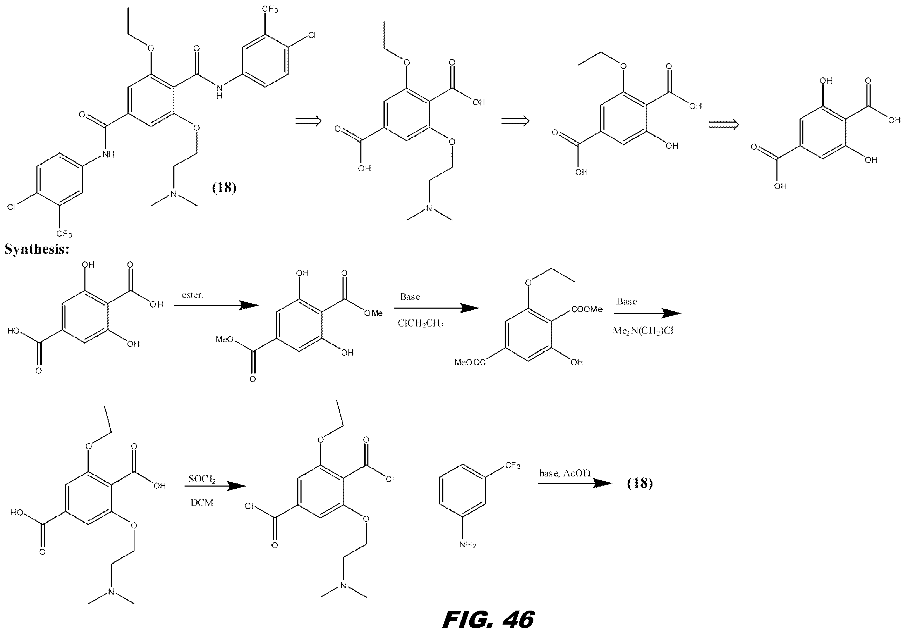

In one embodiment, the HAT Activator compound, 18, can be synthesized according to the scheme depicted in **FIG. 46****.**

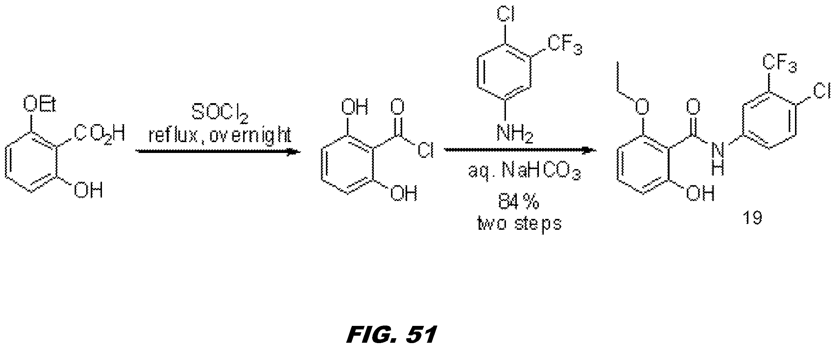

In one embodiment, the HAT Activator compound, 19, can be synthesized according to the scheme depicted in **FIG. 51****.**

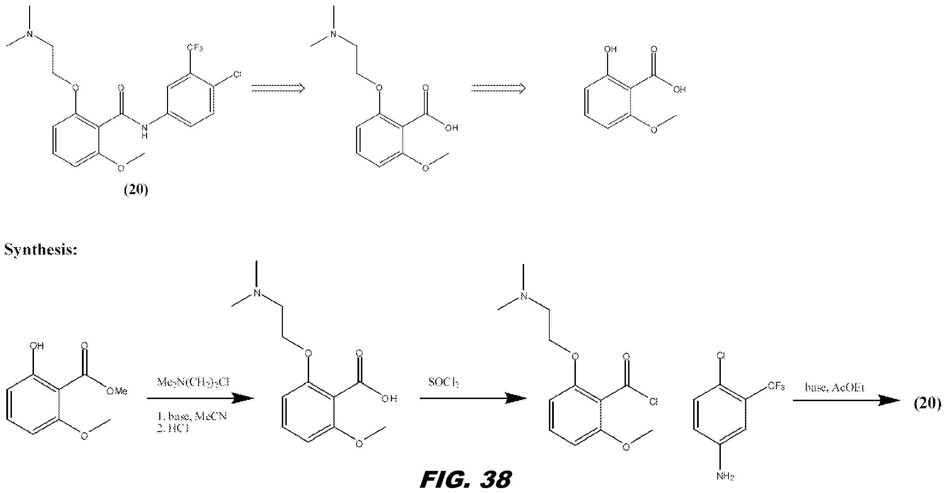

In one embodiment, the HAT Activator compound, 20, can be synthesized according to the scheme depicted in **FIG. 38****.**

The description also discloses reference HAT Activator compounds of 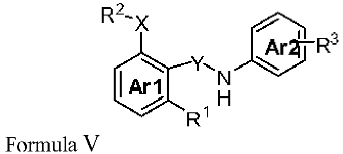 , wherein,
X is S, S(O)₂, NH, O, or C;
Y is -C(O), S(O)₂, or NH-C(O);
AR1 is a 5-membered aromatic ring or a 6-membered aromatic ring containing 1-2 Nitrogens;
AR2 is a 5-membered aromatic ring, a 6-membered aromatic ring or a 6-membered aromatic ring containing 1-2 Nitrogens;
R^{I} is H, Methyl, Ethyl, n-Propyl, Isopropyl, n-butyl, t-butyl, C₈H₁₈, C₁₅H₂₆,
C₁₅H₂₈, C₁₅H₃₀, C₁₅H₃₂, SR⁴, or OR⁴;
R² is H, methyl, ethyl, n-propyl, isopropyl, n-butyl, t-butyl, or (C₁-C₆ alkyl)-CO₂R⁶;
R³ is H, methyl, ethyl, n-propyl, isopropyl, n-butyl, t-butyl, CF₃, CCl₃, Cl₃, F, Cl, I, NO₂, or CN;
R⁴ is (C₁-C₆ alkyl)-N(R⁵)₂- or C₁-C₆ alkyl;
R⁵ is independently hydrogen, C₁-C₆ alkyl, or C₃-C₈ cycloalkyl; and
R⁶ is hydrogen, C₁-C₆ alkyl, or C₃-C₈ cycloalkyl, or a pharmaceutically acceptable salt or hydrate thereof.

In one embodiment, X is O, while Y is N-C=O. In another embodiment, X is S, while Y is N-C=O.

In particular embodiments, the reference compound of Formula (V) is:

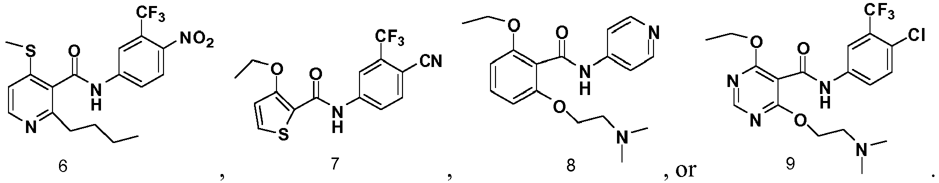

The compounds of the invention can be generally synthesized according to the scheme based on the diagram depicted in **FIG. 29****.**

Pharmaceutically acceptable salts are known in the art, and may be selected from those listed in Berge, et al. ["Pharmaceutical Salts," J. Pharm. Sci., 66(1): 1-19 (Jan. 1977)]. In one embodiment, a pharmaceutically acceptable salt of a compound of Formula (I) is an acid addition salt, for example a hydrochloride, sulfate, or phosphate salt. In another embodiment, a pharmaceutically acceptable salt of a compound of Formula (I) is a base addition salt, for example a sodium, potassium, calcium, or ammonium salt. In another embodiment, the base addition salt is a tetrafluoroboro salt. In one embodiment, a pharmaceutically acceptable salt of a compound of Formula (II) is an acid addition salt, for example a hydrochloride, sulfate, or phosphate salt. In another embodiment, a pharmaceutically acceptable salt of a compound of Formula (II) is a base addition salt, for example a sodium, potassium, calcium, or ammonium salt. In another embodiment, the base addition salt is a tetrafluoroboro salt. In one embodiment, a pharmaceutically acceptable salt of a compound of Formula (III) is an acid addition salt, for example a hydrochloride, sulfate, or phosphate salt. In another embodiment, a pharmaceutically acceptable salt of a compound of Formula (III) is a base addition salt, for example a sodium, potassium, calcium, or ammonium salt. In another embodiment, the base addition salt is a tetrafluoroboro salt. In one embodiment, a pharmaceutically acceptable salt of a compound of Formula (IV) is an acid addition salt, for example a hydrochloride, sulfate, or phosphate salt. In another embodiment, a pharmaceutically acceptable salt of a compound of Formula (IV) is a base addition salt, for example a sodium, potassium, calcium, or ammonium salt. In another embodiment, the base addition salt is a tetrafluoroboro salt. In one embodiment, a pharmaceutically acceptable salt of a compound of Formula (V) is an acid addition salt, for example a hydrochloride, sulfate, or phosphate salt. In another embodiment, a pharmaceutically acceptable salt of a compound of Formula (V) is a base addition salt, for example a sodium, potassium, calcium, or ammonium salt. In another embodiment, the base addition salt is a tetrafluoroboro salt.

The description also provides the use of a HAT activator of formula (I) in methods for reducing inclusion bodies (e.g., amyloid beta (Aβ) protein deposits, native and phosphorylated Tau proteins, native and phosphorylated alpha-synuclein, lipofuscin, cleaved TARDBP (TDB-43), or a combination thereof) in a subject afflicted with a neurodegenerative disease (e.g., a AD, Huntington's Disease, or Parkinson's Disease). The description also provides the use of a HAT activator of formula (I) in methods for treating a neurodegenerative disease in a subject. The descripion further provides the use of a HAT activator of formula (I) in methods for treating cancer in a subject. In some embodiments, the compound administered to a subject is any one of compounds 1-9. In specific embodiments, the HAT Activator compound is YF2, depicted in FIG. 3. The reference compounds of formula (II), (III), (IV) or formula (V) are also mentioned for the same uses. It is also mentioned that the compound administered is a HAT Activator reference compound of Formula (VI): 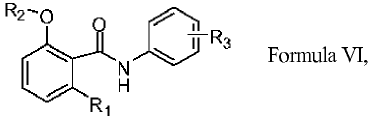 wherein:
R¹ is H, Methyl, Ethyl, n-Propyl, Isopropyl, n-butyl, t-butyl, C₈H₁₈, C₁₅H₂₆, C₁₅H₂₈, C₁₅H₃₀, C₁₅, H₃₂, S, O, SR⁴, or OR⁴;
R² is H, methyl, ethyl, n-propyl, isopropyl, n-butyl, t-butyl, or -C(O)R⁶;
R³ is H, methyl, ethyl, n-propyl, isopropyl, n-butyl, t-butyl, CF₃, CCl₃, Cl₃, F, Cl, I, NO₂, or CN;
R⁴ is -NR⁵;
R⁵ is hydrogen, C₁-C₆ alkyl, or C₃-C₈ cycloalkyl;
R⁶ is hydrogen, C₁-C₆ alkyl, or C₃-C₈ cycloalkyl, C(0)R⁷; and
R⁷ is hydrogen, C₁-C₆ alkyl, or C₃-C₈ cycloalkyl, or a pharmaceutically acceptable salt or hydrate thereof.

In one embodiment, a pharmaceutically acceptable salt of a compound of Formula (VI) is an acid addition salt, for example a hydrochloride, sulfate, or phosphate salt. In another embodiment, a pharmaceutically acceptable salt of a compound of Formula (VI) is a base addition salt, for example a sodium, potassium, calcium, or ammonium salt. In another embodiment, the base addition salt is a tetrafluoroboro salt.

In some embodiments, HAT activator compounds having Formula (I)are first screened for their ability to satisfy one or more of the following characteristics: an EC₅₀ no greater than about 100 nM; a histone acetylation activity *in vitro;* the ability to penetrate the BBB; or a combination thereof.

In one embodiment, the method comprises administering to the subject an effective amount of a composition comprising a HAT Activator compound. In another embodiment, the subject exhibits abnormally elevated amyloid beta plaques, or elevated Tau protein levels, or accumulations of alpha-synuclein, or accumulations of lipofuscin, or accumulation of cleaved TARDBP (TDB-43) levels, or a combination thereof. In some embodiments, the Aβ protein deposit comprises an Aβ40 isomer, an Aβ₄₂ isomer, or a combination thereof In a further embodiment, the subject is afflicted with Alzheimer's disease, Lewy body dementia, inclusion body myositis, Huntington's Disease, Parkinson's Disease, or cerebral amyloid angiopathy. In further embodiments, the subject is afflicted with cancer.

The dosage administered can be a therapeutically effective amount of the composition sufficient to result in amelioration of symptoms of a neurogenerative disease such as, but not limited to reducing inclusion bodies (e.g., amyloid beta (Aβ) protein deposits, native and phosphorylated Tau proteins, native and phosphorylated alpha-synuclein, lipofuscin, cleaved TARDBP (TDB-43), or a combination thereof), or reducing memory loss in a subject. For example, observing at least, about a 25% reduction, at least about a 30% reduction, at least about a 40% reduction, at least about a 50% reduction, at least about a 60% reduction, at least about a 70% reduction, at least about a 80% reduction, at least about a 85% reduction, at least about a 90% reduction, at least about a 95% reduction, at least about a 97% reduction, at least about a 98% reduction, or a 100% reduction in inclusion bodies or memory loss in a subject is indicative of amelioration of symptoms of a neurogenerative disease (for example, including, but not limited to, AD, Huntington's Disease, Parkinson's Disease). This efficacy in reducing inclusion occurrence, can be, for example, a measure of ameliorating symptoms of a neurogenerative disease.

In one embodiment, the therapeutically effective amount is at least about 0.1 mg/kg body weight, at least about 0.25 mg/kg body weight, at least about 0.5 mg/kg body weight, at least about 0.75 mg/kg body weight, at least about 1 mg/kg body weight, at least about 2 mg/kg body weight, at least about 3 mg/kg body weight, at least about 4 mg/kg body weight, at least about 5 mg/kg body weight, at least about 6 mg/kg body weight, at least about 7 mg/kg body weight, at least about 8 mg/kg body weight, at least about 9 mg/kg body weight, at least about 10 mg/kg body weight, at least about 15 mg/kg body weight, at least about 20 mg/kg body weight, at least about 25 mg/kg body weight, at least about 30 mg/kg body weight, at least about 40 mg/kg body weight, at least about 50 mg/kg body weight, at least about 75 mg/kg body weight, at least about 100 mg/kg body weight, at least about 200 mg/kg body weight, at least about 250 mg/kg body weight, at least about 300 mg/kg body weight, at least about 3500 mg/kg body weight, at least about 400 mg/kg body weight, at least about 450 mg/kg body weight, at least about 500 mg/kg body weight, at least about 550 mg/kg body weight, at least about 600 mg/kg body weight, at least about 650 mg/kg body weight, at least about 700 mg/kg body weight, at least about 750 mg/kg body weight, at least about 800 mg/kg body weight, at least about 900 mg/kg body weight, or at least about 1000 mg/kg body weight.

A HAT activator compound can be administered to the subject one time (e.g., as a single injection or deposition). Alternatively, a HAT activator compound of the invention can be administered once or twice daily to a subject in need thereof for a period of from about 2 to about 28 days, or from about 7 to about 10 days, or from about 7 to about 15 days. It can also be administered once or twice daily to a subject for a period of 1, 2, 3, 4, 5, 6, 7, 8, 9, 10, 11, 12 times per year, or a combination thereof.

The dosage administered can vary depending upon known factors such as the pharmacodynamic characteristics of the active ingredient and its mode and route of administration; time of administration of active ingredient; age, sex, health and weight of the recipient; nature and extent of symptoms; kind of concurrent treatment, frequency of treatment and the effect desired; and rate of excretion.

Toxicity and therapeutic efficacy of therapeutic compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic agents that exhibit large therapeutic indices are useful. Therapeutic compositions that exhibit some toxic side effects can be used.

A therapeutically effective dose of a HAT activator compound can depend upon a number of factors known to those of ordinary skill in the art. The dose(s) of a HAT activator compound, for example a compound of Formula (I), Formula (II), Formula (III), Formula (IV), Formula (V), or Formula (VI), can vary, for example, depending upon the identity, size, and condition of the subject or sample being treated, further depending upon the route by which the composition is to be administered, if applicable, and the effect which the practitioner desires the HAT activator compound to have upon a HAT protein or a protein exhibiting intrinsic HAT activity. These amounts can be readily determined by a skilled artisan.

HAT activator compounds of the invention can be incorporated into pharmaceutical compositions suitable for administration. Such compositions can comprise a HAT activator compound (e.g., a compound of HAT activator compounds having Formula (I), or compound YF2 depicted in FIG. 3) and a pharmaceutically acceptable carrier. The compositions can be administered alone or in combination with at least one other agent, such as a stabilizing compound, which can be administered in any sterile, biocompatible pharmaceutical carrier including, but not limited to, saline, buffered saline, dextrose, and water. The compositions
can be administered to a patient alone, or in combination with other agents, drugs or hormones.

According to the invention, a pharmaceutically acceptable carrier can comprise any and all solvents, dispersion media, coatings, antibacterial and antifungal agents, isotonic and absorption delaying agents, and the like, compatible with pharmaceutical administration. The use of such media and agents for pharmaceutically active substances is well known in the art. Any conventional media or agent that is compatible with the active compound can be used. Supplementary active compounds can also be incorporated into the compositions.

Any of the therapeutic applications described herein can be applied to any subject in need of such therapy, including, for example, a mammal such as a dog, a cat, a cow, a horse, a rabbit, a monkey, a pig, a sheep, a goat, or a human.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, and thimerosal. In many cases, it can be useful to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the HAT Activator compound in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

### REFERENCES

S1. Masliah, E., Mechanisms of synaptic dysfunction in Alzheimer's disease. Histol Histopathol, 1995. 10(2): p. 509-19.
S2. Selkoe, D.J., Alzheimer's disease is a synaptic failure. Science, 2002. 298(5594): p. 789-91.
S3. Sant'Angelo, A., F. Trinchese, and O. Arancio, Usefulness of behavioral and electrophysiological studies in transgenic models of Alzheimer's disease. Neurochem Res, 2003. 28(7): p. 1009-15.
S4. Bliss, T.V. and G.L. Collingridge, A synaptic model of memory: long-term potentiation in the hippocampus. Nature, 1993. 361(6407): p. 31-9.
S5. Cullen, W.K., et al., Block of LTP in rat hippocampus in vivo by beta-amyloid precursor protein fragments. Neuroreport, 1997. 8(15): p. 3213-7.
S6. Freir, D.B., C. Holscher, and C.E. Herron, Blockade of long-term potentiation by beta-amyloid peptides in the CA1 region of the rat hippocampus in vivo. J Neurophysiol, 2001. 85(2): p. 708-13.
S7. Itoh, A., et al., Impairments of long-term potentiation in hippocampal slices of beta-amyloid-infused rats. Eur J Pharmacol, 1999. 382(3): p. 167-75.
S8. Kim, J.H., et al., Use-dependent effects of amyloidogenic fragments of (beta)-amyloid precursor protein on synaptic plasticity in rat hippocampus in vivo. J Neurosci, 2001. 21(4): p. 1327-33.
S9. Stephan, A., S. Laroche, and S. Davis, Generation of aggregated beta-amyloid in the rat hippocampus impairs synaptic transmission and plasticity and causes memory deficits. J Neurosci, 2001. 21(15): p. 5703-14.
S10. Vitolo, O.V., et al., Amyloid beta -peptide inhibition of the PKA/CREB pathway and long-term potentiation: reversibility by drugs that enhance cAMP signaling. Proc Natl Acad Sci U S A, 2002. 99(20): p. 13217-21.
S11. Walsh, D.M., et al., Naturally secreted oligomers of amyloid beta protein potently inhibit hippocampal long-term potentiation in vivo. Nature, 2002. 416(6880): p. 535-9.
S12. Puzzo, D., et al., Amyloid-beta peptide inhibits activation of the nitric oxide/cGMP/cAMP-responsive element-binding protein pathway during hippocampal synaptic plasticity. J Neurosci, 2005. 25(29): p. 6887-97.
S13. Selig, D.K., et al., Examination of the role of cGMP in long-term potentiation in the CA1 region of the hippocampus. Learn Mem, 1996. 3(1): p. 42-8.
S14. Prickaerts, J., et al., cGMP, but not cAMP, in rat hippocampus is involved in early stages of object memory consolidation. Eur J Pharmacol, 2002. 436(1-2): p. 83-7.
S15. Paakkari, I. and P. Lindsberg, Nitric oxide in the central nervous system. Ann Med, 1995. 27(3): p. 369-77.
S16. Baratti, C.M. and M.M. Boccia, Effects of sildenafil on long-term retention of an inhibitory avoidance response in mice. Behav Pharmacol, 1999. 10(8): p. 731-7.
S17. van Staveren, W.C., et al., Species differences in the localization of cGMP-producing and NO-responsive elements in the mouse and rat hippocampus using cGMP immunocytochemistry. Eur J Neurosci, 2004. 19(8): p. 2155-68.
S18. Van Staveren, W.C., et al., mRNA expression patterns of the cGMP-hydrolyzing phosphodiesterases types 2, 5, and 9 during development of the rat brain. J Comp Neurol, 2003. 467(4): p. 566-80.
S19. Schultheiss, D., et al., Central effects of sildenafil (Viagra) on auditory selective attention and verbal recognition memory in humans: a study with event-related brain potentials. World J Urol, 2001. 19(1): p. 46-50.
S20. Kemenes, I., et al., Critical time-window for NO-cGMP-dependent long-term memory formation after one-trial appetitive conditioning. J Neurosci, 2002. 22(4): p. 1414-25.
S21. Baltrons, M.A., et al., Regulation of NO-dependent cyclic GMP formation by inflammatory agents in neural cells. Toxicol Lett, 2003. 139(2-3): p. 191-8.
S22. Bon, C.L. and J. Garthwaite, On the role of nitric oxide in hippocampal long-term potentiation. J Neurosci, 2003. 23(5): p. 1941-8.
S23. Trinchese, F., et al., Progressive age-related development of Alzheimer-like pathology in APP/PS1 mice. Ann Neurol, 2004. 55(6): p. 801-14.
S24. Chapman, P.F., et al., Impaired synaptic plasticity and learning in aged amyloid precursor protein transgenic mice. Nat Neurosci, 1999. 2(3): p. 271-6.
S25. Fitzjohn, S.M., et al., Age-related impairment of synaptic transmission but normal long-term potentiation in transgenic mice that overexpress the human APP695SWE mutant form of amyloid precursor protein. J Neurosci, 2001. 21(13): p. 4691-8.
S26. Hsia, A.Y., et al., Plaque-independent disruption of neural circuits in Alzheimer's disease mouse models. Proc Natl Acad Sci U S A, 1999. 96(6): p. 3228-33.
S27. Jolas, T., et al., Long-term potentiation is increased in the CA1 area of the hippocampus of APP(swe/ind) CRND8 mice. Neurobiol Dis, 2002. 11(3): p. 394-409.
S28. Larson, J., et al., Alterations in synaptic transmission and long-term potentiation in hippocampal slices from young and aged PDAPP mice. Brain Res, 1999. 840(1-2): p. 23-35.
S29. Moechars, D., et al., Early phenotypic changes in transgenic mice that overexpress different mutants of amyloid precursor protein in brain. J Biol Chem, 1999. 274(10): p. 6483-92.
S30. Nalbantoglu, J., et al., Impaired learning and LTP in mice expressing the carboxy terminus of the Alzheimer amyloid precursor protein. Nature, 1997. 387(6632): p. 500-5.
S31. Dineley, K.T., et al., Beta-amyloid activates the mitogen-activated protein kinase cascade via hippocampal alpha7 nicotinic acetylcholine receptors: In vitro and in vivo mechanisms related to Alzheimer's disease. J Neurosci, 2001. 21(12): p. 4125-33.
S32. Dineley, K.T., et al., Accelerated plaque accumulation, associative learning deficits, and up-regulation of alpha 7 nicotinic receptor protein in transgenic mice co-expressing mutant human presenilin 1 and amyloid precursor proteins. J Biol Chem, 2002. 277(25): p. 22768-80.
S33. Gong, B., et al., Persistent improvement in synaptic and cognitive functions in an Alzheimer mouse model following rolipram treatment. J. Clin. Invest., 2004. 114: p. 1624-1634.
S34. Yin, J.C., et al., Induction of a dominant negative CREB transgene specifically blocks long-term memory in Drosophila. Cell, 1994. 79(1): p. 49-58.
S35. Bourtchuladze, R., et al., Deficient long-term memory in mice with a targeted mutation of the cAMP-responsive element-binding protein. Cell, 1994. 79(1): p. 59-68.
S36. Bach, M.E., et al., Age-related defects in spatial memory are correlated with defects in the late phase of hippocampal long-term potentiation in vitro and are attenuated by drugs that enhance the cAMP signaling pathway. Proc Natl Acad Sci U S A, 1999. 96(9): p. 5280-5.
S37. Lu, Y.F., E.R. Kandel, and R.D. Hawkins, Nitric oxide signaling contributes to late-phase LTP and CREB phosphorylation in the hippocampus. J Neurosci, 1999. 19(23): p. 10250-61.
S38. McCarty, M.F., Vascular nitric oxide may lessen Alzheimer's risk. Med Hypotheses, 1998. 51(6): p. 465-76.
S39. Troy, C.M., et al., Caspase-2 mediates neuronal cell death induced by beta-amyloid. J Neurosci, 2000. 20(4): p. 1386-92.
S40. Wirtz-Brugger, F. and A. Giovanni, Guanosine 3',5'-cyclic monophosphate mediated inhibition of cell death induced by nerve growth factor withdrawal and beta-amyloid: protective effects of propentofylline. Neuroscience, 2000. 99(4): p. 737-50.
S41. Venturini, G., et al., Beta-amyloid inhibits NOS activity by subtracting NADPH availability. Faseb J, 2002. 16(14): p. 1970-2.
S42. Suhara, T., et al., Abeta42 generation is toxic to endothelial cells and inhibits eNOS function through an Akt/GSK-3beta signaling-dependent mechanism. Neurobiol Aging, 2003. 24(3): p. 437-51.
S43. Colton, C.A., et al., NO synthase 2 (NOS2) deletion promotes multiple pathologies in a mouse model of Alzheimer's disease. Proc Natl Acad Sci U S A, 2006. 103(34): p. 12867-72.
S44. Thatcher, G.R., B.M. Bennett, and J.N. Reynolds, Nitric oxide mimetic molecules as therapeutic agents in Alzheimer's disease. Curr Alzheimer Res, 2005. 2(2): p. 171-82.
S45. Haas, J., et al., Inducible nitric oxide synthase and argininosuccinate synthetase: co-induction in brain tissue of patients with Alzheimer's dementia and following stimulation with beta-amyloid 1-42 in vitro. Neurosci Lett, 2002. 322(2): p. 121-5.
S46. Tran, M.H., et al., Amyloid beta-peptide induces nitric oxide production in rat hippocampus: association with cholinergic dysfunction and amelioration by inducible nitric oxide synthase inhibitors. Faseb J, 2001. 15(8): p. 1407-9.
S47. McCann, S.M., The nitric oxide hypothesis of brain aging. Exp Gerontol, 1997. 32(4-5): p. 431-40.
S48. Xie, Z., et al., Peroxynitrite mediates neurotoxicity of amyloid beta-peptide1-42- and lipopolysaccharide-activated microglia. J Neurosci, 2002. 22(9): p. 3484-92.
S49. Wong, A., et al., Advanced glycation end products co-localize with inducible nitric oxide synthase in Alzheimer's disease. Brain Res, 2001. 920(1-2): p. 32-40.
S50. Wang, Q., M.J. Rowan, and R. Anwyl, Beta-amyloid-mediated inhibition of NMDA receptor-dependent long-term potentiation induction involves activation of microglia and stimulation of inducible nitric oxide synthase and superoxide. J Neurosci, 2004. 24(27): p. 6049-56.
S51. Monsonego, A., et al., Microglia-mediated nitric oxide cytotoxicity of T cells following amyloid beta-peptide presentation to Th1 cells. J Immunol, 2003. 171(5): p. 2216-24.
S52. Contestabile, A., et al., Brain nitric oxide and its dual role in neurodegeneration/neuroprotection: understanding molecular mechanisms to devise drug approaches. Curr Med Chem, 2003. 10(20): p. 2147-74.

### EXAMPLES

Examples are provided below to facilitate a more complete understanding of the invention. The following examples illustrate the exemplary modes of making and practicing the invention. However, the scope of the invention is not limited to specific embodiments disclosed in these Examples, which are for purposes of illustration only, since alternative methods can be utilized to obtain similar results.

### Example 1 - A HAT Activator compound.

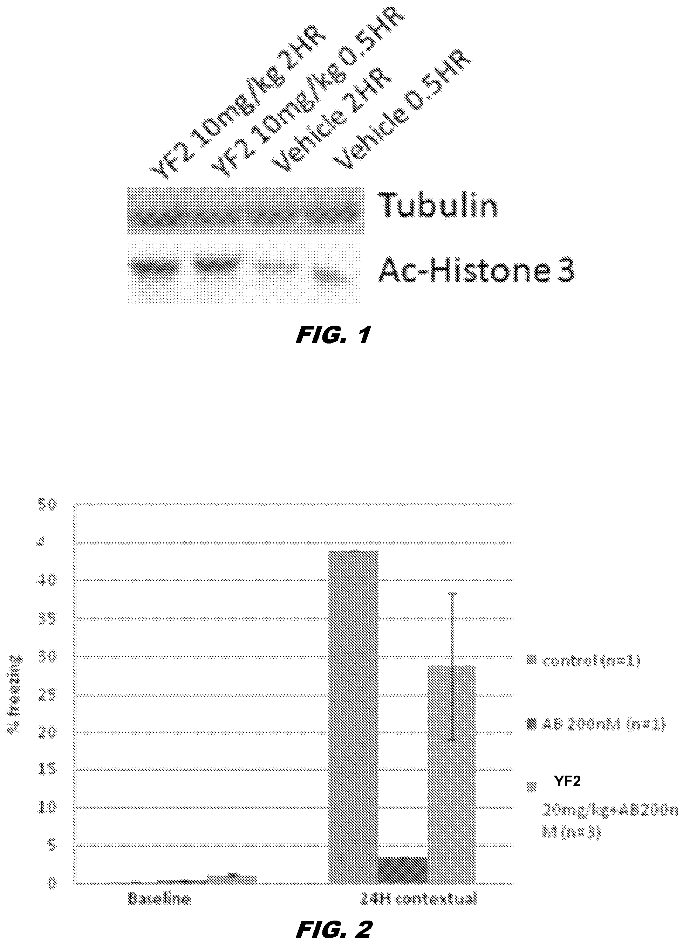

YF2, a Histone Acetyltransferase (HAT) Activator of the invention (**FIG. 3**), is a good drug candidate to ameliorate memory in neurodegenerative diseases (i.e. Alzheimer's disease and Huntington's disease) and treatment for a variety of cancers. When the YF2 compound was administrated to mice (i.p.), the western blot showed that it not only crosses the BBB, but also increases histone 3 acetylation levels of the hippocampus (**FIG. 1**). In the behavior experiments, the title compound ameliorates the contextual fear memory deficit in Aβ42 - infused mice (**FIG. 2**). Aβ42 is a protein that is produced in high amount in AD and is responsible for the impairment of synaptic functions and memory.

### Example 2 - HAT Activator Compound Characteristics

Compound 6J, from Mantelingu *et al,* was synthesized (**FIG. 4**). However, compound 6J had no solubility and precipitated as soon as it was put in H₂O. However, concerns existed that the drug (even if dissolved in 100% DMSO) would precipitate as soon as it was administrated. Thus, a new drug that was soluble was synthesized, "MOM" (**FIG. 5**).

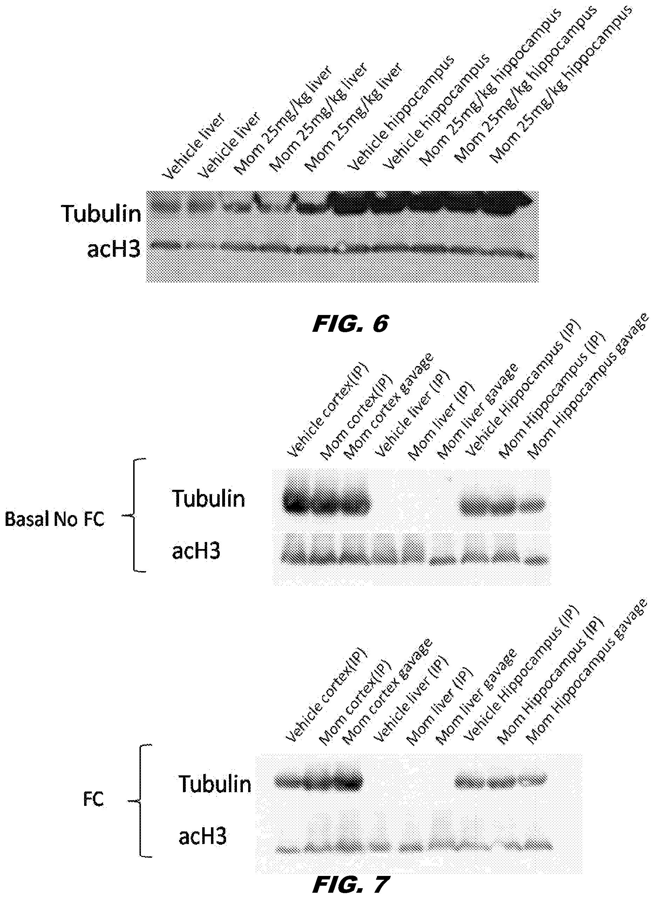

MOM has medium solubility (DMSO 10% in H₂O). MOM was administered 25mg/kg to WT mice (i.p.). The mice liver and hippocampus were extracted 1hr after treatment. The liver showed a very slight increase of AcH3, indicating that the drug has either very little efficacy OR very little membrane permeability (**FIG. 6**). The hippocampus had no increase in AcH3 levels, indicating the drug is either ineffective OR does not cross the blood brain barrier (BBB) (**FIG. 6**). Although MOM failed to increase AcH3 levels in the hippocampus and liver, the experiment was repeated with a new administration (gavage and i.p. 25mg/kg). Fear conditioning treatment of the mice was subsequently carried out to see if the drug is active after induction of learning. In addition to the mouse liver and hippocampus, the mouse cortex was also extracted. Hippocampus, cortex, and liver samples again showed no increase of AcH3 levels, indicating the drug is either ineffective OR does not cross the BBB OR does not cross the cell membrane (**FIG. 7**).

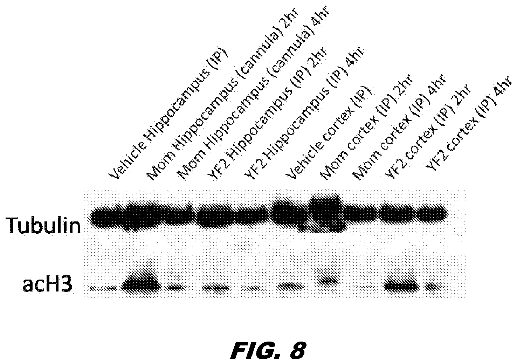

Although MOM failed in increasing AcH3 levels, the experiment was repeated with a different chemical vehicle (10% DMSO and 10% Tween 80). The BBB was bypassed by giving it through cannulas implanted onto the dorsal hippocampus. Both the hippocampus and cortex were extracted at 2hrs or 4hrs after treatment. Compared to vehicle, mice that were administered with MOM via cannula (100 µg/µl per side) showed an increase of AcH4 (lane 1 vs. lanes 2, 3). Mice that were given the drug i.p. showed no increase of AcH3 (**FIG. 8**). This data indicates that the compound MOM does not cross the BBB.

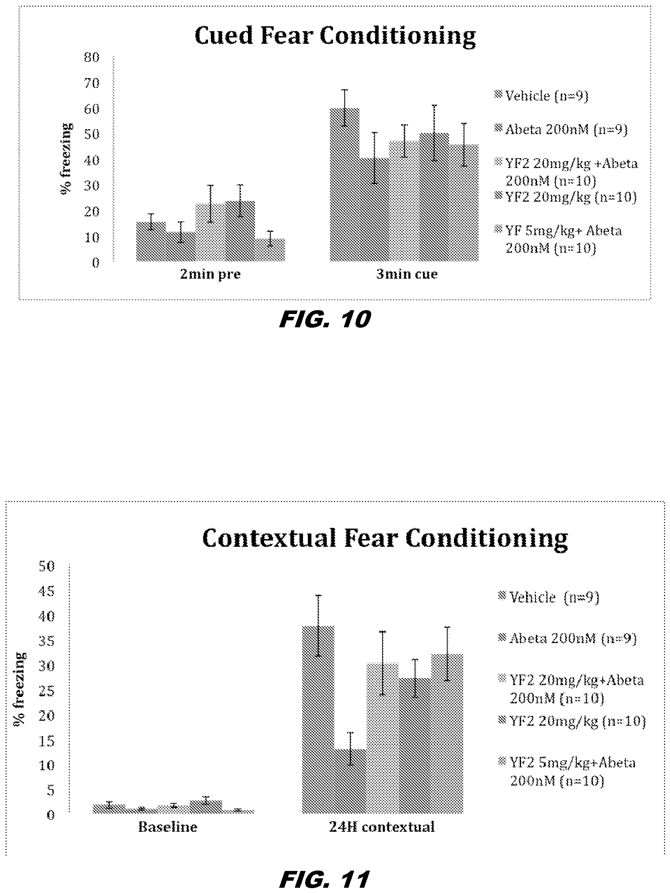

A new compound YF2 (**FIG. 3**), was synthesized. The preparation of YF2 was without a column and 2 phases were visible: clear and oily. YF2 (50 mg/kg, i.p.) was subsequently administered to mice. Two and four hrs after its administration, the mice were sacrificed and hippocampi were extracted. Interestingly, YF2 was able to cross the BBB, penetrate the cells and increase AcH3 (lane 1 vs. lanes 9, 10) (**FIG. 8**). Given that the compound was not 100% clean and needed to be further purified/verified, we synthesized more YF2 and purified it. Purity was verified through Nuclear Magnetic Resonance (NMR). Mice were administered with YF2 (i.p. dissolved in saline) at 5, 10, 20 mg/Kg. Hippocampus extraction was made at 3 different time points (0.5, 1 and 2 hrs post treatment). We then ran a western blotting for AcH3. Except for the 1hr-10mg/kg administration of YF2, YF2 dramatically increased AcH3 levels (**FIG. 10**), indicating that YF2 crosses the BBB and the cell membrane.

### Example 3 - Contextual and Cued Fear conditioning experiments

Contextual and cued fear conditioning was performed to assess whether the compound is capable of ameliorating amyloid-beta (Aβ) induced memory defect. Aβ is a peptide which is elevated in Alzheimer's disease. The hippocampus plays a key role in contextual memory and in Alzheimer's Disease. This type of cognitive test is much faster than other behavioral tasks that require multiple days of training and testing [Q1, Q2]. Our conditioning chamber was in a sound-attenuating box. A clear Plexiglas window allowed the experimenter to film the mouse performance with a camera placed on a tripod and connected to the Freezeframe software (MED Ass. Inc.). To provide background white noise (72 dB), a single computer fan was installed in one of the sides of the sound-attenuating chamber. The conditioning chamber had a 36-bar insulated shock grid floor. The floor was removable, and after each experimental subject, we cleaned it with 75% ethanol and then with water. Only one animal at a time was present in the experimentation room.

For the cued and contextual conditioning experiments, mice were placed in the conditioning chamber for 2 min before the onset of a discrete tone (CS) (a sound that lasted 30 sec at 2800 Hz and 85 dB). In the last 2 sec of the CS, mice were given a foot shock (US) of 0.8 mA for 2 sec through the bars of the floor. After the CS/US pairing, the mice were left in the conditioning chamber for another 30 sec and were then placed back in their home cages. Freezing behavior, defined as the absence of all movement except for that necessitated by breathing, was scored using the Freezeview software.

To evaluate contextual fear learning, freezing was measured for 5 min (consecutively) in the chamber in which the mice was trained 24 hr after training. To evaluate cued fear learning, following contextual testing, the mice were placed in a novel context (triangular cage with smooth flat floor) for 2 min (pre-CS test), after which they were exposed to the CS for 3 min (CS test), and freezing was measured. Sensory perception of the shock was determined through threshold assessment. A sequence of single foot shocks was delivered to animals placed on the same electrified grid used for fear conditioning. Initially, a 0.1 mV shock was delivered for 1 sec, and the animal behavior was evaluated for flinching, jumping, and vocalization. At 30 sec intervals the shock intensity was increased by 0.1 mV to 0.7 mV and then returned to 0 mV in 0.1 mV increments at 30 sec intervals. Threshold to vocalization, flinching, and then jumping was quantified for each animal by averaging the shock intensity at which each animal manifests a behavioral response to the foot shock.

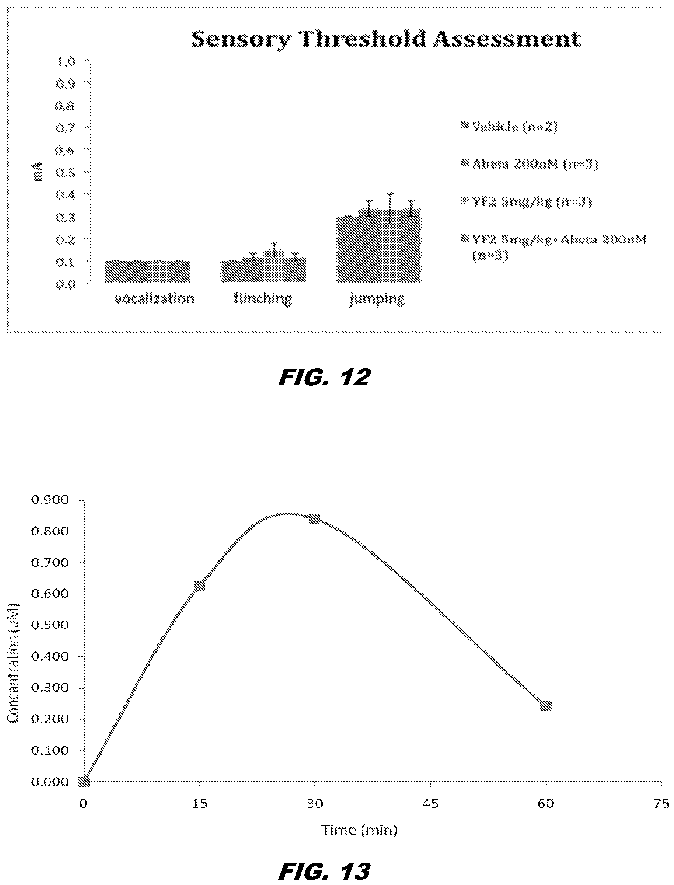

YF2 was i.p. administered to mice (one group of mice was administered with 20 mg/kg, 2hrs before the electric shock, whereas another group was administered with 5 mg/kg, 30 minutes before the electric shock). YF2 at both doses was capable of dramatically increasing the freezing time demonstrating that the compound rescues the defect in contextual memory. The compound alone at the highest concentration (20 mg/kg) did not affect contextual memory (**FIG. 10**) indicating that the compound *per se* is not toxic with respect to memory. Cued memory was not changed in the different groups indicating that YF2 does not affect amygdala function (**FIG. 11**). Finally, no difference was observed among different groups of mice in different sets of experiments in which we assessed sensory threshold in the presence of vehicle, YF2 alone, Aβ alone, or YF2 plus Aβ (**FIG. 12**).

Based on the results obtained during the fear conditioning tests, we decided to determine the kinetics of YF2 in blood to verify the best time point for treatment. To this purpose, we administered the compound (20 mg/kg. i.p.) and then sampled blood from tails at different time points. The kinetics of YF2 shows a peak around 30 minutes post-injection (**FIG. 13**).

### REFERENCES

Q1. Gong, B., et al., Persistent improvement in synaptic and cognitive functions in an Alzheimer mouse model after rolipram treatment. J Clin Invest, 2004. 114(11): p. 1624-34.
Q2. Gong, B., et al., Persistent improvement in synaptic and cognitive functions in an Alzheimer mouse model following rolipram treatment. J. Clin. Invest., 2004. 114: p. 1624-1634.

### Example 4 - Two Day-Radial Arm Water Maze (RAWM)

The task is a hybrid of the Morris Water Maze (MWM) and the radial arm land maze. This task is altered in Aβ-infused mice. The motivation for the animals is the immersion in water. The mouse needed to swim in 6 alleys (arms) radiating from a central area until it found a hidden (submerged) platform at the end of one of the arms, based on visual cues placed in the room. The goal arm was kept constant for all trials, with a different start arm on successive trials, such that the learning criterion was reached in 2 days. The first day of the protocol was a training day. Mice were trained to identify the platform location by alternating between a visible and a hidden platform in a goal arm. The final 3 trials on day 1 and all 15 trials on day 2 used a hidden escape platform to force mice to use spatial cues to identify the location of the goal arm.

To avoid learning limitations imposed by exhausting practice and to avoid fatigue that may result from consecutive trials, spaced practice training were established by running the mice in cohorts of 4 and alternating different cohorts through the 15 training trials over 3 hours testing periods each day. On day 1, a visible platform was placed in a goal location. Mouse 1 of cohort 1 was gently placed in the pool near the perimeter of the wall of the first start arm (specified on a score sheet) and facing the center of the pool. The number of incorrect arm entries (entries in arms with no platform) was counted. If the animal entered the incorrect arm it was gently pulled back to the start arm. Each trial lasted up to 1 min. Failure to select an arm after 15 sec were counted as an error and the mouse was returned to the start arm. After 1 min, if the platform had not been located, the mouse was guided gently through the water by placing a hand behind it to direct it towards the platform. The mouse rested on the platform for 15 sec. After completing the trial, the mouse was removed from the pool, gently towel dried and placed back into its cage under a heat lamp. The goal platform location was different for each mouse.

After all the mice in the first cohort have had a trial to locate a visible platform, the platform was switched from visible to hidden. After each mouse from cohort 1 completed six alternating trials between visible and hidden platforms, the mice was left to rest under a heating source, and mice from the second cohort were tested in the same way. After completing the six alternating trials, mice from cohort 2 returned to their cages to rest.

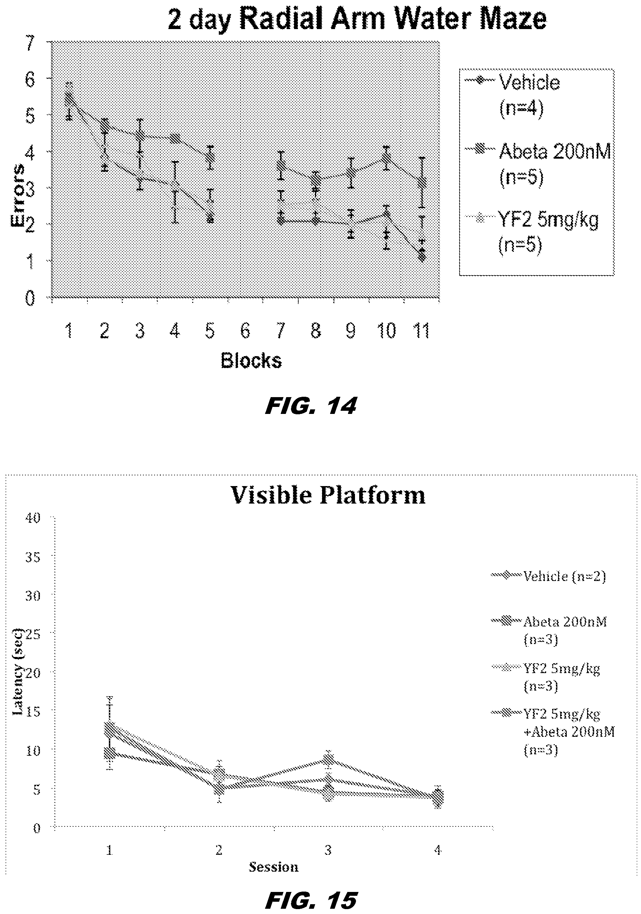

Next, mice from the first cohort completed trials 7-12 again using the alternating visible-hidden platform location. During resting time for mice from the first cohort, mice from the second cohort completed trials 7-12. At this point, all mice had performed 3 hidden platform trials. On day 2, the same procedure was repeated as on day 1 for all 15 trials using only the hidden platform. For data analysis, averages for each mouse were calculated using blocks of 3 trials. As shown in **FIG. 14****,** vehicle-treated mice exhibit ∼1 error over three trials near the end of the second day.

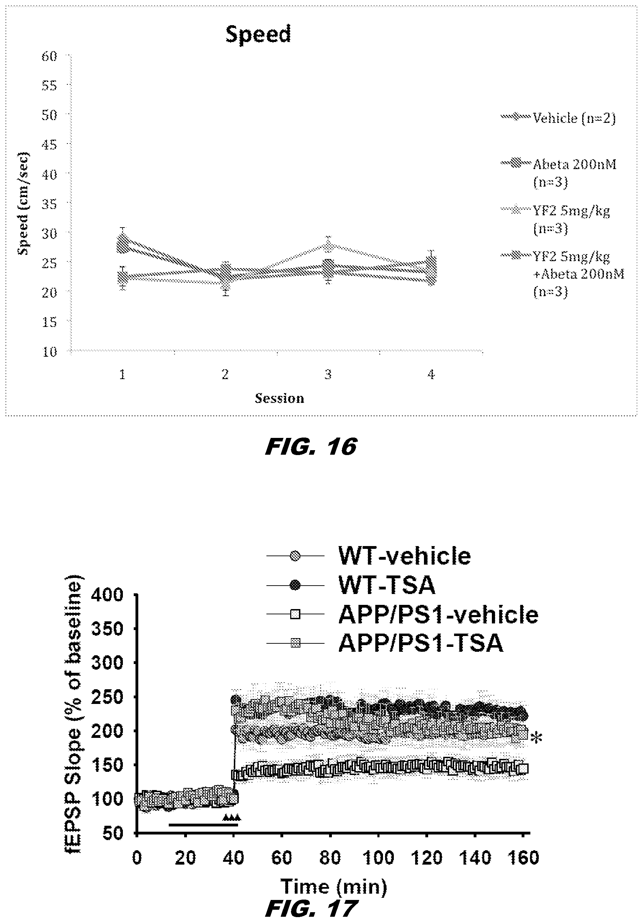

In contrast, Aβ-infused mice [bilateral injections of Aβ₄₂; 200 nM in a final volume of 1 µl over 1 min; twice on each testing day: 15 min prior to the 1^{st} trial (for the 1^{st} group of tests) and 15 min prior to the 7^{th} trial (for the 2^{nd} group of tests) into dorsal hippocampi], failed to learn, making 3-4 errors throughout the training session, with no improvement over trials. Treatment with YF2 [(i.p., 5 mg/kg, 30 min prior to the 1^{st} trial (for the 1^{st} group of tests) and 30 min prior to the 7^{th} trial (for the 2^{nd} group of tests)] rescued the Aβ-induced memory impairment. In addition, YF2 by itself did not affect the performance of the animals with this kind of test. Controls for sensory, motor and motivational skills of the mice that might have influenced the outcome of the test with the 2-day RAWM did not show any difference in the time needed to reach the visible platform and in the swimming speed among the four different groups of mice **(****FIG. 15** and **FIG. 16**).

### Example 5 - Dysregulation of histone acetylation in Alzheimer Disease

We have recently shown that the inhibition of histone de-acetylation through trichostatin A (TSA) ameliorates LTP and contextual fear conditioning (FC) in an amyloid-depositing animal model, the transgenic (Tg) mouse expressing the Swedish mutation in APP(K670M:N671L) together with the mutation in PS1(M146L, line 6.2), termed APP/PS1 mouse^{[A21, A22]}. In addition, CBP HAT activity was essential for enhancing transcription *in vitro* following PS1 stimulation and CBP levels in APP/PS1 mice were significantly lower than in WT mice. Hippocampi from APP/PS1 mice exhibited, after FC training, approximately a 50% reduction in acetylated histone 4 levels, an acetylation that was shown to be important in memory formation^{[A23]}. Based on these findings and without being bound by theory, epigenetic changes, including histone acetylation, play an important role in the Aβ-induced damage of synaptic function and memory associated with AD.

In the brain, CREB phosphorylation is required for CREB ability to bind to CBP and to stimulate memory associated gene expression. CBP functions as a co-activator that facilitates interactions with the basal transcription machinery by working as an acetyltransferase (HAT) that catalyzes acetylation of the histones, causing a loss in chromosomal repression and increase in the transcription of memory associated genes. Unlike HATs, HDACs were found to remove an acetyl group from histones, thus restricting access of the transcriptional machinery to the DNA. Interestingly, HDAC inhibitors have been shown to enhance LTP and contextual fear memory, a form of associative memory in which animals must associate a neutral stimulus with an aversive one[A25].

Also, memory and LTP deficits of CBP+/- mice were reversed by the HDAC inhibitor, SAHA, supporting the potential use of this class of drugs as therapeutic agents against mental retardation in Rubinstein-Taybi syndrome (RTS)[A24]. Nicotinamide, an HDAC III inhibitor, was found to restore cognition in the triple Tg mouse model of AD via a mechanism involving reduction of Thr231-phosphotau [A26]. Moreover, HDAC inhibitors induced sprouting of dendrites, an increased number of synapses, and reinstated learning and access to long-term memories in the CK-p25 Tg mouse model of neuronal loss [A23]. HDAC inhibitors could affect neuronal function through a variety of mechanisms including epigenetic and non-epigenetic changes [A27]. Whether cognitive deficits following Aβ elevation may be induced by epigenetic modification on histone acetylation (via chromatin remodeling) has not been determined.

WT-PS1 stimulates the transcriptional activity ability of CBP whereas its AD M146L mutant did not produce such an effect [A20] indicating that CBP and its HAT activity in AD may be involved. In addition, a CBP mutant lacking HAT activity is not capable of responding to WT-PS1 in terms of increased transcription activating ability. Hence, CBP and its HAT region appear to be essential for enhancing transcription *in vitro* following PS1 stimulation. The inventors find that histone acetylation level of APP/PS1 mice is different than in WT mice, thus identifying AD as a disease of epigenetic etiology.

**Significance.** Although AD was described almost a century ago, the molecular mechanisms that lead to the development of the neuronal pathology are still unknown. Without being bound by theory, epigenetics and histone acetylation might play a fundamental role in AD. Moreover, inhibition of HDAC or conversely, activation of a HAT, can effectively counteract the disease progression.

### To determine if Aβ-induced synaptic and memory dysfunctions are ameliorated by inhibition of histone de-acetylation.

Based on data showing that TSA rescues the reduction of LTP and contextual learning in APP/PS1 mice, we will determine if the inhibitor also rescues these deficits after Aβ exposure in order to separate Aβ effects from other effects of APP and PS1 overexpression.

AD is thought to begin as a synaptic disorder that progressively leads to greater neuronal dysfunction, leading to memory loss [A28]. To obtain evidence in favor of the involvement of epigenetics in Aβ-induced synaptic and memory dysfunction, we performed a series of experiments using the HDAC inhibitor TSA on 3-4 month-old APP/PS1 mice (for a detailed description of the experiments showing the characterization of these mice see our manuscripts [A17, A29].

At this age the Tg mice just start showing the synaptic plasticity and memory impairments [A29]. Basal synaptic transmission (BST) was similar between APP/PS1 and WT mice. We found no difference in BST among the 2 groups as previously shown in mice of this age [A29]. Hippocampal slices were then perfused with TSA (1.65µM) for 30min before inducing LTP through tetanic stimulation of the Schaeffer collateral pathway. Potentiation in TSA treated APP/PS1 slices was far greater than in vehicle-treated APP/PS1 slices (**FIG. 17**). But, TSA did not change the amplitude of LTP in hippocampal slices of WT mice compared to WT slices treated with vehicle alone. Moreover, TSA perfusion did not affect baseline transmission in APP/PS1 and WT slices that received no tetanus.

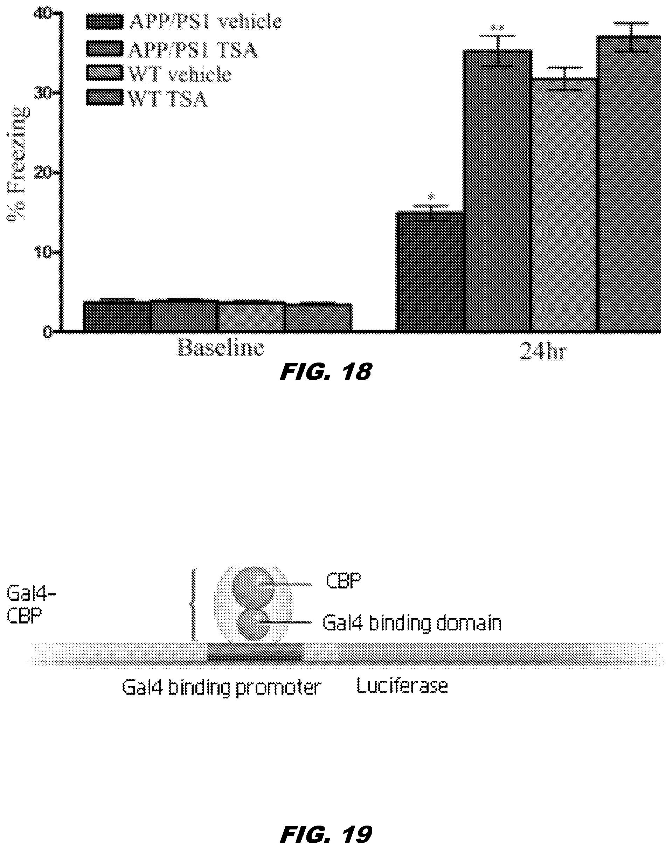

Following experiments on acute effect of TSA in synaptic dysfunction, we determined if TSA is beneficial against impairment of contextual FC in APP/PS1 mice_{[A17]}. 4 month-old mice were divided into 4 groups: APP/PS1 with TSA, APP/PS1 with vehicle, WT with TSA and WT with vehicle. TSA and vehicle control solution were administered i.p. at a concentration of 2 µg/g body weight. We found a similar shock threshold among the various groups of mice. Then, mice were trained to associate neutral stimuli with an aversive one. They were placed in a novel context (FC box), exposed to a white noise cue paired with a mild foot shock, and injected with TSA 2hrs before training. Fear learning was assessed 24 hrs later by measuring freezing behavior - the absence of all movement except for that necessitated by breathing - in response to representation of the context. We found no difference in the freezing behavior among the 4 groups of mice during the training phase of the FC. 24hrs later, freezing behavior was decreased in vehicle-treated APP/PS1 mice compared to vehicle-treated WT littermates in analyzing contextual learning (**FIG. 18**). TSA improved memory in Tg mice.

In future experiments, we will confirm electrophysiological and behavioral findings described in **FIG. 17** and **FIG. 18** using a structurally dissimilar inhibitor of histone-deacetylases, sodium butyrate (NaB). As for TSA experiments, slices from 3-4 month old APP/PS1 mice will be treated with NaB (300 µM) for 30 min prior to applying a theta-burst stimulation to induce LTP. Controls will be performed on slices from APP/PS1 mice treated with vehicle, and WT littermate mice treated with NaB or vehicle. Similarly to TSA, we will also check if NaB (1.2 g/Kg) re-establishes normal contextual fear memory in APP/PS1 mice.

The APP and PS1 transgenes could affect neuronal function through different mechanisms [A30, A31], including direct effects by Aβ. The trafficking and signaling properties of full-length APP and its cleavage products are likely different, which could impact aspects of synaptic function differently. To separate Aβ effects from other effects of APP and PS1 overexpression, we will determine whether Aβ *per se* is responsible for the deficits observed in our studies on Tg mice. Since it has already been described that natural oligomers of human Aβ, in the absence of monomers and fibrils, markedly inhibit LTP *in vivo* [A6], we will apply 200 nM oligomeric Aβ₄₂ concurrently with TSA (1.65µM) for 30 minutes to WT slices prior to inducing LTP. In addition, we will impair contextual fear memory through bilateral infusion of 200 nM oligomeric Aβ₄₂ onto dorsal hippocampi 15 min prior to training for FC, concurrently with TSA (2 µgr/gr body weight, 2 hrs prior to training, i.p.). Without being bound by theory, oligomeric Aβ₄₂ should inhibit LTP and fear memory, and demonstrate that TSA reestablishes normal LTP and contextual fear memory following Aβ₄₂ treatment. TSA alone should not have any effect. In additional experiments we will use NaB (300 µM) plus 200 nM Aβ₄₂ for 30 min prior to applying a theta-burst stimulation to induce LTP. Controls will be performed on slices treated with vehicle. Similarly to TSA, we will also check if NaB (1.2 g/Kg) re-establishes normal contextual fear memory in Aβ₄₂- infused mice. Collectively, these experiments will demonstrate that inhibition of histone deacetylase is beneficial against synaptic and memory dysfunction caused by elevation of oligomeric Aβ.

### To characterize CBP involvement following Aβ elevation.

Based on the observations that i) WT-PS1 no longer promotes transcription on F11 cells transfected with a CBP construct lacking HAT activity, and that ii) CBP levels are reduced in APP/PS1 mice, we will determine if CBP HAT activity and levels are affected in APP/PS1 mice and after Aβ elevation.

We have recently examined if the HAT activity of CBP has any role in the stimulus effect of WT-PS1 by using a construct in which full-length CBP contains a mutation (WY - amino acids 1503-1504 replaced by alanine and serine). This mutation, which completely abolishes its HAT activity [A32], was linked to the DNA binding domain of Gal4 (**FIG. 19**). In contrast to the enhanced promoter activity of full-length WT-CBP, no activity was observed with WY-CBP (**FIG. 20A**). There was no difference in the basal activity between WT and WY Gal-CBP (**FIG. 20B**). Thus, a CBP mutant lacking HAT activity is not capable of responding to WT-PS1 in terms of increased transcription activating ability. Hence, CBP and its HAT region appear to be essential for enhancing transcription *in vitro* following PS1 stimulation. Additionally, we decided to measure endogenous CBP levels in APP/PS1 mice.

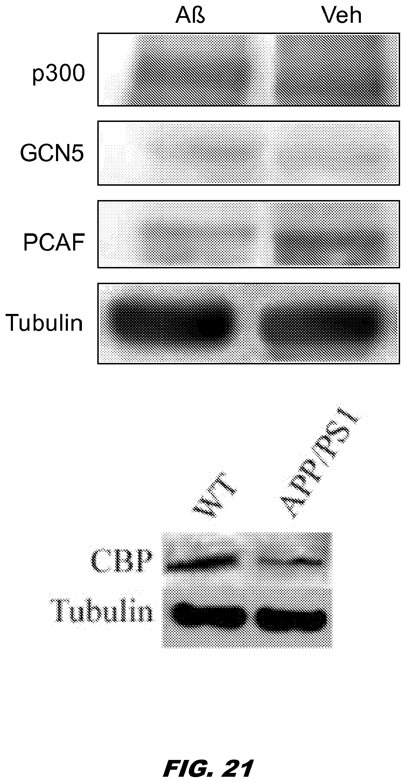

Western blot analysis from the hippocampus of 4 month old APP/PS1 mice revealed a significant decrease in CBP levels compared to WT controls (**FIG. 21**). In future experiments we will replicate these results. Alternative strategies will include measurement of CBP from the nuclear fraction given that changes in the CBP distribution (cytoplasm vs. nucleus) have been observed in F11 cells following overexpression of PS1(M146L) compared to WT-PS1 [A20]. We will also directly measure CBP HAT activity in APP/PS1 mice both in basal condition and 1hr after training for contextual learning compared to their WT littermates. Controls will be performed using a latent inhibition training paradigm to exclude that changes in CBP HAT activity are due to novel context alone or the electric shock instead of the association between them [A25]. In the latent inhibition paradigm animals will be pre-exposed to a novel context prior to receiving the electric shock so that the animal will form a spatial memory that blocks the formation of an associative contextual fear memory. Finally, we will measure HDAC activity using a new fluorimetric assay on hippocampi using the experimental paradigm as in the HAT assay. Without being bound by theory, these experiments will establish if CBP and/or HDACs are altered following overexpression of the APP and PS1 transgenes.

Similar to the electrophysiological and behavioral experiments described herein, we will ask whether TSA administration will rescue changes in CBP levels and CBP HAT activity. We will repeat the same experiments as described herein after treatment with TSA (2 µg/g body weight; i.p), 2 hrs prior to harvesting hippocampi from APP/PS1 mice. Vehicle- and TSA-treated WT littermates will serve as controls. To separate APP and PS1 overexpression effects from Aβ *per se* effects, experiments described herein (including CBP levels, CBP-HAT activity and total HDAC activity with TSA or with vehicle only) will be repeated in the presence of 200 nM oligomeric Aβ₄₂ infused onto dorsal hippocampi of WT mice compared to vehicle infused mice. Without being bound by theory, these experiments will establish if CBP and/or HDACs are altered following Aβ elevation.

### Determine which histones acetylation levels change following Aβ-elevation.

Data have shown ∼50% reduction in acetylated histone 4 levels in hippocampi of APP/PS1 mice. In future experiments, we will extend our investigation to histones 2B and 3 which also play a key role in transcription and memory [A23-A25]. We will finally check if Aβ elevation affects histone acetylation.

We will also test if defects seen in APP/PS1 mice, compared to WT littermates, are due to epigenetic effects. Acetylation of histones 2B, 3 and 4 was shown to play a key role in transcription and memory [A23-A25]. Acetylation of H4 in hippocampus from WT and APP/PS1 animals was measured. Vehicle control solution was administered i.p. 2 hrs before FC training. 1 hr after contextual FC, the APP/PS1 and WT mice were euthanized and hippocampi were extracted. Western blot analysis demonstrated that, compared to WT mice, APP/PS1 animals had an overall reduction of more than 50% in acetylated histone 4 levels (**FIG. 22**). Whether HDAC inhibition might rescue the histone 4 acetylation levels defect observed in APP/PS1 mice was also tested. Injection of TSA (2 µg/g body weight; i.p) 2 hours prior to contextual fear conditioning enhanced H4 acetylation of APP/PS1 mice (**FIG. 22**). Without being bound by theory, AD is likely to be a disease with an epigenetic motif and HDAC inhibitors can elevate decreased levels of histone 4 in an AD mouse model.

In future experiments, we will measure basal acetylation levels of histone 4, as well as controls using the latent inhibition paradigm described herein. In addition, we will extend our studies to histones 2B and 3 by measuring their acetylation in the hippocampus of APP/PS1 and WT littermates of 4 months of age both in basal conditions and following training for FC, with and without TSA as for histone 4. Controls will be also performed using a latent inhibition training paradigm as described herein. Finally, we will confirm these findings using a structurally dissimilar inhibitor of histone-deacetylases, NaB. As for TSA experiments, we will check if NaB (1.2 g/Kg) re-establishes normal histone/s acetylation levels in APP/PS1 mice.

To separate APP and PS1 overexpression effects from Aβ *per se* effects, experiments described herein related to measurements of different histones acetylation levels will be repeated in the presence of 200 nM oligomeric Aβ₄₂ infused onto dorsal hippocampi of WT mice compared to vehicle infused mice. Without being bound by theory, oligomeric Aβ₄₂ will affect histone 4 acetylation levels. Remaining histones acetylation levels should be consistent with findings from Transgenic (Tg) mice. Additional experiments will be performed with NaB. As for TSA experiments, we will check if NaB (1.2 g/Kg) reestablishes normal histone/s acetylation levels in Aβ₄₂-infused mice. Without being bound by theory, these experiments will demonstrate that epigenetic changes, such as reduced histone acetylation, play an important role in the Aβ-induced damage of synaptic function and memory associated with AD.

### Methods

***Animals:*** Double Tg mice will be obtained by crossing APP and PS1 animals (genotyped by PCR)^{[A29]}. For Aβ experiments we will use C57Bl6 mice.

***Aβ preparation:*** Oligomeric Aβ₄₂ will be prepared from commercially available synthetic peptides (American Peptides Co), as described^{[A33, A34]}.

For ***electrophysiology***, 400µm slices will be cut and maintained in an interface chamber at 29°C for 90min prior to recording, as reported^{[A5]}. Briefly, CA1 fEPSPs will be recorded by placing stimulating and the recording electrodes in CA1 *stratum radiatum.* Following BST assessment, a 15min baseline will be recorded every min at an intensity that evokes a response ∼35% of the maximum evoked response. LTP will be induced using θ-burst stimulation (4 pulses at 100Hz, with the bursts repeated at 5Hz and each tetanus including 3 ten-burst trains separated by 15sec).

For ***contextual and cued conditioning***, mice will be placed in the conditioning chamber for 2min before the onset of a discrete tone (CS) (a 30s, 85dB sound at 2800Hz), as described^{[A17]}. In the last 2s of the CS, mice will be given a 2s, 0.60mA foot shock (US) through the bars of the floor. After the CS/US pairing, mice will be left in the conditioning chamber for 30s and will then be placed back in their home cages. Freezing behavior will be scored using the Freezeview software (MED Ass). To evaluate contextual fear learning, freezing will be measured for 5min in the chamber in which the mice will be trained 24hr after training. To evaluate cued fear learning, 24hr after contextual testing, mice will be placed in a novel context for 2min (pre-CS test), after which they will be exposed to the CS for 3min (CS test), and freezing will be measured.

***Sensory perception of the shock*** will be determined through threshold assessment, as described^{[A17]}.

***CBP levels*** will be measured with western blot using specific CBP antibodies. The nuclear fraction will be contained in the pellet obtained from homogenated tissue, centrifuged at 7,700xg for 1min.

***CBP HAT activity*** will be measured by immunoprecipitation from the lysis of hippocampal extracts using CBP antibodies. After isolation, HAT activity will be assessed using indirect enzyme-linked immunosorbent assay kit to detect acetyl residues according to the manufacturer's instruction (Upstate).

For ***HDAC activity assay***, I will use a fluorimetric kit from Biovision (CA), according to the manufacturer instruction.

For ***histone acetylation assay***, Western blot will be performed from snap-frozen in liquid nitrogen hippocampi. Nuclear proteins will be acid-extracted and separated onto a denaturing, 7%-12% acrylamide gel followed by electroblotting onto nitrocellulose. Acetylated histones (H3, H2A and H2B) will be detected using antibodies purchased from Upstate and the Amersham ECF Kit accordingly to the manufacturer protocol.

***Statistical Analyses**:* Experiments will be performed in blind. Results will be analyzed with ANOVA with post-hoc correction with drug or genotype as main effect.

### References

A**1**. Malm et al, Proc Natl Acad Sci U S A, 2006. 103:8852-7.
**A2.** Cleary et al, Nat Neurosci, 2005. 8:79 84.
**A3.** Cullen et al, Neuroreport, 1997. 8:3213-7.
**A4.** Itoh et al, Eur J Pharmacol, 1999. 382:167-75.
**A5.** Vitolo, et al, Proc Natl Acad Sci U S A, 2002. 99:13217-21.
**A6.** Walsh et al, Nature, 2002. 416:535-9.
**A7.** Chen et al, J Neurosci Res, 2000. 60:65-72.
**A8.** Lambert et al, Proc Natl Acad Sci U S A, 1998. 95:6448-53.
**A9.** Kang et al, Nature, 1987. 325:733-6.
**A10.** Sherrington et al, Nature, 1995. 375:754-60.
**A11.** Levy-Lahad et al, Science, 1995. 269:970-3.
**A12.** Czech et al, Prog Neurobiol, 2000. 60:363-84.
**A13.** Takahashi et al, Neurosci Lett, 1999. 260:121-4.
**A14.** Rakyan et al, Biochem J, 2001. 356:1-10.
**A15.** Puzzo et al, J Neurosci, 2005. 25:6887-97.
**A16.** Puzzo et al, in Soc Neurosci. Abstr. 2006. Atlanta.
**A17.** Gong et al, J. Clin. Invest., 2004. 114:1624-1634.
**A18.** Korzus et al, Neuron, 2004. 42:961-72.
**A19.** Saura et al, Neuron, 2004. 42:23-36.
**A20.** Francis et al, Neuroreport, 2006. 17:917-21.
**A21.** Holcomb et al, Nat Med, 1998. 4:97-100.
**A22.** Gong et al, Cell, 2006. 126:775-88.
**A23.** Fischer et al, Nature, 2007. 447:178-82.
**A24.** Alarcon et al, Neuron, 2004. 42:947-59.
**A25.** Levenson et al, J Biol Chem, 2004. 279:40545-59.
**A26.** Green et al, J Neurosci, 2008. 28:11500-10.
**A27.** Kazantsev et al, Nat Rev Drug Discov. 2008. 7:854-68.
**A28.** Masliah, E., Histol Histopathol, 1995. 10:509-19.
**A29.** Trinchese et al, Ann Neurol, 2004. 55:801-14.
**A30.** Kamal et al, Nature, 2001. 414:643-8.
**A31.** Cao et al, Science, 2001. 293:115-20.
**A32.** Bordoli et al, Nucleic Acids Res, 2001. 29:589-97.
**A33.** Stine et al, J Biol Chem, 2003. 278: 11612-22.
**A34.** Puzzo et al, J. Neurosci., 2008. 28:14537-14545.

### Example 6 - Impairment of chromatin remodeling following amyloid elevation

In addition to CREB and CBP link to AD, epigenetic changes might also contribute to AD development. Hippocampal levels of acetylated histone 4, an acetylation important in memory formation [B19], were markedly reduced in an amyloid-depositing animal model after fear conditioning training. Without being bound by theory, changes in histone acetylation play an important role in AD.

Furthermore, after training for fear conditioning, levels of hippocampal acetylated H4 in APP/PS1 mice were dramatically lower than in wild-type littermates, indicating that changes in histone acetylation are involved in memory loss in AD. This observation is consistent with the finding that HDAC inhibition induced sprouting of dendrites, an increased number of synapses, and reinstated learning and access to LTM in the CK-p25 transgenic mouse with neuronal loss [B19]. Although levels of histone acetylation were not investigated, the use of HDAC inhibitors in two additional studies is encouraging. Nicotinamide, an HDAC III inhibitor, was found to restore cognition in the triple transgenic mouse model of AD via a non-epigenetic mechanism involving reduction of cytosolic Thr231-phosphotau [B49]. However, many questions remain to be answered: Does a treatment with HDAC inhibitors rescue the defects in histone acetylation, synaptic plasticity and memory both in transgenic mice with a mild plaque load and those with a more profound amyloid deposition? Can the beneficial effect of HDAC inhibitors be extended to other forms of explicit memory in addition to fear memory, such as reference memory? Are other histones, such as histones H2B and 3 which are also known to play a key role in transcription and memory [B19, B25, B26], abnormally acetylated during memory processes in AD? Given that experiments were performed on mice overexpressing mutated APP and PS1 transgenes which might cause a variety of effects independent of Aβ elevation, is histone acetylation affected following Aβ elevation? Does the histone acetyl-transferase CBP play a role in the reduction of histone acetylation in APP/PS1 mice and following Aβ elevation?

The inventors will also discuss in this Example whether chromatin changes at the level of histone acetylation occur following elevation of Aβ during memory processes. Without being bound by theory, the results obtained will provide a new type of mechanism for Aβ-induced impairment of memory and synaptic function.

The therapies for AD that are currently in use include augmentation of the cholinergic system by usage of acetylcholinesterase inhibitors, or blockage of glutamate neurotoxicity through NMDA antagonists. These agents have a limited efficacy. Major efforts are underway to inhibit tangle formation, to combat inflammation and oxidative damage, and to decrease Aβ load in the brain either by the use of agents that inhibit β and γ secretases or increase α secretase, by the use of drugs that inhibit Aβ oligomerization, or by the use of treatments such as immunization with Aβ that appear to augment the removal of Aβ from the brain. However, the role of APP, Aβ40, and the secretases in normal physiological function might present a problem in providing effective and safe approaches to AD therapy. Drugs interfering with transcription machinery and histone acetylation might represent a new approach to AD treatment to make the cell with its synaptic contacts in which memories are thought to reside, more robust and resistant to the effects of Aβ.

**HDAC inhibition ameliorates deficits in hippocampal long-term potentiation in APP/PS1 mice.** The observation that inspired these studies was the result of a series of experiments in which we tested whether a brief application of the HDAC inhibitor TSA was capable of rescuing the defect in LTP shown by slices from 3-4 month-old APP/PS1 mice (for a detailed description of the experiments showing the characterization of these mice [B50]). At this age the transgenic mice just start showing the synaptic plasticity and memory impairments [B14, B50]). As previously demonstrated [B50], basal synaptic transmission (BST) was similar between APP/PS1 and WT mice. Hippocampal slices were then perfused with TSA (1.65 µM) for 30 min before inducing LTP through tetanic stimulation of the Schaeffer collateral pathway. Potentiation in TSA treated APP/PS1 slices was far greater than in vehicle-treated APP/PS1 slices (two-way ANOVA: F_{1,11}=13.166, p=0.004; **FIG. 17**). On the other hand, TSA did not change the amplitude of LTP in hippocampal slices from WT mice compared to WT slices treated with vehicle alone (F_{1,12}=0.512, p=0.488, **FIG. 17**). Moreover, TSA perfusion did not affect baseline transmission in APP/PS1 and WT slices that received no tetanus (n=3 per each group). Thus, HDAC inhibition is capable of rescuing the defect in LTP in the APP/PS1 animal model of Aβ deposition.

**HDAC inhibition rescues the defect in associative memory in APP/PS1 mice.** Similar to humans affected by AD [B51], APP/PS1 mice exhibit a deficit in associative memory [B46]. In rodents, associative learning can be assessed by contextual fear conditioning. This behavioral task, which is based on the association of a neutral stimulus with an aversive one, is dependent on hippocampal function [B52]. Thus, following experiments on acute effect of TSA in synaptic dysfunction, we determined if TSA is beneficial against impairment of contextual fear conditioning in 3-4 month old APP/PS1 mice [B14]. Animals were divided into 4 groups: APP/PS1 with TSA, APP/PS1 with vehicle, WT with TSA and WT with vehicle. TSA and vehicle control solution were administered i.p. at a concentration of 2 mg/Kg body weight. We found a similar shock threshold among the various groups of mice. Then, mice were trained to associate neutral stimuli with an aversive one. They were placed in a novel context (fear conditioning box), exposed to a white noise cue paired with a mild foot shock, and injected with TSA 2 hrs before training. Fear learning was assessed 24 hrs later by measuring freezing behavior - the absence of all movement except for that necessitated by breathing - in response to representation of the context. We found no difference in the freezing behavior among the 4 groups of mice during the training phase of the fear conditioning (F_{3,47}=0.02997, p=0.9929, n=12-13/group; **FIG. 18**). Twenty-four hours later, we found a decreased freezing behavior in vehicle-treated APP/PS1 mice compared to vehicle-treated WT littermates in the analysis of the contextual learning (F_{3,47}=0.0526, p=0.0280, n=12-13/group; **FIG. 18**).

Freezing in vehicle-treated APP/PS1 mice was about 46% that of vehicle-treated WT mice [vehicle-treated APP/PS1 mice: 14.88 ± 2.97% vs. vehicle-treated WT littermates: 31.68 ± 5.32%; n=13 (11 females plus 2 males) and n=13 (11 females plus 2 males), respectively; t=2.76, p=0.0109; **FIG. 18**]. However, the freezing time was increased in APP/PS1 mice after the injection of TSA to about 236.27% of vehicle-treated APP/PS1 mice [TSA-treated APP/PS1 mice: 35.15 ± 6.99%, n=12 (10 females plus 2 males); t=2.744, p=0.0116; **FIG. 18**]. Statistical analysis revealed that freezing in APP/PS1 mice was not significantly different with respect to both TSA-treated (t=0.1902, p=0.8508) and vehicle-treated WT mice (t=0.3982, p=0.6941). TSA-treated WT mice showed a slight non-significant increase in freezing compared to vehicle-treated WT mice [TSA-treated WT mice: 36.95 ± 6.43%; n=13 (11 females plus 2 males); t=0.6316, p=0.5336, **FIG. 18**]. We next tested cued fear conditioning, a hippocampus-independent task [B46], and did not find a difference in freezing behavior among the 4 groups (both in the pre-CS group, F_{3,25}=0.8763, p=0.4666, and in the CS group, F_{3,24}=0.6398, p=0.5968). This result indicates that the function of the amygdala, which is involved mainly in cued conditioning and is known to be normal in APP/PS1 mice, is not affected by the inhibition of HDACs by TSA, as previously demonstrated [B53]. Taken together, these data show that the inhibition of histone deacetylation is able to re-establish normal associative memory in the APP/PS1 mouse while also restoring normal synaptic plasticity.

**HDAC inhibition rescues the defect in associative memory by Aβ elevation.** The APP and PS1 transgenes could affect neuronal function through different mechanisms [B23, B24], including direct effects by Aβ. Full-length APP and its cleavage products could differently impact H4 acetylation. To separate Aβ effects from other effects of APP and PS1 overexpression, we next determined whether Aβ *per se* is responsible for the deficit in H4 acetylation observed in our studies on transgenic mice. Since it has already been described that natural oligomers of human Aβ, markedly inhibit memory in vivo [B54, B55], we applied a preparation containing oligomeric Aβ42 (200 nM in a volume of 1 µl, bilaterally, slowly over 1 min) into dorsal hippocampi (**FIG. 24A-B**), 15 min prior to training for fear conditioning, with injection of TSA or vehicle (2 mg/kg body weight, 2 hrs prior to training, i.p.). Infusion occurred 5-7 days after implanting cannulas onto dorsal hippocampi. We found no difference in the freezing behavior among the 4 groups of mice during the training phase of the fear conditioning (F_{3,41}=0.04188, p=0.9884, n=8-14/group; **FIG. 24C**). Twenty-four hours later, freezing behavior was decreased in Aβ-infused mice compared to vehicle-infused mice in the analysis of the contextual learning (F_{3,41}=3.619, p=0.0209, n=8-14/group; **FIG. 24C**). Freezing in Aβ-infused mice was about 25% that of vehicle-infused mice [Aβ-infused mice: 8.580 ± 2.119% vs. vehicle-infused mice: 31.46 ± 5.373% n=12 and 11 males, respectively; t=3.147, p=0.0049; **FIG. 24C**]. However, the freezing time was increased in Aβ-infused mice after the injection of TSA to about 300% of Aβ-infused mice (TSA + Aβ treated mice: 26.17 ± 4.747% n=14 males; t=3.066, p=0.0053; **FIG. 24**). Statistical analysis revealed that freezing in Aβ-infused mice that were injected with TSA was not significantly different with respect to both TSA-injected (t=0.1902, p=0.8508) and vehicle-injected mice (t=0.3982, p=0.6941) that were infused with vehicle. TSA-treated mice showed similar amounts of freezing as vehicle-treated mice [TSA-treated mice: 27.01 ± 7.246%; n=8 males; t=0.4335, p= 0.6701, **FIG. 24C****].** We next tested cued fear conditioning, and did not find a difference in freezing behavior among the 4 groups (both in the pre-CS group, F_{3,52}=1.547, p= 0.2135, and in the CS group, F_{3,52}= 0.4838, p= 0.6950). Taken together, these data show that the inhibition of histone deacetylation is able to re-establish normal associative memory following Aβ elevation.

**APP/PS1 mice display a reduced endogenous level of histone 4 acetylation in response to a learning task.** Given that HDAC inhibitors are known to acetylate other molecules besides histones [B49], our next goal was to determine whether the effect of TSA on the defect in fear memory of APP/PS1 mice was linked, at least in part, to chromatin changes at the level of histone acetylation. Acetylation of H4 was shown to play a key role in transcription and memory [B19]. Thus, we asked whether the memory deficit observed in APP/PS1 mice are associated with altered acetylation of H4. We measured acetylation of H4 in hippocampus from 3 to 4 month old wild-type (WT) and APP/PS1 animals, after training for fear conditioning. Vehicle control solution was administered i.p. 2 hrs before training and 1 hour after it mice were euthanized and hippocampi were extracted. Western blot analysis demonstrated that, compared to WT mice, APP/PS1 animals had an overall reduction of more than 50% in acetylated H4 levels (t=2.702, p=0.0355; **FIG. 25A**). Interestingly, in interleaved experiments, injection of the class I/II HDAC inhibitor, TSA (2 mg/kg body weight; i.p, 2 hours prior to training for fear conditioning) enhanced H4 acetylation of APP/PS1 mice (t=3.283, p=0.0168 compared to vehicle-treated APP/PS1 littermates; **FIG. 25A**). Furthermore, H4 acetylation levels in hippocampi from TSA-treated APP/PS1 mice were similar to levels in TSA-treated WT littermates (t=0.2116, p=0.8385; Fig 5A). Finally, as previously demonstrated [B26], TSA increased H4 acetylation levels in WT mice (t=7.659, p=0.0001) compared to hippocampi from vehicle-treated WT littermates; **FIG. 25A**).

We next wanted to determine if there are any changes in the basal acetylation levels of H4 in APP/PS1 mice, compared to wild-type mice. We therefore measured acetylation of H4 in hippocampi from 3 to 4 month old WT and APP/PS1 animals which were exposed to the context without receiving an electrical shock. We found no difference between the two experimental groups in basal acetylated H4 (t=0.076, p=0.9427; **FIG. 25B**). This demonstrates that i) the overexpression of mutated APP and PS1 transgenes affects epigenetic changes at the level of H4 acetylation after contextual fear learning; and ii) TSA is able to elevate the decreased levels of histone 4 in an AD mouse model.

**Role of CBP and its HAT region on transcription following PS1 stimulation.** Studies of the mechanisms underlying memory formation have defined central roles for CRE-dependent gene expression, which is mediated by the transcription factor CREB and the coactivator CBP. CBP creates a bridge between CREB and the basal transcriptional machinery and acetylates histones. Histone acetylation, in turn, induces chromosomal changes resulting in loss of chromosomal repression (see **FIG. 23**). This allows successful transcription of the underlying genes needed for synthesis of proteins underlying memory formation. We have recently examined if the HAT activity of CBP has any role in the stimulus effect of WT-PS1 by using a construct in which full-length CBP contains a mutation (WY - amino acids 1503-1504 replaced by alanine and serine). This mutation, which completely abolishes its HAT activity [B56], was linked to the DNA binding domain of Gal4 (**FIG. 19**). In contrast to the enhanced promoter activity of full-length WT-CBP, no activity was observed with WY-CBP (**FIG. 20A**). There was no difference in the basal activity between WT and WY Gal-CBP (**FIG. 20B**). Thus, these findings indicate that a CBP mutant lacking HAT activity is not capable of responding to WT-PS1 in terms of increased transcription activating ability. Hence, CBP and its HAT region appear to be essential for enhancing transcription *in vitro* following PS1 stimulation.

**APP/PS1 mice display reduced CBP levels.** We next decided to measure endogenous CBP levels in APP/PS1 mice. Western blot analysis from the hippocampus of 4 month old APP/PS1 mice revealed a significant decrease in CBP levels compared to WT controls (**FIG. 21**) consistent with the observation that cerebral CBP levels are reduced in mice lacking functional PSs [B15]. In future experiments, we will replicate these results to increase the number of experiments to firmly establish that overexpression of mutated APP and PS1 transgenes affects CBP levels.

### Aims

### 1. To determine if inhibition of histone de-acetylation rescues synaptic and memory defects following Aβ elevation.

Inhibition of histone de-acetylation through trichostatin A (TSA) rescues the deficits in LTP and contextual fear memory in APP/PS1 mice [B20, B21], a transgenic animal expressing the Swedish mutation in APP(K670M:N671L) together with the mutation in PS1(M146L, line 6.2) [B22]. We will now test whether the structurally dissimilar inhibitor of histone-deacetylases, sodium butyrate (NaB), rescues the synaptic and memory deficits. Given that these observations were obtained in 4 month old APP/PS1 mice when amyloid plaques are just starting to form, we will extend them to 7 month old transgenic animals which have a more profound Aβ deposition. In addition, given that APP and PS1 transgenes could affect neuronal function via different mechanisms [B23, B24], including direct effects by Aβ, we will investigate whether inhibition of histone de-acetylation rescues defects of LTP and contextual fear memory induced by Aβ *per se.* We will also extend these findings to another form of explicit learning, the reference memory.

### Research Design and Methods.

***Rationale.*** A widely accepted concept in the memory field is that memories are stored in the brains as long-lasting changes in synaptic strength. Consistent with this concept AD is thought to begin as a synaptic disorder that progressively leads to greater neuronal dysfunction, leading to memory loss [B57]. Without being bound by theory, HDAC inhibition counteracts the impairment of synaptic plasticity and memory following amyloid elevation. We will test this with an additional and structurally dissimilar inhibitor, NaB, in addition to TSA. Next, given that APP and PS1 transgenes could affect neuronal function through different mechanisms [B23, B24], we will determine whether Aβ *per se* is responsible for the deficit in histone acetylation observed in our studies on transgenic mice. Moreover, we will extend our findings to older APP/PS1 mice when they have a severe plaque load [B50]. Finally, we will extend our findings to another form of explicit memory in addition to fear memory, the reference memory. Without being bound by theory, inhibition of histone deacetylase is beneficial against synaptic and memory dysfunction caused by elevation of oligomeric Aβ.

***Experimental design**.* We will confirm electrophysiological and behavioral findings described in **FIG. 17** and **FIG. 18** using the structurally dissimilar inhibitor NaB. As for TSA experiments, slices from 3-4 month old APP/PS1 mice will be treated with NaB (300 µM) for 30 min prior to applying a theta-burst stimulation to induce LTP. Controls will be performed on slices from APP/PS1 mice treated with vehicle, and WT littermate mice treated with NaB or vehicle. Similarly to TSA, we will also check if NaB (1.2 g/Kg) re-establishes normal contextual fear memory in APP/PS1 mice. In these behavioral experiments, animals were divided into 4 groups: APP/PS1 with NaB, APP/PS1 with vehicle, WT with NaB and WT with vehicle. Controls will be also performed using a latent inhibition training paradigm to exclude that HDAC inhibitors act through an effect on novel context alone or the electric shock instead of the association between them [B26]. In the latent inhibition paradigm animals will be pre-exposed to a novel context prior to receiving the electric shock so that the animal will form a spatial memory that blocks the formation of an associative contextual fear memory [B26].

We will separate APP and PS1 overexpression effects from Aβ *per se* effects. We have already demonstrated that TSA rescues the defect of contextual fear memory induced by a preparation containing oligomeric Aβ42 (see **FIG. 24**). Thus, we will determine whether TSA rescues the defect in LTP induced by 200 nM oligomeric Aβ42. Since it has already been described that natural oligomers of human Aβ, in the absence of monomers and fibrils, markedly inhibit LTP *in vivo* [B29], we will apply 200 nM oligomeric Aβ42 concurrently with TSA (1.65 µM) for 30 minutes to WT slices prior to inducing LTP. In interleaved control experiments we will apply oligomeric Aβ42 alone, or TSA alone, or vehicle.

We will replicate findings described herein using NaB (300 µM) plus 200 nM Aβ42 for 30 min prior to applying a theta-burst stimulation to induce LTP. Controls will be performed on slices treated with vehicle. As for TSA, we will also check if NaB (1.2 g/Kg) rescues defect in contextual fear memory in Aβ42-infused mice. In these behavioral experiments controls will be also performed using a latent inhibition training paradigm to exclude that HDAC inhibitors act through an effect on novel context alone or the electric shock instead of the association between them [B26].

We will repeat all of the experiments described herein with older mice (7 months), when a more severe plaque load exists and synaptic dysfunction includes also BST [B50].

We will extend our findings on contextual fear memory to another form of explicit memory, the reference memory. We have recently implemented a method that has the advantage of taking very short time [B58]. The method is a hybrid of the Morris water maze and the radial arm land maze. The motivation for the animals is the immersion in water. The mouse needs to swim in 6 alleys (arms) radiating from a central area until it finds a hidden (submerged) platform at the end of one of the arms. The goal arm is kept constant for all trials, with a different start arm on successive trials, such that the learning criterion will be reached in 2 days. The number of incorrect arm entries will be. At the end of the task, the mice will perform visible platform testing to exclude the possibility that visual, motor and motivational deficits affect the outcome of the experiments. In these experiments 3-4 and 7 month-old APP/PS1 and WT littermates will be injected with TSA or NaB, 2 hrs prior to performing the task. Vehicle will be used in control experiments. Similar experiments assessing reference memory will be also performed in Aβ42-infused mice (for these experiments mice will be divided into the following groups: Aβ42-infused animals + TSA or NaB or vehicle, vehicle-infused animals + TSA or NaB or vehicle).

Without being bound by theory, we will show that HDAC inhibition rescues the deficits of LTP and memory. If not, as an alternative strategy, we will try higher concentrations of the HDAC inhibitors, or a structurally dissimilar HDAC inhibitor such as MS-275 which has also been shown to cross the blood-brain-barrier [B59]. Then, the rodent will undergo electrophysiological and behavioral testing as described herein. Without being bound by theory, oligomeric Aβ42 will inhibits LTP and fear memory, and demonstrate that TSA ameliorates LTP and contextual fear memory following Aβ42 treatment. TSA alone should not have any effect. Findings from young mice or Aβ-infused animals should be confirmed in 7 month old transgenics. If not, as an alternative strategy, we will try longer treatments with the HDAC inhibitors, starting 1 or 4 weeks before testing. Finally, without being bound by theory, findings with the reference memory task should be in agreement with those obtained with fear conditioning. If so, these results should demonstrate that inhibition of histone deacetylase is beneficial against cognitive loss in general in AD-like animal models. If not, this will be equally interesting as it will demonstrate a dissociation between different types of memory and usage of HDAC inhibitors.

Studies investigating mechanisms underlying LTP in acute hippocampal slices indicate that transcription is not necessary for potentiation occurring prior to 2 hours [B26]. However, we observed an effect of TSA immediately after induction of LTP (FIG. 17). This finding is consistent with the result of Levenson *et al.* [B26]. The LTP induction paradigm they applied induces a form of LTP that is dependent upon transcription for either induction or expression. This was proved by using 5,6-dichlorobenzimidazole riboside (DRB), a transcription inhibitor, in WT mice. DRB was able to block both the early and late phase of a TSA-induced LTP increase, indicating that both the early and late enhancements of LTP by TSA are dependent upon transcriptional modulation [B26].

TSA and other HDAC inhibitors represent a new approach to AD treatment that appears to make the synapse more robust and resistant to the effects of Aβ. With regards to the use of HDAC inhibitors, it has been criticized that inhibition of HDACs might alter gene expression globally and thus affect memory processes in a nonspecific manner. However, Vecsey *et al* [B53] showed that TSA does not globally alter gene expression but instead increases the expression of specific genes during memory consolidation. They were able to show that HDAC inhibitors, including TSA, enhance memory and synaptic plasticity mainly by the activation of key genes that are dependent on CREB transcriptional activation [B53]. Thus, it is likely that TSA may be capable of stopping memory degradation in the presence of Aβ accumulation as well as improving brain functions that have already deteriorated, as in the case of the 3-month-old APP/PS1 mouse. Interestingly, 'rewiring' of the brain and recovery of memory using HDAC inhibitors was recently reported in CK-p25 transgenic mice [B19]. Therefore, it is possible that HDAC inhibitors could be capable of reestablishing neural networks in the AD brain. This indicates that using small molecules to target HDACs in AD patients could facilitate access to long-term memories. HDAC inhibitors with minimized side-effects are currently being developed by the pharmaceutical industry. It remains to be seen if these newer inhibitors can readily enter the brain and if they are as effective as TSA.

HDAC inhibitors could affect neuronal function through a variety of mechanisms including epigenetic and non-epigenetic changes [B19, B60]. Thus, the block of HDACs class I/II may increase the acetylation of non-histone substrates that, in turn, can contribute to the amplification of cellular processes associated with memory. Green et al. [B49] showed that inhibition of class III NAD+-dependent HDACs using vitamin B3 restored cognitive deficits in the triple transgenic AD mice, via a mechanism involving the reduction of Thr231-phosphotau in the cytoplasm.

### 2. To determine if histones 2B, 3 and 4 are abnormally acetylated following Aβ elevation.

Hippocampi from 3-4 month old APP/PS1 mice exhibit, after fear conditioning training, approximately a 50% reduction in acetylated histone 4 (H4) levels, an acetylation that was shown to be important in memory formation [B19]. In future experiments, we will extend our investigation to histones 2B and 3 because they also play a key role in transcription and memory [B19, B25, B26]. Furthermore, we will examine older transgenic mice with a more severe plaque load (7 months). Finally, to separate Aβ effects from other effects of APP and PS1 overexpression, we will check if Aβ infusion into the hippocampi affects acetylation of histones 2B, 3 and 4.

### Research Design and Methods.

***Rationale.*** Overexpression of mutated APP and PS1 transgenes affects changes in H4 acetylation occurring during contextual fear learning (see **FIG. 25**). In addition, HDAC inhibition through TSA is able to elevate the decreased levels of H4 in an AD mouse model. Without being bound by theory, epigenetic mechanisms at the level of histone acetylation may be impaired during learning and memory in AD. Additional histones undergoing changes in acetylation during learning and memory are H2B and H3 [B19, B25]. Therefore, in future experiments we will extend our studies to these histones. Moreover, we will test the structurally dissimilar inhibitor of histone-deacetylases, NaB. Next, given that APP and PS1 transgenes could affect neuronal function through different mechanisms [B23, B24], we will determine whether Aβ *per se* is responsible for the deficit in histone acetylation observed in our studies on transgenic mice. Finally, we will extend our findings to older APP/PS1 mice when they have a severe plaque load [B50].

***Experimental design.*** We will measure acetylation of H2B and H3 in the hippocampus of APP/PS1 and WT littermates of 4 months of age, both in basal conditions and following training for fear conditioning, with TSA (2 mg/kg body weight; i.p, 2 hours prior to training for fear conditioning) or vehicle as for H4. Controls will be performed using a latent inhibition training paradigm to exclude that histone modifications are due to novel context alone or the electric shock instead of the association between them [B26]. Immediately following sacrifice, brain tissue will be dissected and the two hippocampi from each mouse pooled and flash frozen for later homogenization and analysis. Additional controls will use cerebellum.

We will test whether NaB (1.2 g/Kg) is also capable of ameliorating acetylation of H2B, 3 and 4 in the same experimental paradigm and controls as in the TSA experiments described herein in Example 6.

We will repeat all of the experiments described herein Example 6 to measure acetylation of H4, 2A and 3 in the presence of 200 nM oligomeric Aβ42 infused into dorsal hippocampi of WT mice compared to vehicle-infused mice. Controls will be performed using a latent inhibition training paradigm and also using cerebellum.

Findings in Example 6 will be confirmed with the structurally dissimilar NaB. As for TSA experiments, we will check if NaB (1.2 g/Kg) re-establishes normal histones acetylation levels in Aβ42-infused mice. Also for these experiments, controls will be performed on vehicle-infused mice and using a latent inhibition training paradigm, as well as cerebellum.

We will also investigate whether we can ameliorate levels of histone acetylation when profound amyloid load occurs. To address this question which is directly related to the therapeutic possibility of using HDAC inhibitors in advanced stages of AD pathology, we will repeat the experiments as described herein on older APP/PS1 mice (7 months).

Without being bound by theory, oligomeric Aβ42 will affect H4 acetylation levels and oligomeric Aβ42 will produce the same effects on acetylation levels of histones 2B and 3 as in transgenic mice. If not, we will try more prolonged applications of 200 nM oligomeric Aβ42 through Alzet osmotic mini-pumps (1 day, 1 week, 1 month). Problems related to the use of a synthetic preparation containing Aβ42 are greatly alleviated by the use of transgenic animals which produce natural forms of Aβ. Studies on H2B and 3 will provide a more complete picture of the type of epigenetic changes occurring at the level of histone acetylation. Finally, research in older mice will help understanding whether HDAC inhibitors might be used at older disease stages. Taken all together, the studies described herein will help establishing epigenetic changes as events occurring following Aβ elevation.

In addition to histone acetylation, other histone posttranslational modifications (PTMs) such as phosphorylation, sumoylation, ubiquitination, and methylation were shown to regulate transcription [B7]. For example, histone modifications associated with active gene transcription, such as H3 Lys4 methylation and H3 Lys56 acetylation, were found to lead to gene expression. But, histone modifications associated with the inactivation of gene transcription, such as H3 Lys27 methylation and H2A Lys119 ubiquitination were found to cause gene silencing. PTMs that are modified as a consequence of chronic neuronal exposure to oligomeric forms of Aβ42 in mice. As described (see **FIG. 24**), 200 nM oligomeric Aβ42 will be infused into dorsal hippocampi of WT mice. The hippocampi of Aβ42-infused mice will be removed and compared to those of vehicle-infused mice. We will carry mass spectrometry, using immunoprecipitated H2B, 3 and 4, on hippocampi and search for different histone PTMs between Aβ42-infused mice and vehicle-infused animals. To control for non-specific effects, we will have a 3^{rd} group of mice in which we infuse a well studied neurotoxin, gamma-acetylenic GABA, which is not directly related to AD. We will then test several of the most interesting histone PTMs in our APP/PS1 mouse model. These mice will be sacrificed at 1 month (prior to plaque formation), 2 months (as plaques start to form), 3-4 months (at early stages of plaque formation), and 7 months (at late stages of plaque formation) of age to test when this histone PTMs occur. Without being bound by theory, epigenetic changes, such as reduced histone acetylation, are likely to play an important role in the Aβ-induced damage of synaptic function and memory associated with AD.

### 3. To determine if levels and/or activity of the histone acetyl-transferase CBP are modified following Aβ elevation.

CBP HAT activity is essential for enhancing transcription *in vitro* following PS1 stimulation. Furthermore, CBP levels in 3-4 month old APP/PS1 mice were found to be lower than in WT mice. In future experiments, we will extend this observation to older transgenic mice and following hippocampal Aβ infusion. In addition, we will determine if CBP HAT activity is affected both in young and older APP/PS1 mice as well as after Aβ infusion. Finally, we will determine whether stimulation of HAT activity through a recently synthesized HAT agonist, MOM, rescues the deficits in LTP, memory, and histone acetylation following Aβ elevation.

### Research Design and Methods.

***Rationale.*** Endogenous CBP levels are reduced in APP/PS1 mice (see **FIG. 21**). These data are consistent with the observation that cerebral CBP levels are reduced in mice lacking functional PSs [B15]. In addition, given that CBP and its HAT region appear to be essential for enhancing transcription *in vitro* following PS1 stimulation (see **FIG. 20**), we will directly measure CBP HAT activity in APP/PS1 mice both in basal condition and 1 hr after training for contextual learning compared to their WT littermates. If we find a reduction in both CBP levels and/or HAT activity, we will also check whether use of a HAT agonist [B61] rescues the defect in contextual fear memory and histones acetylation. Next, given that APP and PS1 transgenes could affect neuronal function through different mechanisms [B23, B24], we will determine whether Aβ *per se* is responsible for the deficit in CBP levels and/or activity observed in transgenic mice. Finally, we will extend our findings to older APP/PS1 mice when they have a severe plaque load [B50]. Taken together, whether CBP levels and/or activity are altered following overexpression of the APP and PS1 transgenes will be assessed, in addition to whether CBP levels and/or activity are altered following Aβ elevation. The investigators will examine whether HAT agonists rescue defects in synaptic and memory function, as well as histone acetylation.

***Experimental design.*** We will measure CBP levels in the hippocampus of APP/PS1 and WT littermates of 4 months of age. Immediately following sacrifice, brain tissue will be dissected and the two hippocampi from each mouse pooled and flash frozen for later homogenization and analysis. Additional controls will use cerebellum.

We will directly measure CBP HAT activity in APP/PS1 mice both in basal condition and 1 hr after training for contextual learning compared to their WT littermates. Controls will be performed using a latent inhibition training paradigm to exclude that changes in CBP HAT activity are due to novel context alone or the electric shock instead of the association between them [B26]. Additional controls will use cerebellum.

A HAT agonist, such as N-(4-chloro-3-trifluoromethyl-phenyl)-2-ethoxy-benzamide (CTB or also referred to as compound 6J) [B61] was synthesized. Another benzamide HAT agonist, MOM, was synthesized to check if up-regulation of HAT activity rescues the defect in LTP, contextual fear memory and histone acetylation of APP/PS1 mice. We have synthesized the compound **(****FIG. 26A**) and shown that it induces histone acetylation (**FIG. 26B**). We will test whether MOM rescues the LTP defect in hippocampal slices from APP/PS1 mice following the same experimental paradigm as described herein. MOM (10 µM) or vehicle will be applied for 30 min prior to inducing LTP with the θ-burst. WT littermate slices treated with MOM or vehicle will be used as controls. Next, we will check if the agonist rescues the defect in contextual fear memory in APP/PS1 animals following the same experimental paradigm as described herein. We will also check if MOM re-establishes normal reference memory.

Given that MOM does not cross the blood brain barrier, cannulas will be implanted into the dorsal hippocampi of APP/PS1 mice and WT littermates to deliver it directly into the hippocampi. We will infuse 100 µg in 1 µl, slowly over 1 min. The infusion will occur 2 hrs prior to applying the foot shock for fear conditioning. As in the experiments described herein, we will measure the amount of freezing at 24 hrs to assess contextual fear memory followed by cued fear memory at 48 hrs. For the water maze experiments, in turn, we will measure the number of incorrect arm entries. Finally, in a separate set of experiments hippocampi and cerebella will be removed at 1 hr after training for fear conditioning and histone acetylation levels will be measured. If we find that MOM rescues the LTP and memory defects, as well as hippocampal histone acetylation of APP/PS1 mice, we will conclude that HAT agonists might ameliorate the defect in LTP, memory and histone acetylation following overexpression of APP and PS1 transgenes. The protocol will also be carried out for testing the effect of the HAT activator, CTB.

All of the experiments described herein, including CBP levels, HAT activity, and usage of MOM and CTB, will be repeated in the presence of 200 nM oligomeric Aβ42 infused into dorsal hippocampi of WT mice compared to vehicle infused mice.

We will also investigate whether we can ameliorate defect in CBP levels and/or HAT activity when profound amyloid load occurs. To address this question, we will repeat the experiments described herein Example 6 on older APP/PS1 mice (7 months).

Problems related to the use of a synthetic preparation containing Aβ42 are greatly alleviated by the use of transgenic animals which produce natural forms of Aβ. Experiments examining CBP levels will be performed. However, if CBP levels are not affected, we will employ an alternative strategy by measuring CBP from the nuclear fraction. Changes in the CBP distribution have been observed in F11 cells. CBP is mostly found in the nucleus of cells over-expressing WT-PS1, whereas cells over-expressing the mutant form of PS1 show CBP in both the cytoplasm and the nucleus [B18]. Thus, we should be able to determine if changes in CBP localization might play a role in the development of AD.

It is difficult to predict whether CBP HAT activity is changed. Moreover, one cannot exclude the possibility that changes in CBP HAT activity occur at a defined time after training for fear conditioning. Therefore, we will also measure hippocampal HAT activity at different time points (1 min, 5 min, 20 min and 2 hrs) after the electric shock (with controls similar to experiments described herein). Thus, a picture of the changes occurring at the level of the transcription machinery following Aβ elevation should be able to be obtained.

We will measure HDAC activity through a new fluorimetric assay on hippocampi using the experimental paradigm as in the HAT assay (basal, 1 min, 5 min, 20 min and 1 hour after foot shock). An additional alternative possibility is that other HATs are involved in the reduction of histone acetylation. These include GNATs family, MYST family, p300, and ACTR/SRC-1. We will measure levels and activity of these HATs.

Finally, one cannot exclude the possibility that MOM or CTB does not rescue the defect in LTP, memory and histones acetylation in APP/PS1 mice as well as Aβ-infused mice. If so, as an alternative strategy, we will try longer treatments with the HAT agonist, starting a week before testing or at 6 weeks of age prior to plaque appearance for the experiments on double transgenic animals.

### General Methods of the Example

**Animals.** Double transgenic mice will be obtained by crossing APP and PS1 animals (genotyped by PCR) [B20, B21, B50]. For Aβ experiments we will use C57Bl6 mice which will be obtained from a breeding colony. All the mice will be maintained on a 12 h light/dark cycle (with lights on at 6:00 A.M.) in temperature- and humidity-controlled rooms. Food and water will be available ad libitum.

**Electrophysiological studies.** We will cut 400 µm hippocampal slices from C57Bl6 mice and maintain them in an interface chamber at 29° C for 90 min prior to recording, as previously reported [B11]. The bath solution will consist of 124.0 mM NaCl, 4.4 mM KCl, 1.0 mM Na₂HPO4, 25.0 mM NaHCO₃, 2.0 mM CaCl₂, 2.0 mM MgSO₄, and 10.0 mM glucose, continuously bubbled with 95% O₂ and 5% CO₂. As a stimulating electrode we will use a bipolar tungsten electrode, placed at the level of the Schaeffer collateral fibers. As a recording electrode we will use a glass pipette filled with bath solution and placed at the level of CA1 *stratum radiatum.* We will first assess basal synaptic transmission (BST) to plot the stimulus voltages against slopes of fEPSP and determine the intensity of stimulation of our LTP experiments (it should evoke a response ∼35% of the maximum evoked response). Baseline for LTP will be recorded for 15 min, every min. LTP will be elicited using θ-burst stimulation (4 pulses at 100 Hz, with the bursts repeated at 5 Hz and each tetanus including 3 ten-burst trains separated by 15 sec).

**Fear conditioning.** Contextual fear conditioning will be assessed as previously described [B14, B21]. Mice will be placed in a conditioning chamber for 2 min before the onset of a tone (CS) (a 30 s, 85 dB sound at 2800 Hz). In the last 2 s of the CS, mice will be given a 2 s, 0.45 mA foot shock (US) through the bars of the floor. Then, the mice will be left in the conditioning chamber for another 30 s. Freezing behavior, defined as the absence of movement except for that needed for breathing, will be scored using the Freezeview software. Contextual fear learning, a type of memory for which hippocampal function is indispensable, will be evaluated 24 hrs after training by measuring freezing for 5 min in the chamber in which the mice will be trained. Cued fear learning, a type of memory that depends upon amygdala function, will be evaluated 24 hrs after contextual testing by placing mice in a novel context for 2 min (pre-CS test), after which they will be exposed to the CS for 3min (CS test), and freezing will be measured.

Sensory perception of the shock will be determined through threshold assessment. Briefly, the electric current (0.1 mA for 1 s) will be increased at 30 s intervals by 0.1 mA to 0.7 mA. Threshold to flinching (first visible response to shock), jumping (first extreme motor response), and screaming (first vocalized distress) will be quantified for each animal by averaging of the shock intensity at which each animal manifest a behavioral response of that type to the foot shock. No difference in the sensory threshold assessment should be observed among different groups of mice in experiments in which fear conditioning is tested if the experimental procedure does not affect the sensory threshold of the animals.

Moreover, different groups of mice will be checked with the open-field test. The open field will be an arena made of white acrylic with internal dimensions of 72 X 72 X 33 cm (An area measuring 36 X 36 cm in the centre of the open field will be defined as the 'central zone'). Mice will be placed in the center of a standard open field and their behavior monitored for 1 hr and scored for proportion of time in the center compartment vs. periphery, and number of entries into the center compartment. Mice will be returned for a second hour block after 24 hr. No difference in exploratory behavior as demonstrated by a similar percentage of time spent in the center compartment and the number of entries into the center compartment should be observed if the manipulation does not affect exploratory capabilities of the mice.

**Reference memory.** The first day of the protocol will be a training day. Mice will be trained to identify the platform location by alternating between a visible and a hidden platform in a goal arm. The final 3 trials on day 1 and all 15 trials on day 2 will use a hidden escape platform to force mice to use spatial cues to identify the location of the goal arm. To avoid learning limitations imposed by exhausting practice and to avoid fatigue that may result from consecutive trials, spaced practice training will be established by running the mice in cohorts of 4 and alternating different cohorts through the 15 training trials over 3 hours testing periods each day. On day 1, a visible platform will be placed in a goal location. Mouse 1 of cohort 1 will be gently placed in the pool near the perimeter of the wall of the first start arm (specified on a score sheet) and facing the center of the pool. The number of incorrect arm entries (entries in arms with no platform) will be counted. If the animal enters the incorrect arm it is gently pulled back to the start arm. Each trial will last up to 1 minute. Failure to select an arm after 15 seconds will be counted as an error and the mouse will be returned to the start arm. After 1 minute, if the platform has not been located, the mouse will be guided gently through the water by placing a hand behind it to direct it towards the platform. The mouse will rest on the platform for 15 seconds. After completing the trial, the mouse will be removed from the pool, gently towel dried and placed back into its cage under a heat lamp. The goal platform location will be different for each mouse. After all the mice in the first cohort have had a trial to locate a visible platform, the platform will be switched from visible to hidden. After each mouse from cohort 1 completes six alternating trials between visible and hidden platforms, the mice will be left to rest under a heating source, and mice from the second cohort will be tested in the same way. After completing the six alternating trials, mice from cohort 2 will return to their cages to rest. Next, mice from the first cohort will complete trials 7-12 again using the alternating visible-hidden platform location. During resting time for mice from the first cohort, mice from the second cohort will complete trials 7-12. At this point, all mice will have to perform 3 hidden platform trials. On day 2, the same procedure will be repeated as on day 1 for all 15 trials using only the hidden platform. For *data analysis,* the number of errors will be input into statistics software (Statview by SAS, Chicago, IL), and averages for each mouse will be calculated using blocks of 3 trials. As described [B58], we expect that the mice will exhibit 1 or less errors over three trials near the end of the second day. Mice that fail to learn will make 3-5 errors throughout the training session, with no improvement over trials.

At the end of the task visible platform training will be used to test visual, motor and motivation skills of the mice. Both time to reach the platform and swimming speed will be recorded and analyzed with a video-tracking system (HVS 2020, HVS Image, UK).

**Infusion technique.** Following anaesthesia with 20 mg/kg Avertin, mice will be implanted with a 26-gauge guide cannula into the dorsal part of the hippocampi (coordinates: P=2.46 mm, L=1.50 mm to a depth of 1.30 mm) [B62]. The cannulas will be fixed to the skull with acrylic dental cement (Paladur). After 6-8 days we will bilaterally inject 200 pM Aβ₄₂, or vehicle in a final volume of 1 µl over 1 min through infusion cannulas that will be connected to a microsyringe by a polyethylene tube. For Morris water maze, mice will be injected 20 min prior to performing each session and the probe trial, whereas for fear conditioning mice will receive a single injection 20 min before the training. Mice will be handled once a day for 3 days before behavioral experiments. During infusion animals will be handled gently to minimize stress. After infusion, the needle will be left in place for another minute to allow diffusion. After behavioural testing, a solution of 4% methylene blue will be infused into the cannulas. Animals will be sacrificed and their brains removed, frozen, and then cut at -20° with cryostat for histological localization of infusion cannulas.

**Aβ preparation.** Oligomeric Aβ42 will be prepared from commercially available synthetic peptides (American Peptides Co), as described [B63, B64]. Briefly, the lyophilized peptide will be resuspended in cold 1,1,1,3,3,3-hexafluoro-2-propanol (HFIP, Sigma) and aliquoted in polypropylene vials. After 24 hrs the HFIP solution will be allowed to evaporate in a fume hood until a thin film of peptide is formed on the bottom of the vials. Peptide films will be dried under gentle vacuum and stored in sealed vials at -20°C. Prior to use, anhydrous DMSO (Sigma) will be added to obtain a pure monomeric Aβ/DMSO solution that will be sonicated for 10 min [B63]. Oligomeric Aβ₄₂ will be obtained by incubating an aliquot of monomeric Aβ/DMSO solution in sterile PBS at 4°C overnight. The quality of these Aβ preparations will be routinely controlled using Western blot analysis in which Aβ samples will be resolved by Tris-Tricine PAGE under non-denaturing/nonreducing conditions, and then transferred on nitrocellulose membrane. Subsequent Western blotting will be carried out after membrane incubation with the anti-human Aβ monoclonal antibody 6E10 (Signet Lab). The immunostaining will be revealed by horseradish peroxidase chemi-luminescence.

**Histone acetylation assay.** Western blot will be performed from snap-frozen in liquid nitrogen hippocampi and cerebella. Tissue will be homogenized in lysis buffer (62.5 mM Tris-HCl pH 6.8, 3% SDS, 1 mM DTT) and incubated at 4 °C for 10 min, then sonicated before centrifugation at 2,000 rpm for 5 min. Whole cell extracts will be electrophoresed on 10-20% gradient PAGE gel (Invitrogen) and then immunoblotted. Antibodies will be used at a 1:1,000 concentration for immunoblotting. All anti-histone antibodies will be purchased from Millipore. β-III-Tubulin antibody will be purchased from Promega. Immunoblot data will be quantified by measuring the band intensity using imaging software (NIH ImageJ). For quantitative immunoblot analysis, equal amounts of proteins will be loaded into each lane. To confirm equal loading, blots will be reprobed with corresponding pan-antibodies or antibodies for house-keeping proteins such as β-III-Tubulin. For quantification, we always use a signal in the linear range.

**Mass Spectrometry.** Characterization of modifications on immunoprecipitated histones will be carried out by mass spectrometry. Immunoprecipitated histones will be purified by reverse-phase HPLC in the PSR or by SDS-PAGE, then subjected to enzymatic digestion. Resulting peptides will be analyzed by LC-MS/MS on a Waters Qtof mass spectrometer equipped with a Dionex nanflow LC. The standard digestion protocol using trypsin is not feasible due to the number of Lys residues in the N-terminal portion of histones, resulting in peptides too small to be analyzed. Since this is also the region most rich in modifications, a digestion protocol must be used that results in peptides of optimum size (800-2000 Da) for analysis by LC-MS/MS. We will derivatize the Lys residues with propionic anhydride thus limiting tryptic digestion to Arg only. We have already successfully carried out this derivatization and digestion procedure on a commercial preparation of recombinant histone H3.1 and have obtained tryptic peptides from the N-terminal region that are easily analyzed by mass spectrometry. For relative quantitation of histone modifications, we will use a stable isotope-labeling procedure [B65] to modify carboxylic acid groups on the tryptic peptides followed by mass spectrometric analysis.

**CBP levels** will be measured with western blot using specific CBP antibodies. The nuclear fraction will be contained in the pellet obtained from homogenated tissue, centrifuged at 7,700xg for 1min.

**CBP HAT activity** will be measured by immunoprecipitation from the lysis of hippocampal extracts using CBP antibodies. After isolation, HAT activity will be assessed using indirect enzyme-linked immunosorbent assay kit to detect acetyl residues according to the manufacturer's instruction (Upstate).

**HDAC activity** will be measured using a fluorimetric kit from Biovision (CA), according to the manufacturer instruction.

**Statistical Analyses.** Experiments will be performed in blind. Results will be expressed as Standard Error of the Mean (SEM). Level of significance will be set for p<0.05. Results will be analyzed by Student's t test (pairwise comparisons) or ANOVA for repeated measures (multiple comparisons) with treatment condition as main effect. Planned comparisons will be used for post-hoc analysis. For behavior, experiments will be designed in a balanced fashion, and mice will be trained and tested at each of the different conditions in three or four separate experiments. Experiments will be analyzed with ANOVA with treatment as main effect.

### REFERENCES

B1. Lodish H., B.A., Zipursky LS., Matsudaira P., Baltimore D., Darnell J., Molecular Cell Biology. 4 ed. 2000, New York: W. H. Freeman and Company.
B2. Kandel, E.R., The molecular biology of memory storage: a dialog between genes and synapses. Biosci Rep, 2001. 21(5): p. 565-611.
B3. Dash, P.K., B. Hochner, and E.R. Kandel, Injection of the cAMP-responsive element into the nucleus of Aplysia sensory neurons blocks long-term facilitation. Nature, 1990. 345(6277): p. 718-21.
B4. Montarolo, P.G., P. Goelet, V.F. Castellucci, J. Morgan, E.R. Kandel, and S. Schacher, A critical period for macromolecular synthesis in long-term heterosynaptic facilitation in Aplysia. Science, 1986. 234(4781): p. 1249-54.
B5. Bailey, C.H. and M. Chen, Morphological basis of long-term habituation and sensitization in Aplysia. Science, 1983. 220(4592): p. 91-3.
B6. Levenson, J.M. and J.D. Sweatt, Epigenetic mechanisms in memory formation. Nat Rev Neurosci, 2005. 6(2): p. 108-18.
B7. Peterson, C.L. and M.A. Laniel, Histones and histone modifications. Curr Biol, 2004. 14(14): p. R546-51.
B8. Korzus, E., M.G. Rosenfeld, and M. Mayford, CBP histone acetyltransferase activity is a critical component of memory consolidation. Neuron, 2004. 42(6): p. 961-72.
B9. Bourtchuladze, R., B. Frenguelli, J. Blendy, D. Cioffi, G. Schutz, and A.J. Silva, Deficient long-term memory in mice with a targeted mutation of the cAMP-responsive element-binding protein. Cell, 1994. 79(1): p. 59-68.
B10. Yin, J.C., J.S. Wallach, M. Del Vecchio, E.L. Wilder, H. Zhou, W.G. Quinn, and T. Tully, Induction of a dominant negative CREB transgene specifically blocks long-term memory in Drosophila. Cell, 1994. 79(1): p. 49-58.
B11. Vitolo, O.V., A. Sant'Angelo, V. Costanzo, F. Battaglia, O. Arancio, and M. Shelanski, Amyloid beta - peptide inhibition of the PKA/CREB pathway and long-term potentiation: reversibility by drugs that enhance cAMP signaling. Proc Natl Acad Sci U S A, 2002. 99(20): p. 13217-21.
B12. Puzzo, D., O. Vitolo, F. Trinchese, J.P. Jacob, A. Palmeri, and O. Arancio, Amyloid-beta peptide inhibits activation of the nitric oxide/cGMP/cAMP-responsive element-binding protein pathway during hippocampal synaptic plasticity. J Neurosci, 2005. 25(29): p. 6887-97.
B13. Puzzo, D., A. Staniszewski, S. Deng, S. Liu, A. Palmeri, D.W. Landry, and O. Arancio. Sildenafil rescues synaptic and cognitive impairment in a mouse model of Alzheimer's disease. in Soc Neurosci. Abstr. 2006. Atlanta.
B14. Gong, B., O.V. Vitolo, F. Trinchese, S. Liu, M. Shelanski, and O. Arancio, Persistent improvement in synaptic and cognitive functions in an Alzheimer mouse model following rolipram treatment. J. Clin. Invest., 2004. 114: p. 1624-1634.
B15. Saura, C.A., S.Y. Choi, V. Beglopoulos, S. Malkani, D. Zhang, B.S. Shankaranarayana Rao, S. Chattarji, R.J. Kelleher, 3rd, E.R. Kandel, K. Duff, A. Kirkwood, and J. Shen, Loss of presenilin function causes impairments of memory and synaptic plasticity followed by age-dependent neurodegeneration. Neuron, 2004. 42(1): p. 23-36.
B16. Sherrington, R., E.I. Rogaev, Y. Liang, E.A. Rogaeva, G. Levesque, M. Ikeda, H. Chi, C. Lin, G. Li, K. Holman, and et al., Cloning of a gene bearing missense mutations in early-onset familial Alzheimer's disease. Nature, 1995. 375(6534): p. 754-60.
B17. Levy-Lahad, E., E.M. Wijsman, E. Nemens, L. Anderson, K.A. Goddard, J.L. Weber, T.D. Bird, and G.D. Schellenberg, A familial Alzheimer's disease locus on chromosome 1. Science, 1995. 269(5226): p. 970-3.
B18. Francis, Y.I., A. Stephanou, and D.S. Latchman, CREB-binding protein activation by presenilin 1 but not by its M146L mutant. Neuroreport, 2006. 17(9): p. 917-21.
B19. Fischer, A., F. et al., Recovery of learning and memory is associated with chromatin remodelling. Nature, 2007. 447(7141): p. 178-82.
B20. Holcomb, L., et al., Accelerated Alzheimer-type phenotype in transgenic mice carrying both mutant amyloid precursor protein and presenilin 1 transgenes. Nat Med, 1998. 4(1): p. 97-100.
B21. Gong, B., et al., Ubiquitin Hydrolase Uch-L1 Rescues beta-Amyloid-Induced Decreases in Synaptic Function and Contextual Memory. Cell, 2006. 126(4): p. 775-88.
B22. Francis, I.Y., M. Fa', H. Ashraf, H. Zhang, A. Staniszewski, D. Latchman, and O. Arancio, Dysregulation of histone acetylation in the APP/PS1 mouse model of Alzheimer's disease. J Alzheimers Dis, 2009. In press**.**
B23. Kamal, A., A. Almenar-Queralt, J.F. LeBlanc, E.A. Roberts, and L.S. Goldstein, Kinesin-mediated axonal transport of a membrane compartment containing beta-secretase and presenilin-1 requires APP. Nature, 2001. 414(6864): p. 643-8.
B24. Cao, X. and T.C. Sudhof, A transcriptionally [correction of transcriptively] active complex of APP with Fe65 and histone acetyltransferase Tip60. Science, 2001. 293(5527): p. 115-20.
B25. Alarcon, J.M., G. Malleret, K. Touzani, S. Vronskaya, S. Ishii, E.R. Kandel, and A. Barco, Chromatin acetylation, memory, and LTP are impaired in CBP+/- mice: a model for the cognitive deficit in Rubinstein-Taybi syndrome and its amelioration. Neuron, 2004. 42(6): p. 947-59.
B26. Levenson, J.M., et al., Regulation of histone acetylation during memory formation in the hippocampus. J Biol Chem, 2004. 279(39): p. 40545-59.
B27. Cullen, W.K., Y.H. Suh, R. Anwyl, and M.J. Rowan, Block of LTP in rat hippocampus in vivo by beta-amyloid precursor protein fragments. Neuroreport, 1997. 8(15): p. 3213-7.
B28. Itoh, A., T. Akaike, M. Sokabe, A. Nitta, R. Iida, A. Olariu, K. Yamada, and T. Nabeshima, Impairments of long-term potentiation in hippocampal slices of beta-amyloid-infused rats. Eur J Pharmacol, 1999. 382(3): p. 167-75.
B29. Walsh, D.M., I. Klyubin, J.V. Fadeeva, W.K. Cullen, R. Anwyl, M.S. Wolfe, M.J. Rowan, and D.J. Selkoe, Naturally secreted oligomers of amyloid beta protein potently inhibit hippocampal long-term potentiation in vivo. Nature, 2002. 416(6880): p. 535-9.
B30. Pittenger, C. and E.R. Kandel, In search of general mechanisms for long-lasting plasticity: Aplysia and the hippocampus. Philos Trans R Soc Lond B Biol Sci, 2003. 358(1432): p. 757-63.
B31. Lander, et al., Initial sequencing and analysis of the human genome. Nature, 2001. 409(6822): p. 860-921.
B32. Kornberg, R.D. and Y. Lorch, Twenty-five years of the nucleosome, fundamental particle of the eukaryote chromosome. Cell, 1999. 98(3): p. 285-94.
B33. Biel, M., V. Wascholowski, and A. Giannis, Epigenetics--an epicenter of gene regulation: histones and histone-modifying enzymes. Angew Chem Int Ed Engl, 2005. 44(21): p. 3186-216.
B34. de la Cruz, X., S. Lois, S. Sanchez-Molina, and M.A. Martinez-Balbas, Do protein motifs read the histone code? Bioessays, 2005. 27(2): p. 164-75.
B35. Strahl, B.D. and C.D. Allis, The language of covalent histone modifications. Nature, 2000. 403(6765): p. 41-5.
B36. Ng, H.H. and A. Bird, Histone deacetylases: silencers for hire. Trends Biochem Sci, 2000. 25(3): p. 121-6.
B37. Kimura, A., K. Matsubara, and M. Horikoshi, A decade of histone acetylation: marking eukaryotic chromosomes with specific codes. J Biochem, 2005. 138(6): p. 647-62.
B38. de Ruijter, A.J., A.H. van Gennip, H.N. Caron, S. Kemp, and A.B. van Kuilenburg, Histone deacetylases (HDACs): characterization of the classical HDAC family. Biochem J, 2003. 370(Pt 3): p. 737-49.
B39. Yang, X.J. and E. Seto, HATs and HDACs: from structure, function and regulation to novel strategies for therapy and prevention. Oncogene, 2007. 26(37): p. 5310-8.
B40. Gregoretti, I.V., Y.M. Lee, and H.V. Goodson, Molecular evolution of the histone deacetylase family: functional implications of phylogenetic analysis. J Mol Biol, 2004. 338(1): p. 17-31.
B41. Langley, B., et al., Remodeling chromatin and stress resistance in the central nervous system: histone deacetylase inhibitors as novel and broadly effective neuroprotective agents. Curr Drug Targets CNS Neurol Disord, 2005. 4(1): p. 41-50.
B42. Hockly, E., V.M. Richon, B. Woodman, D.L. Smith, X. Zhou, E. Rosa, K. Sathasivam, S. Ghazi-Noori, A. Mahal, P.A. Lowden, J.S. Steffan, J.L. Marsh, L.M. Thompson, C.M. Lewis, P.A. Marks, and G.P. Bates, Suberoylanilide hydroxamic acid, a histone deacetylase inhibitor, ameliorates motor deficits in a mouse model of Huntington's disease. Proc Natl Acad Sci U S A, 2003. 100(4): p. 2041-6.
B43. Guan, J.S., S.J. Haggarty, E. Giacometti, J.H. Dannenberg, N. Joseph, J. Gao, T.J. Nieland, Y. Zhou, X. Wang, R. Mazitschek, J.E. Bradner, R.A. DePinho, R. Jaenisch, and L.H. Tsai, HDAC2 negatively regulates memory formation and synaptic plasticity. Nature, 2009. 459(7243): p. 55-60.
B44. Dineley, K.T., M. Westerman, D. Bui, K. Bell, K.H. Ashe, and J.D. Sweatt, Beta-amyloid activates the mitogen-activated protein kinase cascade via hippocampal alpha7 nicotinic acetylcholine receptors: In vitro and in vivo mechanisms related to Alzheimer's disease. J Neurosci, 2001. 21(12): p. 4125-33.
B45. Dineley, K.T., X. Xia, D. Bui, J.D. Sweatt, and H. Zheng, Accelerated plaque accumulation, associative learning deficits, and up-regulation of alpha 7 nicotinic receptor protein in transgenic mice co-expressing mutant human presenilin 1 and amyloid precursor proteins. J Biol Chem, 2002. 277(25): p. 22768-80.
B46. Gong, B., O.V. Vitolo, F. Trinchese, S. Liu, M. Shelanski, and O. Arancio, Persistent improvement in synaptic and cognitive functions in an Alzheimer mouse model after rolipram treatment. J Clin Invest, 2004. 114(11): p. 1624-34.
B47. Francis, Y.I., J.K. Diss, M. Kariti, A. Stephanou, and D.S. Latchman, p300 activation by Presenilin 1 but not by its M146L mutant. Neurosci Lett, 2007. 413(2): p. 137-40.
B48. Rouaux, C., N. Jokic, C. Mbebi, S. Boutillier, J.P. Loeffler, and A.L. Boutillier, Critical loss of CBP/p300 histone acetylase activity by caspase-6 during neurodegeneration. Embo J, 2003. 22(24): p. 6537-49.
B49. Green, K.N., et al., Nicotinamide restores cognition in Alzheimer's disease transgenic mice via a mechanism involving sirtuin inhibition and selective reduction of Thr231-phosphotau. J Neurosci, 2008. 28(45): p. 11500-10.
B50. Trinchese, F., et al., Progressive age-related development of Alzheimer-like pathology in APP/PS1 mice. Ann Neurol, 2004. 55(6): p. 801-14.
B51. Sperling, R.A., et al., fMRI studies of associative encoding in young and elderly controls and mild Alzheimer's disease. J Neurol Neurosurg Psychiatry, 2003. 74(1): p. 44-50.
B52. Phillips, R.G. and J.E. LeDoux, Differential contribution of amygdala and hippocampus to cued and contextual fear conditioning. Behav Neurosci, 1992. 106(2): p. 274-85.
B53. Vecsey, C.G., et al., Histone deacetylase inhibitors enhance memory and synaptic plasticity via CREB:CBP-dependent transcriptional activation. J Neurosci, 2007. 27(23): p. 6128-40.
B54. Malm, T., et al., beta-Amyloid infusion results in delayed and age-dependent learning deficits without role of inflammation or beta-amyloid deposits. Proc Natl Acad Sci U S A, 2006. 103(23): p. 8852-7.
B55. Cleary, J.P., et al., Natural oligomers of the amyloid-beta protein specifically disrupt cognitive function. Nat Neurosci, 2005. 8(1): p. 79-84.
B56. Bordoli, L., et al., Plant orthologs of p300/CBP: conservation of a core domain in metazoan p300/CBP acetyltransferase-related proteins. Nucleic Acids Res, 2001. 29(3): p. 589-97.
B57. Masliah, E., Mechanisms of synaptic dysfunction in Alzheimer's disease. Histol Histopathol, 1995. 10(2): p. 509-19.
B58. Alamed, J., D.M. Wilcock, D.M. Diamond, M.N. Gordon, and D. Morgan, Two-day radial-arm water maze learning and memory task; robust resolution of amyloid-related memory deficits in transgenic mice. Nat Protoc, 2006. 1(4): p. 1671-9.
B59. Eyupoglu, I.Y., et al., Experimental therapy of malignant gliomas using the inhibitor of histone deacetylase MS-275. Mol Cancer Ther, 2006. 5(5): p. 1248-55.
B60. Kim, D., et al., SIRT1 deacetylase protects against neurodegeneration in models for Alzheimer's disease and amyotrophic lateral sclerosis. EMBO J, 2007. 26(13): p. 3169-79.
B61. Mantelingu, K., et al., Activation ofp300 histone acetyltransferase by small molecules altering enzyme structure: probed by surface-enhanced Raman spectroscopy. J Phys Chem B, 2007. 111(17): p. 4527-34.
B62. Paxinos, G., Mouse brain in stereotaxic coordinates. 2nd ed. 1998, New York: Academic Press.
B63. Stine, W.B., et al., In vitro characterization of conditions for amyloid-beta peptide oligomerization and fibrillogenesis. J Biol Chem, 2003. 278(13): p. 11612-22.
B64. Puzzo, D., et al., Picomolar Amyloid-{beta} Positively Modulates Synaptic Plasticity and Memory in Hippocampus. J. Neurosci., 2008. 28(53): p. 14537-14545.
B65. Garcia, B.A., et al., Chemical derivatization of histones for facilitated analysis by mass spectrometry. Nat Protoc, 2007. 2(4): p. 933-8.

### Example 7: Cell Viability Assays for ACHN, U251, NCI-ADR-RES, A549, Hs578T, CCRF-CEM

### Assay Parameters

Read-out: Cell Proliferation by Cell-TiterGlo, and Cyquant,
Number of Compounds: 1 (YF2)
Concentrations: 10 (0.03, 0.1, 0.25, 0.5, 1, 2.5, 5, 15, 40 and 80 µM)
Reference Compound: Vinblastine (VBL)
Concentrations: 10
(0.001, 0.003, 0.01, 0.03, 0.1, 0.3, 1, 3, and 10 µM)
Time of Drug Treatment: 72 hours
Replicates: 3
Solvent: Aqueous medium

### Materials and Methods

**Cells and culture medium:** All cell lines were purchased from the ATCC and were expanded and archived under liquid nitrogen at CDAS as low passage aliquots. Cells were maintained and passaged in recommended and optimal culture medium (**ACHN**: EMEM, 2 mM L-Gln, 10% FBS; **A549**: Ham's F12, 10% FBS; **U251**: RPMI 1640,2 mM L-Gln, 10% FBS; **Hs578T**: DMEM, 4 mM L-Gln, 1 U/mL of Bovine Insulin, 10% FBS; **CCRF-CEM**: ATCC RPMI, 2 mM L-Gln, 10% FBS; **NCI-ADR-RES**: RPMI 1640, 2 mM L-Gln, 10% FBS). All experiments were carried out with cells which had undergone less than 20 passages. Optimal seed densities were determined for all cell lines. All cells were plated at 1500 cells per well except CCRF-CEM which was plated at 6000 cells per well.

**Drugs:** YF2 was supplied as a 80 mM stock solution in 100% DMSO. Vinblastine was purchased from Sigma (Catalogue Number V-1377) and resuspended at 1 X 10⁻² M in 100% DMSO. All dilutions for both drugs were carried out in culture medium containing 0.2% DMSO such that the final solvent concentration never exceeded 0.1 %.

**Drug treatment:** YF2 tested at 10 concentrations (0.03, 0.1, 0.25, 0.5, 1,2.5, 5, 15,40 and 80 µM) in triplicate wells. Vinblastine was used as a reference control and tested at 10 concentrations in a half-log series (0, 0.001, 0.003, 0.01, 0.03, 0.1, 0.3,1,3, and 10 µM). Cells were resuspended in medium at the appropriate concentration and 180 µl (1500 or 6000 cells) was added to each well following which 20 µl of drug at lOx of the final concentration was added to attain the desired drug concentration in every well. The drug treatment plates were incubated at 37°C for 72 hours, following which cell viability was assayed by the Cell Titer Glo or Cyquant method as described below.

**Cell Titer Glo Assay:** Following the 72 hour drug treatment period, the assay plates were centrifuged, and 100 µL of the medium was aspirated and replaced with 100 J.lL of Cell Titer Glo reagent (Promega) according to the manufacturer's recommended protocol. The reagent was mixed with the cells and the luminescence measured using a Perkin Elmer Envision instrument. The average luminescence signal obtained from wells containing untreated cells which had been incubated for the entire length of the assay period was used to set the 100% viability value. The percent proliferation was calculated as (Test signal)/(Avg. plate background signal) x 100. The % viability was graphed against drug concentration to calculate an IC₅₀ for each drug.

**Cyquant Assay:** Following the 72 hour drug treatment period the assay plates were centrifuged, the medium discarded, and frozen overnight. The plates were assayed using the Cyquant™ reagent (Invitrogen) according to the manufacturer's recommended protocol. The average fluorescence signal obtained from wells containing untreated cells which had been incubated for the entire length of the assay period was used to set the 100% proliferation value. The percent proliferation was calculated as (Test signal)/(Avg. plate background signal) x 100. The % proliferation was graphed against drug concentration to calculate an IC₅₀ for each drug.

### Example 8: YF2 Increases Histone Acetylation by HAT Activation, Not HDAC Inhibition

HDAC inhibition causes an increase in histone acetylation. The inventors examined whether histone acetylation occurred via HDAC inhibition.

The summary of the results is depicted in Table 1. The mean IC₅₀ values of the compounds are summarized in Table 1.

**Table 1. Inhibitory Effects of the Compounds on HDAC Activities**

| **HDACs** | **IC₅₀ (nM) or % Inhibition** | | |
|---|---|---|---|
| | | **YF2** | **SAHA** |
| HDAC1 | | >200µM | 31 |
| HDAC3/NCOR2 | | >200µM | 38 |
| HDAC5FL | | >200µM | >10µM |
| HDAC6 | | >200µM | 30 |
| HADC7 | | >200µM | >10µM |
| HDAC8 | | >200µM | 2,236 |
| HDAC10 | | >200µM | 65 |
| HDAC11 | | >200µM | >10µM |
| Sirtuin 1 | | >200µM | >10µM |
| Sirtuin 2 | | >200µM | >10µM |

The experiments were done blind, where the YF2 compound of the invention corresponds to OA2. The studies show that YF2 has no HDAC inhibition properties. YF2 does not inhibit HDACs.

### Materials and Methods

### Materials:

HDAC Assay Buffer (BPS catalog number 50031)
HDAC Assay Developer (BPS catalog number 50030)
HDAC Substrate 1 (BPS number 50032)
HDAC Substrate 3 (BPS number 50037)
HDAC Class 2a Substrate 1 (BPS number 50040)
SAHA (Cayman Chemical, Ann Arbor, MI, Catalog number 10009929)

**Table 2. Compounds used in the studies**

| Compound I.D. | Compound Supplied | Stock Conc. | Dissolving Solvent | Test Range (nM) | Intermediate Dilution |
|---|---|---|---|---|---|
| OA2* | Solution | 10mM | DMSO | 3 - 200,000 | 10% DMSO in HDAC Assay Buffer |
| SAHA | Powder | 10mM | DMSO | 0.3 - 10,000 | 10% DMSO in HDAC Assay Buffer |

| | | | | | |
|---|---|---|---|---|---|
| *Compound OA2 is cloudy at 2mM in 10% DMSO (The highest test point). **SAHA, and HDACi, is a positive control for HDACs. | | | | | |

### Experimental Conditions

**Table 3. Enzymes and Substrates**

| Assay | Catalog # | Enzyme Used (ng) / Reaction | Substrate |
|---|---|---|---|
| HDAC1 | 50051 | 1.5 | 10 µM of 50037 |
| HADC3/NCOR2 | 50003 | 1.33 | 10 µM of 50037 |
| HDAC5FL | 50045 | 1.25 | 2 µM of 50040 |
| HDAC6 | 50006 | 10 | 10 µM of 50037 |
| HDAC7 | 50007 | 0.3 | 2 µM of 50040 |
| HDAC8 | 50008 | 20 | 2 µM of 50040 |
| HDAC10 | 50010 | 1,300 | 10 µM of 50037 |
| HADC11 | 50011 | 400 | 2 µM of 50040 |
| Sirtuin 1 | 50012 | 400 | 10 µM of 50032 |
| Sirtuin 2 | 50013 | 5,600 | 10 µM of 50032 |

**Assay Conditions.** A series of dilution of the test compounds were prepared with 10% DMSO in assay buffer and 5µl of the dilution was added to a 50µl reaction so that the final concentration of DMSO is 1% in all of reactions. All of the enzymatic reactions were conducted in duplicate at 37°C for 30 minutes except of HDAC11 at room temperature for 3 hours. The 50µl reaction mixture contains HDAC assay buffer, 5µg BSA, an HDAC substrate, an HDAC enzyme and a test compound. After enzymatic reactions, 50µl of HDAC Developer was added to each well and the plate was incubated at room temperature for an additional 20 minutes. Fluorescence intensity was measured at an excitation of 360 nm and an emission of 460 nm using a Tecan Infinite M1000 microplate reader.

**Data Analysis.** HDAC activity assays were performed in duplicates at each concentration. The fluorescent intensity data were analyzed using the computer software, Graphpad Prism. In the absence of the compound, the fluorescent intensity (Fₜ) in each data set was defined as 100% activity. In the absence of HDAC, the fluorescent intensity (F_{b}) in each data set was defined as 0% activity. Compound OA2 has fluorescence at assay condition; therefore the fluorescent intensity at different concentration of OA2 was defined as background (Fb). The percent activity in the presence of each compound was calculated according to the following equation: %activity = (F-F_{b})/(Fₜ-F_{b}), where F= the fluorescent intensity in the presence of the compound.

The values of % activity versus a series of compound concentrations were then plotted using non-linear regression analysis of Sigmoidal dose-response curve generated with the equation Y=B+(T-B)/1+10^{((LogEC50-X)×Hill Slope)}, where Y=percent activity, B=minimum percent activity, T=maximum percent activity, X= logarithm of compound and Hill Slope=slope factor or Hill coefficient. The IC₅₀ value was determined by the concentration causing a half-maximal percent activity.

### Results of Effect of OA2 (YF2 Compound) on HDAC inhibition

**Table 4. HDAC1 Assay - Data for the Effect of OA2 on HDAC1 Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat1 | Repeat2 |
| No CPD | 17721 | 17257 | 796 | 803 | 101.39 | 98.61 |
| 0.5 | 17287 | 17200 | 796 | 798 | 98.80 | 98.28 |
| 1.0 | 17083 | 17178 | 788 | 786 | 97.64 | 98.21 |
| 1.5 | 16949 | 17020 | 830 | 784 | 96.72 | 97.14 |
| 2.0 | 16879 | 16826 | 796 | 779 | 96.42 | 96.10 |
| 2.5 | 16792 | 17072 | 827 | 775 | 95.81 | 97.49 |
| 3.0 | 16943 | 16784 | 829 | 802 | 96.63 | 95.68 |
| 3.5 | 16387 | 17135 | 866 | 827 | 93.12 | 97.60 |
| 4.0 | 16140 | 16336 | 920 | 868 | 91.35 | 92.53 |
| 4.5 | 16432 | 16128 | 1117 | 1035 | 92.01 | 90.19 |
| 5.3 | 24780 | 24451 | 14884 | 13403 | 63.73 | 61.76 |

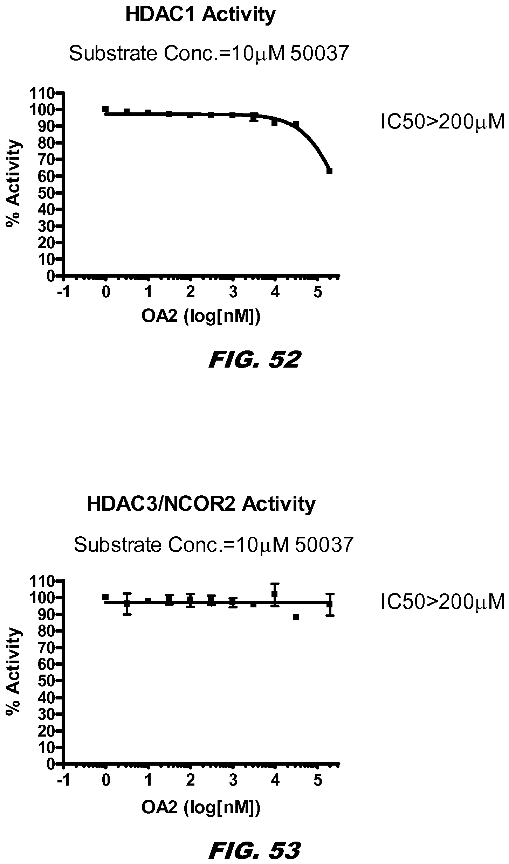

**FIG. 52** corresponds to the results shown in Table 4.

**Table 5. HDAC3/NCOR2 Assay - Data for the Effect of OA2 on HDAC3/NCOR2 Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat1 | Repeat2 |
| No CPD | 10787 | 10452 | 805 | 828 | 101.71 | 98.29 |
| 0.5 | 10928 | 9694 | 813 | 976 | 102.35 | 89.76 |
| 1.0 | 10423 | 10379 | 812 | 818 | 98.01 | 97.56 |
| 1.5 | 10752 | 10231 | 813 | 803 | 101.44 | 96.12 |
| 2.0 | 10827 | 10078 | 809 | 798 | 102.25 | 94.61 |
| 2.5 | 10718 | 10173 | 818 | 803 | 101.07 | 95.51 |
| 3.0 | 10587 | 10073 | 831 | 811 | 99.62 | 94.38 |
| 3.5 | 10362 | 10080 | 854 | 824 | 97.14 | 94.27 |
| 4.0 | 11530 | 10216 | 927 | 898 | 108.31 | 94.90 |
| 4.5 | 9872 | 10001 | 1467 | 1091 | 87.66 | 88.97 |
| 5.3 | 20905 | 22163 | 13408 | 10875 | 89.40 | 102.23 |

**FIG. 53** corresponds to the results shown in Table 5.

**Table 6. HDAC5FL Assay - Data for the Effect of OA2 on HDAC5FL Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat1 | Repeat2 |
| No CPD | 4492 | 4892 | 345 | 348 | 95.40 | 104.60 |
| 0.5 | 4686 | 4386 | 355 | 343 | 99.80 | 92.90 |
| 1.0 | 4802 | 4581 | 341 | 347 | 102.59 | 97.50 |
| 1.5 | 4835 | 4874 | 359 | 342 | 103.20 | 104.10 |
| 2.0 | 5071 | 4991 | 344 | 356 | 108.64 | 106.80 |
| 2.5 | 5068 | 5006 | 344 | 354 | 108.60 | 107.17 |
| 3.0 | 4944 | 4685 | 342 | 354 | 105.76 | 99.80 |
| 3.5 | 4773 | 4686 | 353 | 389 | 101.30 | 99.30 |
| 4.0 | 4987 | 4983 | 449 | 407 | 104.91 | 104.82 |
| 4.5 | 4570 | 4514 | 451 | 398 | 95.40 | 94.11 |
| 5.3 | 9875 | 10983 | 7907 | 5878 | 68.63 | 94.13 |

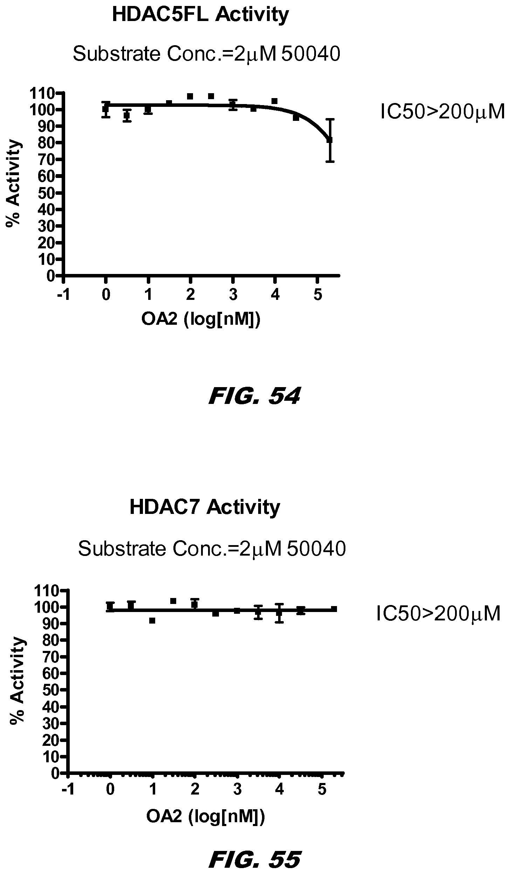

**FIG. 54** corresponds to the results shown in Table 6.

**Table 7. HDAC7 Assay - Data for the Effect of OA2 on HDAC7 Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat 1 | Repeat2 |
| No CPD | 7528 | 7176 | 382 | 377 | 102.52 | 97.48 |
| 0.5 | 7578 | 7200 | 394 | 383 | 103.11 | 97.69 |
| 1.0 | 6756 | 6763 | 385 | 386 | 91.37 | 91.47 |
| 1.5 | 7471 | 7705 | 389 | 381 | 101.63 | 104.98 |
| 2.0 | 7679 | 7196 | 390 | 380 | 104.61 | 97.68 |
| 2.5 | 7071 | 7068 | 385 | 398 | 95.80 | 95.75 |
| 3.0 | 7083 | 7269 | 384 | 392 | 96.02 | 98.69 |
| 3.5 | 7453 | 6898 | 397 | 462 | 100.73 | 92.77 |
| 4.0 | 6801 | 7568 | 416 | 534 | 90.73 | 101.73 |
| 4.5 | 7238 | 7518 | 554 | 565 | 95.78 | 99.80 |
| 5.3 | 9692 | 9912 | 3002 | 2871 | 96.89 | 100.04 |

**FIG. 55** corresponds to the results shown in Table 7.

**Table 8. HDAC8 Assay - Data for the Effect of OA2 on HDAC8 Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat1 | Repeat2 |
| No CPD | 3492 | 3483 | 346 | 346 | 100.14 | 99.86 |
| 0.5 | 3541 | 3581 | 339 | 342 | 101.88 | 103.15 |
| 1.0 | 3519 | 3391 | 349 | 342 | 101.02 | 96.94 |
| 1.5 | 3539 | 3456 | 336 | 331 | 102.04 | 99.40 |
| 2.0 | 3757 | 3425 | 338 | 340 | 108.80 | 98.23 |
| 2.5 | 3451 | 3428 | 335 | 341 | 99.09 | 98.36 |
| 3.0 | 3398 | 2995 | 337 | 347 | 97.28 | 84.45 |
| 3.5 | 3808 | 3407 | 346 | 366 | 109.88 | 97.12 |
| 4.0 | 3361 | 3365 | 433 | 374 | 94.14 | 94.27 |
| 4.5 | 3045 | 3090 | 375 | 364 | 85.17 | 86.60 |
| 5.3 | 6631 | 8117 | 4962 | 4635 | 58.33 | 105.63 |

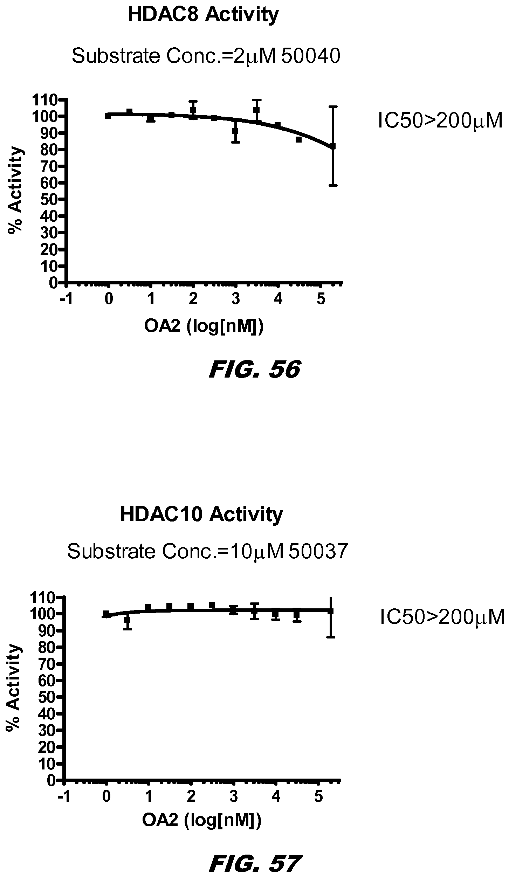

**FIG. 56** corresponds to the results shown in Table 8.

**Table 9. HDAC10 Assay - Data for the Effect of OA2 on HDAC10 Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat1 | Repeat2 |
| No CPD | 11695 | 12141 | 497 | 507 | 98.05 | 101.95 |
| 0.5 | 10894 | 12032 | 492 | 501 | 91.08 | 101.05 |
| 1.0 | 12341 | 12402 | 497 | 492 | 103.77 | 104.31 |
| 1.5 | 12564 | 12368 | 525 | 500 | 105.57 | 103.85 |
| 2.0 | 12262 | 12573 | 500 | 497 | 103.04 | 105.77 |
| 2.5 | 12472 | 12556 | 500 | 493 | 104.90 | 105.64 |
| 3.0 | 11935 | 12471 | 530 | 521 | 99.94 | 104.64 |
| 3.5 | 11622 | 12684 | 501 | 607 | 96.95 | 106.25 |
| 4.0 | 11588 | 12318 | 597 | 547 | 96.50 | 102.89 |
| 4.5 | 11448 | 12305 | 623 | 495 | 95.38 | 102.89 |
| 5.3 | 25769 | 22285 | 12210 | 12714 | 116.56 | 86.05 |

**FIG. 57** corresponds to the results shown in Table 9.

**Table 10. HDAC 11 Assay - Data for the Effect of OA2 on HDAC 11 Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat 1 | Repeat2 |
| No CPD | 2840 | 2860 | 426 | 406 | 99.59 | 100.41 |
| 0.5 | 2761 | 2530 | 411 | 423 | 96.30 | 86.81 |
| 1.0 | 2828 | 2898 | 425 | 415 | 98.93 | 101.81 |
| 1.5 | 2765 | 2851 | 411 | 406 | 96.82 | 100.35 |
| 2.0 | 2812 | 2864 | 408 | 409 | 98.75 | 100.88 |
| 2.5 | 2672 | 2655 | 412 | 408 | 92.93 | 92.24 |
| 3.0 | 2829 | 2806 | 417 | 424 | 98.95 | 98.01 |
| 3.5 | 2719 | 2712 | 427 | 463 | 93.43 | 93.14 |
| 4.0 | 2835 | 2860 | 467 | 524 | 96.12 | 97.14 |
| 4.5 | 3289 | 3064 | 699 | 617 | 108.09 | 98.85 |
| 5.3 | 6249 | 5842 | 2911 | 3158 | 132.07 | 115.35 |

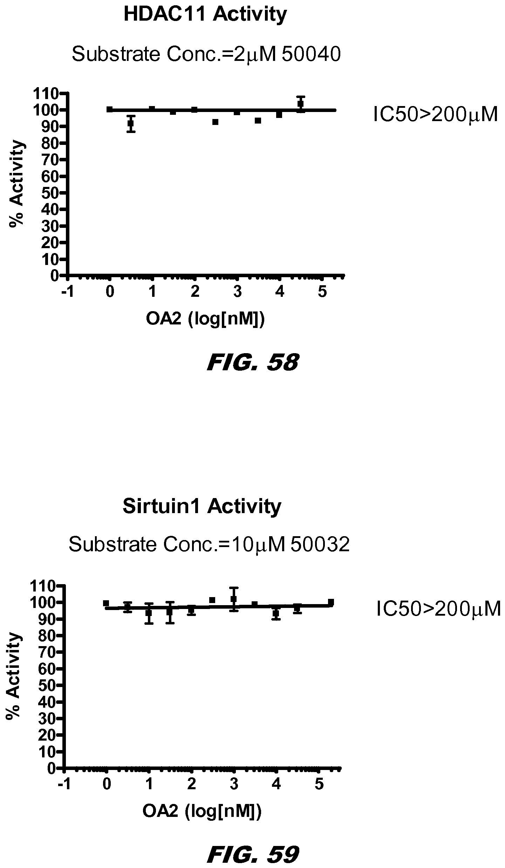

**FIG. 58** corresponds to the results shown in Table 10.

**Table 11. Sirtuin 1 Assay - Data for the Effect of OA2 on Sirtuin 1 Activity**

| OA2 (Log [nM]) | HDAC Activity (Fluorescence count) | | Background (Fluorescence count) | | % Activity | |
|---|---|---|---|---|---|---|
| | Repeat1 | Repeat2 | Repeat1 | Repeat2 | Repeat1 | Repeat2 |
| No CPD | 5823 | 5974 | 412 | 410 | 97.91 | 100.64 |
| 0.5 | 5627 | 5940 | 414 | 420 | 94.26 | 99.92 |
| 1.0 | 5240 | 5913 | 422 | 413 | 87.25 | 99.42 |
| 1.5 | 5980 | 5273 | 418 | 457 | 100.27 | 87.48 |
| 2.0 | 5827 | 5527 | 411 | 411 | 97.98 | 92.56 |
| 2.5 | 6028 | 5987 | 413 | 416 | 101.56 | 100.81 |
| 3.0 | 6454 | 5681 | 422 | 452 | 108.86 | 94.87 |
| 3.5 | 5782 | 5964 | 422 | 426 | 96.93 | 100.23 |
| 4.0 | 5786 | 5408 | 442 | 441 | 96.69 | 89.85 |
| 4.5 | 5976 | 5697 | 502 | 524 | 98.83 | 93.79 |
| 5.3 | 7483 | 7591 | 2022 | 1997 | 99.02 | 100.98 |

**FIG. 59** corresponds to the results shown in Table 11.

**Table 12. Sirtuin 2 Assay - Data for the Effect of OA2 on Sirtuin 2 Activity**

| OA2 (Log [nM]) | HDAC Activity (Fluorescence count) | | Background (Fluorescence count) | | % Activity | |
|---|---|---|---|---|---|---|
| | Repeat 1 | Repeat2 | Repeat1 | Repeat2 | Repeat 1 | Repeat2 |
| No CPD | 3910 | 3919 | 413 | 419 | 99.87 | 100.13 |
| 0.5 | 3835 | 3981 | 420 | 413 | 97.71 | 101.89 |
| 1.0 | 3780 | 3821 | 406 | 422 | 96.21 | 97.38 |
| 1.5 | 3858 | 3954 | 408 | 410 | 98.59 | 101.33 |
| 2.0 | 3712 | 3912 | 420 | 413 | 94.20 | 99.91 |
| 2.5 | 3729 | 3788 | 409 | 420 | 94.74 | 96.43 |
| 3.0 | 3714 | 3861 | 405 | 409 | 94.53 | 98.73 |
| 3.5 | 3806 | 3856 | 422 | 417 | 96.80 | 98.23 |
| 4.0 | 3844 | 3883 | 425 | 426 | 97.71 | 98.83 |
| 4.5 | 3717 | 3811 | 485 | 480 | 92.45 | 95.14 |
| 5.3 | 5686 | 5717 | 2225 | 2245 | 98.64 | 99.53 |

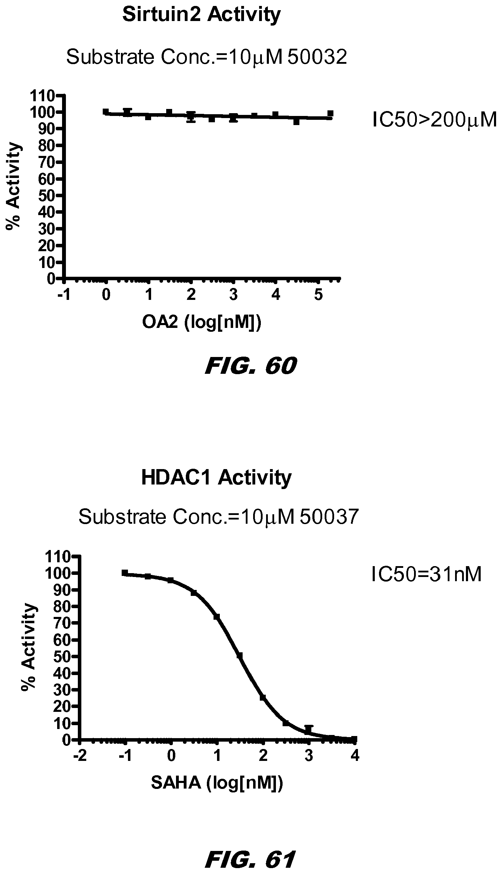

**FIG. 60** corresponds to the results shown in Table 12.

**Table 13. HDAC6 Assay - Data for the Effect of OA2 on HDAC6 Activity**

| OA2 (Log [nM]) | HDAC Activity (Fluorescence count) | | Background (Fluorescence count) | | % Activity | |
|---|---|---|---|---|---|---|
| | Repeat 1 | Repeat2 | Repeat 1 | Repeat2 | Repeat1 | Repeat2 |
| No CPD | 5844 | 5616 | 773 | 733 | 102.29 | 97.71 |
| 0.5 | 5998 | 6074 | 832 | 737 | 104.75 | 106.28 |
| 1.0 | 6006 | 5728 | 747 | 704 | 106.10 | 100_{.}51 |
| 1.5 | 5541 | 6126 | 746 | 706 | 96.75 | 108.50 |
| 2.0 | 5733 | 5981 | 748 | 731 | 100.33 | 105.31 |
| 2_{.}5 | 5678 | 5677 | 763 | 709 | 99.30 | 99.28 |
| 3.0 | 5717 | 5446 | 758 | 716 | 100.06 | 94.62 |
| 3.5 | 5575 | 5616 | 781 | 735 | 96.79 | 97.61 |
| 4.0 | 5516 | 5789 | 828 | 786 | 94.62 | 100.10 |
| 4.5 | 4994 | 5418 | 1081 | 1030 | 79.13 | 87.65 |
| 5.3 | 8327 | 9938 | 4925 | 4721 | 70.40 | 102.77 |

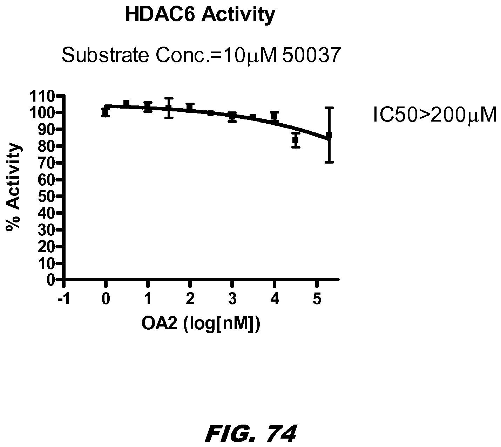

**FIG. 74** corresponds to the results shown in Table 13.

### Results of Effect of SAHA on HDAC inhibition

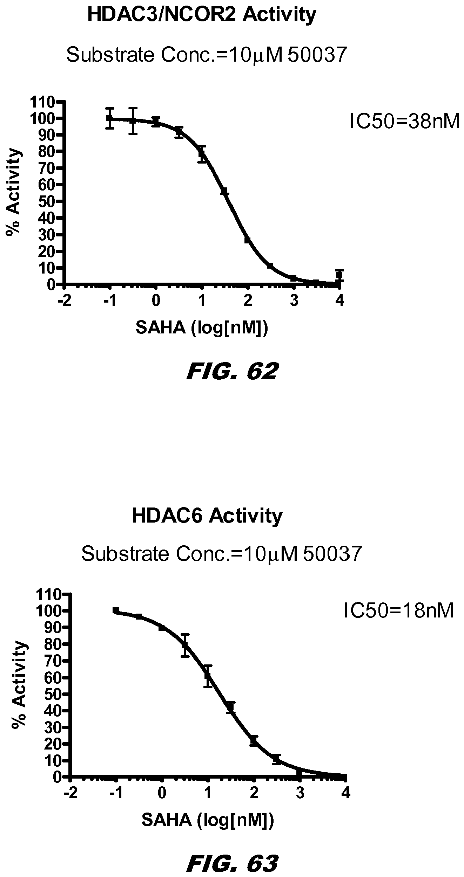

SAHA is an HDAC inhibitor (HDACi). It serves as a positive control for HDACs. **FIGS. 61-63** show the inhibitory effect of SAHA on the HDACs HDAC1, HDAC3/NCOR2, and HDAC6. SAHA also inhibited HDAC5FL, HDAC7, HDAC8, HDAC10, Sirtuin 1, and Sirtuin 2 (see Table 1).

### Example 9: Design of Selective HAT activators

Early amnesic changes in Alzheimer's disease (AD) are thought to be linked to synaptic dysfunction. In this regard, β-amyloid (Aβ), a peptide that is produced in elevated amounts in the disease, has been found to inhibit memory^{[1,2]} and its electrophysiological model, long-term potentiation (LTP)^{[3-8]}. Since memory is modulated by epigenetics through regulation of gene expression, deregulation of one of the epigenetic mechanisms such as histone (H) acetylation, might lead to memory disruption. Reduction of histone acetylation causes the chromatin structure to close, so that the information contained within the DNA might be less amenable to transcription factors and memory formation^{[9]}.

The main strategy that is currently used to up-regulate histone acetylation involves HDAC inhibitors. However, the pleiotropic effect of nonspecific HDAC inhibition may hamper their therapeutic potential ^{[10-13]}. We have demonstrated that hippocampal levels of two HATs, CBP and PCAF, are reduced following Aβ elevation. We have therefore focused towards another target which also up-regulates histone acetylation, the HATs. To this end we have synthesized a HAT activator, YF2. We propose a course of investigation involving the design of selective HAT activators that target specifically CBP and PCAF downstream of Aβ. The aims of our proposal are:
**1)** To design and synthesize new HAT activators which are optimized for AD;.
**2)** To identify compounds with high affinity and good selectivity for selective HAT activators that target specifically CBP and PCAF;
**3)** To determine if new HAT activators have good pharmacokinetic (PK) profile and are safe;
**4)** To select HAT activators that rescue synaptic dysfunction in APP/PS1 mice;
**5)** Further screen HAT activators to determine if they are beneficial against cognitive abnormalities in APP/PS 1 mice.

**BACKGROUND AND SIGNIFICANCE:** The post-translational acetylation status of chromatin is governed by the competing activities of two classes of enzymes, HATs and HDACs. HDAC inhibitors have been shown to enhance LTP and contextual fear memory, a form of associative memory in which animals must associate a neutral stimulus with an aversive one^{[P17]}. Also, memory and LTP deficits of CBP^{+/-} mice were reversed by HDAC inhibition^{[P15]}. The potential of inhibiting HDACs to counteract neurodegenerative disorders has been widely explored^{[14]}. For instance, in a set of experiments, Tsai et al. have reported that HDAC inhibitors induced sprouting of dendrites, increased number of synapses, and reinstated learning and access to long-term memories in the CK-p25 Tg mouse model of neurodegeneration^{[15,16]}. Moreover, in recent studies we have shown that the HDAC inhibitor TSA ameliorates LTP and contextual fear conditioning (FC) in the double Tg APP(K670M:N671L)/PS1(M146L, line 6.2) (APP/PS1) mouse model of amyloid deposition^{[17]}. Bolden et al., (2006, Nature Reviews Drug Discovery, 5:769-84) describe the histone deacetylases family, the reference which is incorporated by reference in its entirety. HATs, however, have been investigated to a lesser extent.

HATs can be divided in two main groups, the nuclear HATs and cytoplasmic HATs^{[18]}. Nuclear A-type HATs can be grouped into at least 4 different families based on sequence conservation within the HAT domain: Gcn5 and p300/CBP associated factor (PCAF), MYST (MOZ, Ybf2/ Sas3, Sas2 and Tip60), p300 and CBP (named for the two human paralogs p300 and CBP) and Rtt109. While the Gcn5/PCAF and MYST families have homologs from yeast to man, p300/CBP is metazoan-specific, and Rtt109 is fungal-specific. Cytoplasmic B-type HATs, such as HAT1, are involved in histone deposition^{[P22]}. Marmorstein and Roth (2001, Curr Opin in Genet and Develop., 11:155-161) list in Table 1 the HAT families and their transcriptional-related functions, the reference which is incorporated by reference in its entirety.

Although other nuclear HAT families have been described, such as the steroid receptor coactivators, TAF250, ATF-2, and CLOCK, their HAT activities have not been investigated as extensively as the major HAT classes^{[18]}. These 4 families show high sequence similarity within families but poor to no sequence similarity between families. Furthermore, the size of the HAT domain of the different families is different^{[P22]}. Interestingly, HATs are highly conserved in mammals^{[P22]}. Of all these HATs, three were shown to be involved in memory: CBP, p300^{[19, 20]}, and PCAF^{[21]}. Interestingly, both CBP and PCAF levels are reduced by Aβ elevation.

HAT activators are a viable approach to enhance histone acetylation. Two scaffolds for HAT activators have been identified. The first one includes CTPB and its derivative CTB^{[22, 23]}. The second one includes only one compound, nemorosone^{[24]}. CTPB/CTB were found to be insoluble and membrane-impermeable^{[22, 23]}. Moreover, CTPB has unfavorable characteristics to be used in CNS diseases (MW equal to 553,29, clogP equal to 12.70) and the clogP of CTB is 5.13. Nemorosone has a MW of 502 and a clogP of 8.42. In summary, ample evidence exists supporting a promising therapeutic role for HAT activators in the treatment of CNS disorders including AD. We will exploit this possibility by developing new chemical entities targeting specifically CBP and PCAF.

### RESEARCH DESIGN AND METHODS:

**1) To design and synthesize new HAT activators which are optimized for AD.** We have designed a new compound based on SAR studies of the CTPB/CTB scaffold, named YF2. YF2 (MW 430.13, clogP 5.15, clogBB 0.17) has increased solubility, membrane permeability and blood-brain barrier permeability, is safe in acute toxicity tests. YF2 rescued the reduction in hippocampal levels of H3 acetylation following Aβ elevation, enhanced enzymatic activity of PCAF, CBP and Gcn5 as well as p300, had excellent selectivity for PCAF, CBP and Gcn5 over HDACs, and was capable of rescuing deficits in fear and reference memory induced by Aβ exposure. We are refining its druggability using a medicinal chemistry (med-chem) approach, focusing on the synthesis of drug-like lead compounds with good PK and ADMET profile. We will design and synthesize HAT activators bearing different moieties at different positions of one of the two aromatic rings of YF2. In particular, we will substitute a dimethylamino group which is likely to be alkylated by microsomes and protect an aromatic ring from oxidative reactions. Additionally, we will explore the HAT binding site i) by evaluating the importance of the amide group between the two aromatic rings, ii) by constraining the molecular structure, iii) by modifying the distance between two aromatic rings that currently form YF2, iv) by replacing one of the two aromatic rings with an eterocyclic ring, and v) by increasing the hindrance of the substituents of one of the two aromatic rings. We will find HAT activators with a) HAT specificity and potency, b) great CNS penetration, and c) safety.
**2) To identify compounds with high affinity and good selectivity for selective HAT activators that target specifically CBP and PCAF.** Following assessment of HAT activator potency (EC₅₀), we will check selectivity with respect to CBP and PCAF. For HAT activators found to show good potency (< 100nM), selectivity (at least 50-fold vs p300, Gcn5, MYST families and HDACs) and solubility, we will use a functional assay to determine if they increase hippocampal H3 and H4 acetylation in adult mice.
**3) To determine whether new HAT activators have good pharmacokinetic (PK) profile and are safe.** Selected HAT activators will be screened against unfavorable PK properties including bioavailability, brain uptake, and BBB penetration. Compounds passing these tests will be evaluated for rudimentary ADMET characteristics including tests of acute and chronic toxicity.
**4) To select HAT activators that rescue LTP in APP/PS1 mice.** We will determine if selected HAT activators ameliorate LTP defect in APP/PS 1 slices using electrophysiological techniques^{[25]}
**5) Further screen HAT activators to examine if they ameliorate cognitive abnormalities in APP/PS1 mice.** We will determine if HAT activators screened in slices can protect APP/PS1 mice against impairments of contextual and reference memory using behavioral assays^{[25]}. Next, we will characterize our compounds for their more global selectivity against targets other than HATs. We will also screen them with a battery of assays focusing on two areas that have resulted in the withdrawal of many drugs from the market: drug-drug interactions (liver metabolism), hERG channel blockage (cardiac dysfunction).

### YF2, ALZHEIMER'S DISEASE, AND DRUG DISCOVERY RESEARCH:

Currently used AD therapies have limited efficacy. Major efforts are underway to inhibit tangle formation, to combat inflammation and oxidative damage, and to decrease Aβ load in the brain^{[26-28]}. However, the role of APP, Aβ, and the secretases in normal physiological function^{[29-31]} might present a problem in providing effective and safe approaches to AD therapy. Developing agents that interact with Aβ targets that lead to neuronal dysfunction is another approach that is currently tested by many laboratories. Without being bound by theory, **HAT activators represent a new class of compounds that might effectively counteract the disease progression.**

### REFERENCES

1. Malm, T., et al., beta-Amyloid infusion results in delayed and age-dependent learning deficits without role of inflammation or beta-amyloid deposits. Proc Natl Acad Sci U S A, 2006. 103(23): p. 8852-7.
2. Cleary, J.P., et al., Natural oligomers of the amyloid-beta protein specifically disrupt cognitive function. Nat Neurosci, 2005. 8(1):p. 79-84.
3. Cullen, W.K., et al., Block of LTP in rat hippocampus in vivo by beta-amyloid precursor protein fragments. Neuroreport, 1997. 8(15): p. 3213-7.
4. Itoh, A., et al., Impairments of long-term potentiation in hippocampal slices of beta-amyloid-infused rats. Eur J Pharmacol, 1999. 382(3):p. 167-75.
5. Vitolo, O.V., et al., Amyloid beta -peptide inhibition of the PKA/CREB pathway and long-term potentiation: reversibility by drugs that enhance cAMP signaling. Proc Natl Acad Sci U S A, 2002. 99(20): p. 13217-21.
6. Walsh, D.M., et al., Naturally secreted oligomers of amyloid beta protein potently inhibit hippocampal long-term potentiation in vivo. Nature, 2002. 416(6880): p. 535-9.
7. Chen, Q.S., et al., Impairment of hippocampal long-term potentiation by Alzheimer amyloid beta-peptides. J Neurosci Res, 2000. 60(1):p. 65-72.
8. Lambert, M.P., et al., Diffusible, nonfibrillar ligands derived from Abeta1-42 are potent central nervous system neurotoxins. Proc Natl Acad Sci USA, 1998. 95(11):p. 6448-53.
9. Rakyan, V.K., et al., The marks, mechanisms and memory of epigenetic states in mammals. Biochem J, 2001. 356(Pt 1): p. 1-10.
10. Gwack, Y., et al., CREB-binding protein and histone deacetylase regulate the transcriptional activity of Kaposi's sarcoma-associated herpesvirus open reading frame 50. J Virol, 2001. 75(4): p. 1909-17.
11. Lu, F., et al., Chromatin remodeling of the Kaposi's sarcoma-associated herpesvirus ORF50 promoter correlates with reactivation from latency. J Virol, 2003. 77(21):p. 11425-35.
12. Knutson, S.K., In vivo characterization of the role of histone deacetylase 3 in metabolic and transcriptional regulation, in Biochemistry. 2008, Vanderbilt: Nashville. p. 167.
13. Rajan, I., et al., Loss of the putative catalytic domain of HDAC4 leads to reduced thermal nociception and seizures while allowing normal bone development. PLoS One, 2009. 4(8): p. e6612.
14. Langley, B., et al., Remodeling chromatin and stress resistance in the central nervous system: histone deacetylase inhibitors as novel and broadly effective neuroprotective agents. Curr Drug Targets CNS Neurol Disord, 2005. 4(1): p. 41-50.
15. Fischer, A., et al., Recovery of learning and memory is associated with chromatin remodelling. Nature, 2007. 447(7141): p. 178-82.
16. Kim, D., et al., SIRT1 deacetylase protects against neurodegeneration in models for Alzheimer's disease and amyotrophic lateral sclerosis. EMBO J, 2007. 26(13): p. 3169-79.
17. Francis, Y.I., et al., Dysregulation of histone acetylation in the APPIPS1 mouse model of Alzheimer's disease. J Alzheimers Dis, 2009. 18(1): p. 131-9.
18. Marmorstein, R. and R.C. Trievel, Histone modifying enzymes: structures, mechanisms, and specificities. Biochim Biophys Acta, 2009. 1789(1): p. 58-68.
19. Roelfsema, J.H. and D.J. Peters, Rubinstein-Taybi syndrome: clinical and molecular overview. Expert Rev Mol Med, 2007. 9(23): p. 1-16.
20. Alarcon, J.M., et al., Chromatin acetylation, memory, and LTP are impaired in CBP+/- mice: a model for the cognitive deficit in Rubinstein-Taybi syndrome and its amelioration. Neuron, 2004. 42(6): p. 947-59.
21. Maurice, T., et al., Altered memory capacities and response to stress in p300/CBP-associated factor (PCAF) histone acetylase knockout mice. Neuropsychopharmacology, 2008. 33(7): p. 1584-602.
22. Mantelingu, K., et al., Activation of p300 histone acetyltransferase by small molecules altering enzyme structure: probed by surface-enhanced Raman spectroscopy. J Phys Chem B, 2007. 111(17): p. 4527-34.
23. Balasubramanyam, K., et al., Small molecule modulators of histone acetyltransferase p300. J Biol Chem, 2003. 278(21): p. 19134-40.
24. Dal Piaz, F., et al., The identification of a novel natural activator of p300 histone acetyltranferase provides new insights into the modulation mechanism of this enzyme. Chembiochem. 11(6): p. 818-27.
25. Trinchese, F., et al., Progressive age-related development of Alzheimer-like pathology in APP/PS1 mice. Ann Neurol, 2004. 55(6): p. 801-14.
26. Nakagami, Y., et al., A novel beta-sheet breaker, RS-0406, reverses amyloid beta-induced cytotoxicity and impairment of long-term potentiation in vitro. Br J Pharmacol, 2002. 137(5): p. 676-82.
27. Walsh, D.M., et al., Certain inhibitors of synthetic amyloid beta-peptide (Abeta) fibrillogenesis block oligomerization of natural Abeta and thereby rescue long-term potentiation. J Neurosci, 2005. 25(10): p. 2455-62.
28. Schenk, D., et al., Immunization with amyloid-beta attenuates Alzheimer-disease-like pathology in the PDAPP mouse. Nature, 1999. 400(6740): p. 173-7.
29. Wu, J., R. Anwyl, and M.J. Rowan, beta-Amyloid-(1-40) increases long-term potentiation in rat hippocampus in vitro. Eur J Pharmacol, 1995. 284(3): p. R1-3.
30. Kowalska, M.A. and K. Badellino, beta-Amyloid protein induces platelet aggregation and supports platelet adhesion. Biochem Biophys Res Commun, 1994. 205(3): p. 1829-35.
31. Mattson, M.P., Z.H. Guo, and J.D. Geiger, Secreted form of amyloid precursor protein enhances basal glucose and glutamate transport and protects against oxidative impairment of glucose and glutamate transport in synaptosomes by a cyclic GMP-mediated mechanism. J Neurochem, 1999. 73(2): p. 532-7.

### Example 10: HAT Agonists for the Treatment of AD

We have demonstrated that hippocampal levels of CREB binding protein (CBP) and p300/CBP associated factor (PCAF), two histone acetyltransferases (HATs) that catalyze histone acetylation and are relevant to memory formation^{[P14-P16]} are reduced following Aβ elevation.

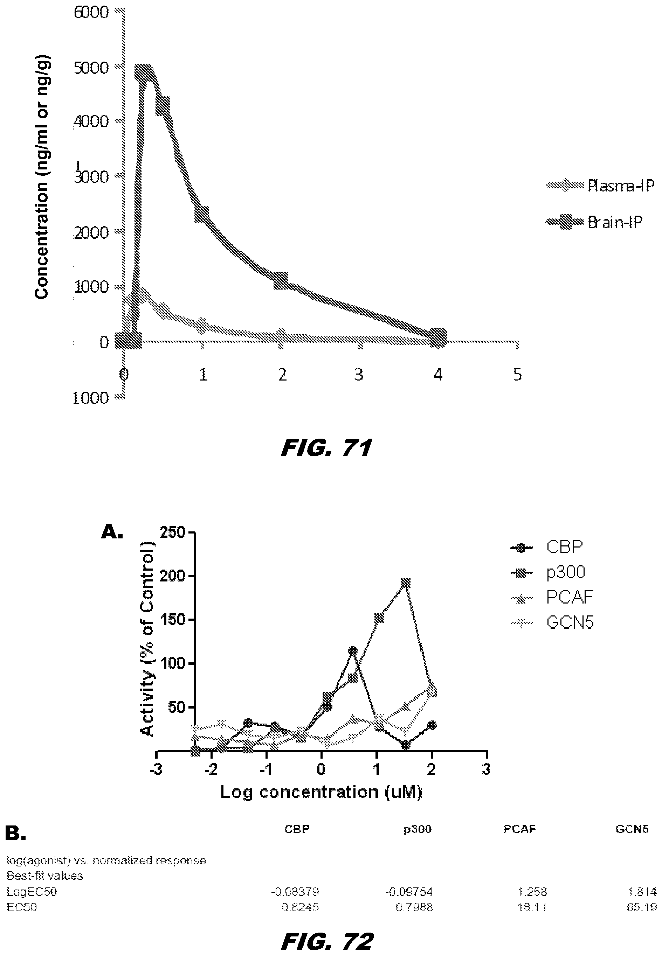

We have designed and synthesized a HAT Activator compound, **YF2 (****FIG. 29**). **YF2** was prepared by Mitsunobu reaction between 2-(dimethylamino)ethanol and **3**, which was obtained through the reaction of the acid **2** and 4-chloro-3-(trifluoromethyl)aniline, in the presence of EDC. The acid was synthesized by ester hydrolysis of **1**. Finally, **YF2** was treated with HCl/Et₂O in order to obtain a water soluble compound. In *In vitro* assays, YF2 has activity versus CBP, PCAF, and GCN5. The EC₅₀'s of YF2 for CBP, PCAF, and GCN5 are 2.75µM, 29.04µM and 49.31µM, respectively. Additionally, YF2 did not interfere with p300 and HDAC activity (HDAC 1, 3, 5, 6, 7, 8, 10, 11, and sirt1-2). YF2 also increases p300 activity as shown in **FIG. 72**.

Encouraged by these results, we then investigated YF2 pharmacokinetic (PK) and blood-brain barrier (BBB) penetration capability. After i.p. and i.v. administration at 20 mg/kg to BALB/c mice, plasma and brain concentrations were determined by LC-MS/MS. The data in Table 14 indicates that YF2 is rapidly absorbed in the brain (Tₘₐₓ at 15min).

**Table 14. Pharmacokinetic parameters of YF2.**

| Parameters | | IP Administration | | | IV Administration | | |
|---|---|---|---|---|---|---|---|
| | | Plasma | Brain | Ratio* | Plasma | Brain | Ratio* |
| Tₘₐₓ | (h) | 0.25 | 0.25 | - | 0.125 | 0.125 | - |
| Cₘₐₓ | (ng/mL or ng/g) | 843 | 4878 | 5.8 | 2132 | 27892 | 13.1 |
| AUC₀₋ₜ | (ng·h/mL or ng·h/g) | 806 | 6493 | 8.1 | 1967 | 22222 | 11.3 |
| AUC_{0-∞} | (ng·h/mL or ng·h/g) | 813 | 6564 | 8.1 | 2020 | 22581 | 11.2 |
| t1/2 | (h) | 0.60 | 0.63 | - | 0.70 | 0.63 | - |
| MRT | (h) | 0.85 | 1.03 | - | 0.84 | 0.74 | - |
| *F* | (%) | 41.0 | 29.2 | - | - | - | - |

| | | | | | | | |
|---|---|---|---|---|---|---|---|
| *Ratio = brain/plasma YF2 EC₅₀'s for CBP, PCAF and GCN5 are: 2.75 µM, 29.04 µM and 49.31 µM, respectively | | | | | | | |

The amount of YF2 in the brain was higher than that in the plasma with an AUC₀₋ₜ ratio of 8.2 and 10.8 for i.p. and i.v. administration, respectively. The elimination half-lives of YF2 in the brain and plasma were ∼40 min. The Tₘₐₓ values in the brain and plasma were similar, indicating that the distribution of YF2 to the brain is also fast. Additionally, in acute toxicity experiments YF2 did not induce any adverse effects up to 300mg/kg (i.p.).

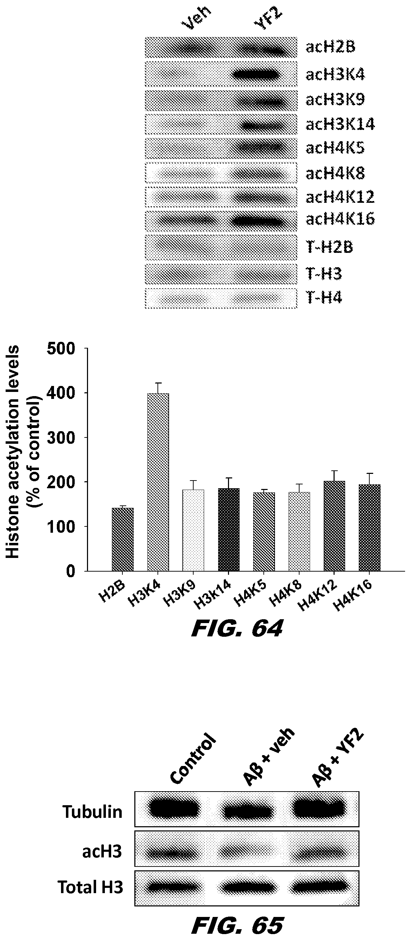

We then tested if YF2 increases histone acetylation in mouse hippocampus. The compound was i.p. administered at 20 mg/Kg, mice were sacrificed 30 min later, and hippocampi were removed and quickly frozen for WB analysis. As shown in **FIG. 64**, YF2 increased acetylation of histone lysines that were shown to be involved in memory formation (H3K4, H3K9, H3K14, H4K5, H4K8, H4K12, H4K16, and H2B)^{[P15,P33]}.

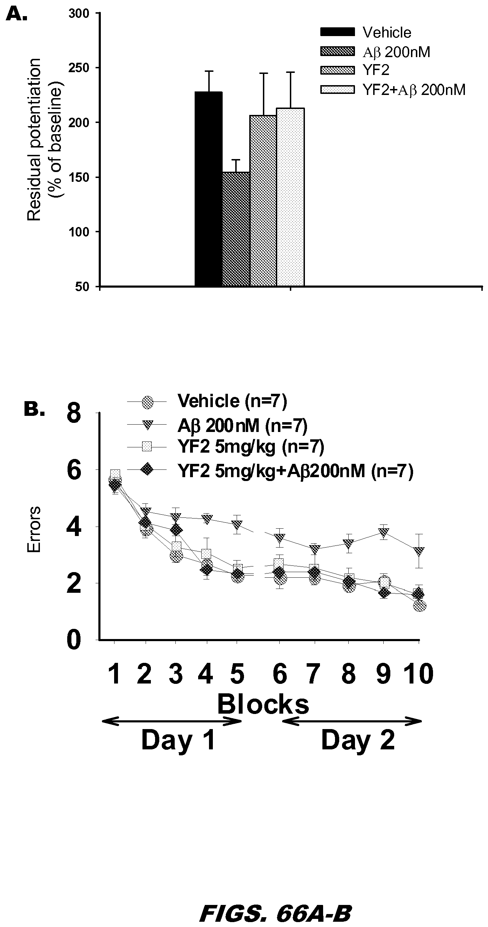

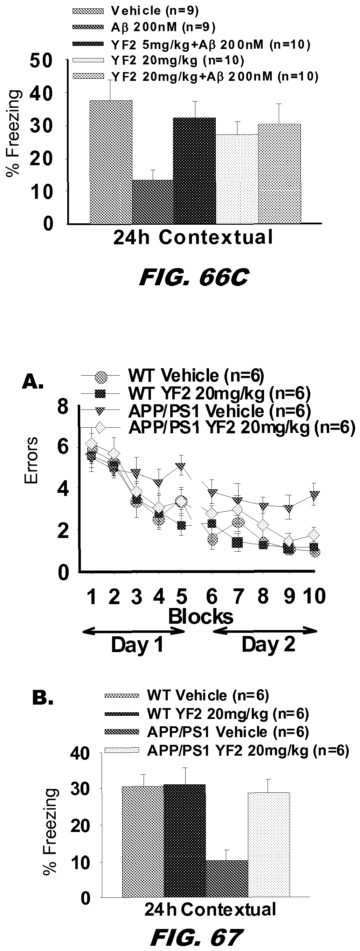

Next, we checked if YF2 could attenuate synaptic and cognitive dysfunction after Aβ elevation. YF2 rescued the defect in H3 acetylation levels following Aβ infusion onto hippocampus (**FIG. 65**). Next, we induced LTP or contextual fear memory as well as reference memory in the presence of oligomeric Aβ₄₂, or vehicle. In the LTP experiments 200 nM Aβ₄₂ or vehicle were perfused through the bath solution for 20 min prior to the application of the θ-burst. In the behavioral experiments 200 nM Aβ₄₂ (in a final volume of 1 µl over 1 min) or vehicle were bilaterally infused 15 min prior to the foot shock or 15 min prior to the 1^{st} trial (for the 1^{st} group of tests in a 2-day radial arm water maze (RAWM) test assessing reference memory^{[P34]}) and 15 min prior to the 7^{th} trial (for the 2^{nd} group of tests of the RAWM) into dorsal hippocampus of the animal that had been pre-implanted with a cannula the week before. Aβ reduced LTP and both reference and contextual fear memory (**FIG. 66**). However, YF2 (1µM, for 30 min prior to the θ-burst in the LTP experiments; 5 or 20mg/Kg, i.p., 15 min before Aβ administration in the behavioral experiments) ameliorated the electrophysiological and behavioral deficits (**FIG. 66**).

Without being bound by theory, given that these experiments were obtained with a synthetic preparation of Aβ₄₂, it is difficult to know if this is the same as Aβ that causes synaptic dysfunction in AD brains. Thus, we validated these experiments in an Aβ-depositing mouse model, the APP/PS1 mouse^{[P35]}. These mice have been well characterized with respect to AD pathology^{[P34-31]} . They start to show synaptic and memory impairment as early as 3 months of age^{[P34]}. Transgenic mice with a double mutation APP (K670M:N671L)/ PS1(M146L) (line 6.2) start to develop large plaques in cortex and hippocampus at the age of 8-10 weeks. They have a reduction in LTP by 3 months of age together with impairment of contextual fear memory and spatial working memory.

As shown in **FIG. 67**, WT mice exhibited <2 errors over two trials near the end of the second day with the RAWM task. APP/PS1 in turn failed to learn and made >3 errors throughout the training session. The treatment with YF2 (20 mg/Kg, i.p. 30 min before training for FC or before the 1^{st} and 2^{nd} group of tests for the RAWM), however, rescued the memory defect in double Tgs without affecting memory in WT mice. We obtained similar results when double Tgs mice performed FC to assess contextual fear memory (**FIG. 67**).

In summary, Histone 3 and 4 acetylation is decreased following Aβ42 elevation. HATs levels (CBP and PCAF) are reduced following Aβ₄₂ elevation. HDAC inhibition is beneficial against damage of synaptic function and memory following Aβ42 elevation. HAT activation is beneficial against memory loss following Aβ₄₂ elevation. We have designed and synthesized a HAT activator, YF2, that shifts the balance of histone 3 acetylation and reinstates normal memory following Aβ elevation.

### Example 11: HAT ACTIVATORS WITH HIGH AFFINITY AND GOOD SELECTIVITY FOR CBP AND/OR PCAF AND/OR P300

Compounds will be tested for HAT activator activity first, using the HAT Assay kit from Active Motif (USA, CA). For this assay, we will use the catalytic domain of human CBP, PCAF, p300 and GCN5 (Enzo Life Sci., USA). The catalytic domains for the remaining HATs will be produced using New England Biolabs K. lactis Protein Expression Kit. In addition to being potent activators of CBP and/or PCAF (EC₅₀<100nM), the candidate compounds must also be selective. When assayed against all other HATs, they must show at least a 50 fold greater potency towards CBP and/or PCAF. Finally, on the most potent/selective compounds that show efficacy in tests of synaptic and memory dysfunction outlined in **Examples 13 and 14** we will check selectivity against HDACs. While performing these tests, we will also evaluate solubility (neutral aqueous buffer > 10 µg/ml).

Our next goal will be to confirm *in vitro* data, using a neuronal preparation. In particular, we will test new HAT activators in primary cultures and adult mice. We will first determine whether the compounds can increase specific histone acetylation in 10 day old cultured hippocampal neurons (prepared as described^{[P47]}). Media will be aspirated and replaced with 0.5 mL of PBS containing the HAT activator. After 30 min at 37°C, cells will be removed and lysed for WB analysis. Compounds that pass the test in hippocampal cultures will next be tested in adult mice, following an assessment of acute toxicity to determine the dose of compound to be administered to the animal (see "toxicity tests" below). Testing in adult mice is essential as cell cultures do not recapitulate the whole body with complex cell-cell interactions and *in vivo* drug PK, BBB penetration, etc (see also **Example 12** below). Animals will be treated with the HAT activator, hippocampi will be collected 30 min after the i.p. injection, and lysed for WB analysis. All experiments will be performed in triplicate. If we find an increase in specific histone acetylation, the candidate compound will be deemed active.

On selected compounds we will also examine interactions with channels, receptors, transporters using the NIMH PDSP program (see http://pdsp.cwru.edu for a list of CNS targets), in addition to ADMET/Tox studies discussed in **Example 12**.

### Example 12: TO DETERMINE WHETHER NEW HAT ACTIVATORS HAVE GOOD PHARMACOKINETIC (PK) PROFILE AND ARE SAFE

We will generate data addressing the rudimentary PK properties and toxicity of the novel HAT activators. If necessary, information obtained through the PK and toxicity studies will be used to guide additional chemical modifications of the new molecules with the ultimate goal of improving bioavailability, brain uptake, and safety. Pharmacokinetic assays that need to be performed will include the measurement of a) bioavailability and b) brain uptake. Mice will be i.p. injected with the activator (for final drug candidates PK tests will be also performed using p.o. and i.v. routes of administration). 5-6 mice/sex will be used for each time-point. For the assessment of bioavailability (concentration of compound in the blood as a function of time after administration), blood samples will be obtained from test animals following a single acute administration (collected at 5min, 15min, 30 min, 1hr, 2hrs, 4hrs, and 24hrs). Blood will be harvested by retro-orbital puncture, collected in heparanized tubes, and plasma obtained by centrifugation. Samples will be analyzed by LC-MS to measure the amounts of the candidate compound and possible metabolites. An indication of brain uptake and BBB penetration will be obtained by tissue extraction of the candidate compound from brain. Briefly, brain homogenates will be centrifuged 11,000rpm for 10 min. An aliquot of the sample will be added to acetonitrile, then injected onto LC-MS/MS for analysis. Similar patterns of brain and plasma concentrations will be indicative of the fact that brain uptake reflects concentration of the blood. A peak brain/blood concentration ratio >1 will indicate that brain uptake for our compound is comparable with that of known CNS drugs in clinical use. For example, the brain/blood ratio for minaprine, a 6-phenylaminopyridazine CNS drug, is >2^{[P48]}.

Next, we will evaluate acute toxicity. All clinical signs, time of onset, duration, reversibility of toxicity and mortalities will be recorded. Animals will be observed periodically during the first 24hrs with continuous monitoring given to the first 4hrs, then at least once a day for 14 days or until they die to check food and liquid intake, weight, as well as locomotion and exploratory behavior. We will also evaluate maximum tolerated dose (MTD) and chronic toxicity. MTD will be computed as the maximum administered dose that does not produce any toxicity effect in terms of malaise or death (body weight will be monitored over time). Chronic toxicity will assessed at the MTD. All clinical signs, time of onset, duration, reversibility of toxicity and mortalities will be recorded. The occurrence of possible chronic toxicity signs will be assessed for at least 1 month after the end of the treatment.

It has been estimated that over half of all drugs fail to reach the market because of ADMET problems^{[P49]}. Therefore, before embarking on a course of costly animal toxicological work and following efficacy assessment (see **Examples 13 and 14),** we will take advantage of recent advances in *in vitro* AD MET testing to screen our best three compounds with a quick, inexpensive battery of assays. We will focus on two areas that have resulted in the withdrawal of many drugs from the market: drug-drug interactions (liver metabolism), hERG channel blockage (cardiac dysfunction). To test for drug-drug interactions related to hepatotoxicity, we will use the Cytochrome P450 inhibition assay (performed by SRI international). hERG channel blockage assay will be performed using the NIMH PDSP program.

### Example 13: Selecting HAT Activators that rescue LTP in APP/PS1 mice

Synaptic dysfunction is a major hallmark of AD^{[P50]}. A qualifying aspect of our drug screening protocol will include a measurement of the effect of the newly-synthesized compounds onto synaptic function. The APP/PS1 mouse presents an impairment of LTP by the age of 3 months^{[P34]} and therefore permits a relatively fast assessment of synaptic function without waiting a long time for mice aging. We will examine LTP because it is a type of synaptic plasticity that is thought to underlie learning and memory. Based on the findings that YF2 rescues the Aβ-induced reduction of LTP, we will screen the compounds indicated by our MedChem studies to select those that can re-establish normal LTP. The compounds will be applied for 30 min using the same experimental protocol as in **FIG. 66A**. Controls will be performed on slices from APP/PS1 mice treated with vehicle, and WT mice treated with compound or vehicle. If the compounds re-establish normal LTP in APP/PS1 slices, **we will conclude that the compounds can rescue impairment of synaptic plasticity in APP/PS1 mice.** We will also investigate another important aspect of the disease, the cognitive impairment (see **Example 14**).

**Animals:** Tg mice will be obtained by crossing APP(K670M:N671L) with PS1(M146L) (line 6.2) animals. The genotype will be identified by PCR on tail samples^{[P51-P53]}

**Electrophysiology** will be performed on males (see description in Gong et al^{[P54]}).

### Statistical Analyses: see Example 14.

### Example 14: FURTHER SCREENING OF HAT ACTIVATORS TO EXAMINE IF THEY AMELIORATE COGNITIVE ABNORMALITIES IN APP/PS1 MICE

Without being bound by theory, treatment with a novel HAT activator indicated by **Example 13** can rescue the cognitive deficits in 3 and 6 month old APP/PS1 mice. As behavioral tasks we will use the RAWM and contextual FC, two types of tests assessing different types of memory (reference ad associative) that are affected in AD patients. The treatment will be performed with the same timing (i.e. 30min before training for fear conditioning or before the 1^{st} and 2^{nd} group of tests for the RAWM). Conditions to be tested include: APP/PS1 and WT treated with HAT activators, APP/PS1 and WT treated with vehicle. After behavioral testing mice will be sacrificed and their blood and brains used for Aβ level, Tau protein, TARDBP and TDB levels, and alpha-synuclein measurements. As a control for effectiveness of HAT activation, we will measure hippocampal acetyl-H4 levels after administration of the compounds 30 min prior to training for fear conditioning and removal of the hippocampi 1hr after the electric shock (APP/PS1 mice have been shown to have a reduction of acetylated H4 after the electric shock^{[P21]}). Finally, we will screen them with a battery of assays focusing on two areas that have resulted in the withdrawal of many drugs from the market: drug-drug interactions, hERG channel blockage (see **Example 12**).

### Animals: see Example 13.

**Behavioral Studies:** Experiments will be performed in blind only on male animals to reduce variability. *A) Spatial memory.* This type of reference memory can be studied with the 2-day RAWM test, as described^{[P56]}; see also **FIG. 66B**). The task is a hybrid of the Morris Water Maze (MWM) and the radial arm land maze. For these experiments, *visible platform testing* will be conducted to exclude that visual, motor and motivation deficits affect the mouse performance, as described^{[P34]}. *B) Fear Conditioning* to examine both contextual and cued learning will be assessed as described^{[P57]}. For these experiments, *threshold assessment test* will performed to check sensory perception of electric foot in different groups of mice^{[P57]}. In addition, the *open-field test* will be conducted to evaluate exploratory as described^{[P58, P59]}.

**Histone acetylation assay:** Western blot will be performed from snap-frozen in liquid nitrogen hippocampi. Tissue will be homogenized in RIPA buffer, then sonicated before centrifugation at 10,000 rpm for 5 min. Whole cell extracts will be electrophoresed on 10-20% gradient PAGE gel (Invitrogen) and then immunoblotted. Antibodies will be used at a 1:1,000 concentration for immunoblotting. All anti-histone antibodies will be purchased from Millipore. Immunoblot data will be quantified by measuring the band intensity using imaging software (NIH ImageJ).

**Determination of Aβ levels** will be performed on homogenates of frozen hemi-brains and plasma as previously described^{[P34]}.

**Determination of alpha-synuclein levels** will be performed on homogenates of frozen hemi-brains using an α-Synuclein ELISA Kit (Catalog # NS400; Millipore, Billerica, MA) according to manufacturer's instructions.

**Determination of TARDBP/TDP-43 levels** will be performed on homogenates of frozen hemi-brains using a Human TAR DNA binding protein 43, TARDBP/TDP-43 ELISA Kit (Catalog # E1951h; Wuhan EIAab Science Co, Wuhan, China) according to manufacturer's instructions.

**Determination of total Tau and phosphorylated Tau (Thr 231) levels** will be performed on homogenates of frozen hemi-brains and plasma using assay and kits according to manufacturer's instructions available from MesoScale Discovery (Gaithersburg, MD) (see http://www.mesoscale.com/catalogsystemweb/webroot/products/assays/alzheimers.aspx).

**Statistics**: Experiments mice will be performed in blind. Results will be expressed as Standard Error Mean (SEM). Level of significance will be set for p<0.05. Results will be analyzed with ANOVA with post-hoc correction with drug or genotype as main effect.

### References

PI. Malm, T., et al., beta-Amyloid infusion results in delayed and age-dependent learning deficits without role of inflammation or beta-amyloid deposits. Proc Natl Acad Sci U S A, 2006. 103(23): p. 8852-7.
P2. Cleary, J.P., et al., Natural oligomers of the amyloid-beta protein specifically disrupt cognitive function. Nat Neurosci, 2005. 8(1): p. 79-84.
P3. Cullen, W.K., et al., Block of LTP in rat hippocampus in vivo by beta-amyloid precursor protein fragments. Neuroreport, 1997. 8(15): p. 3213-7.
P4. Itoh, A., et al., Impairments of long-term potentiation in hippocampal slices of beta-amyloid-infused rats. Eur J Pharmacol, 1999. 382(3): p. 167-75.
P5. Vitolo, O.V., et al., Amyloid beta -peptide inhibition of the PKA/CREB pathway and long-term potentiation: reversibility by drugs that enhance cAMP signaling. Proc Natl Acad Sci U SA, 2002. 99(20):p. 13217-21.
P6. Walsh, D.M., et al., Naturally secreted oligomers of amyloid beta protein potently inhibit hippocampal long-term potentiation in vivo. Nature, 2002. 416(6880):p. 535-9.
P7. Chen, Q.S., et al., Impairment of hippocampal long-term potentiation by Alzheimer amyloid beta-peptides. J Neurosci Res, 2000. 60(1):p. 65-72.
P8. Lambert, M.P., et al., Diffusible, nonfibrillar ligands derived from Abetal-42 are potent central nervous system neurotoxins. Proc Natl Acad Sci USA, 1998. 95(11):p. 6448-53.
P9. Rakyan, V.K., et al., The marks, mechanisms and memory of epigenetic states in mammals. Biochem J, 2001. 356(Pt 1):p. 1-10.
P10. Gwack, Y., et al., CREB-binding protein and histone deacetylase regulate the transcriptional activity of Kaposi's sarcoma-associated herpesvirus open reading frame 50. J Virol, 2001. 75(4):p. 1909-17.
P11. Lu, F., et al., Chromatin remodeling of the Kaposi's sarcoma-associated herpesvirus ORF50 promoter correlates with reactivation from latency. J Virol, 2003. 77(21):p. 11425-35.
P12. Knutson, S.K., In vivo characterization of the role of histone deacetylase 3 in metabolic and transcriptional regulation, in Biochemistry. 2008, Vanderbilt: Nashville. p. 167.
P13. Rajan, I., et al., Loss of the putative catalytic domain of HDAC4 leads to reduced thermal nociception and seizures while allowing normal bone development. PLoS One, 2009. 4(8): p. e6612.
P14. Roelfsema, J.H. and D.J. Peters, Rubinstein-Taybi syndrome: clinical and molecular overview. Expert Rev Mol Med, 2007. 9(23): p. 1-16.
P15. Alarcon, J.M., et al., Chromatin acetylation, memory, and LTP are impaired in CBP+/- mice: a model for the cognitive deficit in Rubinstein-Taybi syndrome and its amelioration. Neuron, 2004. 42(6): p. 947-59.
P16. Maurice, T., et al., Altered memory capacities and response to stress in p300/CBP-associated factor (PCAF) histone acetylase knockout mice. Neuropsychopharmacology, 2008. 33(7): p. 1584-602.
P17. Levenson, J.M., et al., Regulation of histone acetylation during memory formation in the hippocampus. J Biol Chem, 2004. 279(39): p. 40545-59.
P18. Langley, B., et al., Remodeling chromatin and stress resistance in the central nervous system: histone deacetylase inhibitors as novel and broadly effective neuroprotective agents. Curr Drug Targets CNS Neurol Disord, 2005. 4(1): p. 41-50.
P19. Fischer, A., et al., Recovery of learning and memory is associated with chromatin remodelling. Nature, 2007. 447(7141): p. 178-82.
P20. Kim, D., et al., SIRT1 deacetylase protects against neurodegeneration in models for Alzheimer's disease and amyotrophic lateral sclerosis. EMBO J, 2007. 26(13):p. 3169-79.
P21. Francis, Y.I., et al., Dysregulation of histone acetylation in the APPIPS1 mouse model of Alzheimer's disease. J Alzheimers Dis, 2009. 18(1):p. 131-9.
P22. Marmorstein, R. and R.C. Trievel, Histone modifying enzymes: structures, mechanisms, and specificities. Biochim Biophys Acta, 2009. 1789(1):p. 58-68.
P23. Mantelingu, K., et al., Activation of p300 histone acetyltransferase by small molecules altering enzyme structure: probed by surface-enhanced Raman spectroscopy. J Phys Chem B, 2007. 111(17):p. 4527-34.
P24. Balasubramanyam, K., et al., Small molecule modulators of histone acetyltransferase p300. J Biol Chem, 2003. 278(21):p. 19134-40.
P25. Sbardella, G., et al., Identification of long chain alkylidenemalonates as novel small molecule modulators of histone acetyltransferases. Bioorg Med Chem Lett, 2008. 18(9):p. 2788-92.
P26. Dal Piaz, F., et al., The identification of a novel natural activator of p300 histone acetyltranferase provides new insights into the modulation mechanism of this enzyme. Chembiochem. 11(6):p. 818-27.
P27. Nakagami, Y., et al., A novel beta-sheet breaker, RS-0406, reverses amyloid beta-induced cytotoxicity and impairment of long-term potentiation in vitro. Br J Pharmacol, 2002. 137(5): p. 676-82.
P28. Walsh, D.M., et al., Certain inhibitors of synthetic amyloid beta-peptide (Abeta) fibrillogenesis block oligomerization of natural Abeta and thereby rescue long-term potentiation. J Neurosci, 2005. 25(10): p. 2455-62.
P29. Schenk, D., et al., Immunization with amyloid-beta attenuates Alzheimer-disease-like pathology in the PDAPP mouse. Nature, 1999. 400(6740): p. 173-7.
P30. Wu, J., R. Anwyl, and M.J. Rowan, beta-Amytoid-(1-40) increases long-term potentiation in rat hippocampus in vitro. Eur J Pharmacol, 1995. 284(3):p. R1-3.
P31. Kowalska, M.A. and K. Badellino, beta-Amyloid protein induces platelet aggregation and supports platelet adhesion. Biochem Biophys Res Commun, 1994. 205(3): p. 1829-35.
P32. Mattson, M.P., Z.H. Guo, and J.D. Geiger, Secreted form of amyloid precursor protein enhances basal glucose and glutamate transport and protects against oxidative impairment of glucose and glutamate transport in synaptosomes by a cyclic GMP-mediated mechanism. J Neurochem, 1999. 73(2): p. 532-7.
P33. Peleg, S., et al., Altered histone acetylation is associated with age-dependent memory impairment in mice. Science. 328(5979):p. 753-6.
P34. Trinchese, F., et al., Progressive age-related development of Alzheimer-like pathology in APP/PS1 mice. Ann Neurol, 2004. 55(6):p. 801-14.
P35. Holcomb, L., et al., Accelerated Alzheimer-type phenotype in transgenic mice carrying both mutant amyloid precursor protein and presenilin 1 transgenes. Nat Med, 1998. 4(1):p. 97-100.
P36. Kurt, M.A., et al., Neurodegenerative changes associated with beta-amyloid deposition in the brains of mice carrying mutant amyloid precursor protein and mutant presenilin-1 transgenes. Exp Neurol, 2001. 171(1): p. 59-71.
P37. McGowan, E., et al., Amyloid phenotype characterization of transgenic mice overexpressing both mutant amyloid precursor protein and mutant presenilin 1 transgenes. Neurobiol Dis, 1999. 6(4): p. 231-44.
P38. Sant'Angelo, A., F. Trinchese, and O. Arancio, Usefulness of behavioral and electrophysiological studies in transgenic models of Alzheimer's disease. Neurochem Res, 2003. 28(7):p. 1009-15.
P39. Coutts, R.T., P. Su, and G.B. Baker, Involvement of CYP2D6, CYP3A4, and other cytochrome P-450 isozymes in N-dealkylation reactions. J Pharmacol Toxicol Methods, 1994. 31(4): p. 177-86.
P40. Thompson, T.N., Optimization of metabolic stability as a goal of modern drug design. Med Res Rev, 2001. 21(5): p. 412-49.
P41. Chee, C.F., et al., An efficient synthesis of (±)-panduratin A and (±)-isopanduratin A, inhibitors of dengue-2 viral activity. Tetrahedron Letters, 2010. 51:p. 495-498.
P42. Kuehne, M.E. and P.J. Shannon, Reduction of Amides and Lactams to Amines by Reactions with Phosphorus Oxychloride and Sodium Borohydride. J. Org. Chem., 1977. 42:p. 2082-2087.
P43. Mondal, M., V.G. Puranik, and N.P. Argade, Facile Synthesis of 1,3,7-Trihydroxyxanthone and Its Regioselective Coupling Reactions with Prenal: Simple and Efficient Access to Osajaxanthone and Nigrolineaxanthone F. J. Org. Chem., 2006. 71: p. 4992-4995.
P44. Melgar-Fernández, R., et al., Synthesis of Novel Derivatives of (1S,4S)-2,5-Diazabicyclo[2.2.1]heptane and Their Evaluation as Potential Ligands in Asymmetric Catalysis. Eur. J. Org. Chem., 2008: p. 655-672.
P45. Mahmoud, M.R., et al., Synthesis of novel indeno[1,2-c]isoquinolines derivatives. Synthetic Communications, 2010. 40: p. 666-676.
P46. Tsuritani, T., et al., Efficient synthesis of 1,4-Diaryl-5-methyl-1,2,3-triazole, a potential mGluR1 antagonist, and the risk assessment study of arylazides. Organic Process Research & Development, 2009. 13:p. 1407-1412.
P47. Ninan, I. and O. Arancio, Presynaptic CaMKII Is Necessary for Synaptic Plasticity in Cultured Hippocampal Neurons. Neuron, 2004. 42(1):p. 129-41.
P48. Caccia, S., T. Fossati, and A. Mancinelli, Disposition and metabolism of minaprine in the rat. Xenobiotica, 1985. 15(12):p. 1111-9.
P49. Hodgson, J., ADMET--turning chemicals into drugs. Nat Biotechnol, 2001. 19(8):p. 722-6.
P50. Masliah, E., Mechanisms of synaptic dysfunction in Alzheimer's disease. Histol Histopathol, 1995. 10(2): p. 509-19.
P51. Duff, K., et al., Increased amyloid-beta42(43) in brains of mice expressing mutant presenilin 1. Nature, 1996. 383(6602):p. 710-3.
P52. Hsiao, K., et al., Correlative memory deficits, Abeta elevation, and amyloid plaques in transgenic mice. Science, 1996. 274(5284): p. 99-102.
P53. Di Rosa, G., et al., Calpain inhibitors: a treatment for Alzheimer's disease. J Mol Neurosci, 2002. 19(1-2):p. 135-41.
P54. Gong, B., et al., Ubiquitin Hydrolase Uch-L1 Rescues beta-Amyloid-Induced Decreases in Synaptic Function and Contextual Memory. Cell, 2006. 126(4): p. 775-88.
P55. Bowers, E.M., et al., Virtual ligand screening of the p300/CBP histone acetyltransferase: identification of a selective small molecule inhibitor. Chem Biol. 17(5): p. 471-82.
P56. Alamed, J., et al., Two-day radial-arm water maze learning and memory task; robust resolution of amyloid-related memory deficits in transgenic mice. Nat Protoc, 2006. 1(4): p. 1671-9.
P57. Gong, B., et al., Persistent improvement in synaptic and cognitive functions in an Alzheimer mouse model following rolipram treatment. J. Clin. Invest., 2004. 114: p. 1624-1634.
P58. Sipos, E., et al., Beta-amyloid pathology in the entorhinal cortex of rats induces memory deficits: implications for Alzheimer's disease. Neuroscience, 2007. 147(1): p. 28-36.
P59. Puzzo, D., et al., Picomolar amyloid-beta positively modulates synaptic plasticity and memory in hippocampus. J Neurosci, 2008. 28(53): p. 14537-45.

### Example 15: FURTHER SCREENING OF HAT ACTIVATORS TO EXAMINE IF THEY AMELIORATE COGNITIVE ABNORMALITIES IN MOUSE MODELS FOR HUNTINGTON'S DISEASE

We will examine whether treatment with a HAT activator compound indicated by **Example 13** can rescue the cognitive deficits in a mouse model of Huntington's Disease (e.g., FVB-Tg(YAC128)53Hay/J and FVB/NJ-Tg(YAC72)2511Hay/J mice, available from the Jackson Laboratory, Bar Harbor ME). As behavioral tasks, we will use the RAWM and contextual FC, two types of tests assessing different types of memory (reference ad associative). The treatment will be performed with the same timing (i.e. 30min before training for fear conditioning or before the 1^{st} and 2^{nd} group of tests for the RAWM). Conditions to be tested include: Huntington's Disease mice and WT treated with HAT activators, Huntington's Disease mice and WT treated with vehicle. After behavioral testing mice will be sacrificed and their blood and brains used for Huntingtin protein level measurement. As a control for effectiveness of HAT activation, we will measure hippocampal acetyl-H4 levels after administration of the compounds 30 min prior to training for fear conditioning and removal of the hippocampi 1hr after the electric shock. Finally, we will screen them with a battery of assays focusing on two areas that have resulted in the withdrawal of many drugs from the market: drug-drug interactions, hERG channel blockage (see **Example 12**).

**Animals:** Mouse models of Huntington's Disease (e.g., FVB-Tg(YAC128)53Hay/J [Stock no. 004938] and FVB/NJ-Tg(YAC72)2511Hay/J mice [Stock no. 003640]) will be obtained from the Jackson Laboratory (Bar Harbor ME). See also, Hodgson et al., (May 1999) Neuron, Vol. 23, 181-192.

**Behavioral Studies:** Experiments will be performed in blind only on male animals to reduce variability. *A) Spatial memory.* This type of reference memory can be studied with the 2-day RAWM test, as described^{[P56]}). The task is a hybrid of the Morris Water Maze (MWM) and the radial arm land maze. For these experiments, *visible platform testing* will be conducted to exclude that visual, motor and motivation deficits affect the mouse performance, as described^{[P34]}. *B) Fear Conditioning* to examine both contextual and cued learning will be assessed as described^{[P57]}. For these experiments, *threshold assessment test* will performed to check sensory perception of electric foot in different groups of mice^{[P57]}. In addition, the *open-field test* will be conducted to evaluate exploratory as described^{[P58, P59]}.

**Histone acetylation assay:** Western blot will be performed from snap-frozen in liquid nitrogen hippocampi. Tissue will be homogenized in RIPA buffer, then sonicated before centrifugation at 10,000 rpm for 5 min. Whole cell extracts will be electrophoresed on 10-20% gradient PAGE gel (Invitrogen) and then immunoblotted. Antibodies will be used at a 1:1,000 concentration for immunoblotting. All anti-histone antibodies will be purchased from Millipore. Immunoblot data will be quantified by measuring the band intensity using imaging software (NIH ImageJ).

**Determination of huntingtin levels** will be performed on homogenates of frozen hemi-brains and plasma using a Huntingtin (Htt) ELISA Kit (Catalog # ABIN423526; Antibodies-online, Atlanta, GA) according to manufacturer's instructions.

**Statistics:** Experiments mice will be performed in blind. Results will be expressed as Standard Error Mean (SEM). Level of significance will be set for p<0.05. Results will be analyzed with ANOVA with post-hoc correction with drug or genotype as main effect.

### Example 16: FURTHER SCREENING OF HAT ACTIVATORS TO EXAMINE IF THEY AMELIORATE MOTOR ACTIVITY ABNORMALITIES IN MOUSE MODELS FOR PARKINSON'S DISEASE

PD is a degenerative disease with a neuronal death up to 75-95% of the dopamine neurons in the substantia nigra nucleus. We will examine whether treatment with a HAT activator compound indicated by **Example 13** can rescue abnormal motor movements in a mouse model of Parkinson's Disease (PD) (e.g., see Parkinson's Disease mice models available from the Jackson Laboratory, Bar Harbor ME at http://jaxmice.jax.org/list/ ra1594.html; see also Emborg, Journal of Neuroscience Methods 139 (2004) 121-143; Lane Psychopharmacology (2008) 199:303-312; and Meredith et al., Acta Neuropathol (2008) 115:385-398). As behavioral tasks, we will examine, for example, dyskinesia, bradykinesia, tremor, and/or grip force for the evaluation of the compound's efficacy at various stages of PD. Conditions to be tested include: PD mice and WT treated with HAT activators, PD mice and WT treated with vehicle. After behavioral evaluation, mice will be sacrificed and their brains used for aggregated alpha-synuclein protein measurement. As a control for effectiveness of HAT activation, we will measure hippocampal acetyl-H4 levels.

**Animals:** Mouse models of Parkinson's Disease will be obtained from the Jackson Laboratory (Bar Harbor ME). See also, Meredith et al., Acta Neuropathol (2008) 115:385-398.

**Behavioral Studies:** Experiments will be performed in blind only on male animals to reduce variability, according to methods described by Fleming et al., ((2004) The Journal of Neuroscience, 24(42):9434 -9440) and Hwang et al., ((2005) The Journal of Neuroscience, 25(8):2132-2137).

**Histone acetylation assay:** Western blot will be performed from snap-frozen in liquid nitrogen hippocampi. Tissue will be homogenized in RIPA buffer, then sonicated before centrifugation at 10,000 rpm for 5 min. Whole cell extracts will be electrophoresed on 10-20% gradient PAGE gel (Invitrogen) and then immunoblotted. Antibodies will be used at a 1:1,000 concentration for immunoblotting. All anti-histone antibodies will be purchased from Millipore. Immunoblot data will be quantified by measuring the band intensity using imaging software (NIH ImageJ).

**Determination of alpha-synuclein levels** will be performed on homogenates of frozen hemi-brains using an α-Synuclein ELISA Kit (Catalog # NS400; Millipore, Billerica, MA) according to manufacturer's instructions or via standard neuropathological methods (brain tissue histology).

**Statistics:** Experiments mice will be performed in blind. Results will be expressed as Standard Error Mean (SEM). Level of significance will be set for p<0.05. Results will be analyzed with ANOVA with post-hoc correction with drug or genotype as main effect.

## Claims

1. A compound of formula (I): 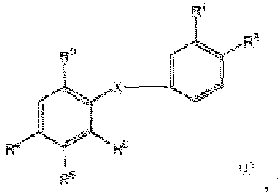 wherein,
R¹ is CF₃;
R² is Cl, or CN;
R³ is H, O-methyl, O-ethyl, S-ethyl, O-cyclopentyl, OCH₂CH₂N(CH₃)₂, or CH₂CH₂CH₂N(CH₃)₂;
R⁴ is H or C(=O)NH-phenyl, wherein phenyl is substituted with one or more halo or CF₃;
R⁵ is H, C₁-C₅-alkyl, OH, OCH₃, O-ethyl, OCH₂CH₂N(CH₃)₂,
CH₂CH₂CH₂N(CH₃)₂, SCH₂CH₂N(CH₃)₂, or OCH₂C(=O)O-alkyl;
R⁶ is H, O-methyl, O-ethyl, OCH₂CH₂N(CH₃)₂; and X is CONH, SONH, SO₂NH, NHC(=O)NH, or NHCO, or a
pharmaceutically acceptable salt or hydrate thereof,
wherein the compound is: 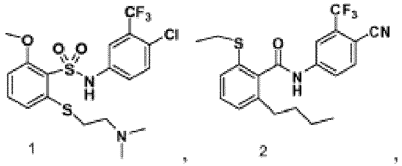 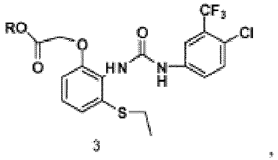 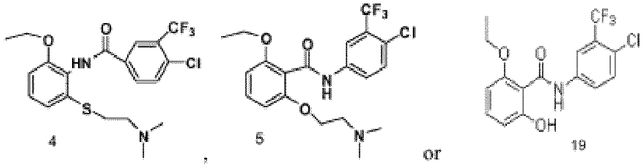 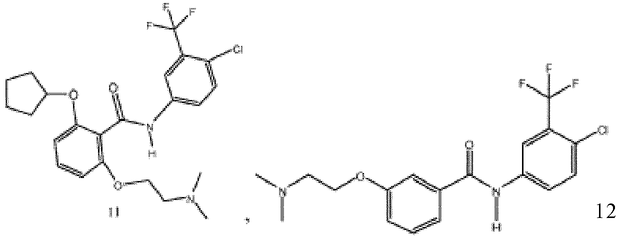 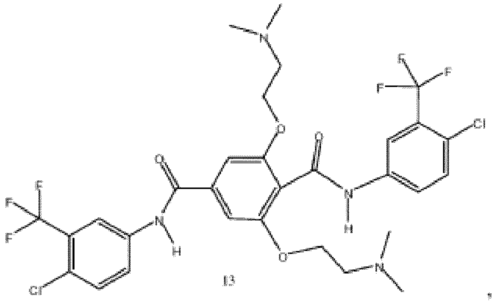 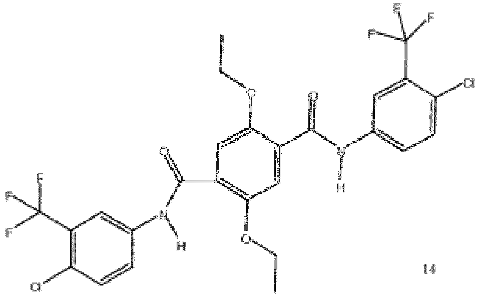 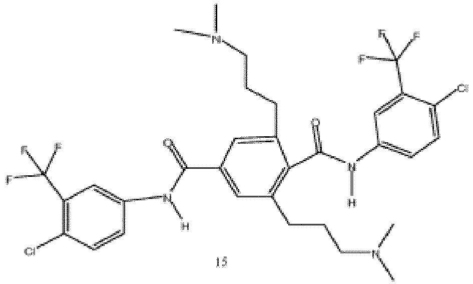 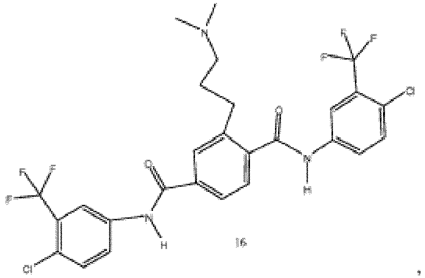 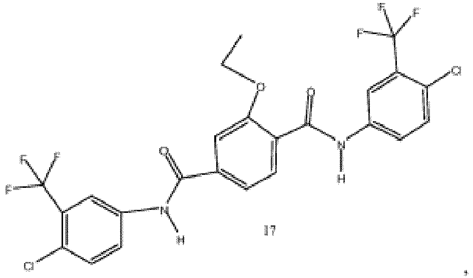 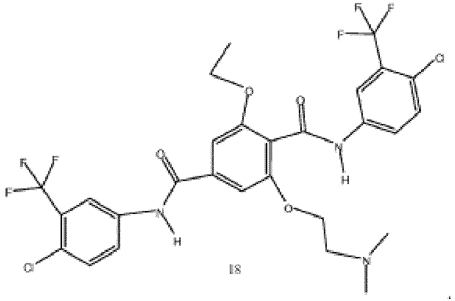
wherein R of compound 3 is H, Methyl, Ethyl, n-Propyl, Isopropyl, n-butyl, t- butyl, C₈H₁₈,C₁₅H₂₆, C₁₅H₂₈, C₁₅H₃₀, or C₁₅H₃₂.

2. The compound of claim 1, wherein the compound is:

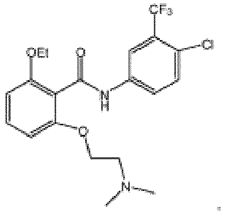

3. A method for screening compounds of Formula (I) in a non-human animal to treat conditions associated with accumulated amyloid-beta peptide deposits, the method comprising:
a) administering a HAT Activator compound of Formula (I) to a non-human animal model of amyloid-beta peptide deposit accumulation; and
b) selecting a HAT Activator compound of Formula (I) that can modulate histone acetylation after administration of the HAT Activator compound in a non-human animal model of amyloid-beta peptide deposit accumulation.

4. A method for identifying a histone acetyltransferase (HAT) activator compound of Formula (I) in a non-human animal to treat conditions associated with accumulated amyloid-beta peptide deposits, wherein the method comprises selecting a HAT Activator compound of Formula (I) having one or more of the following features:
a) the EC50 of the compound is no more than about 1000 nM;
b) the histone acetylation activity in vitro targets histone protein H2, H3, and/or H4; and
c) the compound penetrates the blood brain barrier; or a combination thereof.

5. The method of claim 3, wherein the compound has a molecular mass less than about 500 Da, has a polar surface area less than about 90 A², has less than 8 hydrogen bonds, or a combination thereof, in order to penetrate the blood brain barrier.

6. A compound of Formula I for use in reducing amyloid beta (Aβ) protein deposits in a subject by administering to the subject an effective amount of a composition comprising a HAT Activator compound of Formula (I), thereby decreasing Aβ protein deposits in the subject.

7. The compound of claim 6 for use, wherein the subject exhibits abnormally elevated levels of amyloid beta plaques or
wherein the subject is afflicted with Alzheimer's disease, Lewy body dementia, inclusion body myositis, or cerebral amyloid angiopathy, or
wherein the Aβ protein deposit comprises an Aβ₄₀ isomer, an Aβ₄₂ isomer, or a combination thereof.

8. A compound of Formula I for use in treating Alzheimer's Disease in a subject by administering to a subject a therapeutic amount of a pharmaceutical composition comprising a compound of Formula (I), preferably a HAT activator compound of Formula (I).

9. A compound of Formula I for use in increasing memory retention in a subject afflicted with a neurodegenerative disease by administering to a subject a therapeutic amount of a pharmaceutical composition comprising the compound of Formula (I), or
a compound of Formula I for use in increasing synaptic plasticity in a subject afflicted with a neurodegenerative disease by administering to a subject a therapeutic amount of a composition that increases histone acetylation in the subject, wherein the composition comprises the compound of Formula (I), or
a compound of Formula I for use in treating a neurodegenerative disease in a subject by administering to a subject a therapeutic amount of a pharmaceutical composition comprising the compound of Formula (I), thereby treating the neurodegenerative disease in the subject, or
a compound of Formula I for use in decreasing inclusion bodies in a subject afflicted with a neurodegenerative disorder by administering to the subject an effective amount of a composition comprising a HAT Activator compound of Formula (I), thereby decreasing inclusion bodies in the subject, or
a compound of Formula I for use in ameliorating symptoms of Parkinson's Disease in a subject by administering to a subject a therapeutic amount of a pharmaceutical composition comprising the compound of Formula (I), or
a compound of Formula I for use in ameliorating symptoms of Parkinson's Disease, by administering to a subject a therapeutic amount of a pharmaceutical composition comprising a HAT activator compound of Formula (I), or
a compound of Formula I for use in treating Huntington's Disease in a subject by administering to a subject a therapeutic amount of a pharmaceutical composition comprising the compound of Formula (I), or
a compound of Formula I for use in treating Huntington's Disease by administering to a subject a therapeutic amount of a pharmaceutical composition comprising a HAT activator compound of Formula (I).

10. The compound of Formula I for use of claim 6, 8 or 9, wherein the compound is 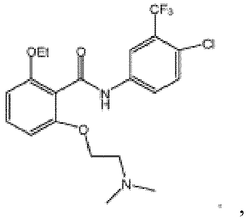 or
wherein the effective amount is at least 1 mg/kg body weight, at least 2 mg/kg body weight, at least 3 mg/kg body weight, at least 4 mg/kg body weight, at least 5 mg/kg body weight, at least 6 mg/kg body weight, at least 7 mg/kg body weight, at least 8 mg/kg body weight, at least 9 mg/kg body weight, at least 10 mg/kg body weight, at least 15 mg/kg body weight, at least 20 mg/kg body weight, at least 25 mg/kg body weight, at least 30 mg/kg body weight, at least 40 mg/kg body weight, at least 50 mg/kg body weight, at least 75 mg/kg body weight, or at least 100 mg/kg body weight, or
wherein the composition crosses the blood brain barrier, or
wherein the neurodegenerative disease comprises Adrenoleukodystrophy (ALD), Alcoholism, Alexander's disease, Alper's disease, Alzheimer's disease, Amyotrophic lateral sclerosis (Lou Gehrig's Disease), Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, Familial fatal insomnia, Frontotemporal lobar degeneration, Huntington's disease, HIV-associated dementia, Kennedy's disease, Krabbe's disease, Lewy body dementia, Neuroborreliosis, Machado- Joseph disease (Spinocerebellar ataxia type 3), Multiple System Atrophy, Multiple sclerosis, Narcolepsy, Niemann Pick disease, Parkinson's disease, Pelizaeus-Merzbacher Disease, Pick's disease, Primary lateral sclerosis, Prion diseases, Progressive Supranuclear Palsy, Rett's syndrome, Tau-positive FrontoTemporal dementia, Tau-negative FrontoTemporal dementia, Refsum's disease, Sandhoff disease, Schilder's disease, Subacute combined degeneration of spinal cord secondary to Pernicious Anaemia, Spielmeyer-Vogt-Sjogren- Batten disease, Batten disease, Spinocerebellar ataxia, Spinal muscular atrophy, Steele-Richardson-Olszewski disease, Tabes dorsalis, or Toxic encephalopathy, or
wherein synaptic plasticity comprises learning, memory, or a combination thereof, or
wherein synaptic plasticity comprises long term
potentiation (LTP).

11. The compound of Formula I for use of claim 6, 8 or 9 wherein the compound is: 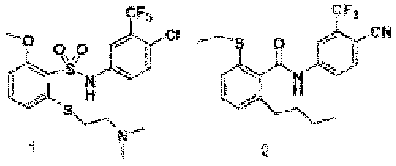 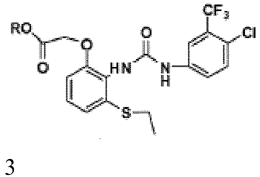 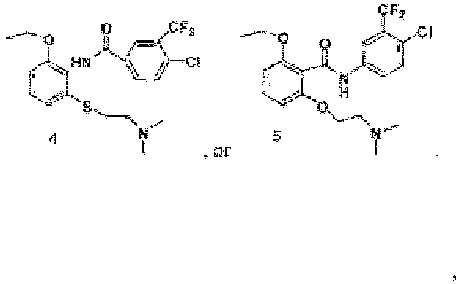 or
wherein the compound is: 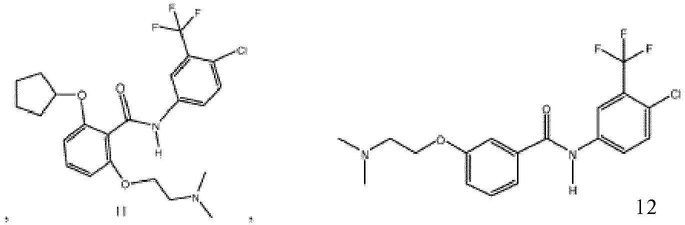 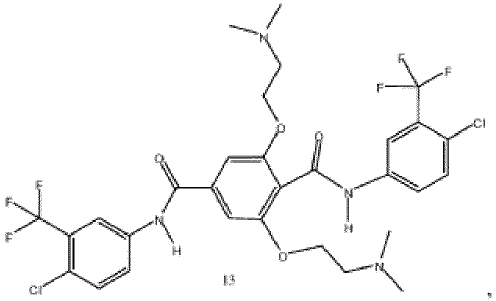 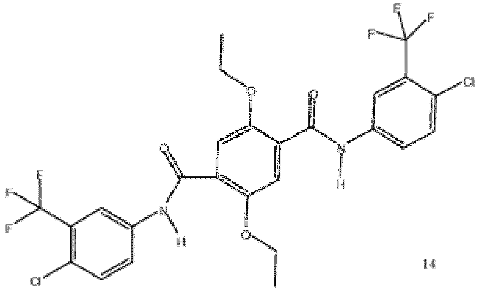 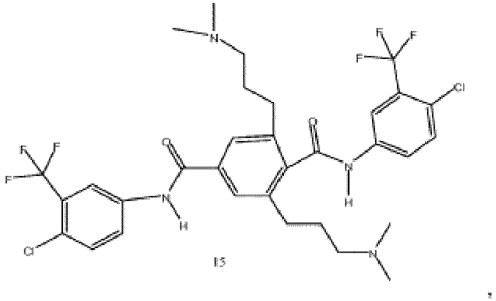 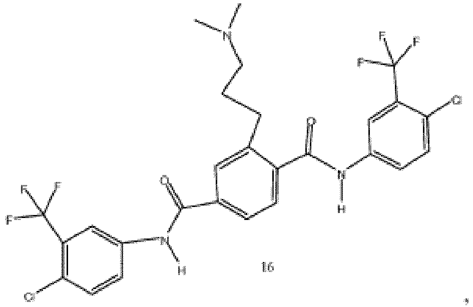 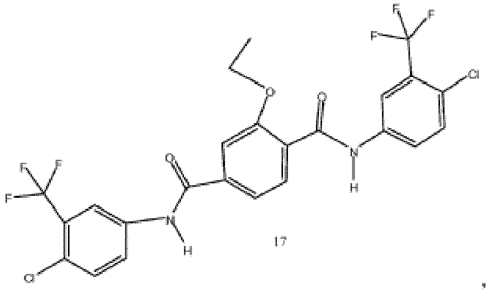 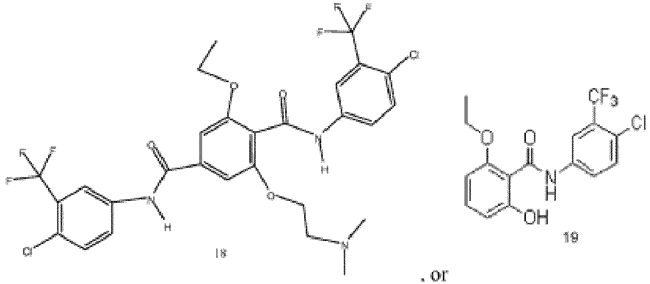 , or
wherein the compound increases histone acetylation.

12. The compound of Formula I for use of claim 11, wherein histone acetylation comprises acetylation of histones H2B, H3, H4, or a combination thereof, or
wherein histone acetylation comprises acetylation of histone lysine residues H3K4, H3K9, H3K14, H4K5, H4K8, H4K12, H4K16, or a combination thereof, or
wherein the inclusion bodies comprise beta-amyloid peptides, native and phosphorylated Tau proteins, native and phosphorylated alpha-synuclein, lipofuscin, cleaved TARDBP (TDB-43), or a combination thereof, or
the compound of Formula I for use of claim 9, wherein the symptoms of Parkinson's Disease comprise tremor, bradykinesia, dyskinesia, rigidity, postural instability, dystonia, akathisia, dementia, impaired gross motor coordination, or a combination thereof, optionally
wherein the postural instability comprises impaired imbalance, impaired coordination, or a combination thereof.

## Patentansprüche

1. Verbindung der Formel (I): 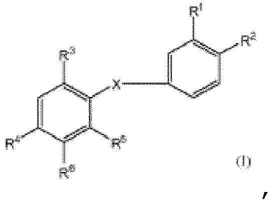 worin:
R¹ CF₃ ist;
R² Cl oder CN ist;
R³ H, O-Methyl, O-Ethyl, S-Ethyl, O-Cyclopentyl, OCH₂CH₂N(CH₃)₂ oder CH₂CH₂CH₂N(CH₃)₂ ist;
R⁴ H oder C(=O)NH-Phenyl ist, wobei das Phenyl mit einem oder mehreren Halogenen oder CF₃ substituiert ist;
R⁵ H, C₁-C₅-Alkyl, OH, OCH₃, O-Ethyl, OCH₂CH₂N(CH₃)₂, CH₂CH₂CH₂N(CH₃)₂, SCH₂CH₂N(CH₃)₂ oder OCH₂C(=O)O-Alkyl ist;
R⁶ H, O-Methyl, O-Ethyl, OCH₂CH₂N(CH₃)₂ ist; und X CONH, SONH, SO₂NH, NHC(=O)NH oder NHCO oder ein pharmazeutisch unbedenkliches Salz oder Hydrat davon ist,
wobei die Verbindung: 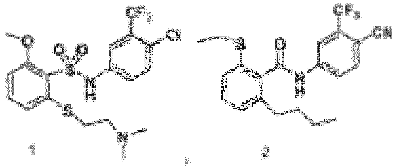 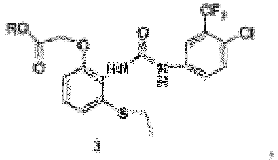 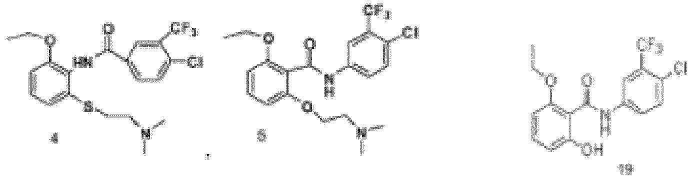 oder 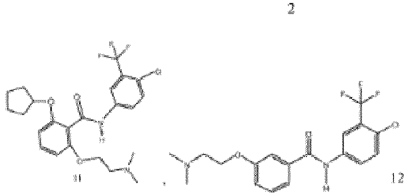 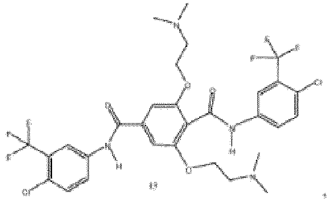 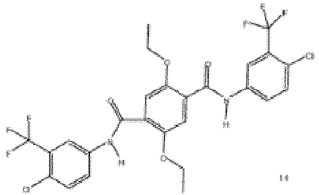 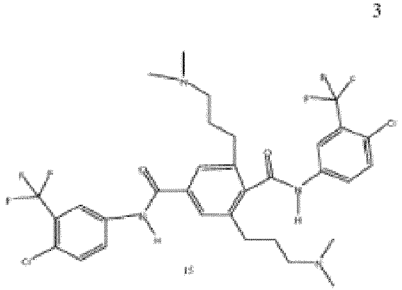 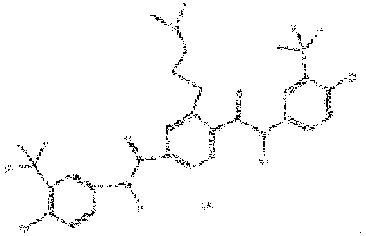 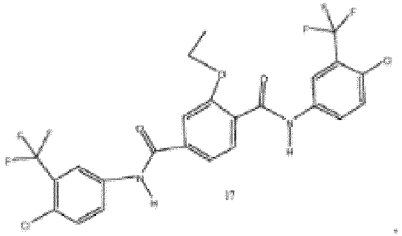 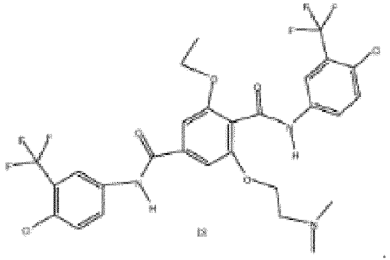 ist,
worin R der Verbindung 3 H, Methyl, Ethyl, n-Propyl, Isopropyl, n-Butyl, t-Butyl, C₈H₁₈, C₁₅H₂₆, C₁₅H₂₈, C₁₅H₃₀ oder C₁₅H₃₂ ist.

2. Verbindung nach Anspruch 1, wobei die Verbindung 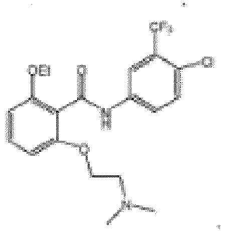 ist.

3. Verfahren zum Screenen von Verbindungen der Formel (I) in einem nicht-menschlichen Tier, um mit akkumulierten Amyloid-Beta-Peptid-Ablagerungen assoziierte Krankheiten zu behandeln, wobei das Verfahren umfasst:
a) Verabreichen einer HAT-Aktivatorverbindung der Formel (I) an ein nicht-menschliches Tiermodell der Amyloid-Beta-Peptid-Ablagerungsakkumulation; und
b) Auswählen einer HAT-Aktivatorverbindung der Formel (I), die die Histon-Acetylierung nach der Verabreichung der HAT-Aktivatorverbindung in einem nicht-menschlichen Tiermodell der Amyloid-Beta-Peptid-Ablagerungsakkumulation modulieren kann.

4. Verfahren zum Identifizieren einer Histon-Acetyltransferase (HAT)-Aktivatorverbindung der Formel (I) in einem nicht-menschlichen Tier, um mit akkumulierten Amyloid-Beta-Peptid-Ablagerungen assoziierte Erkrankungen zu behandeln, wobei das Verfahren das Auswählen einer HAT-Aktivatorverbindung der Formel (I) umfasst, die eines oder mehrere der folgenden Merkmale aufweist:
a) die EC50 der Verbindung beträgt nicht mehr als ungefähr 1000 nM;
b) die Histon-Acetylierungsaktivität in vitro zielt auf das Histon-Protein H2, H3 und/oder H4; und
c) die Verbindung passiert die Blut-Hirn-Schranke;
oder eine Kombination davon.

5. Verfahren nach Anspruch 3, wobei die Verbindung ein Molekulargewicht von weniger als ungefähr 500 Da aufweist, eine polare Oberfläche von weniger als ungefähr 90 A² aufweist, weniger als 8 Wasserstoffverbindungen aufweist, oder eine Kombination davon, um die Blut-Hirn-Schranke zu passieren.

6. Verbindung der Formel I zur Verwendung in der Reduktion von Amyloid-Beta(Aß)-Protein-Ablagerungen in einem Subjekt durch Verabreichen einer wirksamen Menge einer Zusammensetzung, die eine HAT-Aktivatorverbindung der Formel (I) umfasst, an das Subjekt, wodurch (Aß)-Protein-Ablagerungen in dem Subjekt abnehmen.

7. Verbindung nach Anspruch 6 zur Verwendung, wobei das Subjekt abnormal erhöhte Spiegel von Amyloid-Beta-Plaques zeigt oder
wobei das Subjekt an Alzheimer-Krankheit, Lewy-Körper-Demenz, Einschlusskörpermyositis oder zerebraler Amyloidangiopathie leidet, oder
wobei die Aß-Protein-Ablagerung ein Aβ₄₀-Isomer, ein Aβ₄₂-Isomer oder eine Kombination davon umfasst.

8. Verbindung der Formel I zur Verwendung in der Behandlung der Alzheimer-Krankheit bei einem Subjekt durch Verabreichen einer therapeutischen Menge einer pharmazeutischen Zusammensetzung, die eine Verbindung der Formel (I), vorzugsweise eine HAT-Aktivatorverbindung der Formel (I) umfasst, an ein Subjekt.

9. Verbindung der Formel I zur Verwendung in der Steigerung der Gedächtnisleistung bei einem Subjekt, das an einer neurodegenerativen Erkrankung leidet, durch Verabreichen einer therapeutischen Menge einer pharmazeutischen Zusammensetzung, die die Verbindung der Formel (I) umfasst, an ein Subjekt, oder
Verbindung der Formel I zur Verwendung in der Steigerung der synaptischen Plastizität bei einem Subjekt, das an einer neurodegenerativen Erkrankung leidet, durch Verabreichen einer therapeutischen Menge einer pharmazeutischen Zusammensetzung, die die Histon-Acetylierung bei dem Subjekt erhöht, an ein Subjekt, wobei die Zusammensetzung die Verbindung der Formel (I) umfasst, oder
Verbindung der Formel I zur Verwendung in der Behandlung einer neurodegenerativen Erkrankung bei einem Subjekt durch Verabreichen einer therapeutischen Menge einer pharmazeutischen Zusammensetzung, die die Verbindung der Formel (I) umfasst, an ein Subjekt, wodurch die neurodegenerative Erkrankung bei dem Subjekt behandelt wird, oder
Verbindung der Formel I zur Verwendung in der Senkung von Inklusionskörpern bei einem Subjekt, das an einer neurodegenerativen Störung leidet, durch Verabreichen einer wirksamen Menge einer Zusammensetzung, die eine HAT-Aktivatorverbindung der Formel (I) umfasst, an das Subjekt, wodurch die Inklusionskörper bei dem Subjekt abnehmen, oder
Verbindung der Formel I zur Verwendung in der Linderung der Symptome der Parkinson-Krankheit bei einem Subjekt durch Verabreichen einer therapeutischen Menge einer pharmazeutischen Zusammensetzung, die die Verbindung der Formel (I) umfasst, an ein Subjekt, oder
Verbindung der Formel I zur Verwendung in der Linderung der Symptome der Parkinson-Krankheit durch Verabreichen einer therapeutischen Menge einer pharmazeutischen Zusammensetzung, die eine HAT-Aktivatorverbindung der Formel (I) umfasst, an ein Subjekt, oder
Verbindung der Formel I zur Verwendung in der Behandlung der Huntington-Krankheit bei einem Subjekt durch Verabreichen einer therapeutischen Menge einer pharmazeutischen Zusammensetzung, die die Verbindung der Formel (I) umfasst, an ein Subjekt, oder
Verbindung der Formel I zur Verwendung in der Behandlung der Huntington-Krankheit durch Verabreichen einer therapeutischen Menge einer pharmazeutischen Zusammensetzung, die eine HAT-Aktivatorverbindung der Formel (I) umfasst, an ein Subjekt.

10. Verbindung der Formel I zur Verwendung nach Anspruch 6, 8 oder 9, wobei die Verbindung 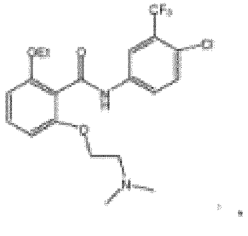 ist, oder
wobei die wirksame Menge mindestens 1 mg/kg Körpergewicht, mindestens 2 mg/kg Körpergewicht, mindestens 3 mg/kg Körpergewicht, mindestens 4 mg/kg Körpergewicht, mindestens 5 mg/kg Körpergewicht, mindestens 6 mg/kg Körpergewicht, mindestens 7 mg/kg Körpergewicht, mindestens 8 mg/kg Körpergewicht, mindestens 9 mg/kg Körpergewicht, mindestens 10 mg/kg Körpergewicht, mindestens 15 mg/kg Körpergewicht, mindestens 20 mg/kg Körpergewicht, mindestens 25 mg/kg Körpergewicht, mindestens 30 mg/kg Körpergewicht, mindestens 40 mg/kg Körpergewicht, mindestens 50 mg/kg Körpergewicht, mindestens 75 mg/kg Körpergewicht oder mindestens 100 mg/kg Körpergewicht beträgt, oder
wobei die Zusammensetzung die Blut-Hirn-Schranke überwindet, oder
wobei die neurodegenerative Erkrankung Adrenoleukodystrophie (ALD), Alkoholismus, Alexander-Krankheit, Alpers-Krankheit, Alzheimer-Krankheit, amyotrophe Lateralsklerose (Lou-Gehrig-Syndrom), Ataxia telangiectasia, Batten-Syndrom (auch bekannt als Spielmeyer-Vogt-Sjögren-Batten-Krankheit), Bovine Spongiform Encephalopathie (BSE), Canavan-Krankheit, Cockayne-Syndrom, corticobasale Degeneration, Creutzfeldt-Jakob-Krankheit, tödliche familiäre Schlaflosigkeit, frontotemporale Lobärdegeneration, Huntington-Krankheit, HIV-assoziierte Demenz, Kennedy-Krankheit, Krabbe-Krankheit, Lewy-Körper-Demenz, Neuroborreliose, Machado-Joseph-Krankheit (spinocerebelläre Ataxie 3), Multisystematrophie, Multiple Sklerose, Narkolepsie, Niemann-Pick-Krankheit, Parkinson-Krankheit, Pelizaeus-Merzbacher-Krankheit, Pick-Krankheit, primäre Lateralsklerose, Prion-Krankheiten, progressive supranukleäre Blickparese, Rett-Syndrom, Tau-positive frontotemporale Demenz, Tau-negative frontotemporale Demenz, Refsum-Syndrom, Sandhoff-Krankheit, Schilder-Krankheit, funikuläre Spinalerkrankung sekundär zu perniziöser Anämie, Spielmeyer-Vogt-Sjögren-Batten-Syndrom, Batten-Syndrom, spinocerebelläre Ataxie, spinale Muskelatrophie, Steele-Richardson-Olszewski-Syndrom, Tabes dorsalis oder toxische Enzephalopathie umfasst, oder
wobei synaptische Plastizität das Lernen, das Gedächtnis oder eine Kombination davon umfasst, oder
wobei synaptische Plastizität Langzeit-Potenzierung (LTP) umfasst.

11. Verbindung der Formel I zur Verwendung nach Anspruch 6, 8 oder 9, wobei die Verbindung 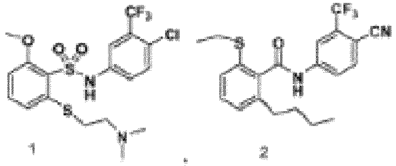 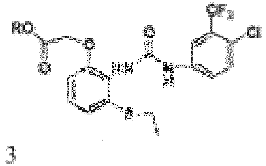 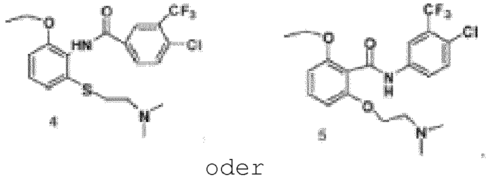 ist, oder
wobei die Verbindung: 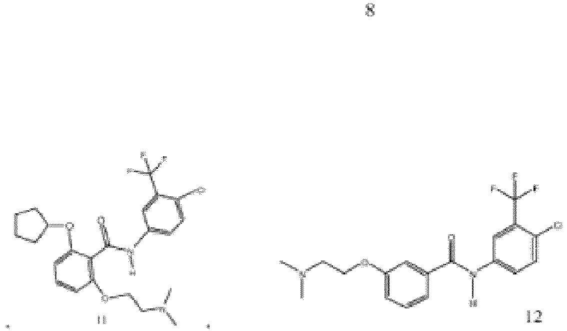 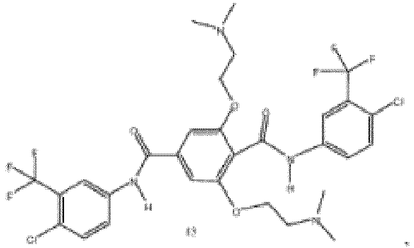 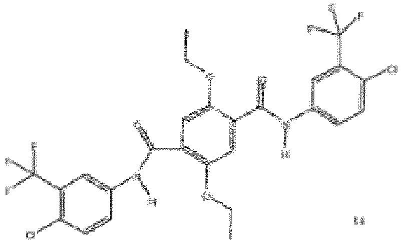 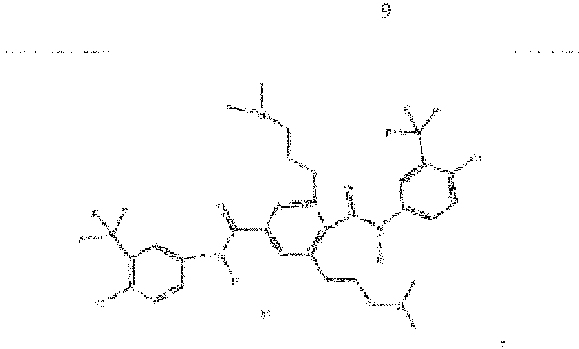 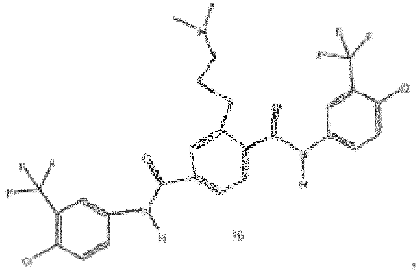 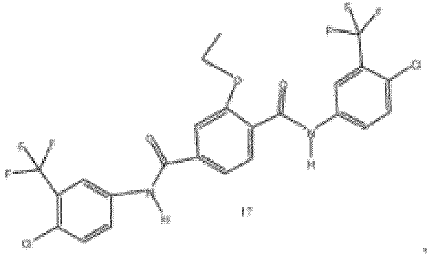 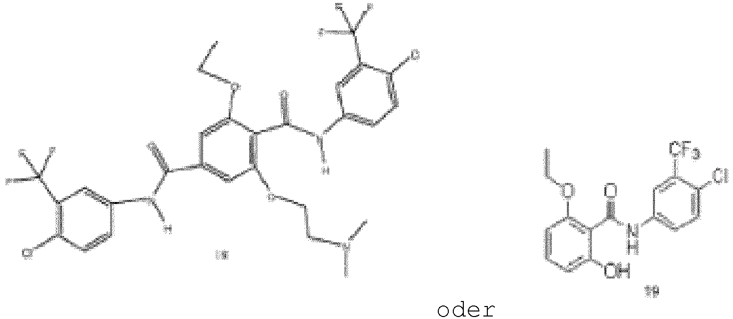 ist, oder
wobei die Verbindung die Histon-Acetylierung erhöht.

12. Verbindung der Formel I zur Verwendung nach Anspruch 11, wobei die Histon-Acetylierung die Acetylierung der Histone H2B, H3, H4 oder eine Kombination davon umfasst, oder
wobei Histon-Acetylierung die Acetylierung von Histon-Lysin-Resten H3K4, H3K9, H3K14, H4K5, H4K8, H4K12, H4K16 oder eine Kombination davon umfasst, oder
wobei die Inklusionskörper Beta-Amyloid-Peptide, native und phosphorylierte Tau-Proteine, natives und phosphoryliertes Alpha-Synuclein, Lipofuscin, gespaltenes TARDBP (TDB-43) oder eine Kombination davon umfasst, oder
Verbindung der Formel I zur Verwendung nach Anspruch 9, wobei die Symptome der Parkinson-Krankheit Tremor, Bradykinesie, Dyskinesie, Rigidität, posturale Instabilität, Dystonie, Akathisie, Demenz, gestörte Grobmotorik/Koordination oder eine Kombination umfassen, wobei
optional die posturale Instabilität, Gleichgewichtsstörungen, Koordinationsstörungen oder eine Kombination davon umfasst.

## Revendications

1. Composé de formule (I) : 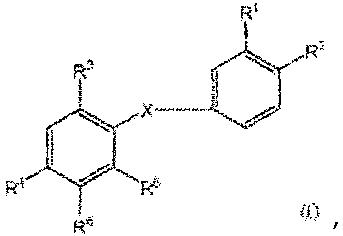 dans lequel
R¹ est CF₃ ;
R² est Cl ou CN ;
R³ est H ou un groupe O-méthyle, O-éthyle, S-éthyle, O-cyclopentyle, OCH₂CH₂N(CH₃)₂ ou CH₂CH₂CH₂N(CH₃)₂ ;
R⁴ est H ou un groupe C(=O)NH-phényle, le groupe phényle étant substitué par un ou plusieurs substituants halogéno ou CF₃ ;
R⁵ est H ou un groupe alkyle en C₁-C₅, OH, OCH₃, O-éthyle, OCH₂CH₂N(CH₃)₂, CH₂CH₂CH₂N(CH₃)₂, SCH₂CH₂N(CH₃)₂ ou OCH₂C(=O)O-alkyle ;
R⁶ est H ou un groupe O-méthyle, O-éthyle, OCH₂CH₂N(CH₃)₂ ; et
X est CONH, SONH, SO₂NH, NHC(=O)NH ou NHCO,
ou sel pharmaceutiquement acceptable ou hydrate de celui-ci,
le composé étant : 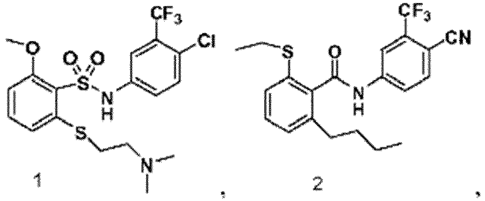 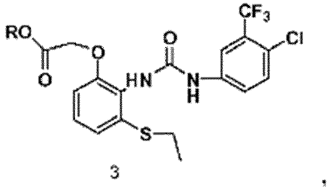 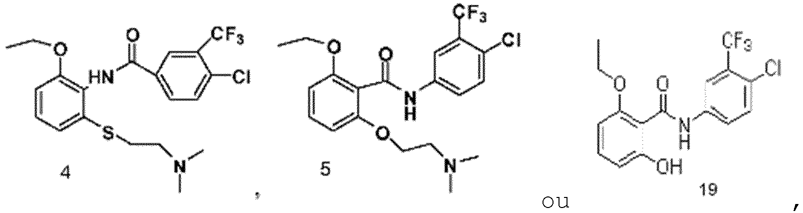 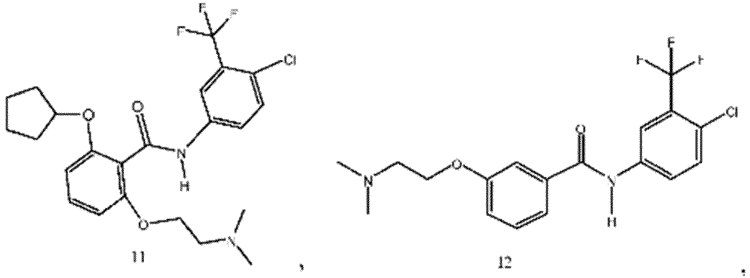 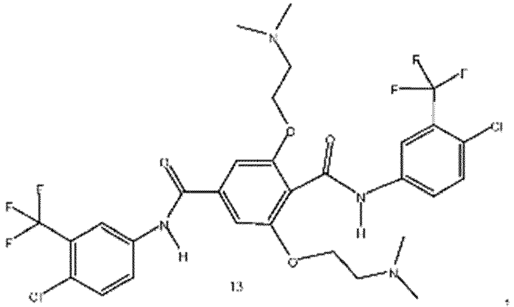 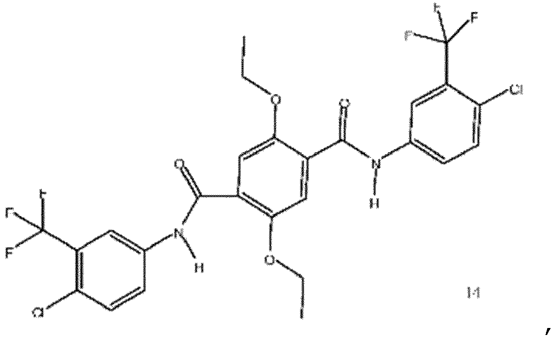 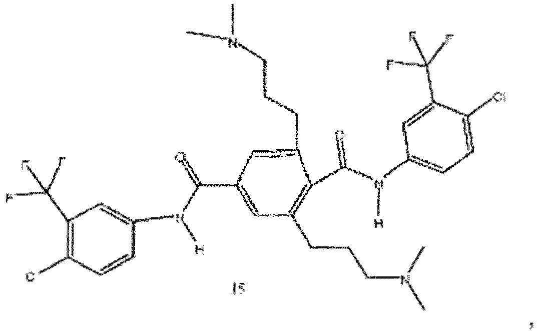 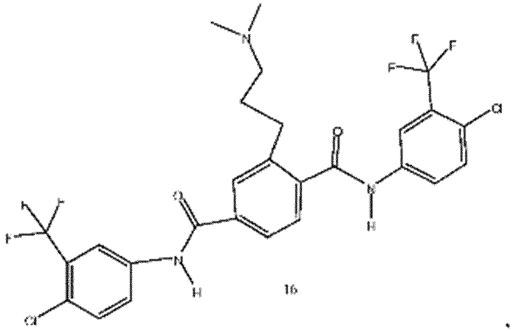 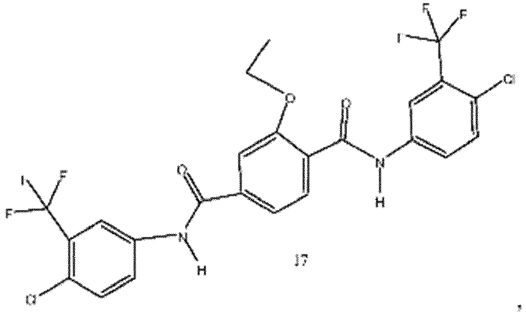 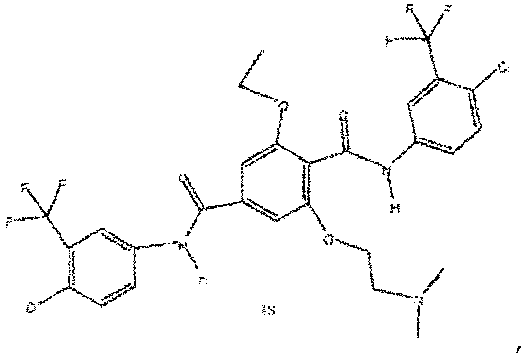 le groupe R du composé 3 étant H ou un groupe méthyle, éthyle, n-propyle, isopropyle, n-butyle, t-butyle, C₈H₁₈, C₁₅H₂₆, C₁₅H₂₈, C₁₅H₃₀ ou C₁₅H₃₂.

2. Composé selon la revendication 1, le composé étant :

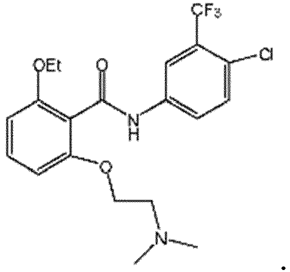

3. Procédé pour le criblage de composés de formule (I) dans un animal non humain pour traiter des affections associées à des dépôts accumulés de peptide bêta-amyloïde, le procédé comprenant :
a) l'administration d'un composé activateur de HAT de formule (I) à un modèle animal non humain d'accumulation de dépôts de peptide bêta-amyloïde ; et
b) la sélection d'un composé activateur de HAT de formule (I) qui peut moduler l'acétylation d'histones après administration du composé activateur de HAT dans un modèle animal non humain d'accumulation de dépôts de peptide bêta-amyloïde.

4. Procédé pour l'identification d'un composé activateur d'histone acétyltransférase (HAT) de formule (I) dans un animal non humain pour traiter des affections associées à des dépôts accumulés de peptide bêta-amyloïde, le procédé comprenant la sélection d'un composé activateur de HAT de formule (I) ayant une ou plusieurs des caractéristiques suivantes :
a) la CE₅₀ du composé est inférieure ou égale à environ 1000 nM ;
b) l'activité d'acétylation d'histones *in vitro* cible la protéine histone H2, H3 et/ou H4 ; et
c) le composé passe à travers la barrière hématoencéphalique ;
ou une combinaison de celles-ci.

5. Procédé selon la revendication 3, dans lequel le composé a une masse moléculaire inférieure à environ 500 Da, une surface polaire inférieure à environ 90 Å², moins de 8 liaisons hydrogène ou une combinaison de cela, afin de passer à travers la barrière hématoencéphalique.

6. Composé de formule I destiné à être utilisé en réduction de dépôts de protéine bêta-amyloïde (Aβ) chez un sujet par administration au sujet d'une quantité efficace d'une composition comprenant un composé activateur de HAT de formule (I), ce qui diminue ainsi les dépôts de protéine Aβ chez le sujet.

7. Composé destiné à être utilisé selon la revendication 6,
le sujet présentant des taux anormalement élevés de plaques de bêta-amyloïde ou
le sujet étant atteint de maladie d'Alzheimer, de démence à corps de Lewy, de myosite à corps d'inclusion ou d'angiopathie amyloïde cérébrale ou
le dépôt de protéine Aβ comprenant un isomère Aβ₄₀, un isomère Aβ₄₂ ou une combinaison de ceux-ci.

8. Composé de formule I destiné à être utilisé en traitement de la maladie d'Alzheimer chez un sujet par administration à un sujet d'une quantité thérapeutique d'une composition pharmaceutique comprenant un composé de formule (I), de préférence un composé activateur de HAT de formule (I).

9. Composé de formule I destiné à être utilisé en augmentation de la fixation mnémonique chez un sujet atteint d'une maladie neurodégénérative par administration à un sujet d'une quantité thérapeutique d'une composition pharmaceutique comprenant le composé de formule (I) ou
composé de formule I destiné à être utilisé en augmentation de la plasticité synaptique chez un sujet atteint d'une maladie neurodégénérative par administration à un sujet d'une quantité thérapeutique d'une composition qui augmente l'acétylation d'histones chez le sujet, la composition comprenant le composé de formule (I), ou
composé de formule I destiné à être utilisé en traitement d'une maladie neurodégénérative chez un sujet par administration à un sujet d'une quantité thérapeutique d'une composition pharmaceutique comprenant le composé de formule (I), ce qui traite ainsi la maladie neurodégénérative chez le sujet, ou
composé de formule I destiné à être utilisé en diminution de corps d'inclusion chez un sujet atteint d'un trouble neurodégénératif par administration au sujet d'une quantité efficace d'une composition comprenant un composé activateur de HAT de formule (I), ce qui diminue ainsi les corps d'inclusion chez le sujet, ou
composé de formule I destiné à être utilisé en amélioration de symptômes de la maladie de Parkinson chez un sujet par administration à un sujet d'une quantité thérapeutique d'une composition pharmaceutique comprenant le composé de formule (I) ou
composé de formule I destiné à être utilisé en amélioration de symptômes de la maladie de Parkinson, par administration à un sujet d'une quantité thérapeutique d'une composition pharmaceutique comprenant un composé activateur de HAT de formule (I), ou
composé de formule I destiné à être utilisé en traitement de la maladie de Huntington chez un sujet par administration à un sujet d'une quantité thérapeutique d'une composition pharmaceutique comprenant le composé de formule (I) ou
composé de formule I destiné à être utilisé en traitement de la maladie de Huntington par administration à un sujet d'une quantité thérapeutique d'une composition pharmaceutique comprenant un composé activateur de HAT de formule (I).

10. Composé de formule I destiné à être utilisé selon la revendication 6, 8 ou 9, le composé étant 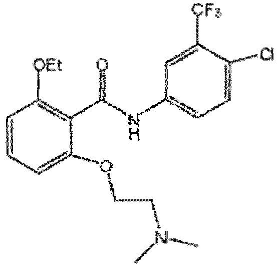 ou
la quantité efficace étant d'au moins 1 mg/kg de poids corporel, d'au moins 2 mg/kg de poids corporel, d'au moins 3 mg/kg de poids corporel, d'au moins 4 mg/kg de poids corporel, d'au moins 5 mg/kg de poids corporel, d'au moins 6 mg/kg de poids corporel, d'au moins 7 mg/kg de poids corporel, d'au moins 8 mg/kg de poids corporel, d'au moins 9 mg/kg de poids corporel, d'au moins 10 mg/kg de poids corporel, d'au moins 15 mg/kg de poids corporel, d'au moins 20 mg/kg de poids corporel, d'au moins 25 mg/kg de poids corporel, d'au moins 30 mg/kg de poids corporel, d'au moins 40 mg/kg de poids corporel, d'au moins 50 mg/kg de poids corporel, d'au moins 75 mg/kg de poids corporel ou d'au moins 100 mg/kg de poids corporel ou
la composition traversant la barrière hématoencéphalique ou
la maladie neurodégénérative comprenant l'adrénoleucodystrophie (ALD), l'alcoolisme, la maladie d'Alexander, la maladie d'Alper, la maladie d'Alzheimer, la sclérose latérale amyotrophique (la maladie de Lou Gehrig), l'ataxie télangiectasie, la maladie de Batten (également appelée maladie de Spielmeyer-Vogt-Sjögren-Batten), l'encéphalopathie spongiforme bovine (ESB), la maladie de Canavan, le syndrome de Cockayne, la dégénérescence cortico-basale, la maladie de Creutzfeldt-Jakob, l'insomnie fatale familiale, la dégénérescence lobaire frontotemporale, la maladie de Huntington, la démence associée au VIH, la maladie de Kennedy, la maladie de Krabbe, la démence à corps de Lewy, la neuroborréliose, la maladie de Machado-Joseph (l'ataxie spinocérébelleuse de type 3), l'atrophie multisystématisée, la sclérose en plaques, la narcolepsie, la maladie de Niemann-Pick, la maladie de Parkinson, la maladie de Pelizaeus-Merzbacher, la maladie de Pick, la sclérose latérale primitive, les maladies à prion, la paralysie supranucléaire progressive, le syndrome de Rett, la démence frontotemporale tau-positive, la démence frontotemporale tau-négative, la maladie de Refsum, la maladie de Sandhoff, la maladie de Schilder, la dégénérescence subaiguë combinée de la moelle épinière consécutive à une anémie pernicieuse, la maladie de Spielmeyer-Vogt-Sjögren-Batten, la maladie de Batten, l'ataxie spinocérébelleuse, l'amyotrophie spinale, la maladie de Steele-Richardson-Olszewski, le tabes dorsalis ou l'encéphalopathie toxique ou
la plasticité synaptique comprenant l'apprentissage, la mémoire ou une combinaison de ceux-ci ou
la plasticité synaptique comprenant la potentialisation à long terme (PLT).

11. Composé de formule I destiné à être utilisé selon la revendication 6, 8 ou 9,
le composé étant : 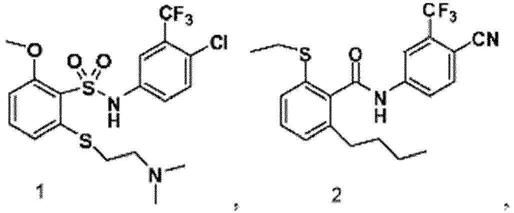 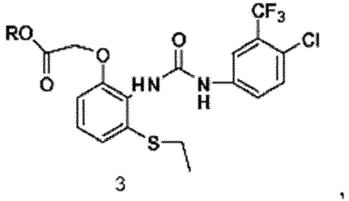 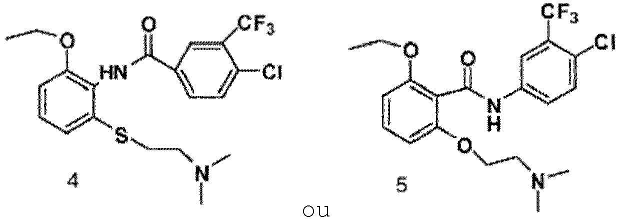 ou
le composé étant : 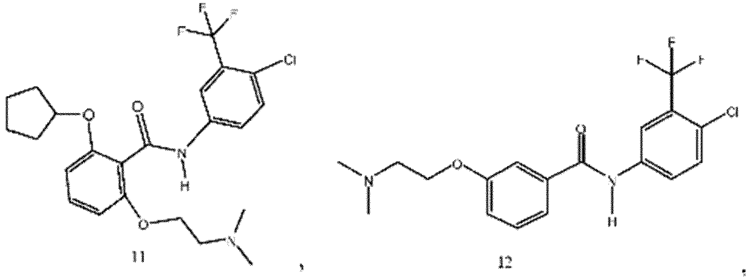 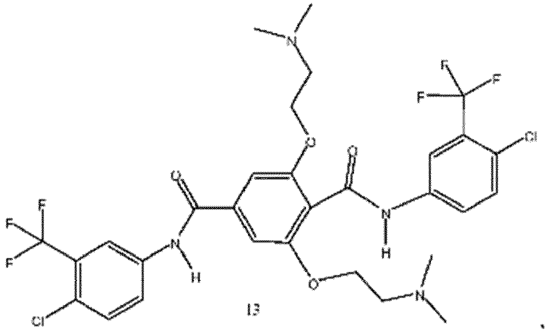 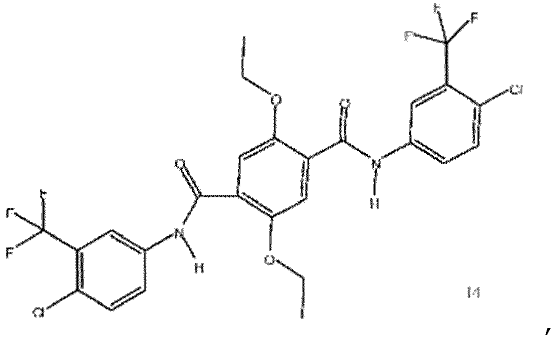 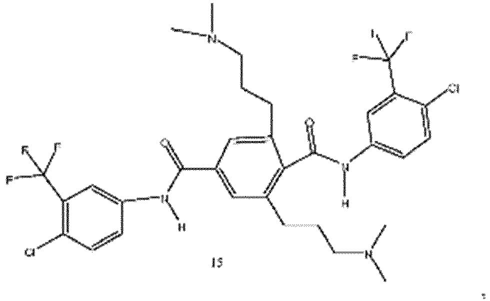 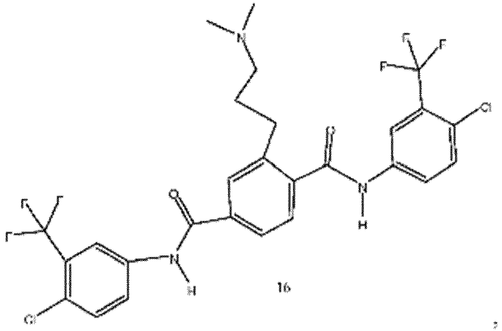 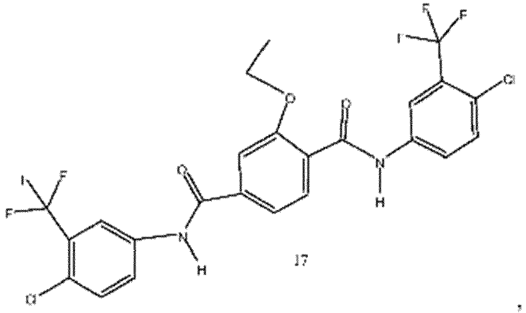 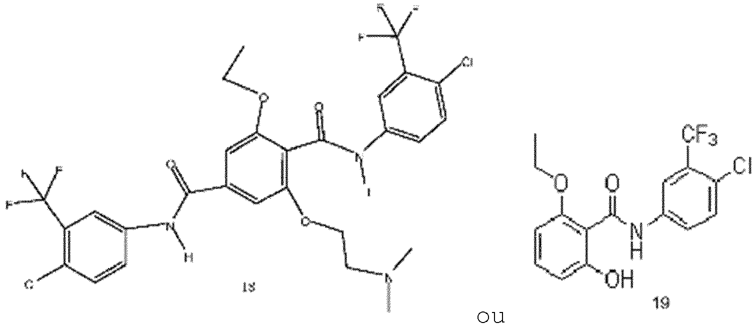 ou
le composé augmentant l'acétylation d'histones.

12. Composé de formule I destiné à être utilisé selon la revendication 11,
l'acétylation d'histones comprenant l'acétylation des histones H2B, H3, H4 ou d'une combinaison de celles-ci ou
l'acétylation d'histones comprenant l'acétylation de résidus de lysine d'histones H3K4, H3K9, H3K14, H4K5, H4K8, H4K12, H4K16 ou d'une combinaison de ceux-ci ou
les corps d'inclusion comprenant des peptides bêta-amyloïdes, des protéines Tau natives et phosphorylées, l'alpha-synucléine native et phosphorylée, la lipofuscine, la TARDBP coupée (TDB-43) ou une combinaison de ceux-ci ou
composé de formule I destiné à être utilisé selon la revendication 9, les symptômes de la maladie de Parkinson comprenant des tremblements, la bradykinésie, la dyskinésie, la rigidité, l'instabilité posturale, la dystonie, l'akathisie, la démence, la mauvaise coordination motrice globale ou une combinaison de ceux-ci, éventuellement
l'instabilité posturale comprenant un mauvais équilibre, une mauvaise coordination ou une combinaison de ceux-ci.


### SEQUENCE LISTING

<110> THE TRUSTEES OF COLUMBIA UNIVERSITY IN THE CITY OF NEW YORK
<120> HISTONE ACETYLTRANSFERASE ACTIVATORS AND USES THEREOF
<130> JL64119P.EPP
<140> EP10836767.3
   <141> 2010-12-10
<150> PCT/US2010/059925
   <151> 2010-12-10
<150> 61/363,009
   <151> 2010-07-09
<150> 61/354,964
   <151> 2010-06-15
<150> 61/355,110
   <151> 2010-06-15
<150> 61/317,765
   <151> 2010-03-26
<150> 61/285,287
   <151> 2009-12-10
<160> 6
<170> PatentIn version 3.5
<210> 1
   <211> 419
   <212> PRT
   <213> Homo sapiens
<400> 1
   Met Ala Gly Phe Gly Ala Met Glu Lys Phe Leu Val Glu Tyr Lys Ser
   1               5                   10                  15
   Ala Val Glu Lys Lys Leu Ala Glu Tyr Lys Cys Asn Thr Asn Thr Ala
               20                  25                  30
   Ile Glu Leu Lys Leu Val Arg Phe Pro Glu Asp Leu Glu Asn Asp Ile
           35                  40                  45
   Arg Thr Phe Phe Pro Glu Tyr Thr His Gln Leu Phe Gly Asp Asp Glu
       50                  55                  60
   Thr Ala Phe Gly Tyr Lys Gly Leu Lys Ile Leu Leu Tyr Tyr Ile Ala
   65                  70                  75                  80
   Gly Ser Leu Ser Thr Met Phe Arg Val Glu Tyr Ala Ser Lys Val Asp
                   85                  90                  95
   Glu Asn Phe Asp Cys Val Glu Ala Asp Asp Val Glu Gly Lys Ile Arg
               100                 105                 110
   Gln Ile Ile Pro Pro Gly Phe Cys Thr Asn Thr Asn Asp Phe Leu Ser
   115 120 125
   Leu Leu Glu Lys Glu Val Asp Phe Lys Pro Phe Gly Thr Leu Leu His
   130 135 140
   Thr Tyr Ser Val Leu Ser Pro Thr Gly Gly Glu Asn Phe Thr Phe Gln
   145 150 155 160
   Ile Tyr Lys Ala Asp Met Thr Cys Arg Gly Phe Arg Glu Tyr His Glu
   165 170 175
   Arg Leu Gln Thr Phe Leu Met Trp Phe Ile Glu Thr Ala Ser Phe Ile
   180 185 190
   Asp Val Asp Asp Glu Arg Trp His Tyr Phe Leu Val Phe Glu Lys Tyr
   195 200 205
   Asn Lys Asp Gly Ala Thr Leu Phe Ala Thr Val Gly Tyr Met Thr Val
   210 215 220
   Tyr Asn Tyr Tyr Val Tyr Pro Asp Lys Thr Arg Pro Arg Val Ser Gln
   225 230 235 240
   Met Leu Ile Leu Thr Pro Phe Gln Gly Gln Gly His Gly Ala Gln Leu
   245 250 255
   Leu Glu Thr Val His Arg Tyr Tyr Thr Glu Phe Pro Thr Val Leu Asp
   260 265 270
   Ile Thr Ala Glu Asp Pro Ser Lys Ser Tyr Val Lys Leu Arg Asp Phe
   275 280 285
   Val Leu Val Lys Leu Cys Gln Asp Leu Pro Cys Phe Ser Arg Glu Lys
   290 295 300
   Leu Met Gln Gly Phe Asn Glu Asp Met Ala Ile Glu Ala Gln Gln Lys
   305 310 315 320
   Phe Lys Ile Asn Lys Gln His Ala Arg Arg Val Tyr Glu Ile Leu Arg
   325 330 335
   Leu Leu Val Thr Asp Met Ser Asp Ala Glu Gln Tyr Arg Ser Tyr Arg
   340 345 350
   Leu Asp Ile Lys Arg Arg Leu Ile Ser Pro Tyr Lys Lys Lys Gln Arg
   355 360 365
   Asp Leu Ala Lys Met Arg Lys Cys Leu Arg Pro Glu Glu Leu Thr Asn
       370                 375                 380
   Gln Met Asn Gln Ile Glu Ile Ser Met Gln His Glu Gln Leu Glu Glu
   385                 390                 395                 400
   Ser Phe Gln Glu Leu Val Glu Asp Tyr Arg Arg Val Ile Glu Arg Leu
                   405                 410                 415
   Ala Gln Glu
<210> 2
   <211> 1682
   <212> DNA
   <213> Homo sapiens
<400> 2
   ctgtgcggtc acttccggcc cgggagcgcg cgggttgatt cgtccttcct cagccgcggg 60
   tgatcgtagc tcggaaatgg cgggatttgg tgctatggag aaatttttgg tagaatataa 120
   gagtgcagtg gagaagaaac tggcagagta caaatgtaac accaacacag caattgaact 180
   aaaattagtt cgttttcctg aagatcttga aaatgacatt agaactttct ttcctgagta 240
   tacccatcaa ctctttgggg atgatgaaac tgcttttggt tacaagggtc taaagatcct 300
   gttatactat attgctggta gcctgtcaac aatgttccgt gttgaatatg catctaaagt 360
   tgatgagaac tttgactgtg tagaggcaga tgatgttgag ggcaaaatta gacaaatcat 420
   tccacctgga ttttgcacaa acacgaatga tttcctttct ttactggaaa aggaagttga 480
   tttcaagcca ttcggaacct tacttcatac ctactcagtt ctcagtccaa caggaggaga 540
   aaactttacc tttcagatat ataaggctga catgacatgt agaggctttc gagaatatca 600
   tgaaaggctt cagacctttt tgatgtggtt tattgaaact gctagcttta ttgacgtgga 660
   tgatgaaaga tggcactact ttctagtatt tgagaagtat aataaggatg gagctacgct 720
   ctttgcgacc gtaggctaca tgacagtcta taattactat gtgtacccag acaaaacccg 780
   gccacgtgta agtcagatgc tgattttgac tccatttcaa ggtcaaggcc atggtgctca 840
   acttcttgaa acagttcata gatactacac tgaatttcct acagttcttg atattacagc 900
   ggaagatcca tccaaaagct atgtgaaatt acgagacttt gtgcttgtga agctttgtca 960
   agatttgccc tgtttttccc gggaaaaatt aatgcaagga ttcaatgaag atatggcgat 1020
   agaggcacaa cagaagttca aaataaataa gcaacacgct agaagggttt atgaaattct 1080
   tcgactactg gtaactgaca tgagtgatgc cgaacaatac agaagctaca gactggatat 1140
   taaaagaaga ctaattagcc catataagaa aaagcagaga gatcttgcta agatgagaaa 1200
   atgtctcaga ccagaagaac tgacaaacca gatgaaccaa atagaaataa gcatgcaaca 1260
   tgaacagctg gaagagagtt ttcaggaact agtggaagat taccggcgtg ttattgaacg 1320
   acttgctcaa gagtaaagat tatactgctc tgtacaggaa gcttgcaaat tttctgtaca 1380
   atgtgctgtg aaaaatctga tgactttaat tttaaaatct tgtgacattt tgcttatact 1440
   aaaagttatc tatctttagt tgaatatttt cttttggaga gattgtatat tttaaaatac 1500
   tgtttagagt ttatgagcat atattgcatt taaagaaaga taaagcttct gaaatactac 1560
   tgcaattgct tcccttctta aacagtataa taaatgctta gttgtgatat gttaatgtgt 1620
   gatgatatga ttcttaaata cttacaataa acctcattct taaatactta aaaaaaaaaa 1680
   aa 1682
<210> 3
   <211> 832
   <212> PRT
   <213> Homo sapiens
<400> 3
   Met Ser Glu Ala Gly Gly Ala Gly Pro Gly Gly Cys Gly Ala Gly Ala
   1               5                   10                  15
   Gly Ala Gly Ala Gly Pro Gly Ala Leu Pro Pro Gln Pro Ala Ala Leu
               20                  25                  30
   Pro Pro Ala Pro Pro Gln Gly Ser Pro Cys Ala Ala Ala Ala Gly Gly
           35                  40                  45
   Ser Gly Ala Cys Gly Pro Ala Thr Ala Val Ala Ala Ala Gly Thr Ala
       50                  55                  60
   Glu Gly Pro Gly Gly Gly Gly Ser Ala Arg Ile Ala Val Lys Lys Ala
   65                  70                  75                  80
   Gln Leu Arg Ser Ala Pro Arg Ala Lys Lys Leu Glu Lys Leu Gly Val
                   85                  90                  95
   Tyr Ser Ala Cys Lys Ala Glu Glu Ser Cys Lys Cys Asn Gly Trp Lys
               100                 105                 110
   Asn Pro Asn Pro Ser Pro Thr Pro Pro Arg Ala Asp Leu Gln Gln Ile
           115                 120                 125
   Ile Val Ser Leu Thr Glu Ser Cys Arg Ser Cys Ser His Ala Leu Ala
       130                 135                 140
   Ala His Val Ser His Leu Glu Asn Val Ser Glu Glu Glu Met Asn Arg
   145                 150                 155                 160
   Leu Leu Gly Ile Val Leu Asp Val Glu Tyr Leu Phe Thr Cys Val His
                   165                 170                 175
   Lys Glu Glu Asp Ala Asp Thr Lys Gln Val Tyr Phe Tyr Leu Phe Lys
               180                 185                 190
   Leu Leu Arg Lys Ser Ile Leu Gln Arg Gly Lys Pro Val Val Glu Gly
           195                 200                 205
   Ser Leu Glu Lys Lys Pro Pro Phe Glu Lys Pro Ser Ile Glu Gln Gly
       210                 215                 220
   Val Asn Asn Phe Val Gln Tyr Lys Phe Ser His Leu Pro Ala Lys Glu
   225                 230                 235                 240
   Arg Gln Thr Ile Val Glu Leu Ala Lys Met Phe Leu Asn Arg Ile Asn
                   245                 250                 255
   Tyr Trp His Leu Glu Ala Pro Ser Gln Arg Arg Leu Arg Ser Pro Asn
               260                 265                 270
   Asp Asp Ile Ser Gly Tyr Lys Glu Asn Tyr Thr Arg Trp Leu Cys Tyr
           275                 280                 285
   Cys Asn Val Pro Gln Phe Cys Asp Ser Leu Pro Arg Tyr Glu Thr Thr
       290                 295                 300
   Gln Val Phe Gly Arg Thr Leu Leu Arg Ser Val Phe Thr Val Met Arg
   305                 310                 315                 320
   Arg Gln Leu Leu Glu Gln Ala Arg Gln Glu Lys Asp Lys Leu Pro Leu
                   325                 330                 335
   Glu Lys Arg Thr Leu Ile Leu Thr His Phe Pro Lys Phe Leu Ser Met
               340                 345                 350
   Leu Glu Glu Glu Val Tyr Ser Gln Asn Ser Pro Ile Trp Asp Gln Asp
           355                 360                 365
   Phe Leu Ser Ala Ser Ser Arg Thr Ser Gln Leu Gly Ile Gln Thr Val
       370                 375                 380
   Ile Asn Pro Pro Pro Val Ala Gly Thr Ile Ser Tyr Asn Ser Thr Ser
   385                 390                 395                 400
   Ser Ser Leu Glu Gln Pro Asn Ala Gly Ser Ser Ser Pro Ala Cys Lys
                   405                 410                 415
   Ala Ser Ser Gly Leu Glu Ala Asn Pro Gly Glu Lys Arg Lys Met Thr
               420                 425                 430
   Asp Ser His Val Leu Glu Glu Ala Lys Lys Pro Arg Val Met Gly Asp
           435                 440                 445
   Ile Pro Met Glu Leu Ile Asn Glu Val Met Ser Thr Ile Thr Asp Pro
       450                 455                 460
   Ala Ala Met Leu Gly Pro Glu Thr Asn Phe Leu Ser Ala His Ser Ala
   465                 470                 475                 480
   Arg Asp Glu Ala Ala Arg Leu Glu Glu Arg Arg Gly Val Ile Glu Phe
                   485                 490                 495
   His Val Val Gly Asn Ser Leu Asn Gln Lys Pro Asn Lys Lys Ile Leu
               500                 505                 510
   Met Trp Leu Val Gly Leu Gln Asn Val Phe Ser His Gln Leu Pro Arg
           515                 520                 525
   Met Pro Lys Glu Tyr Ile Thr Arg Leu Val Phe Asp Pro Lys His Lys
       530                 535                 540
   Thr Leu Ala Leu Ile Lys Asp Gly Arg Val Ile Gly Gly Ile Cys Phe
   545                 550                 555                 560
   Arg Met Phe Pro Ser Gln Gly Phe Thr Glu Ile Val Phe Cys Ala Val
                   565                 570                 575
   Thr Ser Asn Glu Gln Val Lys Gly Tyr Gly Thr His Leu Met Asn His
               580                 585                 590
   Leu Lys Glu Tyr His Ile Lys His Asp Ile Leu Asn Phe Leu Thr Tyr
           595                 600                 605
   Ala Asp Glu Tyr Ala Ile Gly Tyr Phe Lys Lys Gln Gly Phe Ser Lys
       610                 615                 620
   Glu Ile Lys Ile Pro Lys Thr Lys Tyr Val Gly Tyr Ile Lys Asp Tyr
   625                 630                 635                 640
   Glu Gly Ala Thr Leu Met Gly Cys Glu Leu Asn Pro Arg Ile Pro Tyr
                   645                 650                 655
   Thr Glu Phe Ser Val Ile Ile Lys Lys Gln Lys Glu Ile Ile Lys Lys
               660                 665                 670
   Leu Ile Glu Arg Lys Gln Ala Gln Ile Arg Lys Val Tyr Pro Gly Leu
           675                 680                 685
   Ser Cys Phe Lys Asp Gly Val Arg Gln Ile Pro Ile Glu Ser Ile Pro
       690                 695                 700
   Gly Ile Arg Glu Thr Gly Trp Lys Pro Ser Gly Lys Glu Lys Ser Lys
   705                 710                 715                 720
   Glu Pro Arg Asp Pro Asp Gln Leu Tyr Ser Thr Leu Lys Ser Ile Leu
                   725                 730                 735
   Gln Gln Val Lys Ser His Gln Ser Ala Trp Pro Phe Met Glu Pro Val
               740                 745                 750
   Lys Arg Thr Glu Ala Pro Gly Tyr Tyr Glu Val Ile Arg Phe Pro Met
           755                 760                 765
   Asp Leu Lys Thr Met Ser Glu Arg Leu Lys Asn Arg Tyr Tyr Val Ser
       770                 775                 780
   Lys Lys Leu Phe Met Ala Asp Leu Gln Arg Val Phe Thr Asn Cys Lys
   785                 790                 795                 800
   Glu Tyr Asn Pro Pro Glu Ser Glu Tyr Tyr Lys Cys Ala Asn Ile Leu
                   805                 810                 815
   Glu Lys Phe Phe Phe Ser Lys Ile Lys Glu Ala Gly Leu Ile Asp Lys
               820                 825                 830
<210> 4
   <211> 4824
   <212> DNA
   <213> Homo sapiens
<400> 4
   gcggaaaaga ggccgtgggg ggcctcccag cgctggcaga caccgtgagg ctggcagccg      60
   ccggcacgca cacctagtcc gcagtcccga ggaacatgtc cgcagccagg gcgcggagca     120
   gagtcccggg caggagaacc aagggagggc gtgtgctgtg gcggcggcgg cagcggcagc     180
   ggagccgcta gtcccctccc tcctggggga gcagctgccg ccgctgccgc cgccgccacc     240
   accatcagcg cgcggggccc ggccagagcg agccgggcga gcggcgcgct agggggaggg     300
   cgggggcggg gaggggggtg ggcgaagggg gcgggagggc gtggggggag ggtctcgctc     360
   tcccgactac cagagcccga gagggagacc ctggcggcgg cggcggcgcc tgacactcgg     420
   cgcctcctgc cgtgctccgg ggcggcatgt ccgaggctgg cggggccggg ccgggcggct     480
   gcggggcagg agccggggca ggggccgggc ccggggcgct gcccccgcag cctgcggcgc     540
   ttccgcccgc gcccccgcag ggctccccct gcgccgctgc cgccgggggc tcgggcgcct     600
   gcggtccggc gacggcagtg gctgcagcgg gcacggccga aggaccggga ggcggtggct     660
   cggcccgaat cgccgtgaag aaagcgcaac tacgctccgc tccgcgggcc aagaaactgg     720
   agaaactcgg agtgtactcc gcctgcaagg ccgaggagtc ttgtaaatgt aatggctgga 780
   aaaaccctaa cccctcaccc actcccccca gagccgacct gcagcaaata attgtcagtc 840
   taacagaatc ctgtcggagt tgtagccatg ccctagctgc tcatgtttcc cacctggaga 900
   atgtgtcaga ggaagaaatg aacagactcc tgggaatagt attggatgtg gaatatctct 960
   ttacctgtgt ccacaaggaa gaagatgcag ataccaaaca agtttatttc tatctattta 1020
   agctcttgag aaagtctatt ttacaaagag gaaaacctgt ggttgaaggc tctttggaaa 1080
   agaaaccccc atttgaaaaa cctagcattg aacagggtgt gaataacttt gtgcagtaca 1140
   aatttagtca cctgccagca aaagaaaggc aaacaatagt tgagttggca aaaatgttcc 1200
   taaaccgcat caactattgg catctggagg caccatctca acgaagactg cgatctccca 1260
   atgatgatat ttctggatac aaagagaact acacaaggtg gctgtgttac tgcaacgtgc 1320
   cacagttctg cgacagtcta cctcggtacg aaaccacaca ggtgtttggg agaacattgc 1380
   ttcgctcggt cttcactgtt atgaggcgac aactcctgga acaagcaaga caggaaaaag 1440
   ataaactgcc tcttgaaaaa cgaactctaa tcctcactca tttcccaaaa tttctgtcca 1500
   tgctagaaga agaagtatat agtcaaaact ctcccatctg ggatcaggat tttctctcag 1560
   cctcttccag aaccagccag ctaggcatcc aaacagttat caatccacct cctgtggctg 1620
   ggacaatttc atacaattca acctcatctt cccttgagca gccaaacgca gggagcagca 1680
   gtcctgcctg caaagcctct tctggacttg aggcaaaccc aggagaaaag aggaaaatga 1740
   ctgattctca tgttctggag gaggccaaga aaccccgagt tatgggggat attccgatgg 1800
   aattaatcaa cgaggttatg tctaccatca cggaccctgc agcaatgctt ggaccagaga 1860
   ccaattttct gtcagcacac tcggccaggg atgaggcggc aaggttggaa gagcgcaggg 1920
   gtgtaattga atttcacgtg gttggcaatt ccctcaacca gaaaccaaac aagaagatcc 1980
   tgatgtggct ggttggccta cagaacgttt tctcccacca gctgccccga atgccaaaag 2040
   aatacatcac acggctcgtc tttgacccga aacacaaaac ccttgcttta attaaagatg 2100
   gccgtgttat tggtggtatc tgtttccgta tgttcccatc tcaaggattc acagagattg 2160
   tcttctgtgc tgtaacctca aatgagcaag tcaagggcta tggaacacac ctgatgaatc 2220
   atttgaaaga atatcacata aagcatgaca tcctgaactt cctcacatat gcagatgaat 2280
   atgcaattgg atactttaag aaacagggtt tctccaaaga aattaaaata cctaaaacca 2340
   aatatgttgg ctatatcaag gattatgaag gagccacttt aatgggatgt gagctaaatc 2400
   cacggatccc gtacacagaa ttttctgtca tcattaaaaa gcagaaggag ataattaaaa 2460
   aactgattga aagaaaacag gcacaaattc gaaaagttta ccctggactt tcatgtttta 2520
   aagatggagt tcgacagatt cctatagaaa gcattcctgg aattagagag acaggctgga 2580
   aaccgagtgg aaaagagaaa agtaaagagc ccagagaccc tgaccagctt tacagcacgc 2640
   tcaagagcat cctccagcag gtgaagagcc atcaaagcgc ttggcccttc atggaacctg    2700
   tgaagagaac agaagctcca ggatattatg aagttataag gttccccatg gatctgaaaa    2760
   ccatgagtga acgcctcaag aataggtact acgtgtctaa gaaattattc atggcagact    2820
   tacagcgagt ctttaccaat tgcaaagagt acaacccccc tgagagtgaa tactacaaat    2880
   gtgccaatat cctggagaaa ttcttcttca gtaaaattaa ggaagctgga ttaattgaca    2940
   agtgattttt tttcccctct gcttcttaga aactcaccaa gcagtgtgcc taaagcaagg    3000
   tggtttagtt ttttacaaag aattggacat gatgtattga agagacttgt aaatgtaata    3060
   attagcactt ttgaaaaaac aaaaaacctc cttttagctt ttcagatatg tatttaaatt    3120
   gaagtcatag gacattttta ttttatggaa tagattttaa tctatttact actattaagg    3180
   taaattttct atggcatgtc cattagctat ttcatgatag atgattaggg gtttcctcaa    3240
   aacctgtgtg tgaggaaatt gcacacagta gcaaaatttg gggaaatcca taacattttc    3300
   agaccatgaa tgaatgtttc catttttttc taatggaatg tgagagttta cttttatttt    3360
   attctgaagg actttaagga agggatacat gattttaaaa aagcctgtaa gaggtgaaat    3420
   atgtgatgtt tgaagtctct ttatagactt tttatatata ttttttaaaa cactcatcta    3480
   gatgaggtgc tttgagcagt tctgaaaaat gcagttccag gaaagcaact gctttggttc    3540
   ctaaggaaga aattctaaat aatgcaaact tttaaaataa gcatctaggt ttttgataat    3600
   tctgtctact tacaacaaac ttgttagtac ataaccacta ttttaataat tattttctct    3660
   acacaaatgt gtaatatcat atttgacttt gcttatgcag gccataagtt ccaaaagata    3720
   atttccctgc ccacaaaggc ataaacttga aaacacatga gattgaatca acatgcttta    3780
   ataggaaaag atgtatggtc tatatatgta tcaatctggt gaatcctcgt tctaataaag    3840
   gttctttttc ttttctatga tacacacagc cacgctgata atatgcaaat gaacattttc    3900
   ctttatgtct ctccagataa tgtttattgt ctgaggtaaa ttaaattccc accagggttt    3960
   gctgtcagta ttttaacacc cacattagta tatgcgtcca gggtcataac cccctaaaat    4020
   ccatcatgca accttattaa tctgtcttgg gattccagtt tagtgcttgg atttatttcc    4080
   tgattacact acatagaaaa gtgagacatc tgccattccc aactctggga aaaccaacta    4140
   atatacaacc atataaatga aggccatctt gatggtctca acactaattt ttatgatgca    4200
   aatttataca ctgatttttg taaaggacaa agttttaaaa gcgtatttaa cttgatgttt    4260
   tctatcagca taaataaaat ggtcatgaat agtcattaaa aacagttgcc agtgataatc    4320
   tgcatgaagg aaaaagaacc ctgcaaatgg ctattgagtt ggaagtattg tttttgatat    4380
   gtaagagata ttcagaatgc tcacactgaa aatgcctcaa ctttttaaag tgtaagaaac    4440
   caccatgagt ggtgtctaga tttctaatga agaatcatga tacagtttgg attaagtatc    4500
   ttggactggt tttaaacagt gctttgtacc ggatctgctg aagcatctgt ccagctggta    4560
   tcctgtgaaa gtttgttatt ttctgagtag acattcttat agagtattgt ctttaaaatc    4620
   agattgtctc ttctatattg aaagcatttt tatgttttct aatttaaaaa ttaatatttt      4680
   cttatagata ttgtgcaata aagctgaagt agaatgtgtg gtttttgcaa atgctttaac      4740
   agctgataaa aattttacat ttgtaaaatt aatatattgt actggtacaa aatagtttta      4800
   aattatattt taaaaagctt ccaa                                             4824
<210> 5
   <211> 837
   <212> PRT
   <213> Homo sapiens
<400> 5
   Met Ala Glu Pro Ser Gln Ala Pro Thr Pro Ala Pro Ala Ala Gln Pro
   1               5                   10                  15
   Arg Pro Leu Gln Ser Pro Ala Pro Ala Pro Thr Pro Thr Pro Ala Pro
               20                  25                  30
   Ser Pro Ala Ser Ala Pro Ile Pro Thr Pro Thr Pro Ala Pro Ala Pro
           35                  40                  45
   Ala Pro Ala Ala Ala Pro Ala Gly Ser Thr Gly Thr Gly Gly Pro Gly
       50                  55                  60
   Val Gly Ser Gly Gly Ala Gly Ser Gly Gly Asp Pro Ala Arg Pro Gly
   65                  70                  75                  80
   Leu Ser Gln Gln Gln Arg Ala Ser Gln Arg Lys Ala Gln Val Arg Gly
                   85                  90                  95
   Leu Pro Arg Ala Lys Lys Leu Glu Lys Leu Gly Val Phe Ser Ala Cys
               100                 105                 110
   Lys Ala Asn Glu Thr Cys Lys Cys Asn Gly Trp Lys Asn Pro Lys Pro
           115                 120                 125
   Pro Thr Ala Pro Arg Met Asp Leu Gln Gln Pro Ala Ala Asn Leu Ser
       130                 135                 140
   Glu Leu Cys Arg Ser Cys Glu His Pro Leu Ala Asp His Val Ser His
   145                 150                 155                 160
   Leu Glu Asn Val Ser Glu Asp Glu Ile Asn Arg Leu Leu Gly Met Val
                   165                 170                 175
   Val Asp Val Glu Asn Leu Phe Met Ser Val His Lys Glu Glu Asp Thr
               180                 185                 190
   Asp Thr Lys Gln Val Tyr Phe Tyr Leu Phe Lys Leu Leu Arg Lys Cys
           195                 200                 205
   Ile Leu Gln Met Thr Arg Pro Val Val Glu Gly Ser Leu Gly Ser Pro
       210                 215                 220
   Pro Phe Glu Lys Pro Asn Ile Glu Gln Gly Val Leu Asn Phe Val Gln
   225                 230                 235                 240
   Tyr Lys Phe Ser His Leu Ala Pro Arg Glu Arg Gln Thr Met Phe Glu
                   245                 250                 255
   Leu Ser Lys Met Phe Leu Leu Cys Leu Asn Tyr Trp Lys Leu Glu Thr
               260                 265                 270
   Pro Ala Gln Phe Arg Gln Arg Ser Gln Ala Glu Asp Val Ala Thr Tyr
           275                 280                 285
   Lys Val Asn Tyr Thr Arg Trp Leu Cys Tyr Cys His Val Pro Gln Ser
       290                 295                 300
   Cys Asp Ser Leu Pro Arg Tyr Glu Thr Thr His Val Phe Gly Arg Ser
   305                 310                 315                 320
   Leu Leu Arg Ser Ile Phe Thr Val Thr Arg Arg Gln Leu Leu Glu Lys
                   325                 330                 335
   Phe Arg Val Glu Lys Asp Lys Leu Val Pro Glu Lys Arg Thr Leu Ile
               340                 345                 350
   Leu Thr His Phe Pro Lys Phe Leu Ser Met Leu Glu Glu Glu Ile Tyr
           355                 360                 365
   Gly Ala Asn Ser Pro Ile Trp Glu Ser Gly Phe Thr Met Pro Pro Ser
       370                 375                 380
   Glu Gly Thr Gln Leu Val Pro Arg Pro Ala Ser Val Ser Ala Ala Val
   385                 390                 395                 400
   Val Pro Ser Thr Pro Ile Phe Ser Pro Ser Met Gly Gly Gly Ser Asn
                   405                 410                 415
   Ser Ser Leu Ser Leu Asp Ser Ala Gly Ala Glu Pro Met Pro Gly Glu
               420                 425                 430
   Lys Arg Thr Leu Pro Glu Asn Leu Thr Leu Glu Asp Ala Lys Arg Leu
           435                 440                 445
   Arg Val Met Gly Asp Ile Pro Met Glu Leu Val Asn Glu Val Met Leu
       450                 455                 460
   Thr Ile Thr Asp Pro Ala Ala Met Leu Gly Pro Glu Thr Ser Leu Leu
   465                 470                 475                 480
   Ser Ala Asn Ala Ala Arg Asp Glu Thr Ala Arg Leu Glu Glu Arg Arg
                   485                 490                 495
   Gly Ile Ile Glu Phe His Val Ile Gly Asn Ser Leu Thr Pro Lys Ala
               500                 505                 510
   Asn Arg Arg Val Leu Leu Trp Leu Val Gly Leu Gln Asn Val Phe Ser
           515                 520                 525
   His Gln Leu Pro Arg Met Pro Lys Glu Tyr Ile Ala Arg Leu Val Phe
       530                 535                 540
   Asp Pro Lys His Lys Thr Leu Ala Leu Ile Lys Asp Gly Arg Val Ile
   545                 550                 555                 560
   Gly Gly Ile Cys Phe Arg Met Phe Pro Thr Gln Gly Phe Thr Glu Ile
                   565                 570                 575
   Val Phe Cys Ala Val Thr Ser Asn Glu Gln Val Lys Gly Tyr Gly Thr
               580                 585                 590
   His Leu Met Asn His Leu Lys Glu Tyr His Ile Lys His Asn Ile Leu
           595                 600                 605
   Tyr Phe Leu Thr Tyr Ala Asp Glu Tyr Ala Ile Gly Tyr Phe Lys Lys
       610                 615                 620
   Gln Gly Phe Ser Lys Asp Ile Lys Val Pro Lys Ser Arg Tyr Leu Gly
   625                 630                 635                 640
   Tyr Ile Lys Asp Tyr Glu Gly Ala Thr Leu Met Glu Cys Glu Leu Asn
                   645                 650                 655
   Pro Arg Ile Pro Tyr Thr Glu Leu Ser His Ile Ile Lys Lys Gln Lys
               660                 665                 670
   Glu Ile Ile Lys Lys Leu Ile Glu Arg Lys Gln Ala Gln Ile Arg Lys
           675                 680                 685
   Val Tyr Pro Gly Leu Ser Cys Phe Lys Glu Gly Val Arg Gln Ile Pro
       690                 695                 700
   Val Glu Ser Val Pro Gly Ile Arg Glu Thr Gly Trp Lys Pro Leu Gly
   705                 710                 715                 720
   Lys Glu Lys Gly Lys Glu Leu Lys Asp Pro Asp Gln Leu Tyr Thr Thr
                   725                 730                 735
   Leu Lys Asn Leu Leu Ala Gln Ile Lys Ser His Pro Ser Ala Trp Pro
               740                 745                 750
   Phe Met Glu Pro Val Lys Lys Ser Glu Ala Pro Asp Tyr Tyr Glu Val
           755                 760                 765
   Ile Arg Phe Pro Ile Asp Leu Lys Thr Met Thr Glu Arg Leu Arg Ser
       770                 775                 780
   Arg Tyr Tyr Val Thr Arg Lys Leu Phe Val Ala Asp Leu Gln Arg Val
   785                 790                 795                 800
   Ile Ala Asn Cys Arg Glu Tyr Asn Pro Pro Asp Ser Glu Tyr Cys Arg
                   805                 810                 815
   Cys Ala Ser Ala Leu Glu Lys Phe Phe Tyr Phe Lys Leu Lys Glu Gly
               820                 825                 830
   Gly Leu Ile Asp Lys
           835
<210> 6
   <211> 3127
   <212> DNA
   <213> Homo sapiens
<400> 6
   ggttgcccat gcggccctag ggctgggagc gcggcgccgc tctccgctgc gggggaggcc      60
   atggcggaac cttcccaggc cccgaccccg gccccggctg cgcagccccg gccccttcag     120
   tccccagccc ctgccccaac tccgactcct gcacccagcc cggcttcagc cccgattccg     180
   actcccaccc cggcaccagc ccctgcccca gctgcagccc cagccggcag cacagggact     240
   ggggggcccg gggtaggaag tgggggggcc gggagcgggg gggatccggc tcgacctggc     300
   ctgagccagc agcagcgcgc cagtcagagg aaggcgcaag tccgggggct gccgcgcgcc     360
   aagaagcttg agaagctagg ggtcttctcg gcttgcaagg ccaatgaaac ctgtaagtgt     420
   aatggctgga aaaaccccaa gccccccact gcaccccgca tggatctgca gcagccagct     480
   gccaacctga gtgagctgtg ccgcagttgt gagcacccct tggctgacca cgtatcccac     540
   ttggagaatg tgtcagagga tgagataaac cgactgctgg ggatggtggt ggatgtggag     600
   aatctcttca tgtctgttca caaggaagag gacacagaca ccaagcaggt ctatttctac     660
   ctcttcaagc tactgcggaa atgcatcctg cagatgaccc ggcctgtggt ggaggggtcc     720
   ctgggcagcc ctccatttga gaaacctaat attgagcagg gtgtgctgaa ctttgtgcag     780
   tacaagttta gtcacctggc tccccgggag cggcagacga tgttcgagct ctcaaagatg 840
   ttcttgctct gccttaacta ctggaagctt gagacacctg cccagtttcg gcagaggtct 900
   caggctgagg acgtggctac ctacaaggtc aattacacca gatggctctg ttactgccac 960
   gtgccccaga gctgtgatag cctcccccgc tacgaaacca ctcatgtctt tgggcgaagc 1020
   cttctccggt ccattttcac cgttacccgc cggcagctgc tggaaaagtt ccgagtggag 1080
   aaggacaaat tggtgcccga gaagaggacc ctcatcctca ctcacttccc caaattcctg 1140
   tccatgctgg aggaggagat ctatggggca aactctccaa tctgggagtc aggcttcacc 1200
   atgccaccct cagaggggac acagctggtt ccccggccag cttcagtcag tgcagcggtt 1260
   gttcccagca cccccatctt cagccccagc atgggtgggg gcagcaacag ctccctgagt 1320
   ctggattctg caggggccga gcctatgcca ggcgagaaga ggacgctccc agagaacctg 1380
   accctggagg atgccaagcg gctccgtgtg atgggtgaca tccccatgga gctggtcaat 1440
   gaggtcatgc tgaccatcac tgaccctgct gccatgctgg ggcctgagac gagcctgctt 1500
   tcggccaatg cggcccggga tgagacagcc cgcctggagg agcgccgcgg catcatcgag 1560
   ttccatgtca tcggcaactc actgacgccc aaggccaacc ggcgggtgtt gctgtggctc 1620
   gtggggctgc agaatgtctt ttcccaccag ctgccgcgca tgcctaagga gtatatcgcc 1680
   cgcctcgtct ttgacccgaa gcacaagact ctggccttga tcaaggatgg gcgggtcatc 1740
   ggtggcatct gcttccgcat gtttcccacc cagggcttca cggagattgt cttctgtgct 1800
   gtcacctcga atgagcaggt caagggttat gggacccacc tgatgaacca cctgaaggag 1860
   tatcacatca agcacaacat tctctacttc ctcacctacg ccgacgagta cgccatcggc 1920
   tacttcaaaa agcagggttt ctccaaggac atcaaggtgc ccaagagccg ctacctgggc 1980
   tacatcaagg actacgaggg agcgacgctg atggagtgtg agctgaatcc ccgcatcccc 2040
   tacacggagc tgtcccacat catcaagaag cagaaagaga tcatcaagaa gctgattgag 2100
   cgcaaacagg cccagatccg caaggtctac ccggggctca gctgcttcaa ggagggcgtg 2160
   aggcagatcc ctgtggagag cgttcctggc attcgagaga caggctggaa gccattgggg 2220
   aaggagaagg ggaaggagct gaaggacccc gaccagctct acacaaccct caaaaacctg 2280
   ctggcccaaa tcaagtctca ccccagtgcc tggcccttca tggagcctgt gaagaagtcg 2340
   gaggcccctg actactacga ggtcatccgc ttccccattg acctgaagac catgactgag 2400
   cggctgcgaa gccgctacta cgtgacccgg aagctctttg tggccgacct gcagcgggtc 2460
   atcgccaact gtcgcgagta caaccccccg gacagcgagt actgccgctg tgccagcgcc 2520
   ctggagaagt tcttctactt caagctcaag gagggaggcc tcattgacaa gtaggcccat 2580
   ctttgggccg cagccctgac ctggaatgtc tccacctcgg attctgatct gatccttagg 2640
   gggtgccctg gccccacgga cccgactcag cttgagacac tccagccaag ggtcctccgg 2700
   acccgatcct gcagctcttt ctggaccttc aggcaccccc aagcgtgcag ctctgtccca 2760
   gccttcactg tgtgtgagag gtctcctggg ttggggccca gcccctctag agtagctggt 2820
   ggccagggat gaaccttgcc cagccgtggt ggcccccagg cctggtcccc aagagctttg 2880
   gaggcttgga ttcctgggcc tggcccaggt ggctgtttcc ctgaggacca gaactgctca 2940
   ttttagcttg agtgatggct tcaggggttg gaagttcagc ccaaactgaa gggggccatg 3000
   ccttgtccag cactgttctg tcagtctccc ccaggggtgg ggggtatggg gaccattcat 3060
   tccctggcat taatccctta gagggaataa taaagctttt tatttctctg tgaaaaaaaa 3120
   aaaaaaa 3127